(19) Europäisches Patentamt
European Patent Office
Office européen des brevets

(11) **EP 4 515 000 B1**

(12) **EUROPEAN PATENT SPECIFICATION**

(45) Date of publication and mention
of the grant of the patent:
**01.04.2026 Bulletin 2026/14**

(21) Application number: **23722861.4**

(22) Date of filing: **26.04.2023**

(51) International Patent Classification (IPC):
***C12Q 1/6886*** *(2018.01)*

(52) Cooperative Patent Classification (CPC):
**C12Q 1/6886;** C12Q 2600/106; C12Q 2600/156

(86) International application number:
**PCT/EP2023/061011**

(87) International publication number:
**WO 2023/209035 (02.11.2023 Gazette 2023/44)**

(54) **METHODS AND SYSTEMS FOR PREDICTING CANCER THERAPY RESPONSE**

VERFAHREN UND SYSTEME ZUR VORHERSAGE DES ANSPRECHENS AUF EINE KREBSTHERAPIE

PROCÉDÉS ET SYSTÈMES DE PRÉDICTION DE LA RÉPONSE À LA THÉRAPIE DU CANCER

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **26.04.2022 EP 22170144**

(43) Date of publication of application:
**05.03.2025 Bulletin 2025/10**

(73) Proprietor: **F. Hoffmann-La Roche AG**
**4070 Basel (CH)**

(72) Inventors:
• **CHARO, Jehad**
**8952 Schlieren (CH)**
• **FOMIN, Vitalay**
**Little Falls, New Jersey 07424 (US)**
• **SO, WeiQing Venus**
**Little Falls, New Jersey 07424 (US)**

(74) Representative: **Mewburn Ellis LLP**
**Aurora Building**
**Counterslip**
**Bristol BS1 6BX (GB)**

(56) References cited:
WO-A1-2018/183928        WO-A1-2019/207030
US-A1- 2019 219 586        US-A1- 2019 352 723
US-A1- 2020 109 204        US-A1- 2020 109 455

**Description**

This application claims priority from EP22170144.4 filed 26 April 2022.

*Field of the Invention*

The present invention relates to methods for assessment of mutations and combinations thereof that inform cancer treatment, including predicting cancer patient response to therapy.

*Background*

Lung cancer is the leading cause of cancer related mortality worldwide with NSCLC accounting for about 85% of all lung cancer histological subtypes[1,2]. The discovery and FDA approval of check point inhibitors (CPI) completely revolutionized cancer therapy in a variety of malignancies, by achieving prolonged responses[3-7]. Unfortunately, despite the unprecedented prolonged response rates to CPIs the majority of patients are resistant to CPI therapy[8]. Resistance to CPIs can be categorized into two main patient groups: 1. Innate/primary resistant patient group, which never respond or derive clinical benefit from CPI therapy, and 2. Acquired resistance patient group, which initially respond to CPI therapy but eventually develop resistance and have disease progression[8-10]. Since the majority of patients treated with CPI fall into innate or acquired resistance group[8,9,11], there is an urgent and unmet need to understand CPI resistance mechanisms. The mechanistic understanding of CPI resistance will inevitably be followed by development of predictive biomarkers and potential targets for therapeutics discoveries aimed at reverting/preventing resistance to CPI therapy.
Extensive efforts are underway to identify predictive biomarkers utilizing various omics, histopathologic, clinical and computational approaches. These efforts led to the discovery that tumor mutational burden (TMB), microsatellite instability (MSI), PD-L1, JAK1/JAK2, IFNg, PTEN loss, PBRM1, STK11/KEAP1 mutations, antigen processing/presentation loss, WNT/b-catenin signaling can affect patient's response to CPI therapy[12]. While the above biomarkers have led to important advances in the understanding of CPI resistance, the only approved biomarkers are TMB and PDL-1 levels[10.]. However, even these approved biomarkers showed only moderate predictive value[11], and thus unfortunately, do not provide important mechanistic insight behind CPI resistance. In addition, several gene expression signatures were reported to be predictive of CPI response[13-15], and while they increased our biological understanding behind CPI resistance, these signatures do not seem to generalize[16] (at least in melanoma).
US2019/219586, WO2018/183928, US2020/109204, WO2019/207030,
[0006] US2019/352723 and US 2020/109455 describe therapeutic and diagnostic methods for cancer. In particular, methods of predicting responsiveness of a patient suffering from cancer to a treatment comprising a PD-L1 axis binding antagonist based on a tissue mutation burden (tTMB) are disclosed.
The limited genetic and clinical biomarkers to predict CPI response is a major bottle neck in developing novel therapeutics to target CPI resistance and in selection of biomarkers for patient selection.
The present invention has been devised in light of the above considerations. The present invention aims to mitigate problems with prior methods of, in particular, as well as providing certain related advantages.

*Summary of the Invention*

The present inventors have developed methods and related tools for predicting response to immune checkpoint inhibitor (CPI) therapy by utilizing gene mutation profiles as described herein.
Accordingly, in a first aspect the present invention provides a computer-implemented method for predicting the treatment response of a subject having a cancer (e.g. a lung cancer, such as non-small-cell lung cancer (NSCLC)) to an immune checkpoint inhibitor (CPI) therapy, the method comprising:

> providing a mutation profile of the subject, said profile comprising the presence or absence mutations at one or more locations in at least five, six, or at least seven genes selected from the group consisting of: NF1, STK11, TSC2, BRCA2, BRAF, STAG2, U2AF1, BRIP1, PDGFRA, CTNNA1, PDK1, FGF10 and FLT1;

> analysing the mutation profile to classify the profile as matching the mutation profile of a response signature or a resistance signature,

wherein the subject is predicted to be likely to respond to the CPI therapy if the mutation profile for the subject is classified as matching the mutation profile of the response signature and is predicted to be likely not to respond to the CPI therapy if the mutation profile for the subject is classified as matching the mutation profile of the resistance signature.
As explained in greater detail herein, see Example 7 in particular, the present inventors performed a recursive elimination of features and determined that models utilizing - in certain cases - only 5 genes were nevertheless able to provide impressive predictive power for CPI response prediction.

In certain embodiments, the at least five genes may be selected from: NF1, STK11, TSC2, BRCA2, BRAF, STAG2, U2AF1, BRIP1, PDGFRA, CTNNA1 and PDK1.

In particular, said selected genes may include: BRAF, BRIP1, FGF10 and FLT1. This "core" set of four genes was found to be present in an overlap of three inputs.

In some embodiments, the at least five, six or at least seven genes may be selected from: BRAF, BRIP1, CTNNA1, FGF10, FLT1, PDGFRA and STK11.

In some embodiments, the total number of genes making up the mutation profile does not exceed 100, 90, 80, 70, 60, 50, 40, 30, 25, 20, 15, 10, 9 or does not exceed 8 genes.

In some embodiments, the mutations are cancer-specific (i.e. somatic) mutations. In some embodiments, the mutations may be germline mutations. For example, the subject may be a homozygous or heterozygous carrier of a mutation in a gene as specified herein. In particular, the subject may carry one or more copies of a mutation that predisposes the subject to developing cancer and/or which affects the efficacy of a CPI therapy when administered to the subject.

In some embodiments in which one or more of the mutations are germline mutations, the mutation may be in a gene selected from the group consisting of: BRCA1, BRCA2, ATM, TP53, PTEN, STK11, PALB2, RAD51C, VHL, FANCM, EPHB4, CDH1, BRIP1, CHEK1 and CHEK2. Germline mutations in these genes are among those that make up the Flatiron Health-Foundation Medicine NSCLC clinico-genomic database. Mutations in these genes may have a pathogenic impact on cancer (e.g. predisposing an individual to certain cancers, including lung cancers). Examples of specific germline mutations contemplated herein include [format: Gene name, cds_effect, protein_effect]: BRCA1, 181T>G, C61G; BRCA1, 213-11T>G, splice site 213-11T>G; BRCA1, 189A>T, L63F; BRCA2, 2808_2811delACAA, A938fs*21; BRCA2, 9155G>A, R3052Q; BRCA2, 7976G>A, R2659K; ATM, 7271T>G, V2424G; ATM, 8147T>C, V2716A; ATM, 1564_1565delGA, E522fs*43; TP53, 760A>G, I254V; TP53, 784G>A, G262S; TP53, 974G>T, G325V; PTEN, 107delG, G36fs*18; PTEN, 562_563insT, Y188fs*2; PTEN, 741A>C, L247F; STK11, 298C>G, Q100E; STK11, 971C>T, P324L; STK11, 551_552TC>CT, L184P; PALB2, 2507_2509delTCG, V836del; PALB2, 1637T>C, V546A; RAD51C, 1026+5_1026+7delGTA, splice site 1026+5_1026+7delGTA; RAD51C, 404G>C, C135S; VHL, 241C>T, P81S; VHL, 376G>A, D126N; VHL, 628C>T, R210W; FANCM, 1972C>T, R658*; FANCM, 3827C>T, S1276L; FANCM, 5014G>A, D1672N; EPHB4, 1258G>A, V420I; EPHB4, 118G>A, G40R; EPHB4, 1759A>G, T587A; CDH1, 871G>A, D291N; CDH1, 1136C>T, T379M; CDH1, 846G>A, M282I; BRIP1, 799G>A, A267T; BRIP1, 1261G>C, E421Q; BRIP1, 1316G>A, R439Q; CHEK1, 703G>A, D235N; and CHEK2, 349A>G, R117G.

In some embodiments, the at least five genes comprise all of the genes: NF1, STK11, TSC2, STAG2, U2AF1, BRCA2, and PDK1. In some embodiments, the at least five genes comprise all of the genes BRAF, BRIP1, FGF10 and FLT1. In particular, the genes may comprise all of: STK11, BRIP1, CDKN2B, FLT1, FGF10, BRAF, ASXL1, HRAS, IDH1, BARD1, BRCA2, U2AF1 and CTNNA1 or may comprise all of: NF1, STK11, TSC2, BRCA2, BRAF, STAG2, U2AF1, and PDK1. In some embodiments, the genes may comprise all of: NF1, STK11, TSC2, BRCA2, BRAF, STAG2, U2AF1, PDK1, and ATR. In some embodiments, the at least five genes comprise all of the genes: STK11, BRAF, BRIP1, U2AF1 and NF1. In some embodiments, the at least five genes comprise all of the genes: STK11, PDGFRA, BRAF, BRIP1 and CTNNA1. In some embodiments, the at least five genes comprise all of the genes: BRAF, BRIP1, STK11, CDK12, CTNNA1, FAS, NRAS, NOTCH3, PIK3CA, and RAD51C.

In some embodiments, the at least five genes may comprise: NF1, STK11, ASXL1, FGF19, TSC2, BRAF, IDH1, STAG2, BRCA2, PDK1, U2AF1, NKX2-1, and PPP2R1A, and the mutations may be gain of function ("GOF") and/or loss of function ("LOF") mutations selected from: NF1_LOF, STK11_LOF, ASXL1_LOF, FGF19_LOF, TSC2_LOF, BRAF_GOF, IDH1_GOF, STAG2_LOF, BRCA2_LOF, PDK1_LOF, U2AF1_GOF, NKX2-1_LOF, and PPP2R1A_GOF. In some embodiments, the at least five genes comprise: PBRM1, BRIP1, PTEN, CDKN2A, STK11, CDKN2B, U2AF1, CTNNA1, FGF10, FGF19, AKT2, NBN, ALOX12B, BRAF, and NF1, and wherein the mutations are gain of function (GOF) and/or loss of function (LOF) mutations selected from: PBRM1_LOF, BRIP1_LOF, PTEN_LOF, CDKN2A_LOF, STK11_GOF, CDKN2B_LOF, U2AF1_GOF, CTNNA1_LOF, FGF10_GOF, FGF19_LOF, AKT2_GOF, NBN_LOF, ALOX12B_LOF, BRAF_GOF, and NF1_GOF.

In some embodiments, the at least five genes may comprise: BRAF, BRIP1, FGF10, NF1, STK11, MAP3K13, NOTCH3, ALOX12B, U2AF1, RARA, MLH1, FLT1, MAP3K1, MYC, CTNNA1, NBN and ASXL1 and the mutations may be high frequency mutations ("HFM") (which tend to be gain of function ("GOF") mutations) and/or low frequency mutations ("LFM") (which are tend to be loss of function ("LOF") mutations) selected from: BRAF_HFM, BRIP1_LFM, FGF10_HFM, NF1_HFM, STK11_HFM, MAP3K13_LFM, NOTCH3_LFM, ALOX12B_LFM, U2AF1_HFM, RARA_HFM, MLH1_LFM, FLT1_LFM, MAP3K1_LFM, MYC_LFM, CTNNA1_LFM, NBN_LFM, and ASXL1_LFM.

In some embodiments, the at least five genes may comprise: NF1, STK11, ASXL1, KMT2A, NFKBIA, BRAF, TSC2, FGF19, NKX2-1, BRCA2, CDKN2A, PDK1, TP53, NFE2L2, U2AF1, EGFR, PPP2R1A, DNMT3A, and STAG2, and the cancer-specific mutations may be GOF and/or LOF mutations selected from: NF1_LOF, STK11_LOF, ASXL1_LOF, KMT2A_LOF, NFKBIA_GOF, BRAF_GOF_600, TSC2_LOF, FGF19_LOF, NKX2-1_LOF, BRCA2_LOF, CDKN2A_GOF_151, PDK1_LOF, TP53_GOF_282, NFE2L2_GOF_24, U2AF1_GOF_34, EGFR_GOF_719, PPP2R1A_GOF, DNMT3A_GOF_882, and STAG2_LOF. The number following GOF (e.g. BRAF_GOF_600) indicates the position of the mutation in the amino acid sequence of the protein encoded by the gene (e.g. amino acid position 600 in the translated amino acid sequence disclosed at NM_004333). Therefore, a high frequency mutation, as defined herein, which results in an alteration at amino acid position 600 of the encoded BRAF gene product is considered to be a BRAF_GOF_600 mutation. In the case of a "short variant mutation" (e.g. BRAF_GOF_600, SDHA_GOF_531), the stated position is the amino acid position. However, in the case that the mutation is a splicing site mutation or a mutation in a gene promoter, or other mutation that is not attributed to a specific amino acid position, then the number refers to the the coding sequence (CDS) position (where 1 is the start of the CDS). For example, NF1_GOF_1642 refers to a splicing mutation. The position is 334-1+1642=1975 of NM_001042492 (G>T).

In some embodiments, the at least give genes may comprise: CDKN2B, FGF10, BRCA2, FLT1, BRIP1, RARA, DNMT3A, MAP3K13, BRAF, ALOX12B, BRAF, BCL6, XRCC2, EGFR, TSC1, PIK3C2G, TP53, PIK3CA, MLH1 and FAS and the cancer-specific mutations may be HFM and/or LFM mutations selected from: CDKN2B_LFM, FGF10_HFM, BRCA2_LFM, FLT1_LFM, BRIP1_LFM, RARA_HFM, DNMT3A_HFM_771, MAP3K13_LFM, BRAF_HFM_600, ALOX12B_LFM, BRAF_HFM_469, BCL6_LFM, XRCC2_LFM, EGFR_HFM_746, TSC1_LFM, PIK3C2G_HFM, TP53_HFM_331, PIK3CA_HFM, MLH1_LFM and FAS_LFM.

In some embodiments, the at least five genes comprise: BRIP1, CDKN2B, U2AF1, CTNNA1, ALOX12B, EGFR, FAS, and KMT2A, and wherein the cancer-specific mutations are GOF and/or LOF mutations selected from: BRIP1_LOF, CDKN2B_LOF, U2AF1_GOF_34, CTNNA1_LOF, ALOX12B_LOF, EGFR_GOF_746, FAS_LOF, and KMT2A_LOF.

In some embodiments, the at least five genes may comprise: NF1, STK11, TSC2, BRCA2, BRAF, ATRX, STAG2, U2AF1, PDK1, ATR, ASXL1, ERCC4, PAX5, CTNNA1, CD79A, TSC1, NRAS, RARA, PDCD1LG2, NBN, PDGFRB, PDGFRA, CCNE1, JUN, IDH1, CDK4, NKX2-1, PPP2R1A, FH, MDM2, AKT1, NTRK2, FANCG, QKI, BRD4, CDKN1A, CEBPA, FANCL, and SMARCA4.

In some embodiments, the at least five genes may comprise a set of genes set forth in Table 1A or Table 1B and shown in that table as: "binary cluster 1"; "binary cluster 2"; "binary cluster 3"; "binary cluster 4"; "binary cluster 5"; "binary cluster 6"; "binary cluster 7"; "binary cluster 8"; "binary cluster 9"; "binary cluster 10"; "binary cluster 11"; and/or "binary cluster 12".

In some embodiments, the mutations may be, e.g., cancer-specific mutations that are GOF and/or LOF mutations selected from the set of mutations set forth in Table 2A or Table 2B and listed as: GOF/LOF cluster 1; GOF/LOF cluster 2; GOF/LOF cluster 3; GOF/LOF cluster 4; GOF/LOF cluster 5; GOF/LOF cluster 6; GOF/LOF cluster 7; GOF/LOF cluster 8; GOF/LOF cluster 9; GOF/LOF cluster 10; GOF/LOF cluster 11; and/or GOF/LOF cluster 12.

In some embodiments, the mutations may be, e.g., cancer-specific mutations that are GOF and/or LOF mutations selected from the set of mutations set forth in Table 3A or Table 3B and listed as: Hotspot cluster 1; Hotspot cluster 2; Hotspot cluster 3; Hotspot cluster 4; Hotspot cluster 5; Hotspot cluster 6; Hotspot cluster 7; Hotspot cluster 8; Hotspot cluster 9; Hotspot cluster 10; Hotspot cluster 11; and/or Hotspot cluster 12.

In some embodiments, analysing the mutation profile to classify the profile comprises:
inputting data representing the mutation profile of the subject into a machine learning classifier, wherein said machine learning classifier has been trained on a training data set comprising the mutation profiles of at least the same genes as those forming the mutation profile of the subject, as defined above. The mutation profiles of the training set may be from a plurality of samples derived from cancer patients (e.g. lung cancer patients, such as NSCLC patients) known to have responded to CPI therapy and from a plurality of samples derived from cancer patients (e.g. lung cancer patients, such as NSCLC patients) known to have been resistant to CPI therapy. Analysing the mutation profile of the subject to classify the profile further comprises causing the machine learning classifier to classify the mutation profile of the subject as belonging to the response group or the resistance group. For example, the machine learning classifier may take the mutation profile features as inputs and output a probability or a most probable or best fit classification for that mutation profile to the class of CPI responsive or CPI resistant based on similarity or difference between the mutation profile of the subject and the labelled mutation profiles making up the training set. In some cases, the training data set may comprise mutation profiles of at least 50, at least 100 or at least 200 samples derived from cancer patients (e.g. lung cancer patients, such as NSCLC patients) known to have responded to CPI therapy and mutation profiles of at least 50, at least 100 or at least 200 samples derived from cancer patients (e.g. lung cancer patients, such as NSCLC patients) known to have been resistant to CPI therapy. In some cases, the number of patients in each of the classes (CPI responsive and CPI resistant) of the training set may be approximately equal, i.e. a balanced training set.

In some embodiments, the training data set may comprise the Flatiron Health de-identified Clinico-Genomic Database (CGDB) as available on January 1, 2020 and which is described in Singal G, Miller PG, Agarwala V, et al. Association of Patient Characteristics and Tumor Genomics With Clinical Outcomes Among Patients With Non-Small Cell Lung Cancer Using a Clinicogenomic Database (CGDB). JAMA. 2019;321(14):1391-1399.doi:10.1001/jama.2019.3241. In particular, the training set may comprise the nationwide (US-based) de-identified Flatiron Health-Foundation Medicine NSCLC clinico-genomic database (FH-FMI CGDB).

In some embodiments, the machine learning classifier may be selected from the group consisting of: a Naïve Bayes model; a logistic regression model; an artificial neural network, a support vector machine (SVM); a random forest; and a perceptron.

In some embodiments, the presence of a mutation in one or more of the following genes contributes to classifying the profile as matching the mutation profile of a response signature:

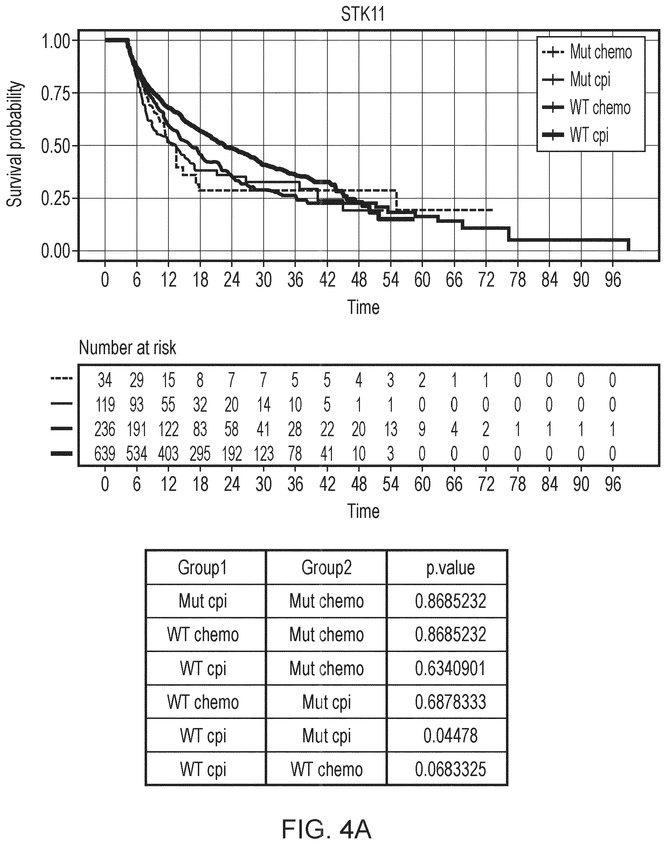

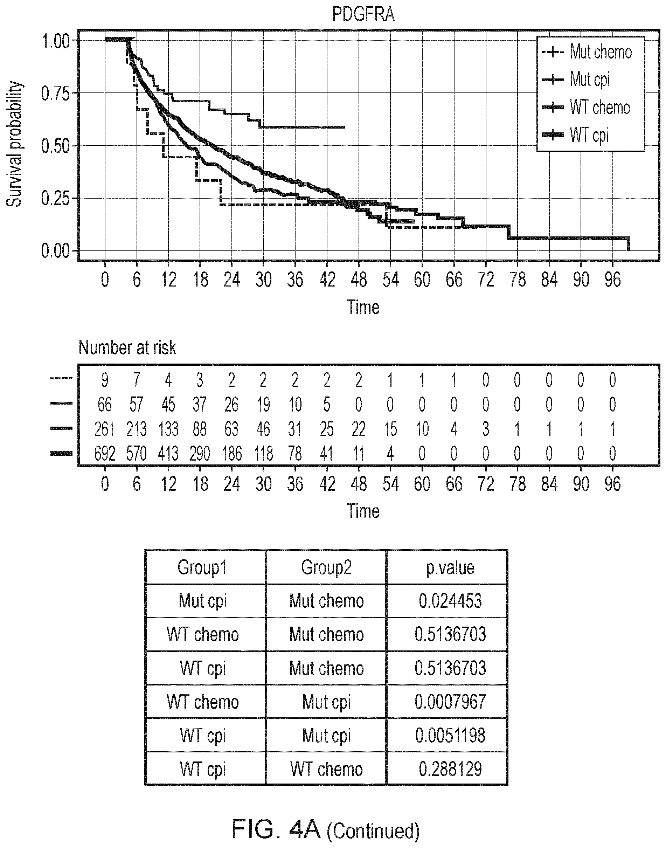

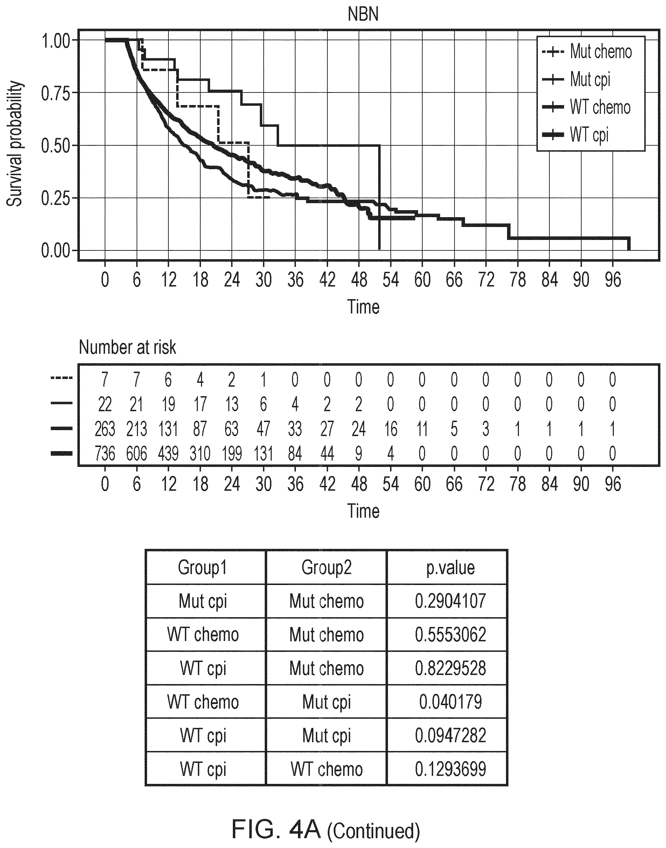

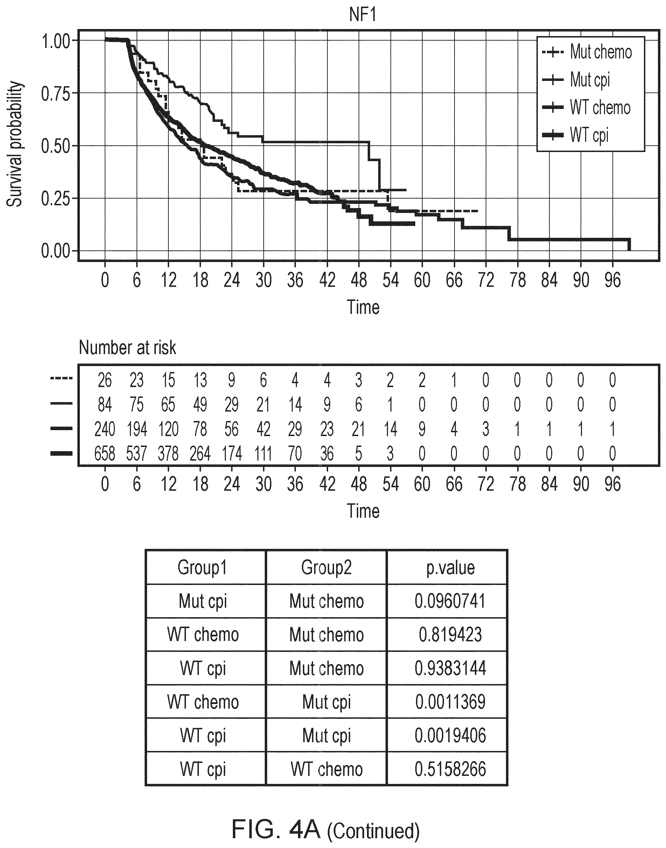

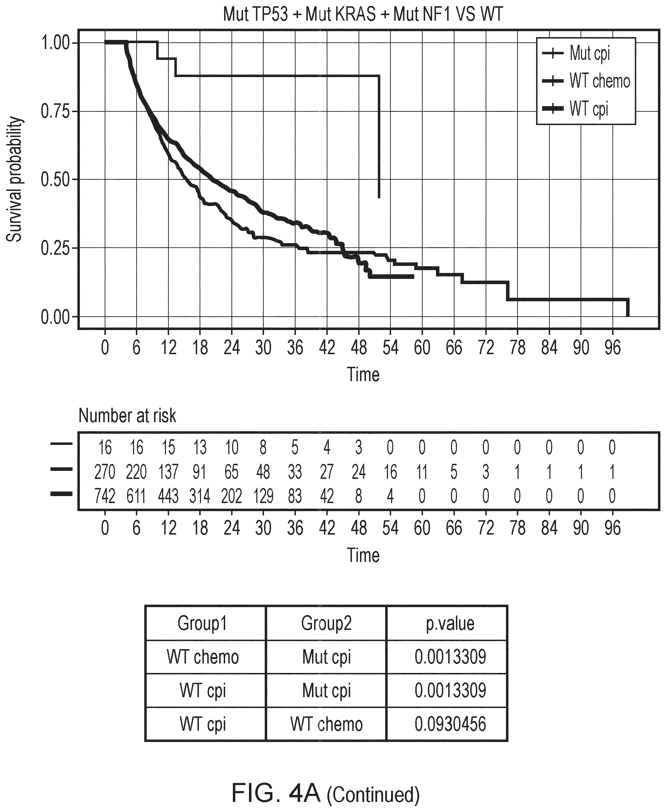

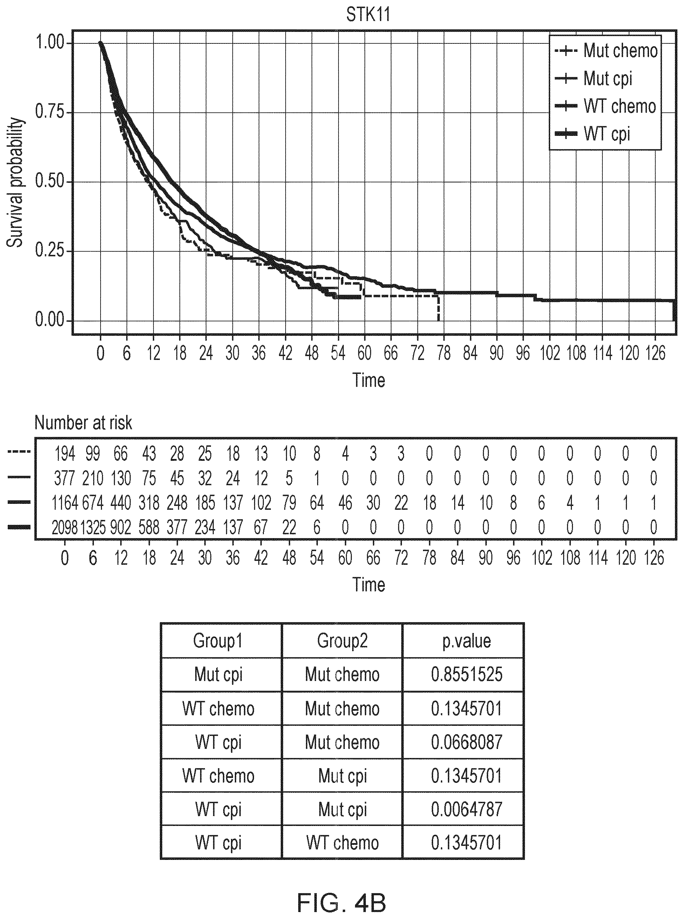

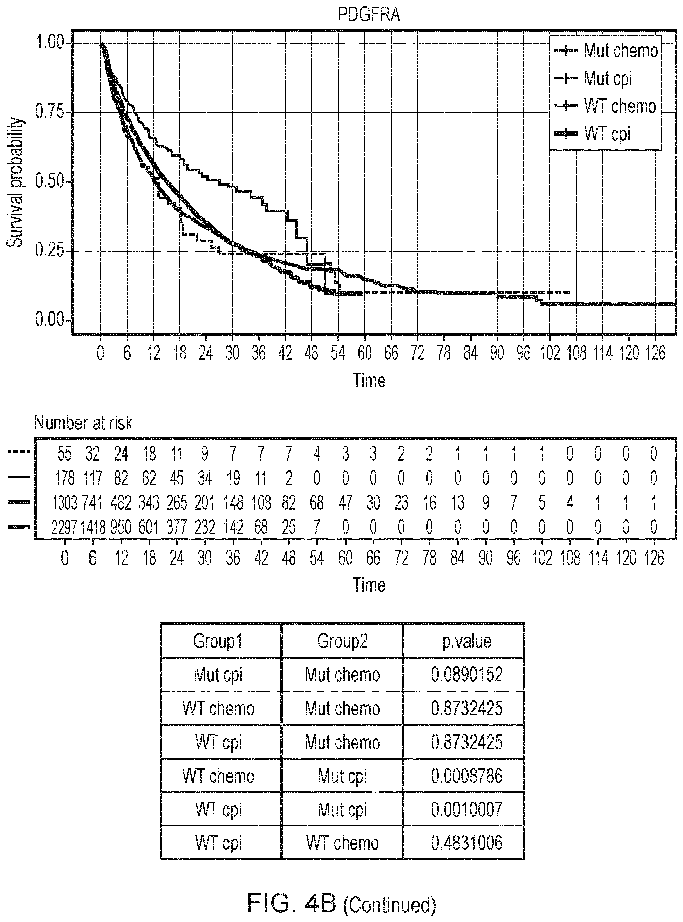

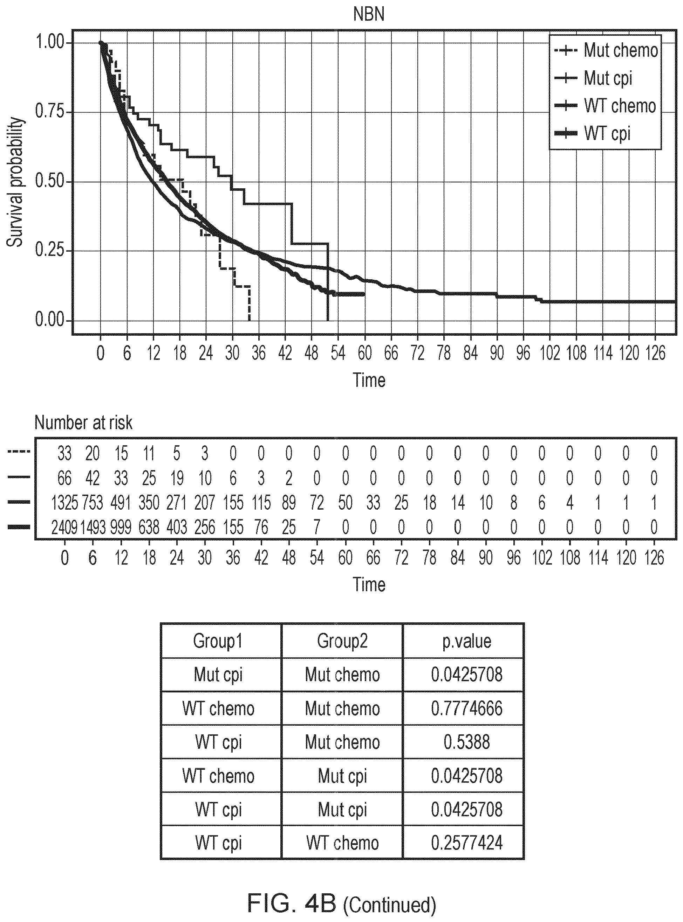

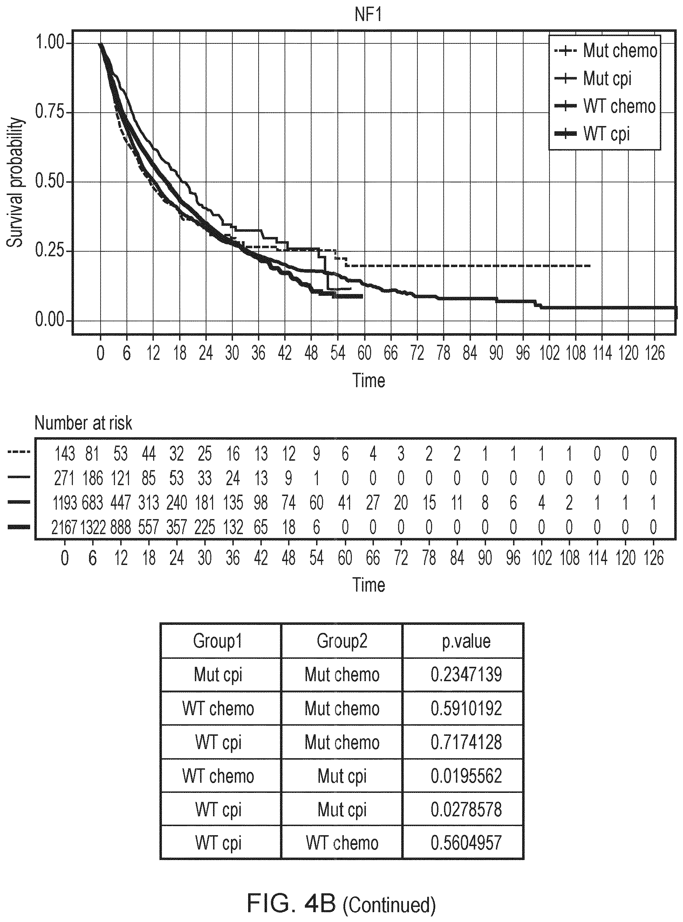

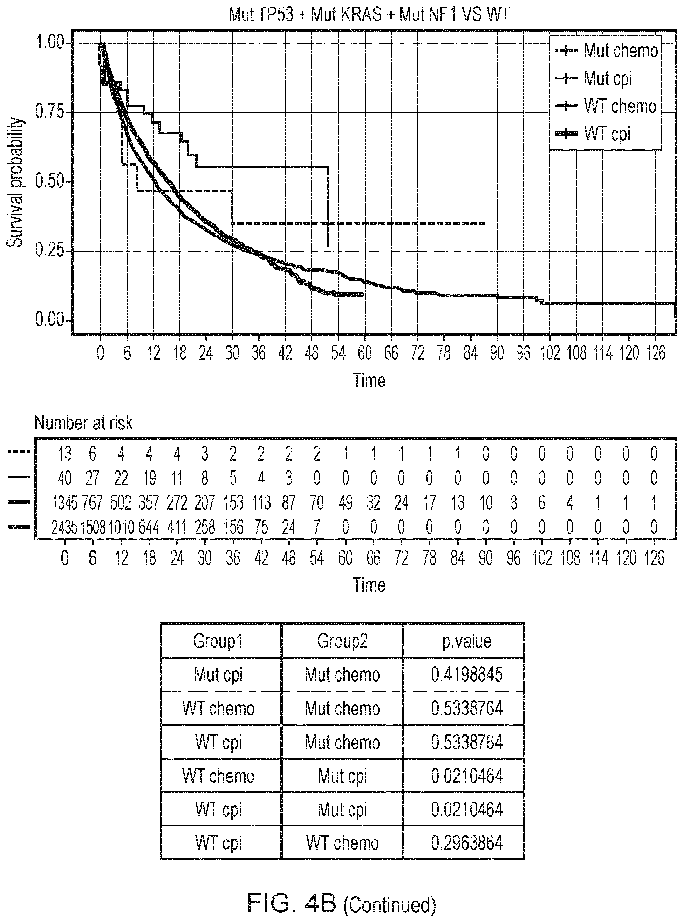

TSC2, U2AF1, FGF23, IDH1, PDCD1LG2, MEF2B, PDK1, BRIP1, QKI, CTNNA1, FUBP1, STAG2, FANCL, PAX5, MLH1, FANCG, AKT1, MPL, BRCA2, ATR, POLE, TSC1, FOXL2, BRAF, ASXL1, NF1, ATRX, NRAS, PDGFRA, SMAD4, NBN, PDGFRB, BRCA1, TP53, and SMARCA4. As shown in Supplementary Figure 4 (see also Figure 4), these genes (as mutation binary input results) were found to exhibit the greatest positive contribution to the CPI treatment response.

In some embodiments, the presence of a mutation in one or more of the following genes contributes to classifying the profile as matching the mutation profile of a response signature:

CTNNA1, ALOX12B, HRAS, FAS, U2AF1, TSC1, MLH1, BRIP1, GNA13, ERRFI1, ACVR1B, IDH1, XRCC2, BRAF, SOX9, HNF1A, PDCD1LG2, ESR1, BRCA2, ASXL1, KEL, TERT, TSC2, BARD1, BCL2, QKI, PDGFRB, BTK, FOXL2, CARD11, PBRM1, EZH2, RAD51C, ABL1, ALK, HSD3B1, POLE, FLT1, NOTCH3, PAX5, MAP3K1, STAT3, GABRA6, TP53, CUL3, NBN, PDGFRA, SDHD, RBM10 and KRAS.

In some embodiments, the presence of one or more of the following GOF and/or LOF mutations contributes to classifying the profile as matching the mutation profile of a response signature:

FGF19_LOF, U2AF1_GOF, NBN_LOF, NRAS_LOF, RAC1_LOF, RB1_GOF, IDH1_GOF, XRCC2_LOF, PAX5_GOF, PDCD1LG2_GOF, CD274_GOF, RAD52_GOF, VEGFA_LOF, CBFB_LOF, AKT1_GOF, FGF23_LOF, PDK1_LOF, MYD88_GOF, CD79B_GOF, PRKAR1A_GOF, BRCA2_GOF, SDHC_LOF, GNA13_LOF, FANCG_GOF, FANCL_LOF, SOX2_LOF, EZH2_LOF, STAG2_LOF, RAD51C_LOF, QKI_LOF, FGF6_GOF, FGF23_GOF, MUTYH_GOF, SYK_LOF, CTNNA1_LOF, CDH1_LOF, PBRM1_LOF, PIK3C2G_GOF, ASXL1_LOF, BRAF_GOF, NF1_LOF, CHEK1_LOF, FUBP1_LOF, ERRFI1_LOF, BRCA2_LOF, HSD3B1_GOF, DNMT3A_GOF, WT1_LOF, PDGFRA_LOF, ATRX_LOF, ATR_LOF, RBM10_GOF, MAP2K2_GOF, BRIP1_GOF, PDCD1LG2_LOF, MEF2B_GOF, CREBBP_GOF, IRF2_GOF, BARD1_GOF, CTNNA1_GOF, MPL_GOF, ACVR1B_LOF, PPARG_LOF, TSC2_LOF, KMT2A_LOF, PTPN11_LOF, PDGFRB_LOF, AKT1_LOF, CHEK2_LOF, NOTCH2_GOF, MUTYH_LOF, TSC2_GOF, MAP3K1_LOF, ARID1A_GOF, MED12_GOF, CBFB_GOF, PIK3R1_GOF, STAG2_GOF, MAP2K4_GOF, FLCN_GOF, TSC1_GOF, and SETD2_GOF. As shown in Supplementary Figure 4 (see also Figure 4), these genes (as mutation HFM/LFM (also known as GOF/LOF) input results) were found to exhibit the greatest positive contribution to the CPI treatment response.

In some embodiments, the presence of one or more of the following GOF and/or LOF mutations contributes to classifying the profile as matching the mutation profile of a response signature:

ALOX12B_LFM, CTNNA1_LFM, HRAS_HFM, MEF2B_LFM, TNFRSF14_LFM, XRCC2_LFM, BRIP1_LFM, U2AF1_HFM, NF1_HFM, FAS_LFM, TSC1_LFM, ERRFI1_LFM, EZH2_LFM, NBN_LFM, MLH1_LFM, GNA13_LFM, FGF19_LFM, ALK_HFM, AMER1_HFM, BRAF_HFM, IDH1_HFM, DNMT3A_HFM, CDK6_LFM, BCL6_LFM, WT1_HFM, SOX9_LFM, SMAD4_LFM, HNF1A_LFM, JAK2_LFM, PAX5_HFM, HSD3B1_LFM, PARP2_LFM, ASXL1_HFM, ESR1_LFM, PIK3C2G_HFM, BRCA2_LFM, ASXL1_LFM, TERT_HFM, WT1_LFM, BARD1_LFM, MAP3K1_LFM, FGFR2_LFM, PPARG_LFM, AXIN1_LFM, PDCD1LG2_HFM, SOX9_HFM, KRAS_LFM, PRKAR1-A_HFM, PDGFRB_LFM, TSC2_LFM, NF2_LFM, FGF6_LFM, PBRM1_LFM, KEL_LFM, FOXL2_LFM, CARD11_LFM, MAP3K13_LFM, FGF6_HFM, NOTCH3_LFM, SF3B1_LFM, CUL3_LFM, FLT1_LFM, CD274_HFM, IDH1_LFM, SPOP_LFM, MYCN_HFM, SRC_LFM, AR_LFM, ERBB2_LFM, CASP8_LFM, PDGFRA_LFM, IRF4_LFM, PTEN_LFM, GNAS_LFM, CCNE1_LFM, NOTCH2_LFM, ATR_LFM, RBM10_LFM, FANCG_HFM, SMO_HFM, EGFR_LFM and SPOP_HFM.

In some embodiments, the presence of one or more of the following GOF and/or LOF mutations contributes to classifying the profile as matching the mutation profile of a response signature:

FGF19_LOF, TP53_GOF_331, NFE2L2_GOF_24, PIK3C2G_GOF_1088, U2AF1_GOF_34, NBN_LOF, NFE2L2_GOF_77, TP53_GOF_376, TP53_GOF_244, AKT1_GOF_17, RAC1_LOF, MYCN_GOF, CDKN2A_GOF_151, DNMT3A_GOF_882, NRAS_LOF, XRCC2_LOF, PAX5_GOF, BRAF_GOF_600, RAD52_GOF, PDCD1LG2_GOF, CD274_GOF, TP53_GOF_159, VEGFA_LOF, CBFB_LOF, PDK1_LOF, KDM5C_GOF_1330, KEAP1_GOF_332, STK11_GOF_37, BRCA2_GOF, KEAP1_GOF_116, MYD88_GOF_265, IDH1_GOF, AR_GOF_457, RB1_GOF, MUTYH_GOF_165, BRAF_GOF_466, POLE_GOF, TP53_GOF_673, CD79B_GOF, ARID1A_GOF_21, PRKAR1A_GOF, DIS3_GOF_458, CDKN2A_GOF_69, FANCL_LOF, FANCG_GOF, SOX2_LOF, SMAD4_GOF, BRAF_GOF_469, TP53_GOF_275, TP53_GOF_192, STAG2_LOF, QKI_LOF, FGF6_GOF, CTNNA1_LOF, MLH1_LOF, CDH1_LOF, ERBB3_LOF, PBRM1_LOF, ASXL1_LOF, KDM5A_GOF, NF1_LOF, CHEK1_LOF, BRCA2_LOF, TP53_GOF_560, WT1_LOF, SDHB_LOF, EGFR_GOF_858, TP53_GOF_215, PDGFRA_LOF, ATRX_LOF, ATR_LOF, IRF2_GOF, EGFR_GOF_771, EGFR_GOF_861, MLH1_GOF, AR_GOF_465, STK11_GOF_221, FAS_GOF, AMER1_GOF_385, RBM10_GOF_503, KEAP1_GOF_153, STK11_GOF_199, PTCH1_GOF, STK11_GOF_163, ATRX_GOF, KEAP1_GOF_509, TP53_GOF_236, KEAP1_GOF_362, HSD3B1_GOF_75, RB1_GOF_576, STK11_GOF_216, FBXW7_GOF_479, KEAP1_GOF_523, JAK3_GOF, PPARG_LOF, PIK3CB_GOF, ACVR1B_LOF, TSC2_LOF, KMT2A_LOF, TP53_GOF_238, AKT1_LOF, PIK3R1_LOF, IRS2_GOF, HRAS_GOF_61, PIK3-CA_GOF_726, AR_GOF_468, TNFRSF14_LOF, NFE2L2_GOF_29, MEN1_GOF, BCL2_LOF, AR_GOF_467, ATM_GOF_337, KEAP1_GOF_544, NTRK3_GOF, NTRK3_GOF_610, HRAS_LOF, CTNNB1_GOF_41, SMARCA4_GOF_1160, KEAP1_GOF_409, SMAD2_GOF_464, PTEN_GOF_59, CTNNB1_GOF_33, CREBBP_GOF_1472, APC_GOF, PTEN_GOF_165, TSC1_GOF, KEAP1_GOF_417, RBM10_GOF, NBN_GOF_219, PIK3CA_GOF_345, AR_GOF_493, ZNF217_GOF_410, AR_GOF_598, STK11_GOF_256, ARID1A_GOF_343, RET_GOF_511, KEAP1_GOF_155, MITF_GOF, CDK8_GOF, DNMT3A_GOF_749, MYD88_GOF, KEAP1_GOF_430, GNAS_GOF_415, KDM5C_GOF, FLT1_GOF, NF1_GOF_1642, KMT2A_GOF_53, SDHA_GOF_531, CDKN2A_GOF_61, CEBPA_GOF_197, STK11_GOF_220, NBN_GOF_680, KEAP1_GOF_450, CHEK2_GOF_392, NKX2-1_GOF_234, SMARCA4_GOF_1157, BCORL1_GOF_94, KEAP1_GOF_493, FLCN_GOF_306, STK11_GOF_168, and MSH6_GOF_1088. As shown in Supplementary Figure 4 (see also Figure 4), these genes (as mutation Hotspot input results) were found to exhibit the greatest positive contribution to the CPI treatment response.

In some embodiments, the presence of one or more of the following GOF and/or LOF mutations contributes to classifying the profile as matching the mutation profile of a response signature:

ALOX12B_LFM, DNMT3A_HFM_771, CTNNA1_LFM, NFE2L2_HFM_24, MEF2B_LFM, TP53_HFM_331, BRAF_HFM_469, XRCC2_LFM, TNFRSF14_LFM, BRIP1_LFM, FUBP1_LFM, U2AF1_HFM_34, FAS_LFM, TP53_HFM_173, TSC1_LFM, ERRFI1_LFM, EZH2_LFM, NBN_LFM, MLH1_LFM, BRAF_HFM_600, FGF19_LFM, GNA13_LFM, ALK_HFM, TP53_HFM_215, TP53_HFM_783, TP53_HFM_560, CDK6_LFM, BCL6_LFM, TP53_HFM_280, BTG1_LFM, WT1_HFM, SOX9_LFM, SMAD4_LFM, NF1_HFM, JAK2_LFM, HSD3B1_LFM, PARP2_LFM, PIK3C2G_HFM, BRCA2_LFM, ASXL1_LFM, TERT_HFM_, BARD1_LFM, MAP3K1_LFM, FGFR2_LFM, SDHA_LFM, IGF1R_LFM, AXIN1_LFM, PDCD1LG2_HFM, PAX5_HFM, TP53_HFM_294, RAD51C_LFM, JUN_LFM, PRKAR1A_HFM, PDGFRB_LFM, TSC2_LFM, NF2_LFM, BTK_LFM, PBRM1_LFM, FOXL2_LFM, MAP3K13_LFM, NOTCH3_LFM, GABRA6_LFM, PIK3CA_HFM_542, TP53_HFM_159, CUL3_LFM, ASXL1_HFM, FLT1_LFM, MITF_LFM, TP53_HFM_249, IDH1_LFM, SRC_LFM, AR_LFM, ERBB2_LFM, SMO_LFM, KEAP1_LFM, PDGFRA_LFM, GATA6_LFM, PTEN_LFM, GNAS_LFM, CCNE1_LFM, ATR_LFM, MERTK_LFM, KMT2A_LFM, SOX9_HFM, GSK3B_HFM, CUL4A_LFM and BCL2L2_LFM.

In some embodiments, the presence of a mutation in one or more of the following genes contributes to classifying the profile as matching the mutation profile of a resistance signature:

GID4, JUN, RAD51, CD79A, STK11, TET2, MAP2K1, CCNE1, CDK4, ERCC4, CEBPA, RARA, CDKN1A, RAD51B, PPP2R1A, CSF1R, FH, NKX2-1, NTRK2, FGFR1, CDK6, MDM2, and BRD4. As shown in Supplementary Figure 4 (see also Figure 4), these genes (as mutation binary input results) were found to exhibit the greatest negative contribution to the CPI treatment response (i.e. greatest contribution to CPI resistance).

In some embodiments, the presence of a mutation in one or more of the following genes contributes to classifying the profile as matching the mutation profile of a resistance signature:

RARA, VHL, GID4, IKBKE, CEBPA, CD79A, CDK12, STK11, BAP1, CDKN2B, FGF10, ARFRP1, CSF1R, FANCC, CDC73, ZNF703, PIK3CA, NKX2-1, TNFAIP3, CCNE1, SOX2, SDHC, MYC, TERC, PARP3, JAK1, DDR2, PARP1, AKT2, PDK1, NFE2L2 and MDM4.

In some embodiments, the presence of one or more of the following GOF and/or LOF mutations contributes to classifying the profile as matching the mutation profile of a resistance signature:

MAP3K1_GOF, FGF14_GOF, PPP2R1A_GOF, CDKN1A_GOF, JAK1_GOF, IRF4_GOF, JUN_LOF, AKT3_GOF, NKX2-1_LOF, TGFBR2_GOF, GABRA6_GOF, BCORL1_GOF, TNFAIP3_GOF, POLD1_GOF, GNAQ_GOF, RAD51C_GOF, BRD4_GOF, RNF43_GOF, FGFR4_GOF, GATA6_GOF, GATA3_GOF, MDM2_LOF, MYC_LOF, RPTOR_GOF, CD79A_LOF, CCNE1_GOF, STK11_LOF, MYCN_LOF, CEBPA_LOF, PARP1_GOF, NOTCH3_GOF, SDHD_GOF, LTK_GOF, DAXX_GOF, ABL1_GOF, PDGFRB_GOF, BTG1_GOF, CHEK1_GOF, GATA4_GOF, JUN_GOF, FLT3_GOF, CUL3_GOF, CDK4_GOF, AKT2_GOF, KDM6A_GOF, MTOR_GOF, BCL2L1_LOF, CD274_LOF, NF2_GOF, SMARCA4_GOF, NFKBIA_GOF, ERCC4_LOF, ZNF703_GOF, STK11_GOF, KEAP1_GOF, ERBB2_GOF, FH_GOF, REL_LOF, RARA_GOF, RAD51_LOF, PTEN_GOF, NTRK2_LOF, CDKN2B_LOF, BAP1_LOF, RARA_LOF, AXL_GOF, CCND2_LOF, CUL4A_LOF, H3F3A_GOF, GRM3_GOF, CDK6_GOF, ARFRP1_LOF, SUFU_LOF, RAD54L_LOF, PIK3CB_LOF, KLHL6_GOF, MAP3K13_GOF, PIK3CA_GOF, CDKN2A_LOF, EZH2_GOF, FANCA_LOF, HGF_LOF, FBXW7_GOF, FGFR2_GOF, AURKA_LOF, HSD3B1_LOF, and SDHC_GOF. As shown in Supplementary Figure 4 (see also Figure 4), these genes (as mutation HFM/LFM (also known as GOF/LOF) input results) were found to exhibit the greatest negative contribution to the CPI treatment response (i.e. greatest contribution to CPI resistance).

In some embodiments, the presence of one or more of the following GOF and/or LOF mutations contributes to classifying the profile as matching the mutation profile of a resistance signature:

MYC_LFM, FANCC_HFM, JAK1_HFM, FGFR2_HFM, GABRA6_HFM, RARA_HFM, ERBB4_HFM, CD79A_LFM, GID4_LFM, IGF1R_HFM, GNA11_HFM, GATA6_HFM, CDC73_HFM, CHEK2_HFM, ARFRP1_HFM, SMARCB1_HFM, FGF10_HFM, CDK12_HFM, IKBKE_LFM, DDR2_HFM, NF2_HFM, AKT3_HFM, CEBPA_LFM, FGF14_HFM, EP300_HFM, CCNE1_HFM, AKT2_HFM, BAP1_LFM, FH_HFM, CEBPA_HFM, ERBB2_HFM, CCND2_LFM, ATM_HFM, PIK3CA_LFM, STK11_HFM, CDKN2B_LFM, PRDM1_LFM, NOTCH3_HFM, CDK12_LFM, ZNF703_HFM, CSF1R_LFM, KLHL6_HFM, BCL2L2_HFM, LYN_HFM, RICTOR_HFM, SOX2_HFM, STK11_LFM, KEAP1_HFM, CYP17A1_LFM, CUL4A_HFM, PRKCI_HFM, PIK3CA_HFM, TERC_HFM, IKZF1_HFM, MEF2B_HFM, NFE2L2_HFM, CDKN2A_LFM, NKX2-1_HFM, TEK_LFM, MYCL_LFM, ROS1_HFM, MCL1_HFM, FGF12_HFM, EZH2_HFM and MDM4_HFM.

In some embodiments, the presence of one or more of the following GOF and/or LOF mutations contributes to classifying the profile as matching the mutation profile of a resistance signature:

EGFR_GOF_746, TP53_GOF_282, EGFR_GOF_747, EGFR_GOF_719, TP53_GOF_195, FGF14_GOF, PPP2R1A_GOF, IRF4_GOF, MAP3K1_GOF, STK11_GOF_291, ERBB2_GOF_776, JAK1_GOF, CDKN1A_GOF, KEAP1_GOF_272, JUN_LOF, AKT3_GOF, NKX2-1_LOF, ATM_GOF, CDKN2A_GOF_100, STK11_GOF_84, FGFR4_GOF, KEAP1_GOF_483, KEAP1_GOF_234, GABRA6_GOF, CDKN2A_GOF_80, MYCN_GOF_44, KEAP1_GOF_260, MAP2K1_GOF_102, PTEN_GOF, PTCH1_GOF_48, GNAQ_GOF, KEAP1_GOF_364, POLD1_GOF, PIK3CA_GOF_1043, BCOR_GOF_679, FH_GOF_476, STK11_GOF_181, TP53_GOF_285, TNFAIP3_GOF, BRD4_GOF, KDM5C_GOF_1546, KEAP1_GOF_274, SMAD4_GOF_351, RNF43_GOF, SF3B1_GOF_666, MTOR_GOF_1834, NOTCH1_GOF, TP53_GOF_272, GATA6_GOF, MDM2_LOF, GATA3_GOF, PIK3CA_GOF_1047, TP53_GOF_278, CD79A_LOF, RPTOR_GOF, ZNF703_GOF, CCNE1_GOF, STK11_LOF, MYCN_LOF, PARP1_GOF, CEBPA_LOF, MAP2K1_GOF_121, SMARCA4_GOF_1243, MED12_GOF, KEAP1_GOF_135, AR_GOF_69, BTG1_GOF, MAP3K1_GOF_5, TYRO3_GOF, ERBB2_GOF_755, CBL_GOF_1096, STK11_GOF_176, EGFR_GOF_790, RAF1_GOF, KEAP1_GOF_244, STK11_GOF_464, STK11_GOF_308, KDM6A_GOF, PDGFRB_GOF, BRAF_GOF_464, PIK3C2G_GOF_129, FBXW7_GOF_505, KEAP1_GOF_470, ALOX12B_GOF, FLT3_GOF, AMER1_GOF_625, IDH2_GOF_140, GNAS_GOF_407, KEAP1_GOF_186, BCOR_GOF_1526, NFE2L2_GOF_27, STK11_GOF_251, CHEK1_GOF, HRAS_GOF_13, KEAP1_GOF_236, GATA4_GOF, MET_GOF_2888, KMT2D_GOF_755, RAD51C_GOF_21, SMARCA4_GOF_1162, STK11_GOF_734, MAP3K1_GOF_949, PALB2_GOF, BCORL1_GOF_883, KEAP1_GOF, ARID1A_GOF_515, AR_GOF_469, EGFR_GOF_763, AR_GOF_70, PPARG_GOF, VHL_GOF, PARP3_GOF, CDK4_GOF, AKT2_GOF, TP53_GOF_920, NFE2L2_GOF_30, CHEK2_GOF, SMAD2_LOF, SMARCB1_GOF, TP53_GOF_298, SDHA_GOF_457, FH_GOF, CDC73_GOF, NFKBIA_GOF, ERCC4_LOF, RARA_GOF, RAD51B_LOF, NTRK2_LOF, CUL4A_LOF, STK11_GOF_57, TP53_GOF_234, STK11_GOF_242, CHEK2_GOF_367, RAD51D_GOF, CTCF_LOF, EPHA3_GOF, RAD54L_LOF, PIK3CB_LOF, KLHL6_GOF, PIK3CA_GOF, DDR2_GOF, MAP3K13_GOF, PIK3CA_GOF_545, HGF_LOF, NFE2L2_GOF_31, MET_GOF_3028, BCL6_GOF, TP53_GOF_342, and CDKN2A_GOF_83. As shown in Supplementary Figure 4 (see also Figure 4), these genes (as mutation Hotspot input results) were found to exhibit the greatest negative contribution to the CPI treatment response (i.e. greatest contribution to CPI resistance).

In some embodiments, the presence of one or more of the following GOF and/or LOF mutations contributes to classifying the profile as matching the mutation profile of a resistance signature:

EGFR_HFM_746, MYC_LFM, FANCC_HFM, JAK1_HFM, NFE2L2_HFM_79, GABRA6_HFM, RARA_HFM, ERBB4_HFM, CD79A_LFM, NF2_HFM, GNA11_HFM, CHEK2_HFM, GID4_LFM, NFE2L2_HFM_29, IGF1R_HFM, GATA6_HFM, CDC73_HFM, FGFR2_HFM, CHEK2_HFM_157, STK11_HFM_242, TP53_HFM_152, STK11_HFM_53, ARFRP1_HFM, TP53_HFM_282, FGF10_HFM, CDK12_HFM, IKBKE_LFM, DDR2_HFM, NKX2-1_LFM, AKT3_HFM, ERBB2_HFM_775, TP53_HFM_920, CEBPA_LFM, DIS3_HFM, GATA3_HFM, EP300_HFM, PIK3CA_HFM_1047, FGF14_HFM, ARAF_HFM_181, AKT2_HFM, CCNE1_HFM, BAP1_LFM, FH_HFM, TP53_HFM_154, PIK3CA_HFM, VHL_LFM, ERBB3_HFM, H3F3A_HFM, CCND2_LFM, PIK3CA_LFM, CDKN2B_LFM, PRDM1_LFM, STK11_HFM_194, TP53_HFM_135, KEAP1_HFM_320, ZNF703_HFM, CSF1R_LFM, KLHL6_HFM, BCL2L2_HFM, LYN_HFM, SOX2_HFM, STK11_LFM, SDHC_HFM, EPHA3_HFM, CUL4A_HFM, STK11_HFM, MAP3K13_HFM, BCL6_HFM, TERC_HFM, NFE2L2_HFM_28, JAK3_HFM, TP53_HFM_272, CDKN2A_LFM, TEK_LFM, ROS1_HFM, NFKBIA_HFM and FGF19_HFM.

In some embodiments, the CPI response is a durable response, such as a lack of disease progression for at least 270 days following CPI therapy. In some embodiments, CPI resistance is an innate resistance, such as a lack of response to CPI therapy and/or a confirmed disease progression with 270 days of CPI therapy. In some embodiments, CPI resistance may comprise so-called acquired resistance to CPI therapy. Preferably, CPI resistance comprises innate resistance to CPI therapy, as described in further detail in the Methods section of the Examples, herein.

In a second aspect, the present invention provides a method for classifying a sample obtained from a subject having a cancer (e.g. a lung cancer, such as NSCLC), said sample comprising nucleic acid, optionally wherein said sample is derived from one or more cancer cells of the subject, the method comprising:

analysing the sample to obtain a mutation profile for the subject, said mutation profile comprising the presence or absence of mutations at one or more locations in at least five, six, or at least seven genes as defined in accordance with the first aspect of the invention. In particular, the at least five genes may be selected from the group consisting of: NF1,

7

STK11, TSC2, BRCA2, BRAF, STAG2, U2AF1, BRIP1, PDGFRA, CTNNA1, PDK1, FGF10, and FLT1; and analysing the mutation profile to classify the profile as matching the mutation profile of a checkpoint inhibitor (CPI) response signature or a CPI resistance signature, wherein the sample is classified as being derived from a CPI therapy responsive subject if the mutation profile is classified as matching the mutation profile of the CPI response signature and is characterised as being derived from a CPI therapy resistant subject if the mutation profile is classified as matching the mutation profile of the CPI resistance signature.

In some embodiments, the mutations at one or more locations are cancer-specific mutations.

In some embodiments, analysing the sample to obtain the mutation profile for the subject comprises nucleic acid sequencing. For example, the sample may be sequenced to provide at least exonic coverage of the at least five, six, seven or all of the genes as defined in connection with the first aspect of the invention.

In some embodiments, the sample comprises a tumour tissue sample (e.g. tumour biopsy sample), a circulating tumour cell or a cell-free sample comprising cell-free DNA, circulating tumour DNA (ctDNA) and/or circulating tumour RNA (ctRNA) (e.g. a blood, plasma, urine, CSF, or other liquid biological sample).

In some embodiments, analysing the mutation profile to classify the profile as matching the mutation profile of a response signature or a resistance signature comprises carrying out the method of the first aspect of the invention.

In accordance with any aspect of the present invention, the method may further comprise analysing one or more additional markers of CPI response derived from the subject to supplement and/or corroborate the prediction of CPI response or resistance. In particular, the one or more additional markers of CPI response may be selected from the group consisting of: age, disease stage, histology, smoking history, race, gender, tumour mutational burden (TMB), microsatellite instability (MSI), PD-L1 expression, JAK1/JAK2, IFNg, PTEN loss, PBRM1, STK11/KEAP1 mutations, antigen processing/presentation loss, and WNT/b-catenin signalling.

In a third aspect, the present invention provides a system for predicting the treatment response of a subject having a cancer (e.g. a lung cancer, such as NSCLC) to an immune checkpoint inhibitor (CPI) therapy, the system comprising:

at least one processor; and
at least one non-transitory computer readable medium containing instructions that, when executed by the at least one processor, cause the at least one processor to perform operations comprising:

obtaining a mutation profile for the subject, said profile comprising the presence or absence of one or more mutations at one or more locations in at least five, six or seven genes as defined in connection with the first aspect of the invention, for example, at least five genes selected from the group consisting of: NF1, STK11, TSC2, BRCA2, BRAF, STAG2, U2AF1, BRIP1, PDGFRA, CTNNA1, PDK1, FGF10, and FLT1;
analysing the mutation profile to classify the profile as matching the mutation profile of a CPI response signature or a CPI resistance signature,

wherein the subject is predicted to be likely to respond to the CPI therapy if the mutation profile for the subject is classified as matching the mutation profile of the CPI response signature and is predicted to be likely not to respond to the CPI therapy if the mutation profile for the subject is classified as matching the mutation profile of the CPI resistance signature.

In some embodiments, said instructions, when executed by the at least one processor, cause the at least one processor to perform the method of the first aspect of the invention.

In a fourth aspect, the present invention provides one or more computer readable media comprising instructions that, when executed by one or more processors, cause the one or more processors to perform the steps of the method of the first aspect of the invention.

In a fifth aspect, the present invention provides a pharmaceutical composition comprising an immune checkpoint inhibitor (CPI) for use in a method of treatment of a subject having a cancer (e.g. a lung cancer, such as NSCLC), wherein the subject has been predicted to respond to said CPI therapy by a method of the first or second aspect of the invention.

In some embodiments, the method of treatment comprises the step of predicting whether the subject will respond to the CPI therapy by a method of the first or second aspect of the invention.

In some embodiments, the immune checkpoint inhibitor comprises an inhibitor of PD-L1, PD-1, and/or CTLA-4, optionally wherein the inhibitor comprises an antibody. In particular, the immune checkpoint inhibitor may be selected from the group consisting of: nivolumab, pembrolizumab, atezolizumab, durvalumab, avelumab, ipilimumab, and cemiplimab.

The invention includes the combination of the aspects and preferred features described except where such a combination is clearly impermissible or expressly avoided.

### *Summary of the Figures*

Embodiments and experiments illustrating the principles of the invention will now be discussed with reference to the accompanying figures in which:

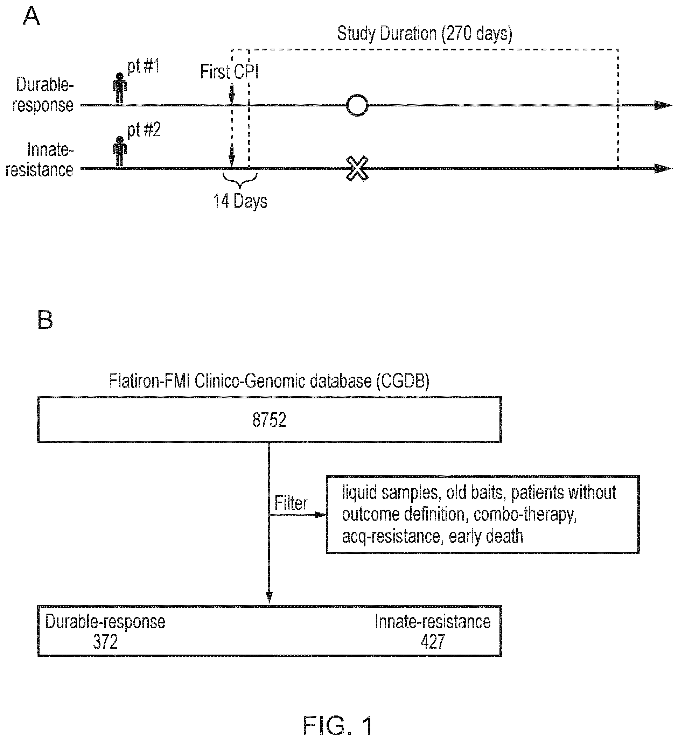

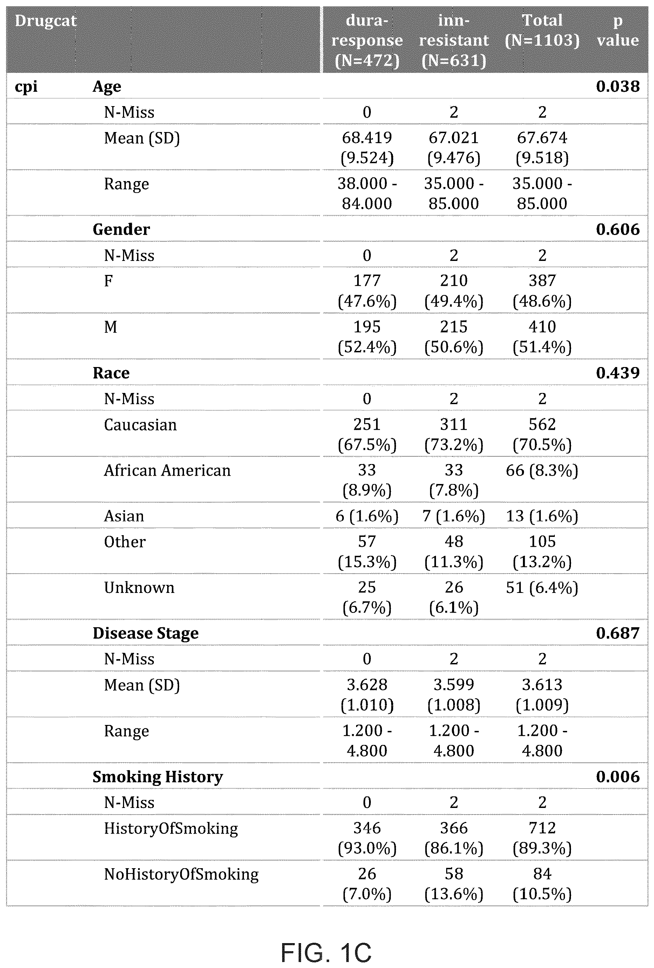

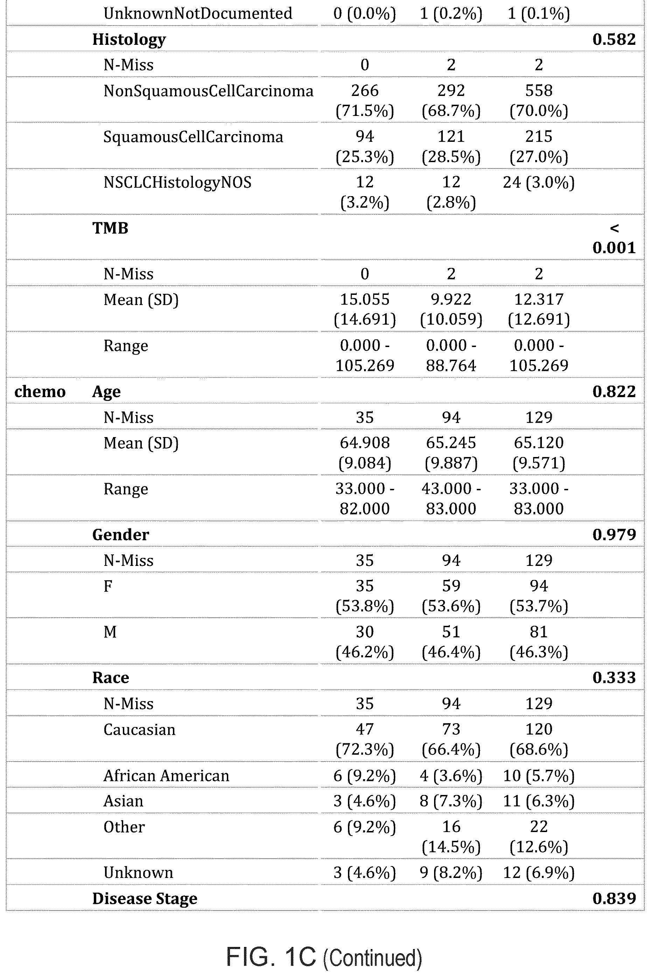

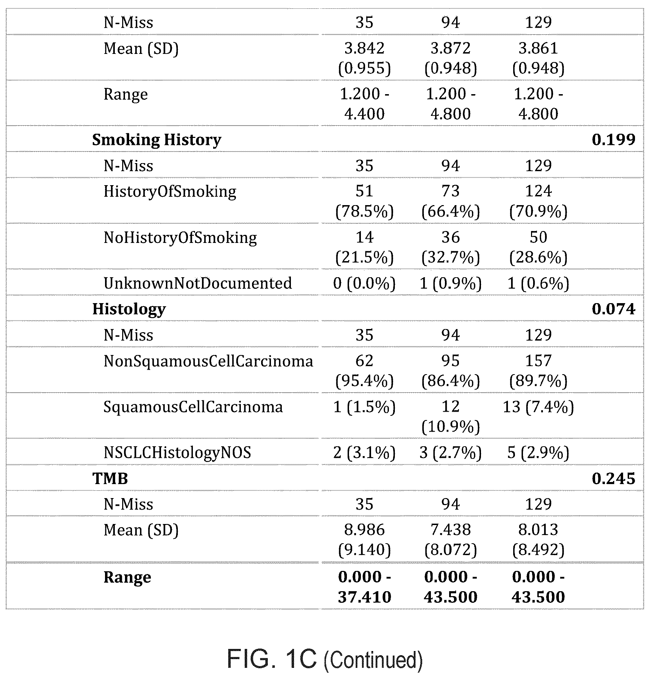

**Figure 1: Patient's treatment outcome group definition and cohort selection from CGDB A.** Schematic representation of response definition for durable-response and innate-resistance. The long blue arrows represent patient journey over time. The study period (270 days) in which treatment outcomes were investigated is marked by green dotted lines. Green vertical arrows represent the first time a patient was treated with a CPI and the grayed out area represents buffer time (14 days) during which treatment outcomes are ignored because they were likely resulting from the previous treatment. Green circle represents clinical benefit from CPI therapy (CR, PR, SD), while red X represents disease progression. **B.** Schematic representing the number of patients selected based on the criteria depicted in the scheme (more details in methods) C. Clinical characteristics of the cohort

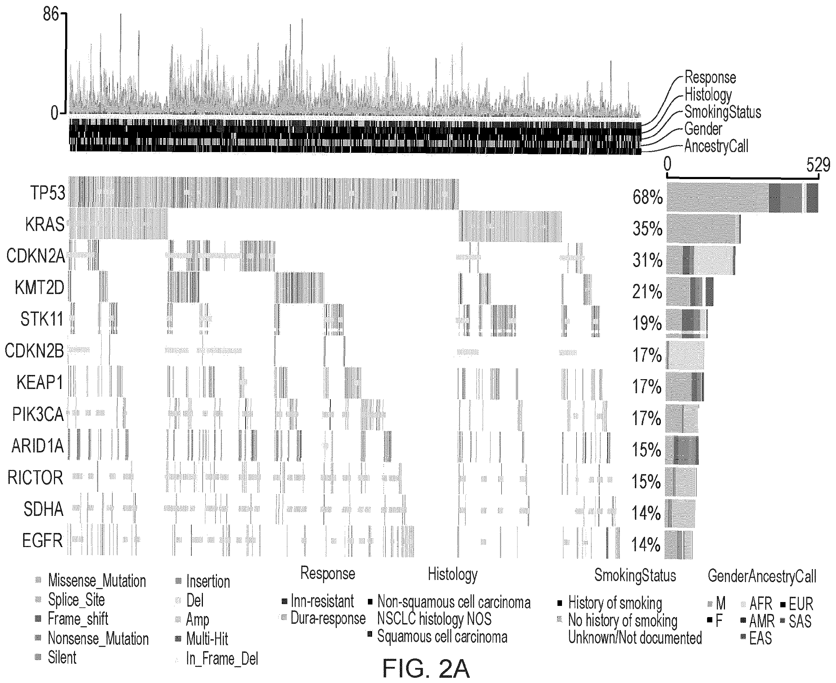

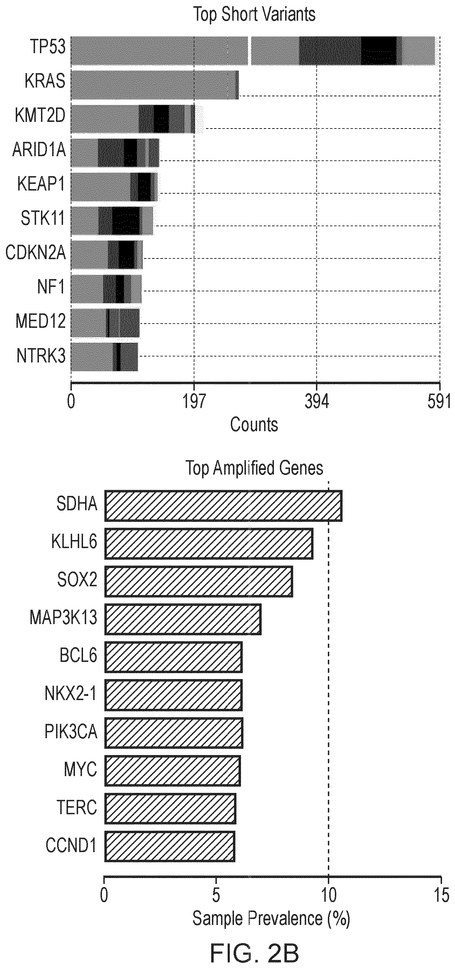

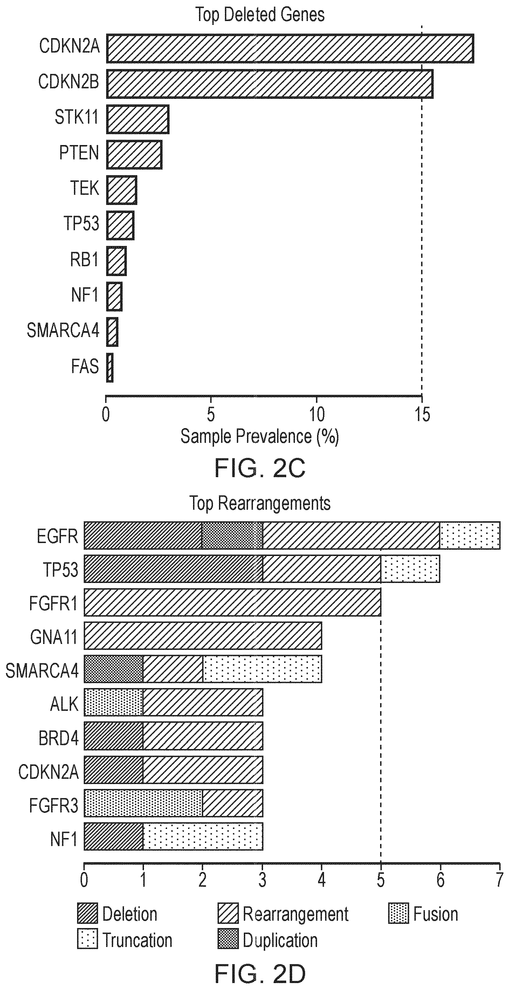

**Figure 2: Mutational landscape of the selected cohort**
**A.** Oncoplot including all patients in the CPI cohort (n=1150), depicting the top 12 altered genes and some clinical characteristics. Each column represents a single patient and each row represents a gene. The bar plot on the top of the figure represents the number of mutations in each patient with each color representing the type of mutations (Missense, splice site, frame shift, etc). Middle of the figure represents a heatmap with clinical characteristics that include response, histology, smoking status, gender and ancestry call, with color scheme depicted at the bottom of the figure. The bottom part represents the oncoplot with different colors in each row representing a specific mutation in the depicted patient. The different colors represent different types of mutations (color scheme as in **A**) with the color scheme depicted at the bottom of the figure. The right side of the oncoplot shows a bar graph summarizing the total count of the indicated mutation types and the stacking represents the proportion of each type of mutation. **B.** Bar graph showing the top short variants found in our cohort (n=1150) with color stacking representing the different types of mutation within each gene (same color scheme as in A) **C.** The left panel shows prevalence (in percent) of the top 10 deleted genes (green bar graph) and lower left panel is showing prevalence (in percent) of top 10 amplified genes (orange bar graph). **D.** Bar graph representing prevalence (in percent) of top 10 rearrangements, with color scheme (bottom figure legend) representing different types of rearrangements.

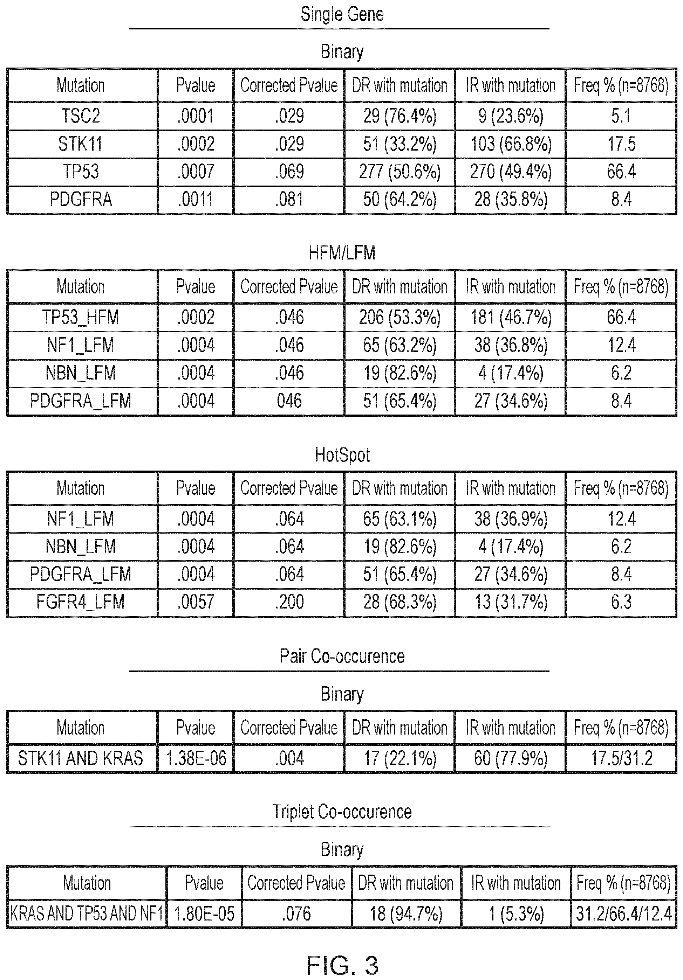

**Figure 3: Mutation association with response or resistance**
Tables representing mutations that were found to be significantly or marginally-significantly enriched with treatment outcome (durable-response or innate-resistance). The first section (depicted as single gene) represents statistical test results on single gene levels analysing the three different mutation classifications (binary, HFM/LFM, Hotspot). The middle section (depicted as pair co-occurrence) represents statistical test results on a pair of co-occurring mutations showing results from binary mutation classification. Bottom section (represented as triplet co-occurrence) represents statistical test results on triplet co-occurring mutations showing results from binary mutation classification. For each section genes associated with durable-response are green, and genes associated with innate-resistance are red. Columns show: Mutation, showing the mutated gene name. P-value, showing p-value derived by Fisher Exact Test. Corrected P-value, showing false discovery rate (FDR) corrected p-value. DR with mutations, shows the number of durable-response (DR) patients with mutation in the gene of interest (among 1150 patients), and in brackets the percent of patients having the mutation with DR (#DR/#IR+#DR). IR with mutation, shows the number of innate-resistant (IR) patients with the specific mutation in the gene of interest, in brackets same as in DR with mutation column. Freq %, represents the percent of patients having any mutation in the specific gene, calculated across 8768 patients. Any gene/row with FDR value below 0.05, was filled in green.

**Figure 4: OS analysis of the 1150 patients with mutations found to be significantly associated with CPI outcomes**
**A.** Kaplan-Meier survival curves of overall survival (OS) in patients treated with CPI or Chemo with or without mutations in genes found to be significant/marginally-significant in Figure 3. Each Kaplan-Meier plot also shows the number of patients (in the Number at risk table) at each time point (Time in month), and includes a significance table with p-values when comparing each patient group (depicted as Group1 and Group2) at the bottom of each plot. **B.** Same as in **A,** with the exception of using the extended CGDB database of 3362 patients treated with CPI and Chemo.

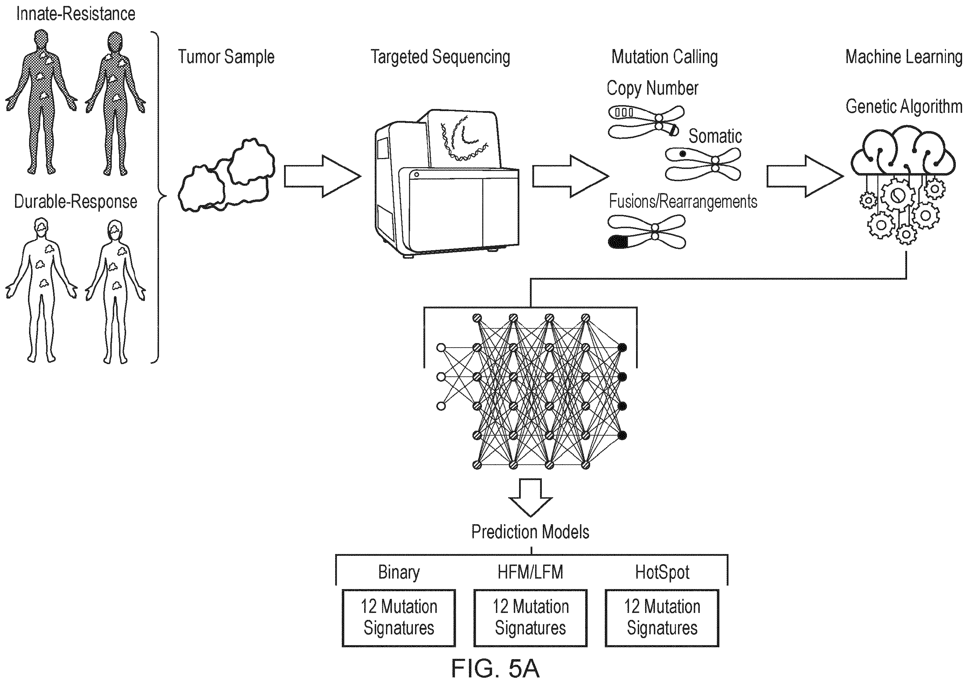

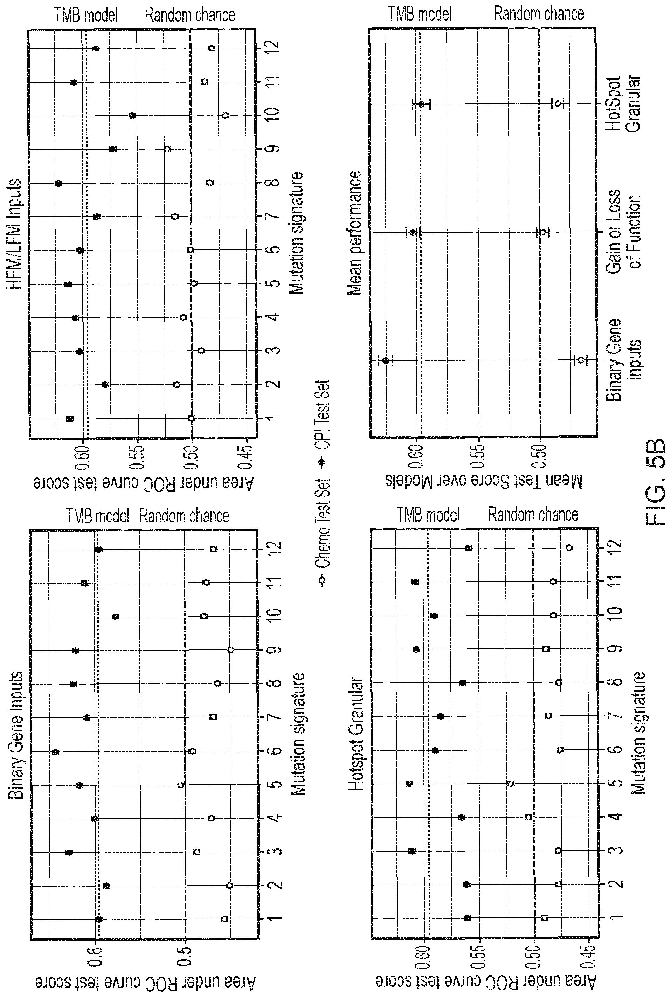

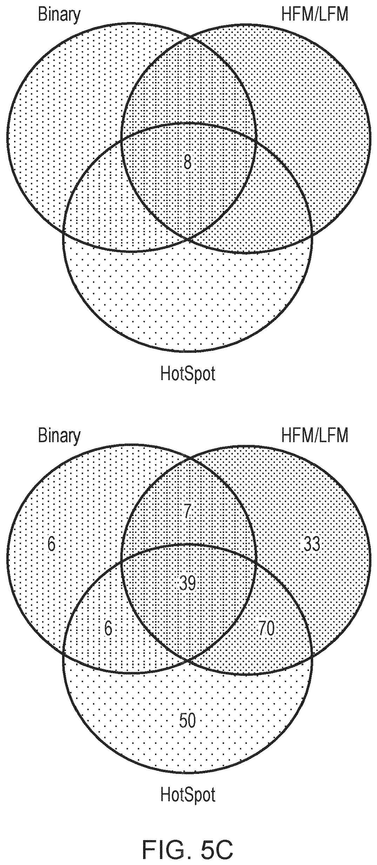

**Figure 5: ML pipeline identifies 36 predictive genetic signatures with a core of shared genes**
**A.** Schematic depicting the machine learning pipeline. **B.** Plot showing the Area under the ROC Curve (AUC) from held out (not used in training) test set (n=173) for each of the input types (Binary, HFM/LFM and Hotspot granular) for 36 different genetic signatures with blue and orange dots representing CPI and Chemo treated patients respectively.

Each blue dot represents AUC derived from held out test set for an individual genetic signature in patients treated with CPI therapy, while orange dots represent Area under ROC Curve score derived from same genetic signature but in patients treated with chemotherapy. Error bars in blue and orange were derived from cross validation scores and are standard error of the mean (SEM). For each graph the black dotted horizontal line represents the scores for only TMB model and the orange dotted line represents the 50 percent accuracy score (depicted as random chance). Lower right panel shows average AUC scores of the 12 genetic signatures for each of the three inputs, with error bars as SEM. C. Left Venn diagram representing the gene overlap between the 36 genetic signatures across binary, HFM/LFM and Hotspot granular inputs, with 8 genes representing genes that are included in every single genetic signature (36). The right Venn diagram represents overlap between the unique genes within the 12 genetic signatures across the three inputs (58 binary genes, 149 HFM/LFM genes, and 165 hotspot genes).

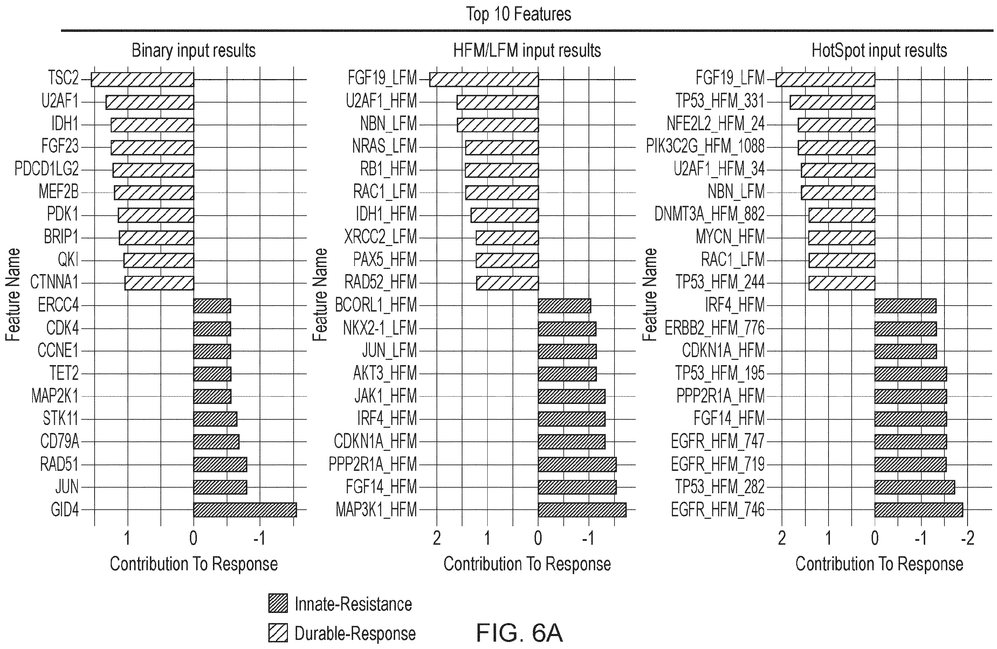

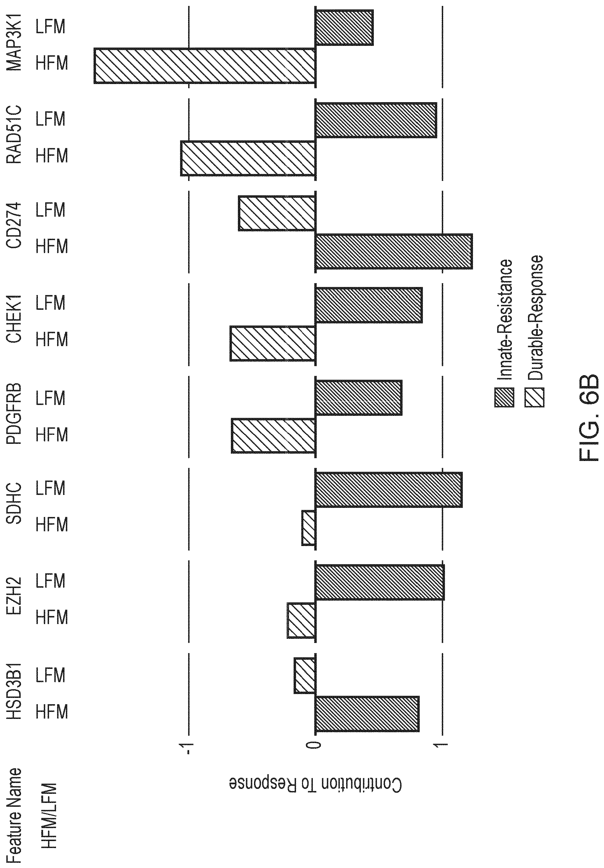

**Figure 6: Relative contribution of mutations to CPI response in 36 genetic signatures**
**A.** Waterfall plot of the top 10 linear coefficients (represent feature importance) derived from linear conversion of the 36 ML derived genetic signatures sorted by feature importance with positive values indicate association with durable-response (green bars) and negative values with innate-resistance (red bars). Left panel represents all the unique genes in binary input. Middle panel represents HFM/LFM feature importance and right most panel represents feature importance from Hotspot granular input. **B.** Plot representing linear coefficients in genes in which HFM and LFM mutations have divergent effects on CPI response, with red associated with innate-resistance and blue with durable-response.

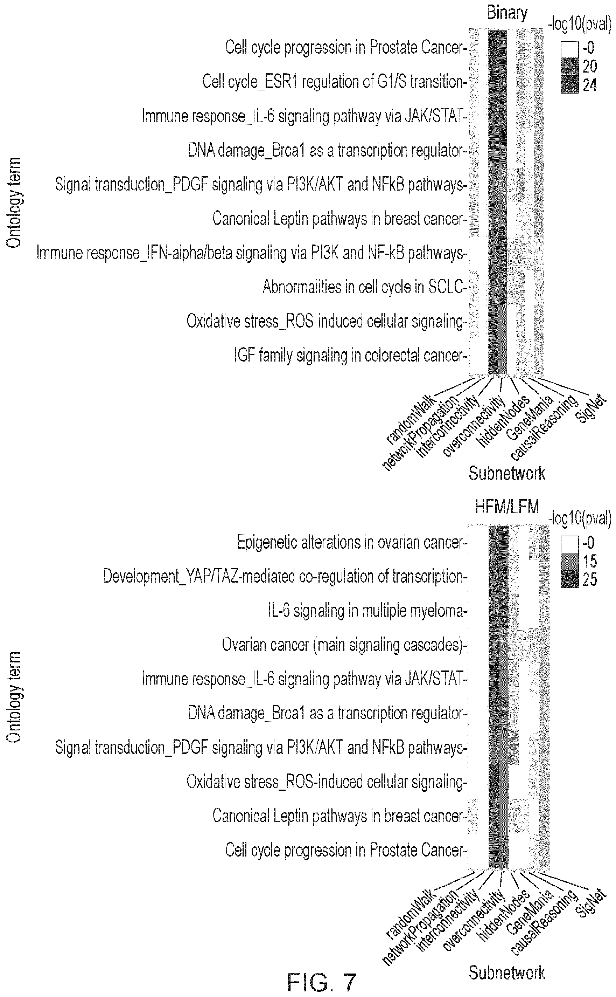

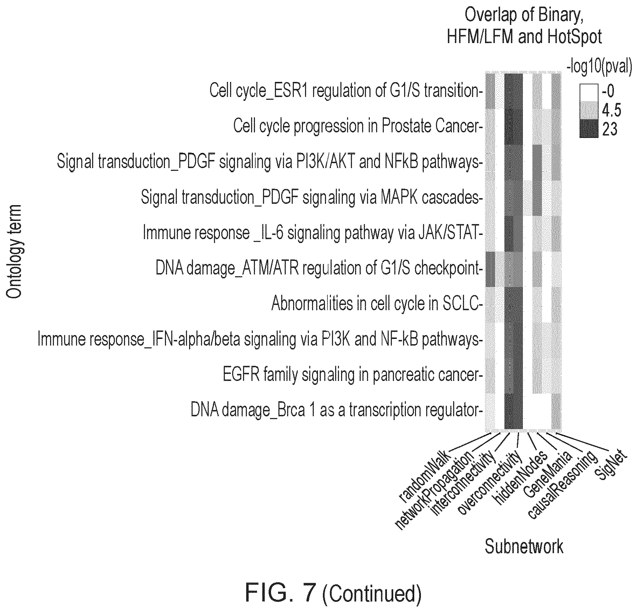

**Figure 7: Pathway analysis of predictive genetic signatures reveals immune response and other biological pathways associated with CPI response.**
Top 10 pathways derived from CBDD pathway analysis utilizing 8 different network-based algorithms. The results represent the pathways that have lowest P-value (derived from hypergeometric test) across algorithms. The Binary and HFM/LFM mutations are the two top panels. Lower panel (depicted as Overlap of Binary, HFM/LFM and Hotspot) represents the topology assisted pathway analysis of the 39 gene overlap between binary, HFM/LFM and Hotspot granular genetic signatures.

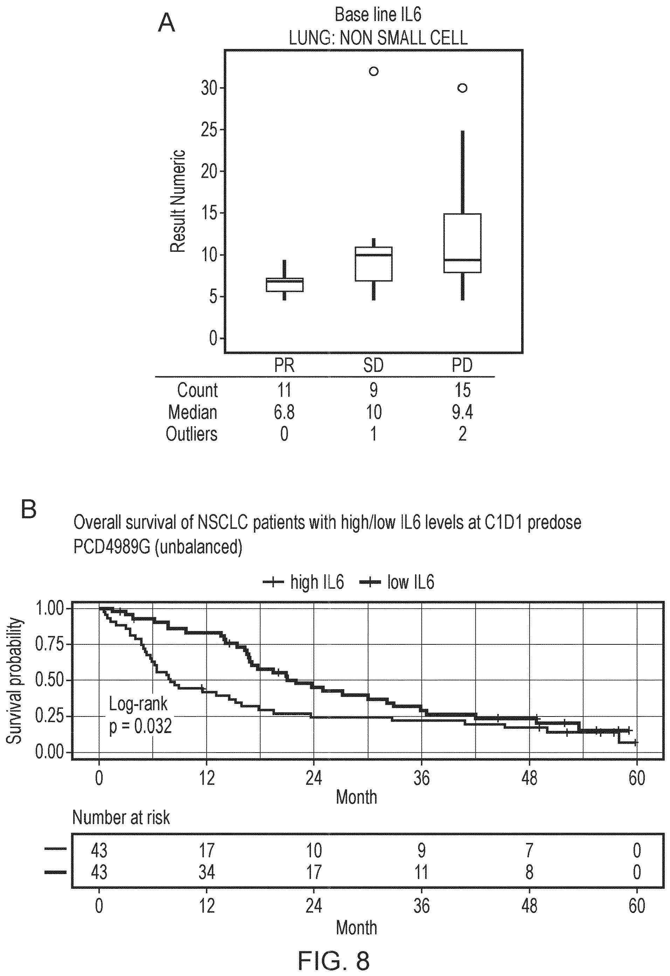

**Figure 8: Validating the role of IL6 identified from the pathway analysis at the protein level in atezolizumab clinical study. High serum levels of IL-6 is associated with progressive disease in patients treated with Atezolizumab**
**A.** Boxplot depicting IL6 levels at baseline in patients who atezolizumab response was assessed by the RECIST criteria (CR, PR, SD or PD). **B.** Kaplan-Meier curves of OS in patients treated with Atezolizumab (trial PCD4989G) comparing patients with high (red) and low (blue) IL-6 serum levels, with the number of patient's in each month shown in the Number in risk table below the OS plot.

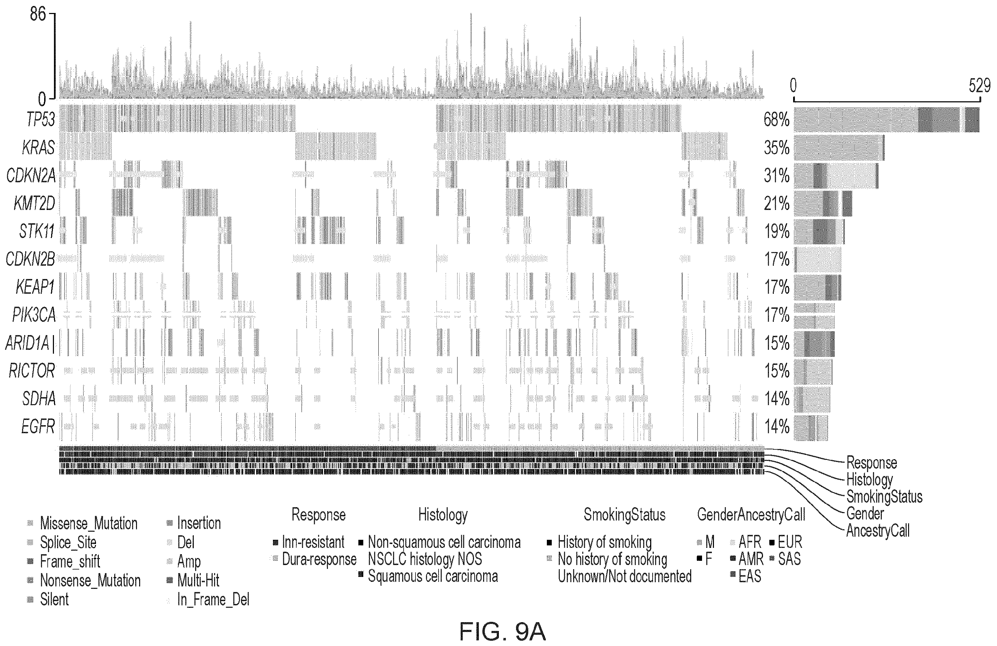

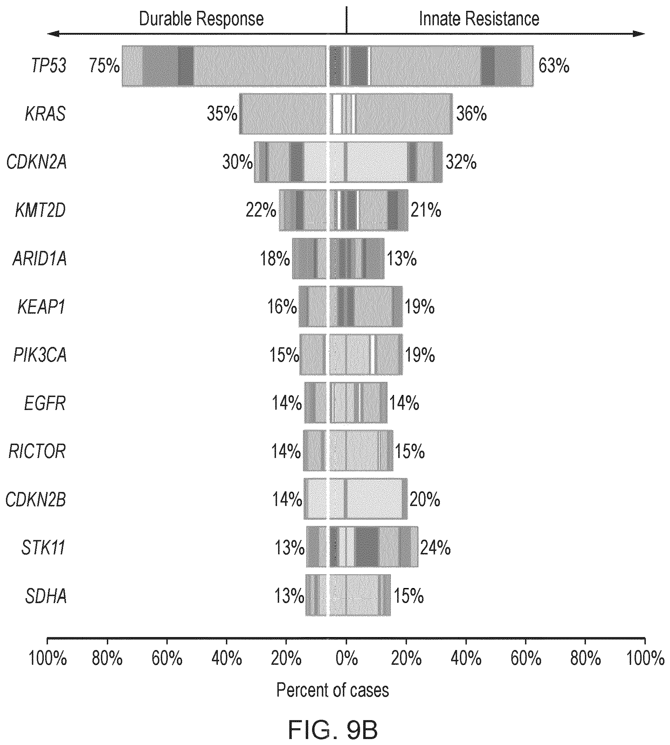

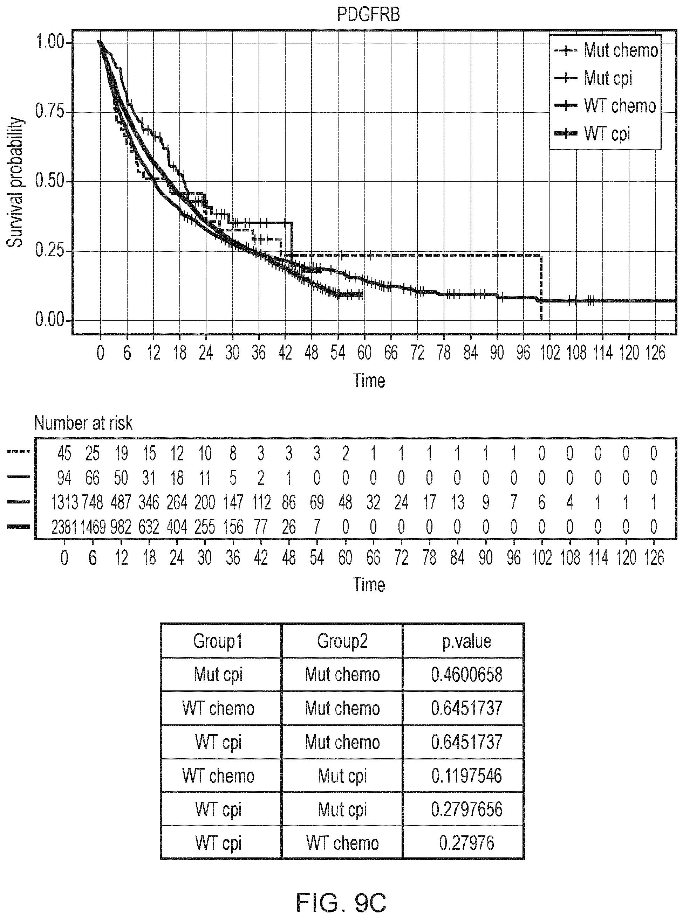

**Figure 9: Mutational landscape of the selected cohort grouped by response, and OS analysis of PDGFRB**
**A.** Oncoplot of the CPI cohort (n=1150) depicting the top 12 altered genes and selected clinical characteristics, segregated by response (depicted as response in lower part of oncoplot). Each column represents a single patient and each row represents a specific gene (name of gene listed on the left side). The top of the figure the histogram represents the number of mutations in each patient with each color representing the type of mutations (as indicated in figure). The middle part represents the oncoplot with different colors in each row represent a specific mutation in a specific patient, and different colors represent different types of mutations (as indicated in the figure), with the right side of the oncoplot showing a bar graph summarizing the number of mutations and the proportion of each type of mutation. Bottom of the figure represents selected clinical characteristics that include response group, histology, smoking status, gender and ancestry call, with color scheme depicted in figure legend. **B.** Stacked bar plot comparing prevalence of top 12 mutations between durable response (left) and innate-resistance (right side). Each color in the stacked bar plot represents a different type of mutations with same color scheme depicted in A. **C.** Kaplan-Meier survival overall survival (OS) curve in patients treated with CPI or Chemo for PDGFRB, details same as in figure 4.

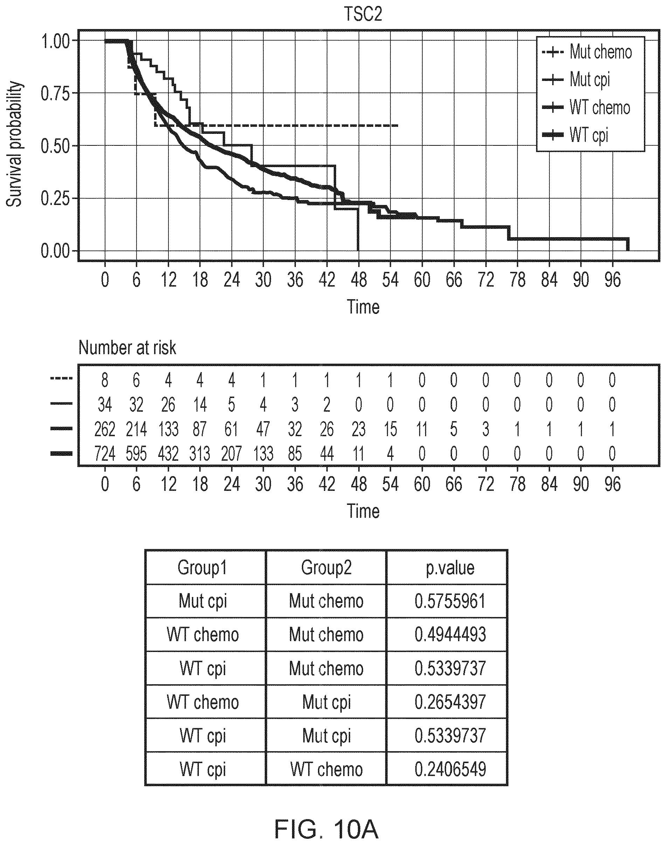

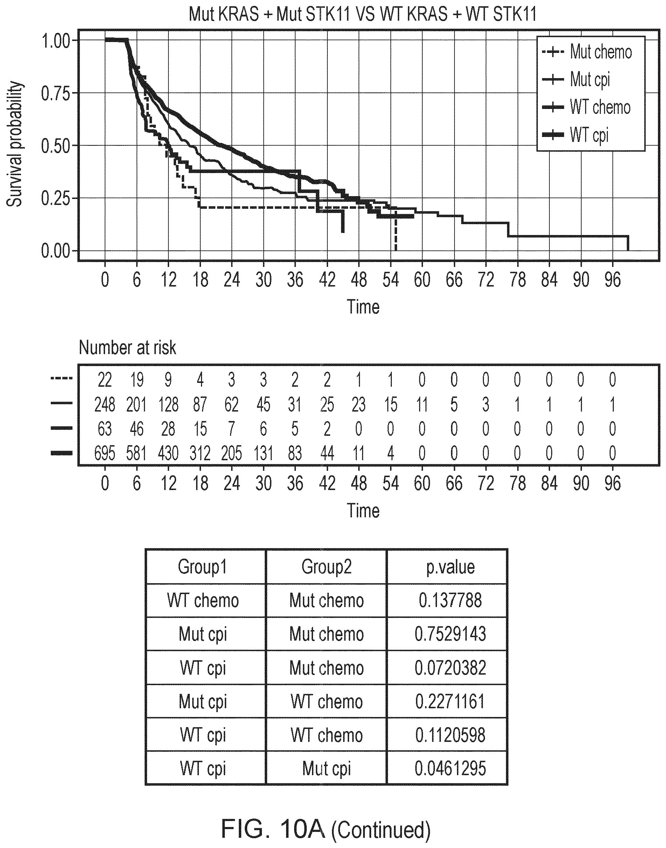

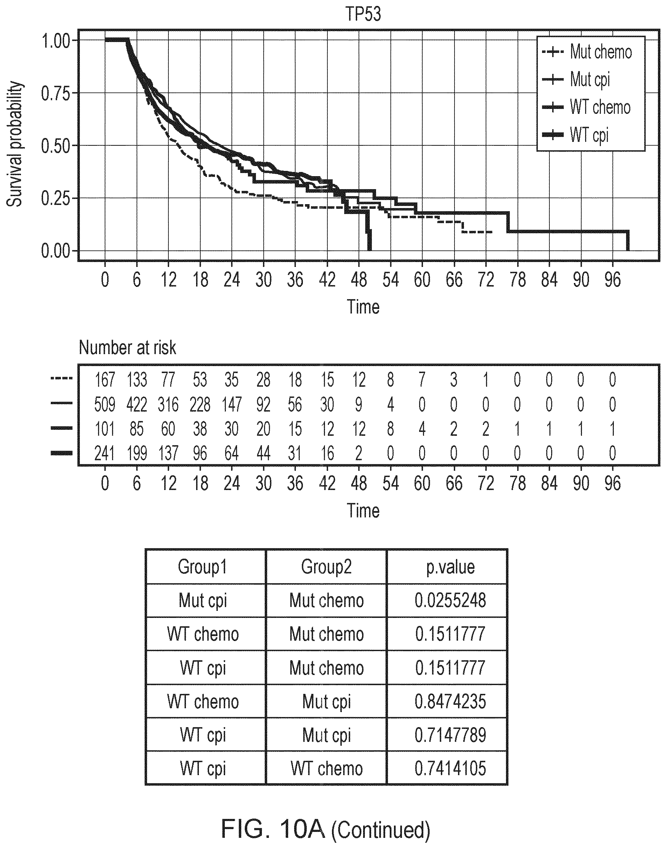

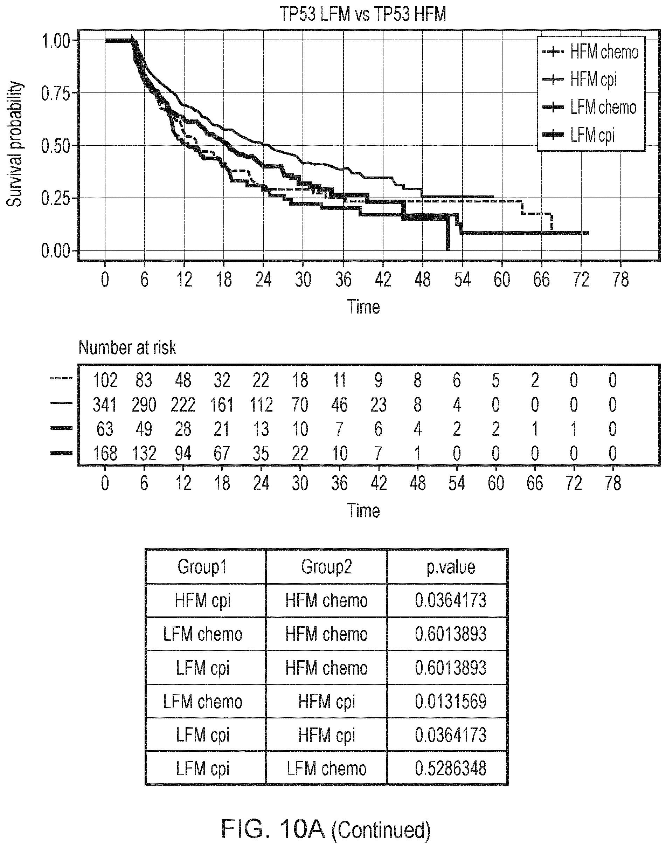

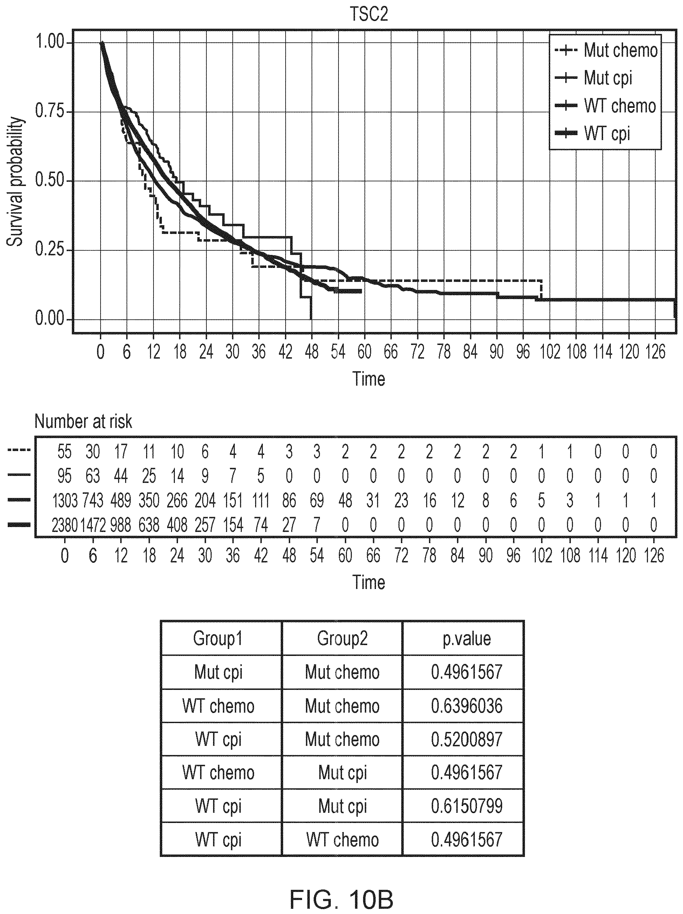

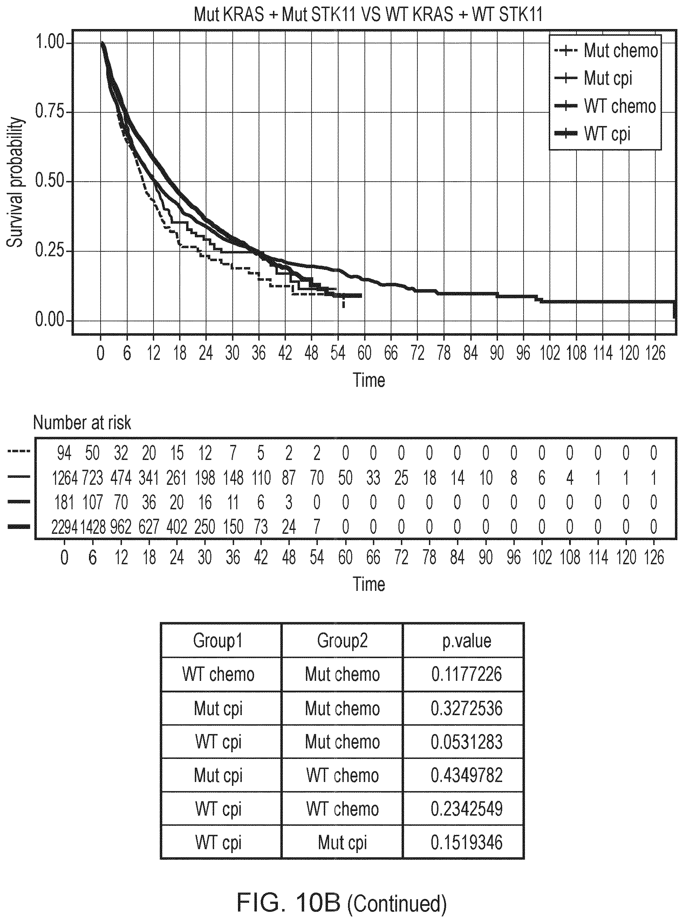

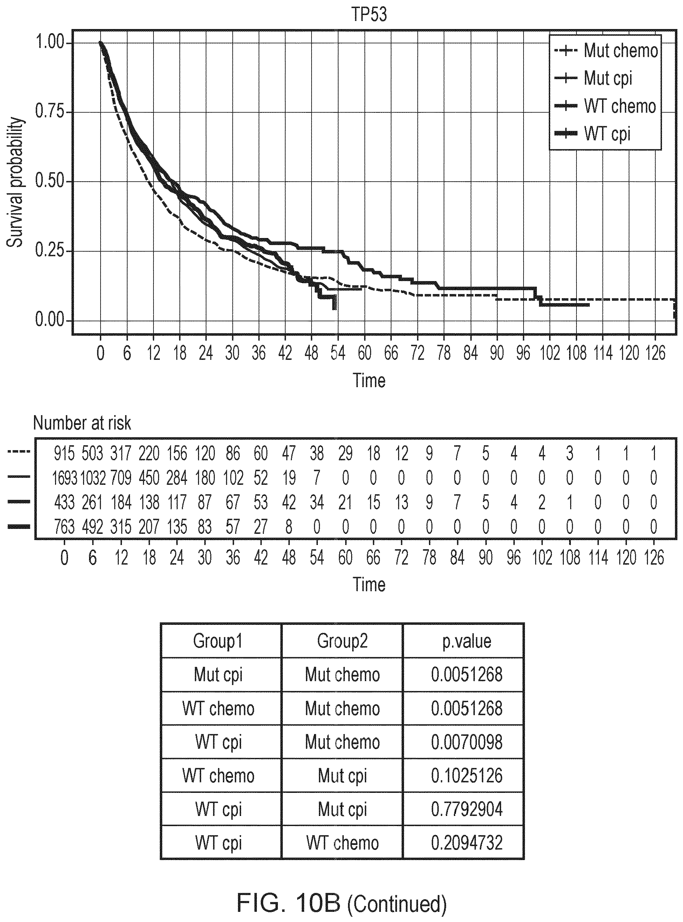

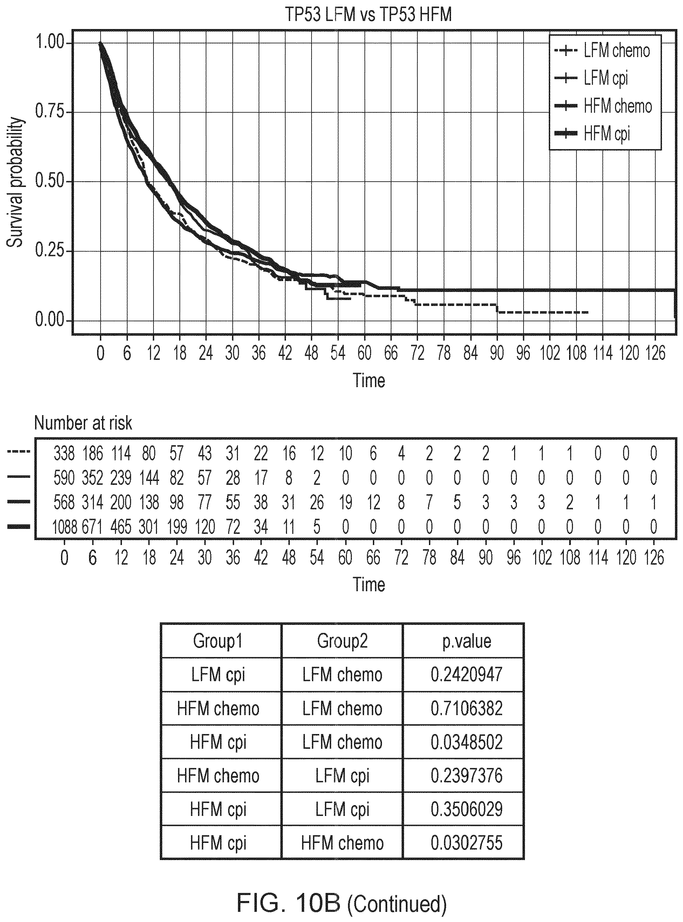

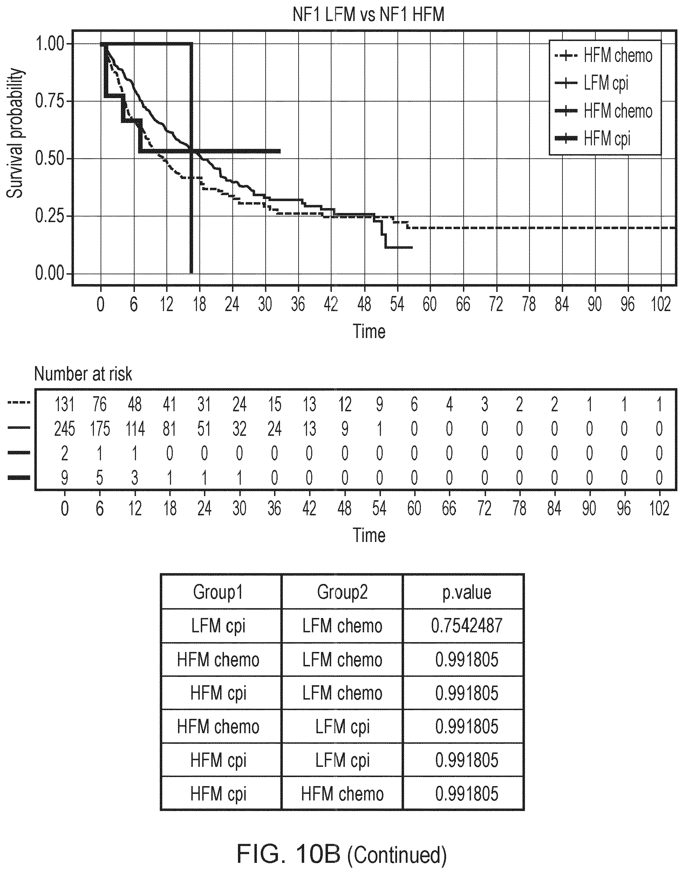

**Figure 10: OS analysis of the extended cohort patients with mutations found to be significantly associated with CPI outcomes**
**A.** Kaplan-Meier survival curves of overall survival (OS) in patients treated with CPI or Chemo with or without mutations in genes found to be significant/marginally-significant in Figure 3. Each Kaplan-Meier plot also shows the number of patients (in the Number at risk table) at each time point (Time in month), and includes a significance table with p-values when comparing each patient group (depicted as Group1 and Group2) at the bottom of each plot. **B.** Same as in **A,** with the exception of using the extended CGDB database of 3362 patients treated with CPI and Chemo.

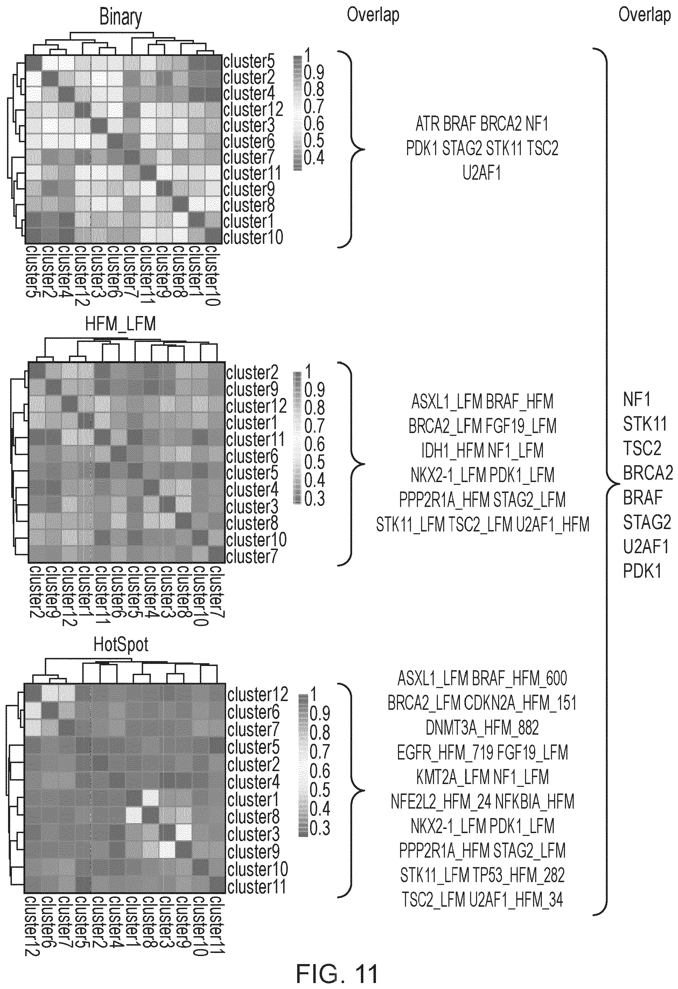

**Figure 11: Uniqueness and diversity of the 36 predictive genetic signatures**
Heatmap representing the Pearson Correlation between the 12 genetic signatures within each of the three input classifications (binary, HFM/LFM and Hotspot granular). To the right of the heatmaps (the "Overlap" middle column) are genes depicted as overlap, between the 12 genetic signatures within each input category. The last column represents the overlap between the 36 genetic signatures across the three input categories.

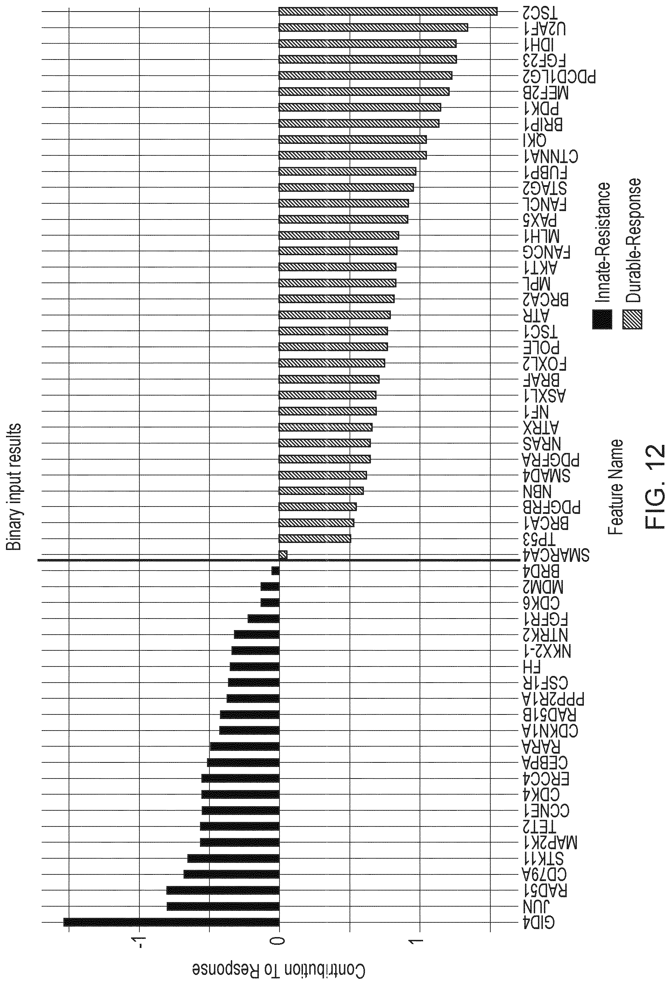

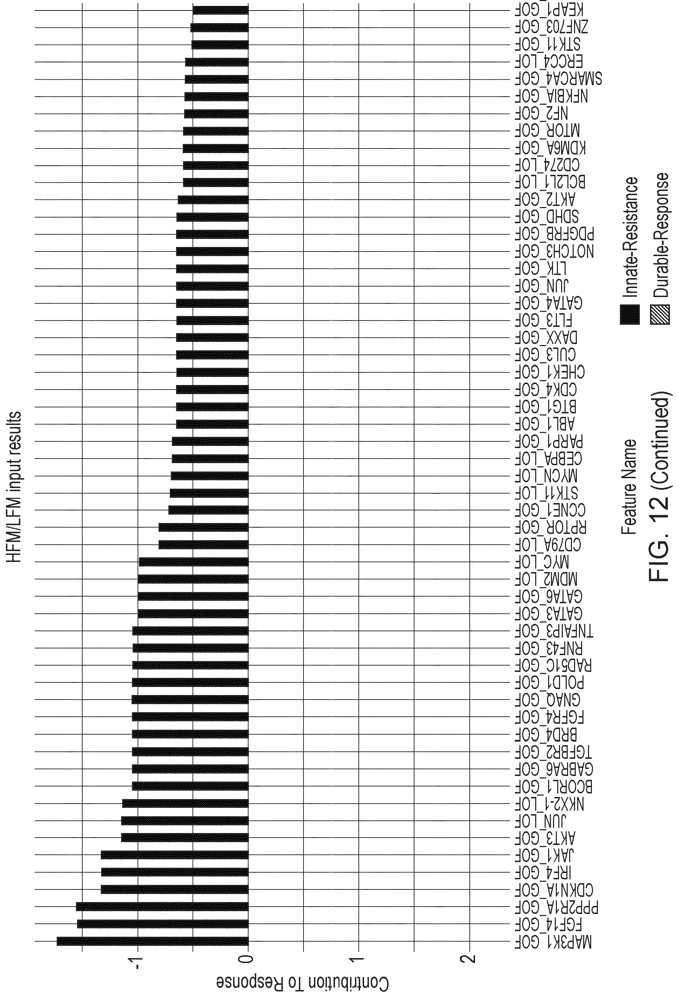

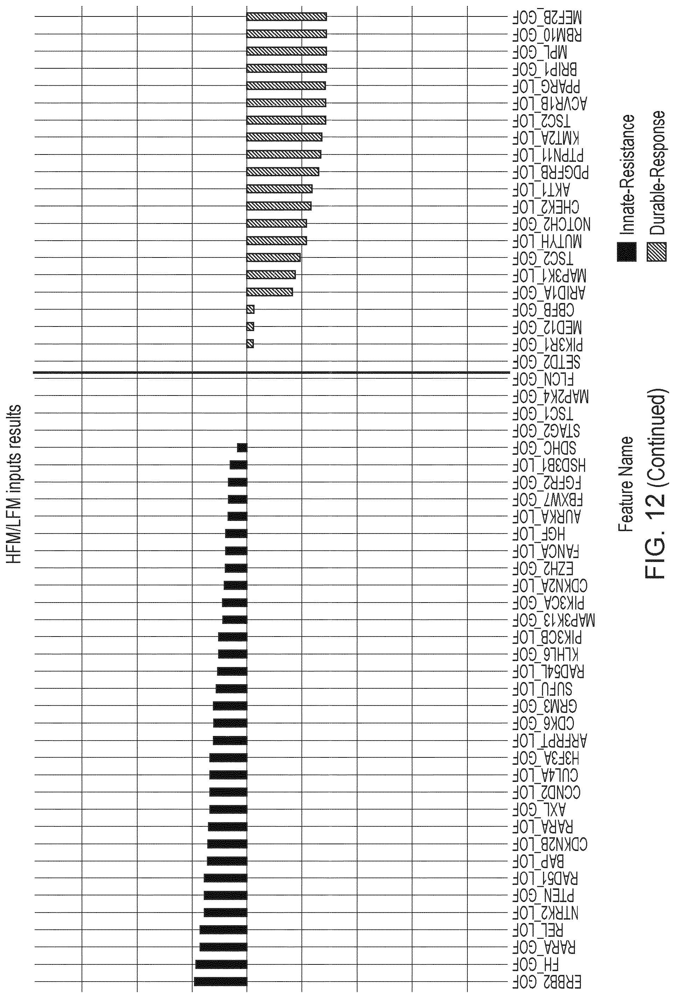

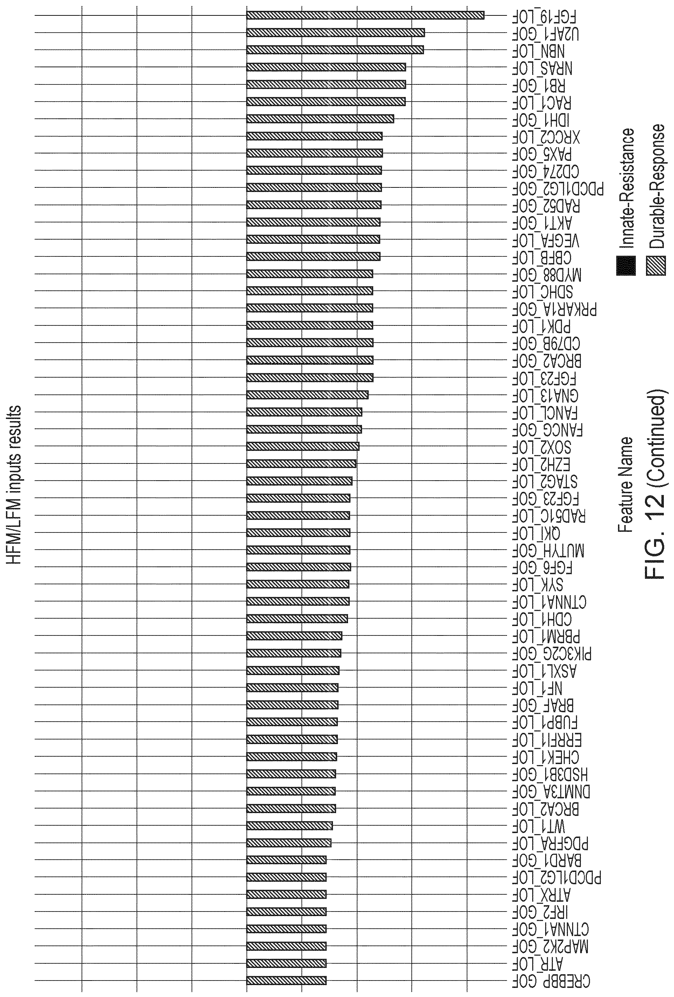

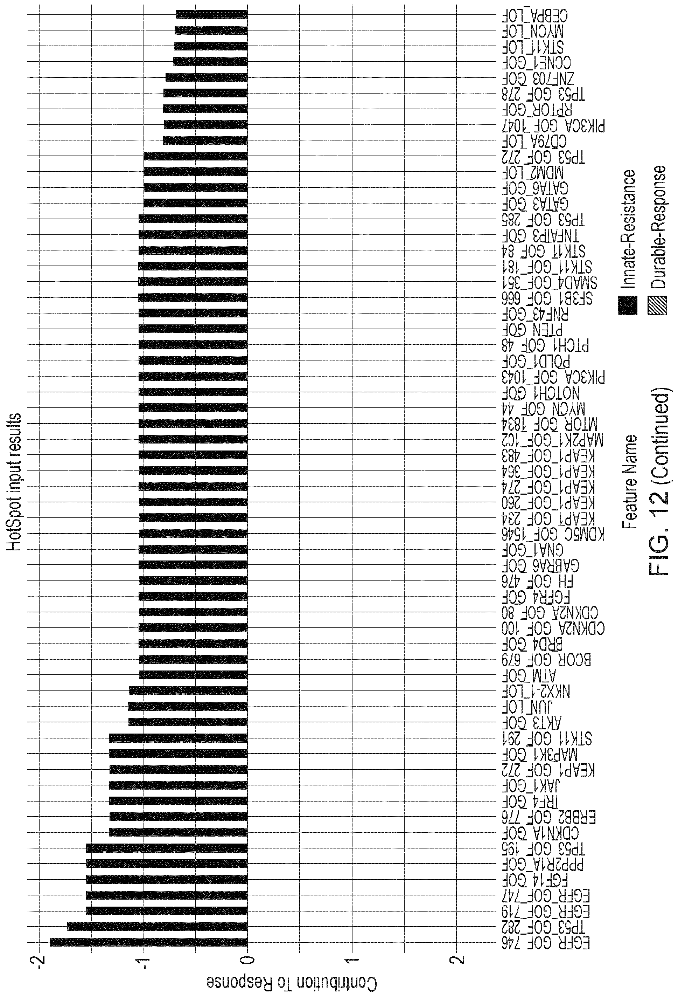

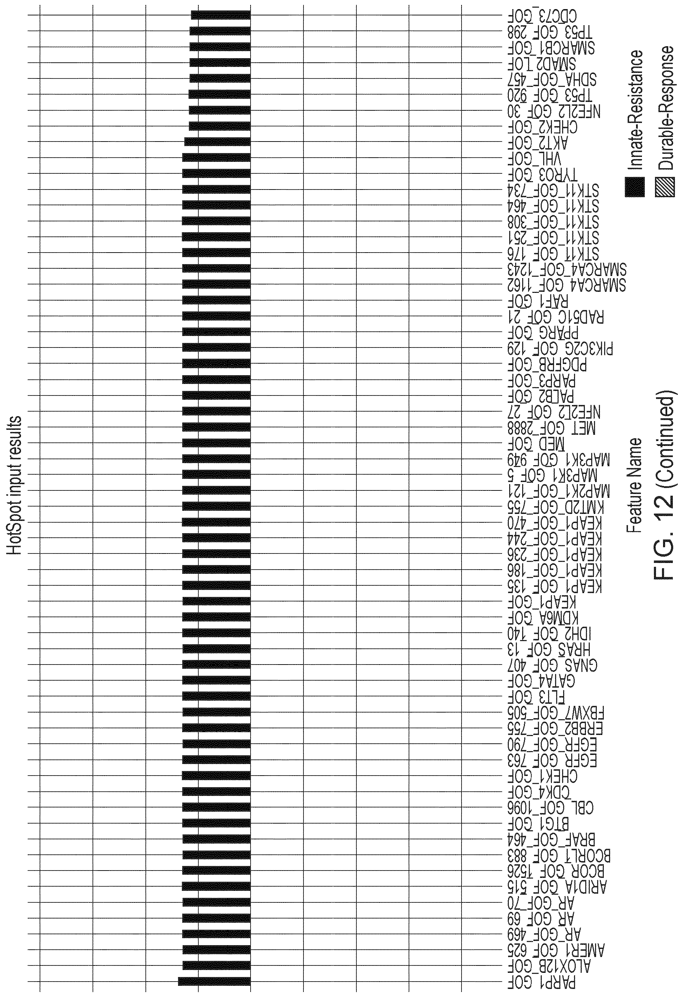

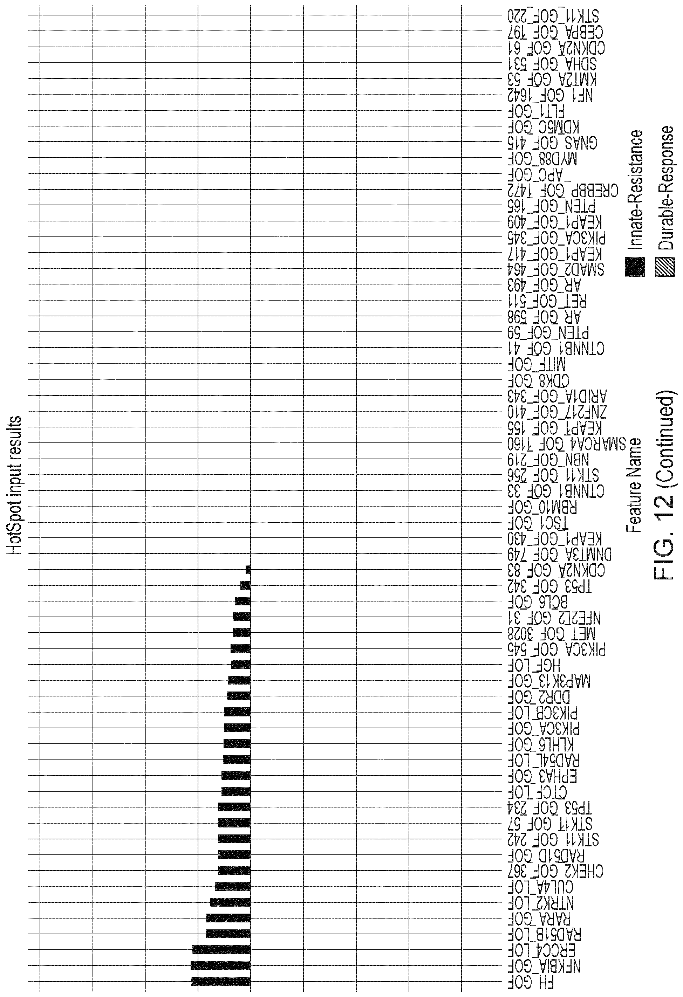

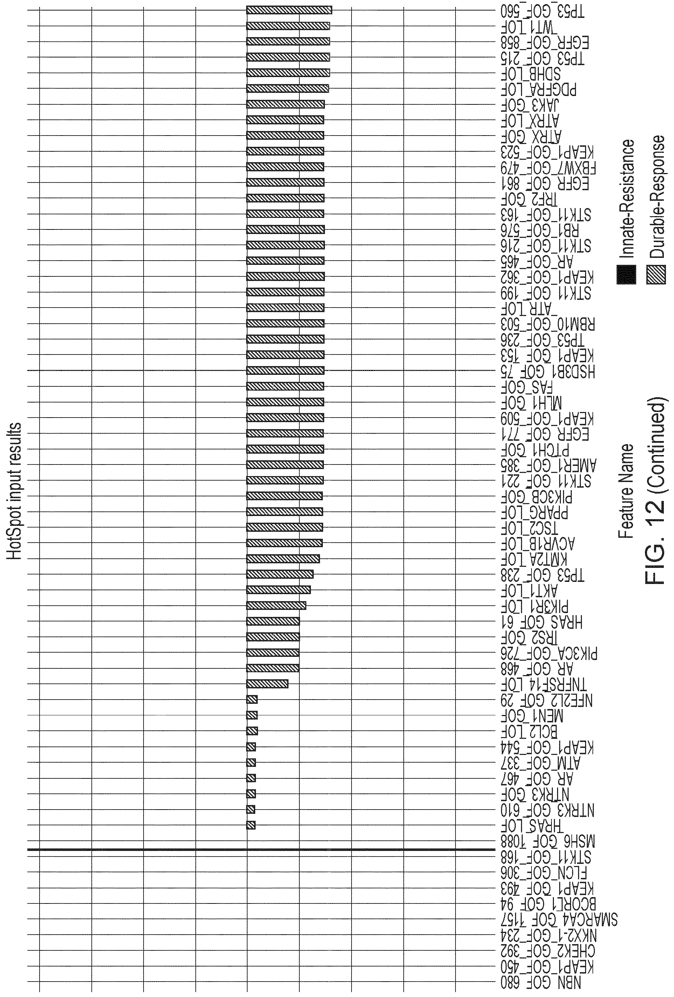

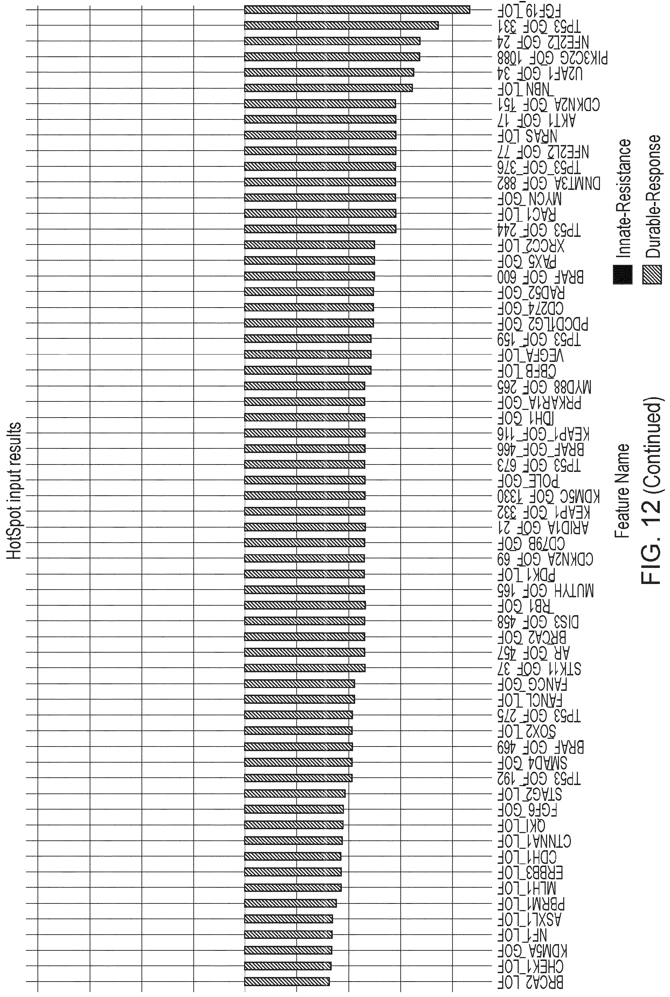

**Figure 12: Linear conversion of 36 predictive models reveals feature importance (full list)**
Waterfall plot of all the linear coefficients (depicted as feature importance) derived from linear conversion of the 36 ML derived genetic signatures sorted by feature importance with positive values indicate association with durable-response (green bars) and negative values indicate association with innate-resistance (red bars). Top panel represents all the unique genes in binary input separated by features contributing to durable response (green) and features contributing to resistance (red). Middle panel represents HFM/LFM feature importance and bottom panel represents feature importance from Hotspot granular input. Black vertical line seperates between the responses.

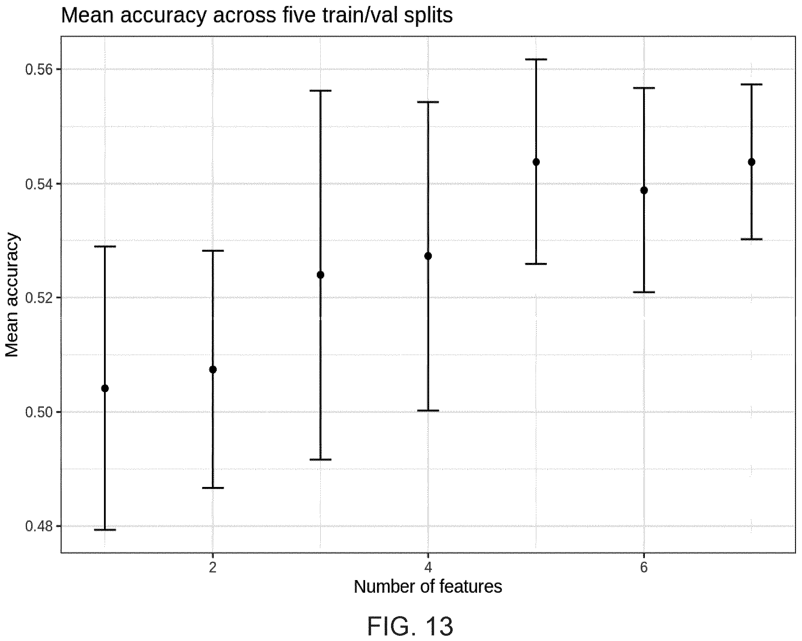

**Figure 13: Mean accuracy across five training/validation splits.**
The plot shows mean accuracy (y-axis) plotted against number of features (x-axis; from 1 feature to 7 features) for results from recursive elimination of features from 8 (which has accuracy of 0.5455 for patients with NSCLC treated with mono CPI and 0.4832 for chemotherapy patients). For the 7-gene feature set, the mean $\pm$ standard deviation of the accuracy for CPI are 0.547 $\pm$ 0.0136; for chemo are 0.491 $\pm$ 0.0186. Error bars indicate the standard deviation of the accuracy from 5 random training/validation data splits. The seven-genes are: NF1, STK11, TSC2, STAG2, U2AF1, BRCA2, PDK1.

### *Detailed Description of the Invention*

Aspects and embodiments of the present invention will now be discussed with reference to the accompanying figures. Further aspects and embodiments will be apparent to those skilled in the art.

A "sample" as used herein may be a cell or tissue sample, a biological fluid, an extract (e.g. a DNA extract obtained from the subject), from which genomic material can be obtained for genomic analysis, such as genomic sequencing (e.g. whole genome sequencing, whole exome sequencing). The sample may be a cell, tissue or biological fluid sample obtained from a subject (e.g. a biopsy). Such samples may be referred to as "subject samples". In particular, the sample may be a blood sample, or a tumour sample, or a sample derived therefrom. The sample may be one which has been freshly obtained from a subject or may be one which has been processed and/or stored prior to genomic analysis (e.g. frozen, fixed or subjected to one or more purification, enrichment or extraction steps). The sample may be a cell or tissue culture sample. As such, a sample as described herein may refer to any type of sample comprising cells or genomic material derived therefrom, whether from a biological sample obtained from a subject, or from a sample obtained from e.g. a cell line. In embodiments, the sample is a sample obtained from a subject, such as a human subject. The sample is preferably from a mammalian (such as e.g. a mammalian cell sample or a sample from a mammalian subject, such as a cat, dog, horse, donkey, sheep, pig, goat, cow, mouse, rat, rabbit or guinea pig), preferably from a human (such as e.g. a human cell sample or a sample from a human subject). Further, the sample may be transported and/or stored, and collection may take place at a location remote from the genomic sequence data acquisition (e.g. sequencing) location, and/or any computer-implemented method steps described herein may take place at a location remote from the sample collection location and/or remote from the genomic data acquisition (e.g. sequencing) location (e.g. the computer-implemented method steps may be performed by means of a networked computer, such as by means of a "cloud" provider).

The subject may have a cancer which comprises a solid tumour (primary and/or metastatic In some cases, the cancer may be a cancer for which CPI therapy has been approved as a treatment option. In particular, the cancer may comprise Advanced Urothelial Carcinoma, Breast Cancer, Colorectal Cancer, Advanced Endometrial Cancer, Gastric Cancer, Hepatocellular Carcinoma, Head and Neck Cancer, Melanoma, Malignant Pleural Mesothelioma, Non-Small Cell Lung Cancer (NSCLC), Renal Cell Carcinoma or Small-Cell Lung Cancer (SCLC). In some cases, the cancer may be a cancer for which CPI therapy has not (yet) been approved as a treatment option. In particular, the cancer may be selected from Acute Myeloid Leukemia, Chronic Lymphocytic Leukemia, Diffuse Large B-Cell Lymphoma, Follicular Lymphoma, Mantle Cell Lymphoma, Multiple Myeloma, Ovarian Cancer, Metastatic Pancreatic Cancer, and Metastatic Prostate Cancer. It is specifically contemplated herein that the cancer may be a lung cancer, such as NSCLC or SCLC.

A "mixed sample" refers to a sample that is assumed to comprise multiple cell types or genetic material derived from multiple cell types. Within the context of the present disclosure, a mixed sample is typically one that comprises tumour cells, or is assumed (expected) to comprise tumour cells, or genetic material derived from tumour cells. Samples obtained from subjects, such as e.g. tumour samples, are typically mixed samples (unless they are subject to one or more purification and/or separation steps). Typically, the sample comprises tumour cells and at least one other cell type (and/or genetic material derived therefrom). For example, the mixed sample may be a tumour sample. A "tumour sample" refers to a sample derived from or obtained from a tumour. Such samples may comprise tumour cells and normal (non-tumour) cells. The normal cells may comprise immune cells (such as e.g. lymphocytes), and/or other normal (non-tumour) cells. The lymphocytes in such mixed samples may be referred to as "tumour-infiltrating lymphocytes" (TIL). A tumour may be a solid tumour or a non-solid or haematological tumour. A tumour sample may be a primary tumour sample, tumour-associated lymph node sample, or a sample from a metastatic site from the subject. A sample comprising tumour cells or genetic material derived from tumour cells may be a bodily fluid sample. Thus, the genetic material derived from tumour cells may be circulating tumour DNA or tumour DNA in exosomes. Instead or in addition to this, the sample may comprise circulating tumour cells. A mixed sample may be a sample of cells, tissue or bodily fluid that has been processed to extract genetic material. Methods for extracting genetic material from biological samples are known in the art. A mixed sample may have been subject to one or more processing steps that may modify the proportion of the multiple cell types or genetic material derived from the multiple cell types in the sample. For example, a mixed sample comprising tumour cells may have been processed to enrich the sample in tumour cells. Thus, a sample of purified tumour cells may be referred to as a "mixed sample" on the basis that small amounts of other types of cells may be present, even if the sample may be assumed, for a particular purpose, to be pure (i.e. to have a tumour fraction of 1 or 100%).

A "normal sample" or "germline sample" refers to a sample that is assumed not to comprise tumour cells or genetic material derived from tumour cells. A germline sample may be a blood sample, a tissue sample, or a purified sample such as a sample of peripheral blood mononuclear cells from a subject. Similarly, the terms "normal", "germline" or "wild type" when referring to sequences or genotypes refer to the sequence / genotype of cells other than tumour cells. A germline sample may comprise a small proportion of tumour cells or genetic material derived therefrom, and may nevertheless be assumed, for practical purposes, not to comprise said cells or genetic material. In other words, all cells or genetic material may be assumed to be normal and/or sequence data that is not compatible with the assumption may be ignored.

The term "sequence data" refers to information that is indicative of the presence and preferably also the amount of genomic material in a sample that has a particular sequence. Such information may be obtained using sequencing technologies, such as e.g. next generation sequencing (NGS), for example whole exome sequencing (WES), whole genome sequencing (WGS), or sequencing of captured genomic loci (targeted or panel sequencing), or using array technologies, such as e.g. copy number variation arrays, or other molecular counting assays. When NGS technologies are used, the sequence data may comprise a count of the number of sequencing reads that have a particular sequence. When non-digital technologies are used such as array technology, the sequence data may comprise a signal (e.g. an intensity value) that is indicative of the number of sequences in the sample that have a particular sequence, for example by comparison to an appropriate control. Sequence data may be mapped to a reference sequence, for example a reference genome, using methods known in the art (such as e.g. Bowtie (Langmead et al., 2009)). Thus, counts of sequencing reads or equivalent non-digital signals may be associated with a particular genomic location (where the "genomic location" refers to a location in the reference genome to which the sequence data was mapped). Further, a genomic location may contain a mutation, in which case counts of sequencing reads or equivalent non-digital signals may be associated with each of the possible variants (also referred to as "alleles") at the particular genomic location. The process of identifying the presence of a mutation at a particular location in a sample is referred to as "variant calling" and can be performed using methods known in the art (such as e.g. the GATK HaplotypeCaller, https://gatk.broadinstitute.org/hc/en-us/articles/360037225632-Hap lotypeCaller). For example, sequence data may comprise a count of the number of reads (or an equivalent non-digital signal) which match a germline (also sometimes referred to as "reference") allele at a particular genomic location, and a count of the number of reads (or an equivalent non-digital signal) which match a mutated (also sometimes referred to as "alternate") allele at the genomic location.

Further, sequence data may be used to infer copy number profiles along a genome, using methods known in the art. Copy number profiles may be allele specific. In the context of the present invention, copy number profiles are preferably allele specific and tumour / normal sample specific. In other words, the copy number profiles used in the present invention are preferably obtained using methods designed to analyse samples comprising a mixture of tumour and normal cells, and to produce allele-specific copy number profiles for the tumour cells and the normal cells in a sample. Allele specific copy number profiles for mixed samples may be obtained from sequence data (e.g. using read counts as described above), using e.g. ASCAT (Van Loo et al., 2010). Other methods are known and equally suitable. Preferably, within the context of the present invention, the method used to obtain allele-specific copy number profiles is one that reports a plurality of possible copy number solutions and an associated quality/confidence metric. For example, ASCAT outputs a goodness-of-fit metric for each combination of values of ploidy (ploidy for a whole tumour sample, not segment-specific) and purity for which a corresponding allele-specific copy number profile was evaluated. Note that the tumour-specific copy number profiles generated by such methods represent an average or summary of the entire tumour cell population (i.e. it does not account for heterogeneity within the tumour population).

The term "total copy number" refers to the total number of copies of a genomic region in a sample. The term "major copy number" refers to the number of copies of the most prevalent allele in a sample. Conversely, the term "minor copy number" refers to the number of copies of the allele other than the most prevalent allele in a sample. Unless indicated otherwise, these terms refer to the inferred major and major copy numbers (and total copy numbers) for an inferred tumour copy number profile. The term "normal copy number" or "normal total copy number" refers to the number of copies of a genomic region in the normal cells in a sample. Normal cells typically have two copies of each chromosome (unless the cell is genetically male and the chromosome is a sex chromosome), and hence the normal copy number may in embodiments be assumed to be equal to 2 (unless the genomic region is on the X or Y chromosome and the sample under analysis is from a male subject, in which case the normal copy number may be assumed to be equal to 1). Alternatively, the normal copy number for a particular genomic region may be determined using a normal sample.

*Methods for classification based on gene mutations*

In some embodiments, the present invention provides methods for classifying, prognosticating, predicting treatment response (e.g. to CPI therapy) or monitoring cancer in subjects. In particular, data obtained from analysis DNA sequencing may be evaluated using one or more pattern recognition algorithms. Such analysis methods may be used to form a predictive model, which can be used to classify test data. For example, one convenient and particularly effective method of classification employs multivariate statistical analysis modelling, first to form a model (a "predictive mathematical model") using data ("modelling data") from samples of known subgroup (e.g., from subjects known to have a particular CPI response), and second to classify an unknown sample (e.g., "test sample") to the appropriate response group.

Pattern recognition methods have been used widely to characterize many different types of problems ranging, for example, over linguistics, fingerprinting, chemistry and psychology. In the context of the methods described herein, pattern recognition is the use of multivariate statistics, both parametric and non-parametric, to analyse data, and hence to classify samples and to predict the value of some dependent variable based on a range of observed measurements. There are two main approaches. One set of methods is termed "unsupervised" and these simply reduce data complexity in a rational way and also produce display plots which can be interpreted by the human eye. However, this type of approach may not be suitable for developing a clinical assay that can be used to classify samples derived from subjects independent of the initial sample population used to train the prediction algorithm.

The other approach is termed "supervised" whereby a training set of samples with known class or outcome is used to produce a mathematical model which is then evaluated with independent validation data sets. Here, a "training set" of mutation data is used to construct a statistical model that predicts correctly the "subgroup" of each sample. This training set is then tested with independent data (referred to as a test or validation set) to determine the robustness of the computer-based model. These models are sometimes termed "expert systems," but may be based on a range of different mathematical procedures such as support vector machine, decision trees, k-nearest neighbour and naïve Bayes. Supervised methods can use a data set with reduced dimensionality (for example, the first few principal components), but typically use unreduced data, with all dimensionality. In all cases the methods allow the quantitative description of the multivariate boundaries that characterize and separate each subtype in terms of its intrinsic mutation profile. It is also possible to obtain confidence limits on any predictions, for example, a level of probability to be placed on the goodness of fit. The robustness of the predictive models can also be checked using cross-validation, by leaving out selected samples from the analysis.

The terms "tumour-specific mutation", "somatic mutation" or simply "mutation" are used interchangeably and refer to a difference in a nucleotide sequence (e.g. DNA or RNA) in a tumour cell compared to a healthy cell from the same subject. A germline mutation, by contrast, occurs in germ cells and is passed on to offspring, such that the mutation is present in essentially all cells of the individual. A germline mutation may be a mutation that predisposes the individual carrying the mutation to developing a cancer (e.g. a mutation in the gene TP53, or the BRCA1 gene or BRCA2 gene). In particular, the germline mutation may be in a gene selected from the group consisting of: BRCA1, BRCA2, ATM, TP53, PTEN, STK11, PALB2, RAD51C, VHL, FANCM, EPHB4, CDH1, BRIP1, CHEK1 and CHEK2. Germline mutations in these genes are among those that make up the Flatiron Health-Foundation Medicine NSCLC clinico-genomic database. Mutations in these genes may have a pathogenic impact on cancer (e.g. predisposing an individual to certain cancers, including lung cancers). Examples of specific germline mutations contemplated herein include [format: Gene name, cds_effect, protein_effect]: BRCA1, 181T>G, C61G; BRCA1, 213-11T>G, splice site 213-11T>G; BRCA1, 189A>T, L63F; BRCA2, 2808_2811delACAA, A938fs*21; BRCA2, 9155G>A, R3052Q; BRCA2, 7976G>A, R2659K; ATM, 7271T>G, V2424G; ATM, 8147T>C, V2716A; ATM, 1564_1565delGA, E522fs*43; TP53, 760A>G, I254V; TP53, 784G>A, G262S; TP53, 974G>T, G325V; PTEN, 107delG, G36fs*18; PTEN, 562_563insT, Y188fs*2; PTEN, 741A>C, L247F; STK11, 298C>G, Q100E; STK11, 971C>T, P324L; STK11, 551_552TC>CT, L184P; PALB2, 2507_2509delTCG, V836del; PALB2, 1637T>C, V546A; RAD51C, 1026+5_1026+7delGTA, splice site 1026+5_1026+7delGTA; RAD51C, 404G>C, C135S; VHL, 241C>T, P81S; VHL, 376G>A, D126N; VHL, 628C>T, R210W; FANCM, 1972C>T, R658*; FANCM, 3827C>T, S1276L; FANCM, 5014G>A, D1672N; EPHB4, 1258G>A, V420I; EPHB4, 118G>A, G40R; EPHB4, 1759A>G, T587A; CDH1, 871G>A, D291N; CDH1, 1136C>T, T379M; CDH1, 846G>A, M282I; BRIP1, 799G>A, A267T; BRIP1, 1261G>C, E421Q; BRIP1, 1316G>A, R439Q; CHEK1, 703G>A, D235N; and CHEK2, 349A>G, R117G.

As a result of a somatic mutation, the difference in the nucleotide sequence can result in the expression of a protein which is not expressed by a healthy cell from the same subject. For example, a mutation may be a single nucleotide variant (SNV), multiple nucleotide variant (MNV), a deletion mutation, an insertion mutation, a translocation, a missense mutation, a translocation, a fusion, a splice site mutation, or any other change in the genetic material of a tumour cell. A mutation may result in the expression of a protein or peptide that is not present in a healthy cell from the same subject. Mutations may be identified by exome sequencing, RNA-sequencing, whole genome sequencing and/or targeted gene panel sequencing and or routine Sanger sequencing of single genes, followed by sequence alignment and comparing the DNA and/or RNA sequence from a tumour sample to DNA and/or RNA from a reference sample or reference sequence (e.g. the germline DNA and/or RNA sequence, or a reference sequence from a database). Suitable methods are known in the art.

As used herein a "gain of function" or "GOF" mutation may be a high frequency mutation (HFM) as defined herein. Therefore, GOF and HFM may be used interchangeably. A "loss of function" or "LOF" mutation may be a low frequency mutation (LFM) as defined herein. Therefore, LOF and LFM may be used interchangeably. In particular, HFM and LFM (and GOF/LOF, accordingly) may be defined according to the following classification scheme: 1. the total number of amino acids mutated per gene was calculated; 2. the frequency of mutations in each gene was calculated (i.e., how many patients had any mutation in that gene). 3. From #1 and #2 the average amino acid mutation rate was calculated:

$$Average\ amino\ acid\ mutation = \frac{(gene\ level\ mutation\ frequency\ (\#2))}{(Total\ amino\ acids\ mutated\ in\ the\ gene\ (\#1))}$$

4. The HFM label was assigned to any mutation that had 2x the average mutations per that specific amino acid and had more than/equal to 9 mutations in that gene. The LFM label was assigned to any mutation that had lower than 2x the average mutations per that specific amino acid and/or had less than 9 mutations. Any mutation in the TERT promoter was classified as HFM. Amplifications were considered as HFM and deletions as LFM. The rationale behind this was that LFM tend to be loss of function (LOF) and HFM tend to be gain of function (GOF).

The Hotspot granular classification as used herein employs the same definition as described above for HFM/LFM, but adds the amino acid mutation location to any HFM. For example, TP53_178, refers to a HFM in TP53 located at amino acid 178, wherein the amino acid position number refers to the encoded protein sequence. Any HFM that lacks the information about amino acid location is defined as an amplification mutation. The patient population in which the determinations of high frequency or low frequency, as set out above, may be a population such as the approximately 10,000 non-small cell lung cancer patients from the Flatiron Health-Foundation Medicine NSCLC de-identified clinico-genomics database (JAMA 2019;321(14):1391-1399. doi:10.1001/jama.2019.3241), TCGA datasets (https://www.cancer.gov/about-nci/organization/ccg/research/structural-genomics/tcga) and/or from an internal clinic-genomics database. In particular, the patient population may be that described in Singal G, Miller PG, Agarwala V, et al. Association of Patient Characteristics and Tumor Genomics With Clinical Outcomes Among Patients With Non-Small Cell Lung Cancer Using a Clinicogenomic Database (CGDB). JAMA. 2019;321(14):1391-1399. doi:10.1001/jama.2019.3241 (the entire contents of which is expressly incorporated herein by reference, including the de-identified CGDB).

An "indel mutation" refers to an insertion and/or deletion of bases in a nucleotide sequence (e.g. DNA or RNA) of an organism. Typically, the indel mutation occurs in the DNA, preferably the genomic DNA, of an organism. An indel mutation may be a frameshift indel mutation. A frameshift indel mutation is a change in the reading frame of the nucleotide sequence caused by an insertion or deletion of one or more nucleotides. Such frameshift indel mutations may generate a novel open-reading frame which is typically highly distinct from the polypeptide encoded by the non-mutated DNA/RNA in a corresponding healthy cell in the subject.

A "neoantigen" (or "neo-antigen") is an antigen that arises as a consequence of a mutation within a cancer cell. Thus, a neoantigen is not expressed (or expressed at a significantly lower level) by normal (i.e. non-tumour) cells. A neoantigen may be processed to generate distinct peptides which can be recognised by T cells when presented in the context of MHC molecules. Neoantigens may be used as the basis for cancer immunotherapies. References herein to "neoantigens" are intended to include also peptides derived from neoantigens. The term "neoantigen" as used herein is intended to encompass any part of a neoantigen that is immunogenic. An "antigenic" molecule as referred to herein is a molecule which itself, or a part thereof, is capable of stimulating an immune response, when presented to the immune system or immune cells in an appropriate manner. The binding of a neoantigen to a particular MHC molecule (encoded by a particular HLA allele) may be predicted using methods which are known in the art. Examples of methods for predicting MHC binding include those described by Lundegaard et al., O'Donnel et al., and Bullik-Sullivan et al. For example, MHC binding of neoantigens may be predicted using the netMHC-3 (Lundegaard et al.) and netMHCpan4 (Jurtz et al.) algorithms. A neoantigen that has been predicted to bind to a particular MHC molecule is thereby predicted to be presented by said MHC molecule on the cell surface.

A cancer immunotherapy (or simply "immunotherapy") refers to a therapeutic approach comprising administration of an immunogenic composition (e.g. a vaccine), a composition comprising immune cells, or an immunoactive drug, such as e.g. a therapeutic antibody, to a subject. The term "immunotherapy" may also refer to the therapeutic compositions themselves. In the context of the present invention, the immunotherapy typically targets a neoantigen. For example, an immunogenic composition or vaccine may comprise a neoantigen, neoantigen presenting cell or material necessary for the expression of the neoantigen. As another example, a composition comprising immune cells may comprise T and/or B cells that recognise a neoantigen. The immune cells may be isolated from tumours or other tissues (including but not limited to lymph node, blood or ascites), expanded ex vivo or in vitro and re-administered to a subject (a process referred to as "adoptive cell therapy"). Instead or in addition to this, T cells can be isolated from a subject and engineered to target a neoantigen (e.g. by insertion of a chimeric antigen receptor that binds to the neoantigen) and re-administered to the subject. As another example, a therapeutic antibody may be an antibody which recognises a neoantigen.

A composition as described herein may be a pharmaceutical composition which additionally comprises a pharmaceutically acceptable carrier, diluent or excipient. The pharmaceutical composition may optionally comprise one or more further pharmaceutically active polypeptides and/or compounds. Such a formulation may, for example, be in a form suitable for intravenous infusion.

References to "an immune cell" are intended to encompass cells of the immune system, for example T cells, NK cells, NKT cells, B cells and dendritic cells. In a preferred embodiment, the immune cell is a T cell. An immune cell that recognises a neoantigen may be an engineered T cell. A neoantigen specific T cell may express a chimeric antigen receptor (CAR) or a T cell receptor (TCR) which specifically binds a neoantigen or a neoantigen peptide, or an affinity-enhanced T cell receptor (TCR) which specifically binds a neoantigen or a neoantigen peptide (as discussed further hereinbelow). For example, the T cell may express a chimeric antigen receptor (CAR) or a T cell receptor (TCR) which specifically binds to a neo-antigen or a neo-antigen peptide (for example an affinity enhanced T cell receptor (TCR) which specifically binds to a neo-antigen or a neo-antigen peptide). Alternatively, a population of immune cells that recognise a neoantigen may be a population of T cell isolated from a subject with a tumour. For example, the T cell population may be generated from T cells in a sample isolated from the subject, such as e.g. a tumour sample, a peripheral blood sample or a sample from other tissues of the subject. The T cell population may be generated from a sample from the tumour in which the neoantigen is identified. In other words, the T cell population may be isolated from a sample derived from the tumour of a patient to be treated, where the neoantigen was also identified from a sample from said tumour. The T cell population may comprise tumour infiltrating lymphocytes (TIL).

The term "Antibody" (Ab) includes monoclonal antibodies, polyclonal antibodies, multispecific antibodies (e.g., bispecific antibodies), and antibody fragments that exhibit the desired biological activity. The term "immunoglobulin" (Ig) may be used interchangeably with "antibody". Once a suitable neoantigen has been identified, for example by a method according to the invention, methods known in the art can be used to generate an antibody.

An "immunogenic composition" is a composition that is capable of inducing an immune response in a subject. The term is used interchangeably with the term "vaccine". The immunogenic composition or vaccine described herein may lead to generation of an immune response in the subject. An "immune response" which may be generated may be humoral and/or cell-mediated immunity, for example the stimulation of antibody production, or the stimulation of cytotoxic or killer cells, which may recognise and destroy (or otherwise eliminate) cells expressing antigens corresponding to the antigens in the vaccine on their surface.

As used herein "treatment" refers to reducing, alleviating or eliminating one or more symptoms of the disease which is being treated, relative to the symptoms prior to treatment. "Prevention" (or prophylaxis) refers to delaying or preventing the onset of the symptoms of the disease. Prevention may be absolute (such that no disease occurs) or may be effective only in some individuals or for a limited amount of time.

As used herein, the terms "computer system" includes the hardware, software and data storage devices for embodying a system or carrying out a method according to the above described embodiments. For example, a computer system may comprise a central processing unit (CPU), input means, output means and data storage, which may be embodied as one or more connected computing devices. Preferably the computer system has a display or comprises a computing device that has a display to provide a visual output display (for example in the design of the business process). The data storage may comprise RAM, disk drives or other computer readable media. The computer system may include a plurality of computing devices connected by a network and able to communicate with each other over that network. It is explicitly envisaged that computer system may consist of or comprise a cloud computer.

As used herein, the term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

Accordingly, the exemplary embodiments of the invention set forth above are considered to be illustrative and not limiting.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps. It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### *Examples*

### <u>METHODS</u>

### Data Sources

The patent data set was obtained from the Flatiron Health de-identified Clinico-Genomic Database (CGDB) as available on January 1, 2020 and which is described in Singal G, Miller PG, Agarwala V, et al. Association of Patient Characteristics and Tumor Genomics With Clinical Outcomes Among Patients With Non-Small Cell Lung Cancer Using a Clinicogenomic Database (CGDB). JAMA. 2019;321(14):1391-1399. doi:10.1001/jama.2019.3241. In particular, the de-identified Flatiron Health-Foundation Medicine NSCLC clinico-genomic database (FH-FMI CGDB). Patient treatment data between January 2011 and December 2019 (data collection cut-off date) were used for the analyses that follow.

### Defining Patient Treatment Outcome Groups

### *Research Design and Patient Cohorts*

To better distinguish predictive (specific to CPI) from prognostic effects (independent of which treatment), we analysed standard-of-care chemotherapy cohorts. To better delineate the two effects, we also removed patients with early deaths (patients who died in the first 18 weeks after treatment start). This is because it has been reported that the CPI and chemotherapy survival curves did not differentiate until after about 18 weeks (Journal of Thoracic Oncology. 2018;13:1156-1170)

Patients were categorized into two main outcome groups: durable-response and innate-resistance groups (Figure 1B).

We used real-world progression (rwP; progressed or not on certain date) (Advances in Therapy, 2019;36(8):2122-2136) and real-world response (rwR; CR, PR, SD or PD on certain date) (Advances in Therapy, 2021;38:1843-1859) for defining the response groups.

A patient was considered to have "durable-response" if there was tumor response and no disease progression starting from 14 days after CPI treatment start to the end of the study duration. A patient was considered to have "innate-resistance" if there was disease progression without any tumor response during the study duration. To study the clinical benefit of CPI therapy and to have a more balanced number of patients in each response group, CR, PR and SD were considered as having tumor response from the rwR data. Having disease progression included rwP, death or a change to a non-CPI treatment line within the study duration. For study duration determination, sensitivity analysis using study durations ranging from 120 to 365 days, in ~2-3-month increments, were performed. Study duration of 270 days resulted in an optimal balance in patient number in each response groups and is a clinically relevant duration. Disease progression within the first 14 days after CPI treatment was ignored, since it might not reflect the effect of the current treatment (recommendation by Flatiron Health).

### Treatment data

Checkpoint inhibitor (CPI) analysis included monotherapies nivolumab, pembrolizumab, atezolizumab, durvalumab and avelumab. Chemotherapy patients from FH-FMI databases included patients with all the drugs annotated by Flatiron Health as "chemotherapy" who did not have "immunotherapy" in the patient's record in the database. For patients who had multiple lines of CPI or chemotherapy, their first CPI or chemotherapy records were used for analysis.

**TMB**

Tumor mutation burden (TMB, number of mutations per Mb) calculated from targeted DNA sequencing using FoundationOne panel with solid baitsets (JAMA (2019) doi:10.1001/jama.2019.3241) on tumor biopsies from all analysed patients was provided by FMI (Foundation Medicine Inc). TMB data from the most recent specimens collected before treatment start was used. Research Use Only (RUO) calculations based on FMI's research algorithm used at the time of collection were analysed (Genome Med. (2017) doi:10.1186/s13073-017-0424-2.).

**Data preparation**

After associating a CPI resistance label to each patient, the mutations for each mono-CPI patient are filtered to remove synonymous mutations and then aggregated into a categorization: per-gene, as gain or loss of function per-gene, and as hotspots. There are 427 innate resistance patients and 372 durable response patients, with 288 mutations present when aggregated per-gene, 460 mutations present when aggregated as loss or gain of function per-gene, and 528 mutations when aggregated as hotspots. The input dataset is randomly split into training and test subsets, stratified by CPI resistance label, leaving 977 training patients and 173 test patients.

**Pathway analysis using CBDD**

For pathway analysis the predictive genes were used as input to six different network-based algorithms implemented in CBDD R package, that utilizes the Metabase network and pathway data. The algorithms used were network propagation, interconnectivity, overconnectivity, hidden nodes, gene mania and causal reasoning. The top 100 nodes resulting from each algorithm were then used to run a pathway enrichment analysis on the Metabase pathways.

**Cohort balancing; Baseline and lab value bias evaluation**

In order to assess potential bias in the absence of explicit patient re-weighting, the number of correctly predicted patients in the test set categorized by baseline and lab values were compared with a Fisher's Exact Test[3]. For lab values, the median of the most recent 3 lab tests predating treatment within 1 year and up to 4 weeks after treatment start was used. Patients were divided into tertiles by the lab value measurements and the Fisher's test was applied comparing each pair of tertiles. After applying a False Discovery Rate correction[4] no statistically significant bias in any of the baseline or lab value covariates was found ($P < 0.05$), indicating that none of the models predict baseline or lab value quantities potentially correlated with CPI resistance rather than CPI resistance itself.

**Categorizing mutations using Binary, HFM/LFM and Hotspot granular categories**

In Binary classification, we consider any mutation within a particular gene as mutated (synonymous mutations were filtered) and genes without any mutations are considered WT. In HFM/LFM classification the following was flow was used to define the HFM and LFM categories: 1. We calculated the total number of amino acids mutated per gene 2. We calculated the frequency of mutations in each gene (I.e., how many patients had any mutation in that gene). 3. From #1 and #2 we calculated the average amino acid mutation rate (average amino acid mutation = gene level mutation frequency (#2) / Total amino acids mutated in the gene (#1). 4. HFM were assigned to any mutation that had 2x the average mutations per that specific amino acid and had more than/equal to 9 mutations in that gene. LFM was assigned to any mutation that had lower than 2x the average mutations per that specific amino acid and or had less than 9 mutations. Any mutation in the TERT promoter was classified as HFM. Amplifications were considered as HFM and deletions as LFM, the rationale behind this was that LFM tend to be loss of function and high Frequency mutations tend to be gain of function. In Hotspot granular classification, same as in HFM/LFM, but adding the amino acid mutation location to any HFM ( TP53_178, meaning that HFM in TP53 in amino acid 178.

***EXAMPLE 1 - GENETIC ALGORITHM FEATURE SELECTION***

Genetic Algorithms (GA) can be adapted for use as a feature selection technique[5,6] .In this study, we define a GA individual as a subset of the available input features of the dataset, represented as a binary mask over all features. The fitness of each individual is calculated based on the predictive power of a model which uses only the features contained in the subset and is trained to predict the binary CPI resistance category, 'dura-response' or 'inn-resistance'.

Naive Bayes models with a Bernoulli prior are used. Naive Bayes models were chosen for the simplicity of their internal state, resistance to over-training, and interpretability. Random Forest and other ensemble based methods were attempted but found to require heavy hyperparameter tuning to avoid over-training during the genetic algorithm search. The Bernoulli prior is appropriate for binary input data and includes a penalty term for the feature not appearing, differentiating it from a multinomial prior.

For each GA individual, the fitness is the cross-validation (CV) accuracy score of a Naive Bayes model on the training set. The accuracy score is class-balanced to avoid favoring 'dura-response' over 'inn-resistance' or vice versa. The cross-validation uses stratification to keep the same fraction of 'dura-response' and 'inn-resistance' patients in each fold. The number of folds used was 5, a compromise to keep the number of patients in each fold high while keeping the number of folds high enough to be confident in the result. The training data is shuffled for each individual before cross validation to avoid overfitting on CV folds during GA optimization.

In each generation of the GA, individuals are selected for mutation or crossover using fitness proportionate selection[7], which samples individuals probabilistically based on their fitness in order to maintain diversity in the GA breeding pool. The breeding pool of each generation is supplemented by a set of the highest fitness individuals from all previous generations. Mutation occurs by randomly removing or adding features to the subset while conserving the average number of features. The average number of features are conserved during mutation by partitioning the probability of mutation between adding features and removing features in order to retain (on average) the same number of features removed and added. Without this partition, mutation would tend to increase the size of models with less than 50% of the total features used and decrease the size of models with more than 50% of the features used regardless of the fitness of the result. Crossover occurs by randomly selecting each feature flag of the binary mask from two previous individuals and does not include further correction: crossover on average will produce offspring with the number of features halfway between each parent.

The GA procedure was run with the following parameters set. The GA is run for 200 generations each with a population of 1000 individuals. Larger populations and larger numbers of generations were not found to produce different results, as the GA was able to find an optima within this time. The first generation is generated randomly such that on average 10% of the features are included in each individual. This fraction was chosen to correspond roughly to the number of features at the end of the GA. During mutation, on average 1% of features are removed or added to an individual. The top 200 individuals over all generations are added to the breeding pool for each generation for a total breeding pool size of 1200 in each generation after the first. To generate each successive generation, 600 individuals are formed by mutating an individual from the breeding pool while the remaining 400 of each generation are formed by crossover of two previous individuals, a relative fraction chosen to slightly favor mutation in order to increase diversity in the population.

The GA is run 10 times for each mutational input categorization (binary per-gene, gain or loss of function, hotspot). Since each run of the GA tends to find separate local optima, these 10 runs along with the clustering technique described below are used to identify multiple local optima that are too distant for a single GA run to identify.

After 10 runs of 200 generations with a population size of 1000 there are 2,000,000 GA individuals which are available. The top 5% (100,000) of all individuals, based on their CV score, are selected and then clustered according to the similarity of the features they contain. The clustering is done using a simple KMeans clustering with 12 clusters to account for the expected 10 separate local optima (one from each run of the GA) plus some leeway for outliers.

In each cluster, the set of features which appear in more than 50% of cluster members is considered the characteristic set of features for that cluster. A final model is then trained on each of the 12 characteristic sets and evaluated on the test set.

**Feature Importance Calculation**

In order to visualize the importance of individual mutations on the prediction outcomes, the internal state of the 36 Bernoulli Naive Bayes models were converted into their equivalent linear (logistic regression) coefficients[8]. This conversion for each gene mutation feature is outlined below.

$$coef(resp) = \ln\left(\frac{P(mut|resp)}{1 - P(mut|resp)}\right)$$

$$coef(resist) = \ln\left(\frac{P(mut|resist)}{1 - P(mut|resist)}\right)$$

$$coef = coef(resp) - coef(resist)$$

Where "P(mut|resp)" is the probability of mutation for the response group, "P(mut|resist)" is the probability of mutation for the resistance group. "Coef(resp)" is the linear coefficient for the feature in the response group, while "coef(resist)" is the linear coefficient for the feature in the resistance group. "coef" is the final linear coefficient of the feature. "coef" > 0 indicates association of the feature with response while "coef" < 0 indicates association with resistance. The sum of coefficients of all the features determines the prediction of response or resistance for a patient.

*When $\Sigma_{mut}coef > threshold$,* response is predicted over resistance.

Where threshold is the intercept of the model, which is a trained parameter of the model in addition to the coefficients.

Notice that the value for each mutation is independent of all other mutations, therefore they are a feature of the training set itself. Bernoulli Naive Bayes models incorporate indirect mutation-mutation interactions through the model intercept/threshold (not derived here).

## Mutation Co-Occurrence Analysis

In order to assess potential relationships between co-occurrence of mutation-pairs / triples and CPI resistance, a series of Fisher's exact tests were performed, unless in the 2x2 contingency table there were zero count(s), then Mantel-Haenszel method with continuity correction of 0.5 was used. Multiple testing adjustment of p-values used False Discovery Rate method. The contingency table structure for the number of patients with mutation pairs / triples is as below:

|  | 'dura-response' | 'inn-resistance' |
|---|---|---|
| All gene mutation features mutated |  |  |
| Not all mutated (any or none mutated) |  |  |

Using this corrected block diagram ensures that a significant single-mutation effect does not imply a significant mutational-pair effect and that each test is uncorrelated with each other and FDR corrections are appropriate. An additional requirement that each row/column sum of the block diagram must be at least 5 was applied to remove very rare mutational combinations.

For each of the mutation aggregations described above (binary gene, loss or gain of function, and granular hotspot) the mutation co-occurrence Fisher's Exact Test was computed and a False Discovery Rate correction applied.

## *EXAMPLE 2 - PERFORMANCE OF 36 SIGNATURES*

While we identified several previously unreported mutations affecting CPI treatment response (e.g., NBN, PDGFRA, NF1, and the co-occurring mutations in TP53, KRAS and NF1), attempting to understand the biological mechanism(s) behind CPI resistance/response requires broadening the analysis beyond the aforementioned genes. Therefore, we investigated whether a genetic signature(s) (i.e., a collection of tumor mutations) can predict response to CPI. Since TMB is considered to be an established and important biomarker that correlates with response to CPI therapy, we used a TMB-only model as a benchmark of our future results. The TMB-only model trained on our NSCLC cohort showed an AuC score of 0.59 (Figure 5C). Next, we utilized a machine learning (ML) approach which applied Naive Bayes models with a Bernoulli prior as the model architecture, embedded in a genetic algorithm (GA) for feature selection (Figure 5A,) to reveal predictive mutational signatures. We used GA because of the large set of available features (Binary:288, HFM/LFM:460, Hotspot:528) relative to the number of patients (n = 1150), as it can efficiently find the most predictive feature combinations (out of Binary:2^288, HFM/LFM:2^460, Hotspot:2^528) while including multi-feature interactions. Naive Bayes models were chosen for the simplicity of their internal state, resistance to over-training, and interpretability[33]. Analysis using our ML method on 977 patients (training set) resulted in 36 genetic signatures (12 for each input: binary, HFM/LFM and Hotspot) (Tables 1 to 3) with cross-validation AuC score ranges of 0.69-0.8 (Tables 8 to 10). In order to ensure generalizability and control overfitting, we held out 173 patients (test set) and used the 36 genetic signatures to predict CPI response. The generalizability analysis resulted in AUC score ranges of 0.55-0.64 (Figure 5B, Tables 8 to 10), which supports the predictive power and generalizability of the genetic signatures. Importantly, we investigated the specificity of the genetic signatures on CPI response by utilizing the 36 genetic signatures to predict chemotherapy response in 304 chemo treated patients. Our results indicated that the average AuC scores for predicting response in chemo patients utilizing the 36 mutational signatures was $0.48\pm0.1$, which is consistent with random chance, indicating that the mutational signatures are CPI response specific (Figure 5B). Importantly, comparing the AuC scores between TMB-only and our binary mutational signature resulted in comparable results with AuC scores of 0.61 and 0.59 for binary and TMB respectively (Figure 5C). These results are consistent with previously published AUC values for TMB[34,35] and provide support that our genetic signatures perform as well as the established TMB biomarker in prediction of CPI response. Altogether, our results indicate that our ML workflow performs as well as TMB, but provides the important advantage of interpretability, as the mutations deemed predictive can be explored to understand the biology behind response/resistance, which is not possible with a TMB-only prediction model.

### EXAMPLE 3 - OVERLAPS

Since each of the three ML inputs generated 12 separate predictive mutational signatures (Tables 1 to 3), we first confirmed that each genetic signature within the input is diverse (I.e., not large overlap between signatures) (Figure 11) Next, we assessed feature (mutations) popularity across the 12 mutational signatures within each of the 3 ML inputs, as the most frequently selected mutations might indicate the importance of the mutation on CPI response ( Table 4 and Table 5). We identified genes that were shared between the 12 independent mutational signatures within the 3 ML inputs sets (Table 5). We found that for the binary, HFM/LFM and Hotspot inputs, there was an overlap of 9, 13, 19 genes respectively within the 12 mutational signatures (Table 4). Among these overlaps, 8 mutations were shared across all 36 genetic signatures (Figure 5D, Table 4). The overlap within each ML input across the 12 independent signatures suggests that the 9, 13, and 19 (Table below) genes are necessary in generating a predictive mutational signature and play an important role in CPI response.

| Input | Unique | Within input overlap | AVG |
|---|---|---|---|
| Binary | 58 | 9 | 24 |
| GOF/LOF | 149 | 13 | 52 |
| Hotspot | 165 | 19 | 87 |
| Input overlap | 39 | 8 | |

Moreover, the 8 shared genes (NF1, STK11, TSC2, BRCA2, BRAF, STAG2, U2AF1 and PDK1) shared by all genetic signatures represent a core set of mutations-arguably the most important-in predicting/affecting CPI response (Figure 5D, Table 4). Next, we assessed the number of unique mutations selected as predictive within each of the 3 ML input across the 12 mutational signatures. Binary, HFM/LFM and hotspot mutational signatures contained 58, 149, and 165 unique features (genes/mutations), and had 39 genes overlapping between them (Figure 5D, Table 5), further suggesting a core of mutations important for CPI response prediction. Altogether, our ML approach revealed a significant number of genes, many of which are previously unreported to play a role in immunotherapy response.

### EXAMPLE 4 - RELATIVE CONTRIBUTIONS OF INDIVIDUAL GENE MUTATIONS IN OVERALL CPI RESPONSE

One of the advantages of using Naïve Bayes models with a Bernoulli prior is that it is a linear model (logistic regression)[36]. This enables interpretability of the mutational signatures in two important ways: 1. enables us to quantify the relative contribution of each mutation to the prediction of CPI response (i.e., relative importance), and 2. allows us to associate each mutation with the specific response it predicts (i.e., does a mutation associate with durable-response or innate-resistance). Using the equivalent logistic regression formulation of the Naive Bayes models, we were able to assign each feature (mutation) to a corresponding CPI response group with a numeric contribution, allowing us to sort the mutations by their contribution/importance to CPI response (Figure 6A, Figure 12, Table 6 and Table 7). In Binary mutational signatures we found that TSC2 mutations were the top contributor to durable-response and GID4 was the top contributor to innate-resistance (Figure 6A, Table 6 and Table 7). In HFM/LFM mutational signatures we found that FGF19 LFM mutations were the top contributor to durable response and MAP3K1 HFM mutations were the top contributor to innate resistance (Figure 6A, Table 6 and Table 7). In Hotspot mutational signatures we found that FGF19 LFM was the top contributor to durable-response and EGFR mutation at amino acid 746 was the top contributor to innate-resistance. Interestingly, we observed a set of genes that had opposite effects on CPI response, depending on the type of mutation (Figure 6B, Table 6 and Table 7). For example, in HFM/LFM input, we found that HFM mutations in PDGFRB were associated with durable-response, while LFM mutations were associated with innate-resistance (Figure 6B). Furthermore, in Hotspot inputs, we found that certain TP53 HFM mutations were associated with durable-response, while other TP53 HFM mutations were associated with innate-resistance (Figure 6B). Altogether these results provide a previously unreported link between certain mutations and CPI response, and revealed that mutations within the same gene can lead to opposite responses.

## EXAMPLE 5 - PATHWAYS

While it is important to identify individual mutations that affect CPI response, there is an unmet need to understand the biology/biological-processes behind these mutations and how they affect CPI response. In order to shed some light on the biological processes behind the aforementioned predictive genetic signatures, we investigated if these signatures fall into meaningful biological process. As traditional pathway enrichment analysis looks at biological pathways as a collection of genes, we wanted to apply network/topology-based pathway analysis that takes gene-gene (or protein-protein) interactions into account when performing pathway analysis. To that end, we used 8 different algorithms to perform topology assisted pathway analysis, utilizing the Computational Biology Methods for Drug Discovery (CBDD) R package37 developed by Clarivate. This approach provides the benefit of discovering additional pathways that would otherwise not be detected in an enrichment only approach. Network/topology pathway analysis for binary input revealed that the top 10 pathways across 8 algorithms were associated with cell cycle, IL-6, DNA damage response/repair (DDR), PDGF, Leptin, and IFN alpha/beta signaling (Figure 7). For HFM/LFM input the top 10 pathways were associated with epigenetic changes, YAP/TAZ, IL-6, DDR, PDGF, leptin and cell cycle related signaling. Since there is overlap of 39 genes between the three ML inputs (binary, HFM/LFM, hotspot), we checked which pathways are enriched in this overlap. Network/Topology based pathway analysis of the 39 gene overlap between the three inputs revealed that the top 10 pathways were associated with cell cycle, ESR1, PDGF, IL-6, DDR, IFN-alpha/beta, and EGFR signaling (Figure 7). We chose not to preform pathway analysis on the Hotspot granular genetic signature as it included 58% of possible genes, and can potentially bias our analysis. Altogether, our pathway analysis reveals several previously reported (e.g., IL-6, IFN-alpha/beta, DDR) and unreported tumor intrinsic pathways that may be involved in CPI response (e.g., YAP/TAZ, leptin and PDGF signaling)

## EXAMPLE 6 - IL-6 *SIGNALLING*

Since IL-6 signaling appeared in multiple pathway analysis results, we investigated its importance in NSCLC patients treated with Atezolizumab. We found that serum levels of IL-6 were elevated in both stable disease (SD) and progressive disease (PD) patients when compared to partial response (PR) patients in both Response evaluation criteria in solid tumors (RECIST) and immune-related response criteria (irRC). Furthermore, OS analysis revealed a significant survival increase in patients with lower IL-6 serum levels in NSCLC patients treated with Atezolizumab, which is consistent with previous reports. Altogether, the confirmation of the involvement of IL-6 in resistance mechanism supports the validity of our ML pipeline, which allows further insight into the biological pathways and mechanism(s) behind CPI resistance/response.

## EXAMPLE 7 - RECURSIVE ELIMINATION OF FEATURES

In order to assess the effect of reducing the number of features (i.e. genes) on the predictive accuracy of classification, a recursive elimination strategy was adopted. As shown in Figure 13, recursive elimination of features from 8 (which has accuracy of 0.5455 for patients with NSCLC treated with mono CPI and 0.4832 for chemotherapy patients) was conducted to assess performance (mean accuracy) of 7-gene, 6-gene, 5-gene, 4-gene, 3-gene, 2-gene and 1-gene models. For the 7-gene feature set, the mean $\pm$ standard deviation (sd) of the accuracy for CPI are $0.547 \pm 0.0136$; for chemotherapy are $0.491 \pm 0.0186$. Error bars indicate the standard deviation of the accuracy from 5 random training/validation data splits. The seven-genes are: NF1, STK11, TSC2, STAG2, U2AF1, BRCA2, PDK1. Without wishing to be bound by any particular theory, it is presently believed that reduction below 5 features (i.e. the 4-gene and below models) exhibit notably decreased mean accuracy. Therefore, models involving 5 features or greater may be chosen for their improved accuracy. The comparison between accuracy of prediction of CPI response vs. that of chemotherapy response evidences the specific nature of the CPI response predictive models as disclosed herein.

## EXAMPLE 8 - CGDB SUBSET OF MINIMAL GENES FOR PREDICTING DURABLE RESPONSE VS. INNATE RESISTANCE

The present inventors conducted further analysis to determine optimized minimal gene sets that maintain reasonable predictive performance and below which predictive performance is negatively impacted. This led to the following feature (gene) sets, each of which exhibited performance (mean accuracy) in the present data set that was comparable to other feature sets described herein, including feature sets involving larger number of genes and/or mutations.

* binary gene input (10-gene set): BRAF, BRIP1, STK11, CDK12, CTNNA1, FAS, NRAS, NOTCH3, PIK3CA and RAD51C.

\* HFM/LFM (GoF/LoF) (15-gene set): PBRM1_LOF, BRIP1_LOF, PTEN_LOF, CDKN2A_LOF, STK11_GOF, CDKN2B_LOF, U2AF1_GOF, CTNNA1_LOF, FGF10_GOF, FGF19_LOF, AKT2_GOF, NBN_LOF, ALOX12B_LOF, BRAF_GOF and NF1_GOF.

\* Hotspot (8-gene set): BRIP1_LOF, CDKN2B_LOF, U2AF1_GOF_34, CTNNA1_LOF, ALOX12B_LOF, EGFR_GOF_746, FAS_LOF and KMT2A_LOF.

Furthermore, the present inventors have tested a set of 5 genes selected with prior knowledge and achieved 57% AUC (prediction performance), and without those 5 genes, accuracy drops ~3% from using all features.

The two lists of 5 genes are shown below. The predictive performance of each was approximately the same.

First 5-gene set: STK11, BRAF, BRIP1, U2AF1 and NF1

Second 5-gene set: STK11, PDGFRA, BRAF, BRIP1 and CTNNA1.

## Tables

**Table 1A:** Cluster information for binary mutations

| Binary | | | | | |
|---|---|---|---|---|---|
| **cluster1** | **cluster2** | **cluster3** | **cluster4** | **cluster5** | **cluster6** |
| NF1 | NF1 | NF1 | NF1 | STK11 | STK11 |
| STK11 | STK11 | STK11 | STK11 | NF1 | TSC2 |
| TSC2 | TSC2 | BRAF | TSC2 | TSC2 | BRAF |
| ATR | TP53 | TSC2 | STAG2 | BRAF | ATR |
| STAG2 | BRCA2 | ATR | BRAF | STAG2 | NBN |
| BRAF | BRAF | NBN | NBN | ATR | NF1 |
| U2AF1 | ATRX | STAG2 | ATR | PDGFRA | PDGFRA |
| ATRX | STAG2 | BRCA2 | BRCA2 | U2AF1 | U2AF1 |
| BRCA2 | FGF23 | U2AF1 | U2AF1 | BRCA2 | BRCA2 |
| TET2 | U2AF1 | PDGFRB | GID4 | NBN | STAG2 |
| GID4 | TET2 | FGF23 | PDK1 | PDK1 | PDK1 |
| RARA | POLE | PDK1 | FGF23 | GID4 | TET2 |
| PDK1 | BRIP1 | GID4 | IDH1 | CCNE1 | GID4 |
| NBN | PDK1 | PDGFRA | PPP2R1A | CDK4 | FOXL2 |
| FOXL2 | ATR | CD79A | JUN | FOXL2 | CDK4 |
| IDH1 | ASXL1 | CCNE1 | ATRX | PDGFRB | PPP2R1A |
| CTNNA1 | ERCC4 | FOXL2 | FH | MLH1 | CCNE1 |
| FGF23 | PAX5 | TET2 | CD79A | CD79A | CSF1R |
| MPL | CTNNA1 | CTNNA1 | ASXL1 | FGF23 | ASXL1 |
| AKT1 | MPL | JUN | CTNNA1 | RAD51 | CTNNA1 |
| CCNE1 | GID4 | RAD51 | TET2 | IDH1 | RAD51 |
| FANCL | CD79A | IDH1 | PDCD1LG2 | CSF1R | IDH1 |
| ERCC4 | TSC1 | CDK4 | FOXL2 | | FGF23 |
| SMARCA4 | MAP2K1 | CSF1R | MDM2 | | CDK6 |
| | NRAS | NKX2-1 | MEF2B | | FUBP1 |

(continued)

| Binary | | | | | |
|---|---|---|---|---|---|
| **cluster1** | **cluster2** | **cluster3** | **cluster4** | **cluster5** | **cluster6** |
| | RARA | | CCNE1 | | AKT1 |
| | PDCD1LG2 | | PDGFRB | | FGFR1 |
| | | | CDK4 | | NTRK2 |
| | | | | | MLH1 |
| **cluster7** | **cluster8** | **cluster9** | **cluster10** | **cluster11** | **cluster12** |
| ATR | STK11 | NF1 | NF1 | STK11 | STK11 |
| NF1 | TSC2 | STK11 | STK11 | NF1 | NF1 |
| STK11 | BRAF | TP53 | TSC2 | TSC2 | TSC2 |
| TSC2 | BRCA2 | TSC2 | ASXL1 | TP53 | STAG2 |
| STAG2 | PDGFRA | BRCA2 | ATR | POLE | ATR |
| BRCA2 | NF1 | BRAF | BRAF | BRCA2 | ATRX |
| NBN | U2AF1 | U2AF1 | BRCA2 | ATRX | BRAF |
| BRAF | MLH1 | ATR | U2AF1 | BRAF | ASXL1 |
| PDK1 | ASXL1 | ATRX | CDK4 | STAG2 | U2AF1 |
| U2AF1 | POLE | BRIP1 | RARA | TET2 | BRCA2 |
| TET2 | PDK1 | SMAD4 | CTNNA1 | U2AF1 | TET2 |
| IDH1 | RARA | ASXL1 | STAG2 | FGF23 | PDK1 |
| GID4 | STAG2 | IDH1 | PDK1 | BRIP1 | GID4 |
| CD79A | BRIP1 | STAG2 | CCNE1 | ASXL1 | MPL |
| CTNNA1 | CDK4 | FGF23 | MLH1 | PDK1 | IDH1 |
| FGF23 | PDCD1LG2 | TET2 | CDKN1A | ERCC4 | CTNNA1 |
| CCNE1 | FANCG | GID4 | PPP2R1A | ATR | PPP2R1A |
| AKT1 | CCNE1 | CTNNA1 | MPL | PAX5 | RARA |
| ATRX | TET2 | PDK1 | ATRX | CTNNA1 | NBN |
| ASXL1 | ATR | PDCD1LG2 | RAD51B | TSC1 | CDK4 |
| PDGFRB | PPP2R1A | QKI | ERCC4 | MPL | TSC1 |
| JUN | FUBP1 | FOXL2 | IDH1 | PDCD1LG2 | MEF2B |
| | | MPL | AKT1 | RARA | FGF23 |
| | | CD79A | POLE | BRCA1 | CEBPA |
| | | TSC1 | CEBPA | CEBPA | CDK6 |
| | | MEF2B | MEF2B | CD79A | |
| | | FUBP1 | PDCD1LG2 | GID4 | |
| | | CCNE1 | GID4 | CDK4 | |
| | | BRD4 | | MAP2K1 | |
| | | | | NRAS | |
| | | | | SMAD4 | |
| | | | | MEF2B | |

**Table 1B:** Cluster information for binary mutations - updated following additional analysis

| Binary | | | | | |
|---|---|---|---|---|---|
| **cluster 1** | **cluster 2** | **cluster 3** | **cluster 4** | **cluster 5** | **cluster 6** |
| STK11 | BRAF | ASXL1 | BRAF | BRAF | BRAF |
| BRIP1 | CDKN2B | BRAF | BRIP1 | STK11 | TERT |
| CDKN2B | FLT1 | BRCA2 | STK11 | BRIP1 | CDKN2B |
| FLT1 | BRIP1 | CDKN2B | CDKN2B | FGF10 | STK11 |
| FGF10 | STK11 | FLT1 | ASXL1 | CDKN2B | FLT1 |
| RBM10 | ASXL1 | STK11 | CARD11 | BRCA2 | CARD11 |
| BRAF | FGF10 | BARD1 | FLT1 | FLT1 | ASXL1 |
| ASXL1 | TP53 | BRIP1 | BARD1 | ASXL1 | BRCA2 |
| TP53 | U2AF1 | U2AF1 | BRCA2 | BTK | BRIP1 |
| TSC2 | BARD1 | FAS | FGF10 | TERT | FGF10 |
| IKBKE | RBM10 | CTNNA1 | NOTCH3 | U2AF1 | IKBKE |
| HRAS | PBRM1 | IDH1 | HRAS | MLH1 | HRAS |
| PBRM1 | MLH1 | HRAS | PIK3CA | HRAS | BARD1 |
| IDH1 | CTNNA1 | NKX2-1 | TSC1 | IDH1 | ABL1 |
| RAD51C | BRCA2 | TSC1 | TERT | NOTCH3 | CTNNA1 |
| BARD1 | FAS | TSC2 | U2AF1 | PDGFRB | NOTCH3 |
| BRCA2 | IDH1 | ALOX12B | CTNNA1 | CTNNA1 | U2AF1 |
| MLH1 | RAD51C | GNA13 | GABRA6 | BARD1 | TSC2 |
| U2AF1 | TERT | CARD11 | SOX9 | NKX2-1 | GABRA6 |
| CEBPA | TSC2 | PBRM1 | IDH1 | ALOX12B | KEL |
| BTK | NKX2-1 | VHL | VHL | TSC1 | FAS |
| TSC1 | FOXL2 | MLH1 | ALOX12B | HSD3B1 | IDH1 |
| CSF1R | BTK | TERT | ABL1 | FOXL2 | TSC1 |
| CARD11 | GNA13 | IKBKE | CCNE1 | GNA13 | GNA13 |
| GABRA6 | HRAS | FGF10 | TSC2 | KRAS | BTK |
| RARA | CUL3 | KRAS | POLE | RAD51C | POLE |
| CDK12 | CD79A | ABL1 | GNA13 | CDK12 | CD79A |
| NKX2-1 | CCNE1 | ACVR1B | IKBKE | XRCC2 | ACVR1B |
| CD79A | GABRA6 | TNFAIP3 | CEBPA | VHL | NFE2L2 |
| SOX9 | HSD3B1 | FOXL2 | PARP1 | PDGFRA | SDHC |
| NOTCH3 | RARA | CEBPA | QKI | TNFAIP3 | CEBPA |
| SOX2 | SDHC | RAD51C | STAT3 | CD79A | ZNF703 |
| ACVR1B | MYC | CCNE1 | MDM4 | GID4 | NKX2-1 |
| CTNNA1 | JAK1 | CSF1R | SDHD | PDK1 | AKT2 |
| EZH2 | IKBKE | POLE | NFE2L2 | EZH2 | VHL |
| ESR1 | | CD79A | PDGFRB | SOX9 | PARP3 |
| FAS | | | FANCC | SDHC | |
| ALOX12B | | | | DDR2 | |

(continued)

| Binary | | | | | |
|---|---|---|---|---|---|
| **cluster 1** | **cluster 2** | **cluster 3** | **cluster 4** | **cluster 5** | **cluster 6** |
| STAT3 | | | | FAS | |
| | | | | TSC2 | |
| | | | | IKBKE | |
| | | | | MAP3K1 | |
| | | | | NFE2L2 | |
| **cluster 7** | **cluster 8** | **cluster 9** | **cluster 10** | **cluster 11** | **cluster 12** |
| BRAF | BRAF | BRAF | STK11 | ASXL1 | BRAF |
| CDKN2B | STK11 | STK11 | BRAF | BRAF | FLT1 |
| STK11 | CDKN2B | FLT1 | CDKN2B | STK11 | STK11 |
| ASXL1 | FLT1 | CDKN2B | BRIP1 | CDKN2B | BRIP1 |
| BRCA2 | BRIP1 | CARD11 | FLT1 | BRCA2 | CDKN2B |
| BRIP1 | FGF10 | BRCA2 | ASXL1 | FLT1 | FGF10 |
| FLT1 | ASXL1 | ASXL1 | BARD1 | BRIP1 | ASXL1 |
| FAS | CARD11 | BRIP1 | U2AF1 | CTNNA1 | TP53 |
| MLH1 | IKBKE | BARD1 | BRCA2 | FGF10 | RBM10 |
| U2AF1 | NOTCH3 | NOTCH3 | CTNNA1 | MLH1 | BRCA2 |
| IDH1 | HRAS | HRAS | MLH1 | U2AF1 | BARD1 |
| CTNNA1 | U2AF1 | U2AF1 | RAD51C | IDH1 | TSC2 |
| BARD1 | BARD1 | ABL1 | RARA | IKBKE | U2AF1 |
| FOXL2 | PBRM1 | IKBKE | NKX2-1 | CEBPA | PBRM1 |
| FGF10 | NKX2-1 | FGF10 | FGF10 | NOTCH3 | HRAS |
| RAD51C | CTNNA1 | CTNNA1 | HRAS | BARD1 | TSC1 |
| HRAS | VHL | FAS | FAS | HRAS | IKBKE |
| KEL | CCNE1 | TSC2 | PBRM1 | SOX9 | IDH1 |
| PDGFRB | IDH1 | KEL | IDH1 | VHL | MLH1 |
| CD79A | BRCA2 | TSC1 | IKBKE | TSC1 | RAD51C |
| PBRM1 | BTK | IDH1 | TSC2 | TERT | CEBPA |
| VHL | FAS | BTK | PDGFRB | KEL | BTK |
| HSD3B1 | ALOX12B | GABRA6 | CD79A | HSD3B1 | CSF1R |
| BTK | KEL | ACVR1B | FOXL2 | ALOX12B | CTNNA1 |
| ACVR1B | RARA | POLE | CEBPA | BAP1 | SOX9 |
| ALOX12B | TSC2 | CD79A | ALOX12B | ALK | RARA |
| TERT | PDGFRB | GNA13 | NBN | PDGFRB | ALOX12B |
| CDK12 | TSC1 | CEBPA | CUL3 | XRCC2 | GABRA6 |
| ALK | RAD51C | ZNF703 | BAP1 | CARD11 | CD79A |
| BAP1 | MLH1 | NFE2L2 | MYC | CDC73 | ARFRP1 |
| EZH2 | CDK12 | SDHC | SDHC | SOX2 | CARD11 |
| PDK1 | ERRFI1 | AKT2 | RBM10 | FOXL2 | GNA13 |

(continued)

| cluster 7 | cluster 8 | cluster 9 | cluster 10 | cluster 11 | cluster 12 |
|---|---|---|---|---|---|
| | POLE | NKX2-1 | BCL2 | ERRFI1 | NKX2-1 |
| | PARP3 | PARP3 | PARP3 | KRAS | HNF1A |
| | MAP3K1 | VHL | PAX5 | PDCD1LG2 | CUL3 |
| | ZNF703 | | GNA13 | | GID4 |
| | CD79A | | TERC | | FAS |
| | SDHC | | PDK1 | | |

**Table 2A:** Cluster information for GOF/LOF mutations

| GOF/LOF | | | | | |
|---|---|---|---|---|---|
| **cluter1** | **cluster2** | **cluster3** | **cluster4** | **cluster5** | **cluster6** |
| NF1_LOF | NF1_LOF | NF1_LOF | NF1_LOF | NF1_LOF | NF1_LOF |
| STK11_LOF | STK11_LOF | STK11_LOF | STK11_LOF | STK11_LOF | STK11_LOF |
| KMT2A_LO F | BRAF_GOF | BRAF_GOF | FGF19_LOF | ASXL1_LOF | BRAF_GOF |
| ASXL1_LOF | ASXL1_LOF | TSC2_LOF | BRAF_GOF | BRAF_GOF | TSC2_LOF |
| FGF19_LOF | BRCA2_LO F | ATRX_LOF | STK11_GO F | ATRX_LOF | STAG2_LO F |
| TSC2_LOF | TSC2_LOF | BRCA2_LO F | ATRX_LOF | FGF19_LOF | FGF19_LOF |
| BRAF_GOF | STAG2_LO F | FGF19_LOF | BRCA2_LO F | STK11_GO F | PDK1_LOF |
| IDH1_GOF | NKX2-1_LOF | STAG2_LO F | TSC2_LOF | TSC2_LOF | ASXL1_LOF |
| STAG2_LO F | ATR_LOF | ASXL1_LOF | NFKBIA_G OF | BRCA2_LO F | STK11_GO F |
| STK11_GO F | FGF19_LOF | PDK1_LOF | ASXL1_LOF | KMT2A_LO F | PDGFRA_L OF |
| BRCA2_LO F | PDK1_LOF | STK11_GO F | CDK4_GOF | STAG2_LO F | IDH1_GOF |
| PAX5_GOF | KMT2A_LO F | PDGFRA_L OF | JAK1_GOF | NKX2-1_LOF | ATRX_LOF |
| SOX2_LOF | NFKBIA_G OF | RB1_GOF | JUN_LOF | PDK1_LOF | U2AF1_GO F |
| PDK1_LOF | STK11_GO F | NKX2-1_LOF | IDH1_GOF | IDH1_GOF | NKX2-1_LOF |
| RB1_GOF | RB1_GOF | IDH1_GOF | PDK1_LOF | RB1_GOF | NBN_LOF |
| ATRX_LOF | PDGFRA_L OF | CDK4_GOF | KMT2A_LO F | FANCG_GO F | PPP2R1A_ GOF |
| PDCD1LG2 _GOF | U2AF1_GO F | U2AF1_GO F | STAG2_LO F | RAD52_GO F | NRAS_LOF |
| GATA3_GO F | IDH1_GOF | NFKBIA_G OF | NKX2-1_LOF | CDK4_GOF | PIK3C2G_G OF |
| U2AF1_GO F | CEBPA_LO F | JUN_LOF | PPP2R1A_ GOF | ATR_LOF | JUN_LOF |
| CTNNA1_L OF | PPP2R1A_ GOF | PBRM1_LO F | PAX5_GOF | MUTYH_GO F | JAK1_GOF |
| NKX2-1_LOF | PAX5_GOF | QKI_LOF | U2AF1_GO F | U2AF1_GO F | NTRK2_LO F |
| CEBPA_LO F | AKT3_GOF | GATA3_GO F | PIK3C2G_G OF | PPP2R1A_ GOF | ACVR1B_L OF |
| PIK3C2G_G OF | PIK3C2G_G OF | JAK1_GOF | FGF23_LOF | MAP3K1_L OF | BRCA2_GO F |
| PPP2R1A_ GOF | VEGFA_LO F | PIK3C2G_G OF | ZNF703_G OF | NRAS_LOF | CEBPA_LO F |
| HSD3B1_G OF | FGF14_GO F | MYC_LOF | ATR_LOF | VEGFA_LO F | FANCL_LO F |

(continued)

| GOF/LOF | | | | | |
|---|---|---|---|---|---|
| cluter1 | cluster2 | cluster3 | cluster4 | cluster5 | cluster6 |
| JAK1_GOF | GATA6_GO F | EZH2_LOF | CDH1_LOF | CDKN2B_L OF | BRD4_GOF |
| PBRM1_LO F | SOX2_LOF | MYD88_GO F | PBRM1_LO F | EZH2_GOF | RB1_GOF |
| GATA6_GO F | MYCN_LOF | KMT2A_LO F | MAP3K1_L OF | TNFAIP3_G OF | FGF23_LOF |
| NFKBIA_G OF | HGF_LOF | FGF14_GO F | CCNE1_GO F | CREBBP_G OF | REL_LOF |
| VEGFA_LO F | LTK_GOF | CD79A_LO F | CBFB_LOF | DAXX_GOF | PDCD1LG2 _GOF |
| JUN_LOF | PTPN11_LO F | PPP2R1A_ GOF | FGFR4_GO F | CDK6_GOF | VEGFA_LO F |
| FGF14_GO F | NTRK2_LO F | PAX5_GOF | PPARG_LO F | BRCA2_GO F | MAP3K1_G OF |
| CDK6_GOF | FH_GOF | CEBPA_LO F | KEAP1_GO F | PDCD1LG2 _GOF | RNF43_GO F |
| CD274_LOF | CD79A_LO F | SDHC_LOF | MYCN_LOF | GATA6_GO F | MYCN_LOF |
| KLHL6_GO F | PRKAR1A_ GOF | CD274_GO F | AURKA_LO F | FLT3_GOF | QKI_LOF |
| SYK_LOF | MEF2B_GO F | MAP3K1_G OF | GATA3_GO F | CHEK1_GO F | TGFBR2_G OF |
| BRCA2_GO F | PARP1_GO F | ZNF703_G OF | IRF4_GOF | ACVR1B_L OF | BRCA2_LO F |
| CUL4A_LO F | PDCD1LG2 _GOF | CD79B_GO F | RB1_GOF | DNMT3A_G OF | CD274_GO F |
| GABRA6_G OF | SETD2_GO F | NBN_LOF | BRCA2_GO F | FGFR2_GO F | KLHL6_GO F |
| ACVR1B_L OF | GNAQ_GOF | KEAP1_GO F | TGFBR2_G OF | AKT3_GOF | HGF_LOF |
| BCL2L1_LO F | CHEK1_GO F | MAP2K2_G OF | NRAS_LOF | CUL3_GOF | BCORL1_G OF |
| MYD88_GO F | ERCC4_LOF | GRM3_GOF | GATA6_GO F | CEBPA_LO F | GATA4_GO F |
| TGFBR2_G OF | ABL1_GOF | VEGFA_LO F | FANCA_LO F | H3F3A_GO F | PDGFRB_L OF |
| RAD51_LO F | CUL4A_LO F | CUL3_GOF | CDKN2B_L OF | POLD1_GO F | RARA_GOF |
| ERCC4_LOF | FUBP1_LO F | SYK_LOF | FLCN_GOF | BAP1_LOF | TNFAIP3_G OF |
| RAC1_LOF | CDK6_GOF | NRAS_LOF | ACVR1B_L OF | CTNNA1_L OF | BRIP1_GOF |
| AKT1_GOF | TGFBR2_G OF | SUFU_LOF | MYC_LOF | CD79A_LO F | CDK6_GOF |
| MAP3K1_G OF | PTEN_GOF | AKT2_GOF | RPTOR_GO F | NOTCH3_G OF | PAX5_GOF |
| PARP1_GO F | JAK1_GOF | MED12_GO F | NBN_LOF | PDGFRB_G OF | PDCD1LG2 _LOF |
| MYC_LOF | SDHC_LOF | CREBBP_G OF | ARID1A_G OF | | CDK4_GOF |

| GOF/LOF | | | | | |
|---|---|---|---|---|---|
| **cluter1** | **cluster2** | **cluster3** | **cluster4** | **cluster5** | **cluster6** |
| RAD51C_G OF | RAC1_LOF | BCORL1_G OF | AKT1_LOF | | MAP2K4_G OF |
| FGF23_LOF | FGFR4_GO F | SMARCA4_ GOF | CEBPA_LO F | | |
| EZH2_GOF | | | CD274_GO F | | |
| AKT1_LOF | | | CUL3_GOF | | |
| PRKAR1A_ GOF | | | MTOR_GOF | | |
| NF2_GOF | | | FLT3_GOF | | |
| FGF23_GO F | | | MUTYH_LO F | | |
| CDKN2B_L OF | | | CHEK2_LO F | | |
| ABL1_GOF | | | CDKN2A_L OF | | |
| CHEK1_LO F | | | RARA_LOF | | |
| SDHC_GOF | | | | | |
| **cluster7** | **cluter8** | **cluster9** | **cluster10** | **cluster11** | **cluster12** |
| NF1_LOF | NF1_LOF | NF1_LOF | NF1_LOF | NF1_LOF | NF1_LOF |
| STK11_LOF | BRAF_GOF | STK11_LOF | STK11_LOF | STK11_LOF | STK11_LOF |
| BRAF_GOF | STK11_LOF | BRAF_GOF | BRAF_GOF | STK11_GO F | ASXL1_LOF |
| ASXL1_LOF | ASXL1_LOF | BRCA2_LO F | ATR_LOF | ASXL1_LOF | BRAF_GOF |
| BRCA2_LO F | TSC2_LOF | ASXL1_LOF | KMT2A_LO F | BRCA2_LO F | FGF19_LOF |
| ATRX_LOF | STAG2_LO F | KMT2A_LO F | TSC2_LOF | BRAF_GOF | STAG2_LO F |
| PAX5_GOF | ATRX_LOF | NKX2-1_LOF | ASXL1_LOF | PBRM1_LO F | KMT2A_LO F |
| FGF19_LOF | NKX2-1_LOF | TSC2_LOF | PDK1_LOF | STAG2_LO F | TSC2_LOF |
| KMT2A_LO F | STK11_GO F | PDK1_LOF | FGF19_LOF | FGF19_LOF | IDH1_GOF |
| TSC2_LOF | BRCA2_LO F | STAG2_LO F | NKX2-1_LOF | RB1_GOF | BRCA2_LO F |
| PDK1_LOF | FGF19_LOF | ATR_LOF | SOX2_LOF | IDH1_GOF | STK11_GO F |
| IDH1_GOF | ATR_LOF | PDGFRA_L OF | IDH1_GOF | PDK1_LOF | PDK1_LOF |
| STK11_GO F | PDGFRA_L OF | FGF19_LOF | BRCA2_LO F | CTNNA1_L OF | RB1_GOF |

(continued)

| cluster7 | cluter8 | cluster9 | cluster10 | cluster11 | cluster12 |
|---|---|---|---|---|---|
| NKX2-1_LOF | PDK1_LOF | PIK3C2G_G OF | CEBPA_LO F | U2AF1_GO F | PAX5_GOF |
| ATR_LOF | RB1_GOF | NFKBIA_G OF | PPP2R1A_ GOF | PPP2R1A_ GOF | PIK3C2G_G OF |
| STAG2_LO F | IDH1_GOF | STK11_GO F | FGF23_LOF | JUN_LOF | PDCD1LG2 _GOF |
| U2AF1_GO F | NFKBIA_G OF | RB1_GOF | PDCD1LG2 _GOF | NKX2-1_LOF | ATRX_LOF |
| PIK3CA_GO F | U2AF1_GO F | VEGFA_LO F | KLHL6_GO F | TSC2_LOF | SOX2_LOF |
| JAK1_GOF | PPP2R1A_ GOF | PAX5_GOF | SYK_LOF | ERRFI1_LO F | U2AF1_GO F |
| KLHL6_GO F | GATA3_GO F | CEBPA_LO F | NBN_LOF | ATRX_LOF | CEBPA_LO F |
| MAP3K13_ GOF | JAK1_GOF | PPP2R1A_ GOF | STAG2_LO F | PIK3C2G_G OF | NKX2-1_LOF |
| PIK3C2G_G OF | JUN_LOF | MYCN_LOF | NFKBIA_G OF | CHEK2_LO F | PPP2R1A_ GOF |
| NBN_LOF | ERCC4_LO F | U2AF1_GO F | GATA6_GO F | XRCC2_LO F | GATA3_GO F |
| CDK4_GOF | FGF14_GO F | AKT3_GOF | U2AF1_GO F | PDCD1LG2 _GOF | NFKBIA_G OF |
| HGF_LOF | IRF4_GOF | GATA6_GO F | GNA13_LO F | RNF43_GO F | HSD3B1_G OF |
| RAD51C_L OF | CDH1_LOF | IDH1_GOF | PAX5_GOF | SOX2_LOF | CTNNA1_L OF |
| NRAS_LOF | HGF_LOF | FGF14_GO F | MAP3K1_G OF | JAK1_GOF | JAK1_GOF |
| CDK6_GOF | ZNF703_G OF | SOX2_LOF | KDM6A_GO F | PAX5_GOF | CD274_LOF |
| MDM2_LOF | MAP3K1_G OF | HGF_LOF | GATA3_GO F | BRCA2_GO F | GATA6_GO F |
| FH_GOF | MYCN_LOF | ERCC4_LO F | PARP1_GO F | CDKN1A_G OF | PBRM1_LO F |
| PPP2R1A_ GOF | JUN_GOF | PTPN11_LO F | RARA_GOF | MYD88_GO F | KLHL6_GO F |
| GNA13_LO F | VEGFA_LO F | NTRK2_LO F | HGF_LOF | FGF23_LOF | CDK6_GOF |
| AXL_GOF | BRCA2_GO F | FUBP1_LO F | PDGFRB_G OF | QKI_LOF | SYK_LOF |
| RB1_GOF | FANCG_GO F | PARP1_GO F | JAK1_GOF | GATA3_GO F | FGF14_GO F |
| CTNNA1_L OF | ACVR1B_L OF | GNAQ_GOF | MYCN_LOF | BCL2L1_LO F | AKT1_GOF |
| CCND2_LO F | PIK3C2G_G OF | FH_GOF | NOTCH2_G OF | RARA_GOF | JUN_LOF |
| GATA3_GO F | BARD1_GO F | PRKAR1A_ GOF | RAD51C_G OF | AKT2_GOF | MYC_LOF |
| TGFBR2_G OF | CEBPA_LO F | LTK_GOF | TGFBR2_G OF | VEGFA_LOF | TGFBR2_G OF |
| TSC2_GOF | NBN_LOF | MEF2B_GO F | RAD52_GO F | MAP3K13_ GOF | CUL4A_LO F |

30

| cluster7 | cluter8 | cluster9 | cluster10 | cluster11 | cluster12 |
|---|---|---|---|---|---|
| MYC_LOF | MPL_GOF | TGFBR2_G OF | VEGFA_LO F | HSD3B1_G OF | BRCA2_GO F |
| RAD54L_LO F | PBRM1_LO F | JAK1_GOF | RBM10_GO F | AKT3_GOF | RAC1_LOF |
| VEGFA_LO F | QKI_LOF | RAD51C_L OF | CUL3_GOF | CCNE1_GO F | MYD88_GO F |
| CCNE1_GO F | FGFR4_GO F | CHEK1_GO F | RAC1_LOF | BRD4_GOF | GABRA6_G OF |
| CD274_GO F | CHEK1_GO F | SETD2_GO F | ERBB2_GO F | PIK3R1_GO F | MAP3K1_G OF |
| MAP3K1_G OF | | ABL1_GOF | PIK3CB_LO F | FGF6_GOF | VEGFA_LO F |
| SOX2_LOF | | PTEN_GOF | XRCC2_LO F | MYC_LOF | BCL2L1_LO F |
| GATA6_GO F | | CUL4A_LO F | CTNNA1_G OF | ABL1_GOF | ACVR1B_L OF |
| ARFRP1_L OF | | IRF2_GOF | LTK_GOF | GABRA6_G OF | PRKAR1A_ GOF |
| DAXX_GOF | | BTG1_GOF | NRAS_LOF | PDCD1LG2 _LOF | ABL1_GOF |
| SDHD_GOF | | CD79A_LO F | CBFB_GOF | STAG2_GO F | PARP1_GO F |
| PDGFRB_G OF | | WT1_LOF | **IRF4_GOF** | | CUL3_GOF |
| CTNNA1_G OF | | PDCD1LG2 _GOF | JUN_LOF | | FGFR2_GO F |
| TSC1_GOF | | HSD3B1_L OF | FBXW7_GO F | | RAD51_LO F |
| | | | FGF14_GO F | | RAD51C_G OF |

**Table 2B:** Cluster information for GOF/LOF mutations - updated following additional analysis

| HFM/LFM | | | | | |
|---|---|---|---|---|---|
| cluster1 | cluster2 | cluster3 | cluster4 | cluster5 | cluster6 |
| BRAF_HFM | BRAF_HFM | BRAF_HFM | BRAF_HFM | BRAF_HFM | BRAF_HFM |
| BRIP1_LFM | CDKN2B_LFM | NOTCH3_LFM | CDKN2B_LFM | BRIP1_LFM | FGF10_HFM |
| FGF10_HFM | FGF10_HFM | CDKN2B_LFM | BRIP1_LFM | CDKN2B_LFM | CDKN2B_LFM |
| NF1_HFM | BRIP1_LFM | PTEN_LFM | NOTCH3_LFM | PTEN_LFM | TERT_HFM |
| STK11_HFM | NF1_HFM | BCL6_LFM | FGF10_HFM | FGF10_HFM | MAP3K13_LFM |
| MAP3K13_LFM | RARA_HFM | NF1_HFM | NF1_HFM | NF1_HFM | MLH1_LFM |
| TERT_HFM | PTEN_LFM | STK11_HFM | STK11_HFM | STK11_HFM | BRCA2_LFM |
| PDGFRA_LFM | STK11_HFM | BRIP1_LFM | FGFR2_LFM | NOTCH3_LFM | BRIP1_LFM |
| NOTCH3_LFM | TSC1_LFM | ASXL1_LFM | PBRM1_LFM | FLT1_LFM | NF1_HFM |
| CDKN2B_LFM | TERT_HFM | PIK3C2G_HFM | NBN_LFM | IKBKE_LFM | KEAP1_HFM |
| PTEN_LFM | FGFR2_HFM | MYC_LFM | MAP3K1_LFM | MYC_LFM | NOTCH3_LFM |
| IKBKE_LFM | MYC_LFM | ALOX12B_LFM | PTEN_LFM | ALOX12B_LFM | DDR2_HFM |
| ALOX12B_LFM | IKBKE_LFM | PDGFRA_LFM | ALOX12B_LFM | MLH1_LFM | RARA_HFM |
| TSC1_LFM | MAP3K13_LFM | FGF10_HFM | MLH1_LFM | MAP3K1_LFM | U2AF1_HFM |
| BARD1_LFM | ALOX12B_LFM | PDGFRB_LFM | TERT_HFM | MAP3K13_LFM | BARD1_LFM |
| PDGFRB_LFM | NOTCH3_LFM | KEAP1_HFM | ZNF703_HFM | ZNF703_HFM | MYC_LFM |
| U2AF1_HFM | ASXL1_LFM | FGFR2_HFM | MAP3K13_LFM | TSC1_LFM | PDGFRB_LFM |
| RARA_HFM | ZNF703_HFM | CHEK2_HFM | RARA_HFM | BRCA2_LFM | FGFR2_HFM |
| MLH1_LFM | NBN_LFM | RARA_HFM | BRCA2_LFM | TERT_HFM | PIK3C2G_HFM |
| FLT1_LFM | JAK1_HFM | CD79A_LFM | HRAS_HFM | BARD1_LFM | PDGFRA_LFM |
| ZNF703_HFM | MLH1_LFM | TERT_HFM | KEL_LFM | NF2_LFM | ZNF703_HFM |
| BRCA2_LFM | PDGFRA_LFM | GATA6_HFM | FLT1_LFM | DDR2_HFM | PIK3CA_LFM |
| MAP3K1_LFM | FLT1_LFM | MAP3K13_LFM | MYC_LFM | ASXL1_LFM | FLT1_LFM |
| CHEK2_HFM | PDGFRB_LFM | PIK3CA_LFM | FGFR2_HFM | RARA_HFM | ASXL1_LFM |
| MYC_LFM | DDR2_HFM | ESR1_LFM | HNF1A_LFM | U2AF1_HFM | PTEN_LFM |
| DDR2_HFM | TNFRSF14_LFM | FLT1_LFM | CTNNA1_LFM | ARFRP1_HFM | ALOX12B_LFM |
| CTNNA1_LFM | BCL6_LFM | MLH1_LFM | IKBKE_LFM | CTNNA1_LFM | TSC1_LFM |
| FGFR2_HFM | FGFR2_LFM | TSC1_LFM | ASXL1_LFM | PIK3C2G_HFM | BCL6_LFM |
| NBN_LFM | PAX5_HFM | U2AF1_HFM | CHEK2_HFM | WT1_LFM | CD79A_LFM |
| GATA6_HFM | CTNNA1_LFM | ZNF703_HFM | U2AF1_HFM | PBRM1_LFM | KLHL6_HFM |
| ASXL1_LFM | GATA6_HFM | ARFRP1_HFM | FANCC_HFM | JAK1_HFM | JAK2_LFM |
| BCL6_LFM | CD79A_LFM | FOXL2_LFM | JAK1_HFM | FOXL2_LFM | SMAD4_LFM |
| STK11_LFM | MAP3K1_LFM | JAK2_LFM | KEAP1_HFM | RICTOR_HFM | TNFRSF14_LFM |
| HRAS_HFM | ASXL1_HFM | FGF19_LFM | FOXL2_LFM | NBN_LFM | CUL3_LFM |
| TNFRSF14_LFM | BAP1_LFM | BARD1_LFM | ASXL1_HFM | HRAS_HFM | FAS_LFM |
| KLHL6_HFM | HRAS_HFM | CTNNA1_LFM | PIK3CA_LFM | FGFR2_LFM | ERBB4_HFM |
| GNAS_LFM | IDH1_HFM | TSC2_LFM | AKT2_HFM | SPOP_LFM | ARFRP1_HFM |

(continued)

| HFM/LFM | | | | | |
|---|---|---|---|---|---|
| **cluster1** | **cluster2** | **cluster3** | **cluster4** | **clusters** | **clusters** |
| PIK3CA_LFM | FOXL2_LFM | XRCC2_LFM | ERBB2_HFM | FGF19_LFM | ASXL1_HFM |
| NF2_LFM | ERBB2_HFM | PRKCI_HFM | ESR1_LFM | CEBPA_LFM | STK11_HFM |
| XRCC2_LFM | CD274_HFM | PAX5_HFM | GNAS_LFM | TERC_HFM | FOXL2_LFM |
| TSC2_LFM | BARD1_LFM | CDKN2A_LFM | EZH2_LFM | ERBB2_LFM | CEBPA_LFM |
| ARFRP1_HFM | ARFRP1_HFM | FAS_LFM | FH_HFM | GATA6_HFM | PARP2_LFM |
| FANCC_HFM | TSC2_LFM | SMAD4_LFM | WT1_HFM | NF2_HFM | AKT2_HFM |
| JAK1_HFM | IGF1R_HFM | BRCA2_LFM | GID4_LFM | TSC2_LFM | CSF1R_LFM |
| FH_HFM | BRCA2_LFM | DDR2_HFM | GABRA6_HFM | AR_LFM | CDK6_LFM |
| ASXL1_HFM | U2AF1_HFM | ERBB2_HFM | RICTOR_HFM | CHEK2_HFM | AMER1_HFM |
| WT1_LFM | GNA11_HFM | EZH2_LFM | CARD11_LFM | ERBB4_HFM | IKBKE_LFM |
| PDCD1LG2_HFM | PIK3CA_LFM | KLHL6_HFM | GATA6_HFM | IKZF1_HFM | SRC_LFM |
| JAK2_LFM | ATM_HFM | GNA13_LFM | CEBPA_LFM | MEF2B_LFM | CTNNA1_LFM |
| GABRA6_HFM | CHEK2_HFM | FANCC_HFM | PDGFRB_LFM | FGFR2_HFM | CCND2_LFM |
| SF3B1_LFM | FANCC_HFM | GNAS_LFM | LYN_HFM | CCNE1_LFM | EZH2_LFM |
| NOTCH3_HFM | IKZF1_HFM | IKBKE_LFM | SMO_HFM | PIK3CA_LFM | GATA6_HFM |
| AKT2_HFM | CDK12_LFM | SPOP_HFM | IDH1_HFM | TNFRSF14_LFM | FGFR2_LFM |
| CDK6_LFM | AR_LFM | AKT2_HFM | HSD3B1_LFM | BAP1_LFM | SMARCB1_HFM |
| FGF19_LFM | SMARCB1_HFM | NBN_LFM | FGF19_LFM | WT1_HFM | CHEK2_HFM |
| CCNE1_HFM | SMAD4_LFM | MEF2B_HFM | AXIN1_LFM | ERRFI1_LFM | IDH1_LFM |
| CSF1R_LFM | FGF19_LFM | TNFRSF14_LFM | PRKAR1A_HFM | | MEF2B_HFM |
| FGF6_HFM | BCL2L2_HFM | RICTOR_HFM | | | ALK_HFM |
| NFE2L2_HFM | ROS1_HFM | ASXL1_HFM | | | MAP3K1_LFM |
| | PRKAR1A_HFM | NKX2-1_HFM | | | HSD3B1_LFM |
| | FANCG_HFM | CDK6_LFM | | | ERRFI1_LFM |
| | PIK3C2G_HFM | MAP3K1_LFM | | | NBN_LFM |
| | EGFR_LFM | FANCG_HFM | | | FH_HFM |
| | | EZH2_HFM | | | CUL4A_HFM |
| | | RBM10_LFM | | | MYCL_LFM |
| | | HRAS_HFM | | | EP300_HFM |
| | | WT1_HFM | | | |
| **cluster7** | **cluster8** | **cluster9** | **cluster10** | **cluster11** | **cluster12** |
| FGF10_HFM | BRAF_HFM | BRAF_HFM | BRAF_HFM | CDKN2B_LFM | BRIP1_LFM |
| BRAF_HFM | BRIP1_LFM | BRIP1_LFM | CDKN2B_LFM | BRAF_HFM | BRAF_HFM |
| CDKN2B_LFM | NBN_LFM | CDKN2B_LFM | BRIP1_LFM | FGF10_HFM | FGF10_HFM |
| TERT_HFM | NOTCH3_LFM | NOTCH3_LFM | FGF10_HFM | BRIP1_LFM | CDKN2B_LFM |

| cluster7 | cluster8 | cluster9 | cluster10 | cluster11 | cluster12 |
|---|---|---|---|---|---|
| BRIP1_LFM | NF1_HFM | NF1_HFM | NOTCH3_LFM | TERT_HFM | STK11_HFM |
| NOTCH3_LFM | TSC2_LFM | FGF10_HFM | PTEN_LFM | NF1_HFM | NF1_HFM |
| MAP3K13_LFM | FGF10_HFM | TERT_HFM | TERT_HFM | STK11_HFM | ASXL1_LFM |
| BRCA2_LFM | STK11_HFM | MAP3K13_LFM | TSC2_LFM | PTEN_LFM | BARD1_LFM |
| MLH1_LFM | ASXL1_LFM | ASXL1_LFM | NF1_HFM | NOTCH3_LFM | MAP3K13_LFM |
| KEAP1_HFM | U2AF1_HFM | STK11_HFM | U2AF1_HFM | MYC_LFM | BAP1_LFM |
| U2AF1_HFM | PIK3C2G_HFM | FGFR2_HFM | STK11_HFM | RARA_HFM | MYC_LFM |
| NF1_HFM | FLT1_LFM | ALOX12B_LFM | FGFR2_HFM | FLT1_LFM | RARA_HFM |
| DDR2_HFM | MAP3K1_LFM | KEAP1_HFM | MAP3K13_LFM | MAP3K13_LFM | MLH1_LFM |
| RARA_HFM | MYC_LFM | PDGFRB_LFM | MYC_LFM | ASXL1_LFM | U2AF1_HFM |
| PIK3CA_LFM | BARD1_LFM | RARA_HFM | TSC1_LFM | MLH1_LFM | FGFR2_LFM |
| PDGFRB_LFM | ZNF703_HFM | MYC_LFM | PDGFRB_LFM | TSC1_LFM | ALOX12B_LFM |
| MYC_LFM | MLH1_LFM | PIK3C2G_HFM | FOXL2_LFM | IKBKE_LFM | FGFR2_HFM |
| FLT1_LFM | NF2_LFM | PTEN_LFM | NBN_LFM | FGFR2_HFM | MAP3K1_LFM |
| BARD1_LFM | RARA_HFM | BCL6_LFM | KEAP1_HFM | GATA6_HFM | NOTCH3_LFM |
| PIK3C2G_HFM | PIK3CA_HFM | U2AF1_HFM | SMAD4_LFM | ZNF703_HFM | PAX5_HFM |
| KLHL6_HFM | ALOX12B_LFM | GATA6_HFM | MAP3K1_LFM | PDGFRA_LFM | NF2_LFM |
| FGFR2_HFM | DDR2_HFM | FLT1_LFM | MLH1_LFM | DDR2_HFM | EZH2_LFM |
| ZNF703_HFM | ERBB2_HFM | CTNNA1_LFM | RARA_HFM | FGFR2_LFM | ARFRP1_HFM |
| PDGFRA_LFM | NKX2-1_HFM | MLH1_LFM | ALOX12B_LFM | TNFRSF14_LFM | HRAS_HFM |
| PTEN_LFM | CTNNA1_LFM | TSC1_LFM | WT1_LFM | JAK1_HFM | PARP2_LFM |
| TSC1_LFM | HRAS_HFM | CD79A_LFM | ASXL1_HFM | BCL6_LFM | FOXL2_LFM |
| ASXL1_LFM | IKBKE_LFM | SMAD4_LFM | PIK3CA_LFM | ALOX12B_LFM | FAS_LFM |
| ALOX12B_LFM | ATR_LFM | FOXL2_LFM | CHEK2_HFM | PAX5_HFM | GNA13_LFM |
| CD79A_LFM | PDGFRB_LFM | BARD1_LFM | JAK1_HFM | HRAS_HFM | IKBKE_LFM |
| BCL6_LFM | ESR1_LFM | PIK3CA_LFM | BCL6_LFM | U2AF1_HFM | JAK1_HFM |
| TNFRSF14_LF M | WT1_LFM | CHEK2_HFM | HSD3B1_LFM | CTNNA1_LFM | ERBB2_HFM |
| SMAD4_LFM | SOX2_HFM | JAK2_LFM | GNAS_LFM | PDGFRB_LFM | SF3B1_LFM |
| ASXL1_HFM | GID4_LFM | FAS_LFM | CDC73_HFM | CD79A_LFM | ZNF703_HFM |
| JAK2_LFM | FANCC_HFM | ZNF703_HFM | ASXL1_LFM | BAP1_LFM | NBN_LFM |
| CUL3_LFM | MAP3K13_LFM | FGF19_LFM | BARD1_LFM | NBN_LFM | FLT1_LFM |
| FAS_LFM | FAS_LFM | ARFRP1_HFM | ARFRP1_HFM | FOXL2_LFM | DDR2_HFM |
| ARFRP1_HFM | FH_HFM | PAX5_HFM | FANCC_HFM | BARD1_LFM | ESR1_LFM |
| STK11_HFM | KEL_LFM | CDKN2A_LFM | JAK2_LFM | ASXL1_HFM | CTNNA1_LFM |
| CEBPA_LFM | CHEK2_HFM | ESR1_LFM | ERBB2_HFM | CD274_HFM | PDGFRB_LFM |
| FOXL2_LFM | FGF19_LFM | PRKCI_HFM | PDGFRA_LFM | IDH1_HFM | PTEN_LFM |
| ERBB4_HFM | TNFRSF14_LF M | TSC2_LFM | CTNNA1_LFM | TSC2_LFM | PPARG _LFM |
| AMER1_HFM | PBRM1_LFM | | PARP2_LFM | ARFRP1_HFM | NOTCH2_LFM |

**EP 4 515 000 B1**

(continued)

| cluster7 | cluster8 | cluster9 | cluster10 | cluster11 | cluster12 |
|---|---|---|---|---|---|
| IKBKE_LFM | RICTOR_HFM | XRCC2_LFM | PDCD1LG2_HFM | IGF1R_HFM | DNMT3A HFM |
| CDK6_LFM | JAK1_HFM | EZH2_LFM | GID4_LFM | GNA11_HFM | TERC_HFM |
| CHEK2_HFM | FGF6_LFM | KLHL6_HFM | CCNE1_HFM | ERBB2_HFM | KLHL6_HFM |
| CTNNA1_LFM | SF3B1_LFM | GNA13_LFM | IKBKE_LFM | CCNE1_HFM | CEBPA_LFM |
| ALK_HFM | ERBB4_HFM | BRCA2_LFM | PRDM1_LFM | MAP3K1_LFM | TSC1_LFM |
| PARP2_LFM | ASXL1_HFM | MAP3K1_LFM | MDM4_HFM | CHEK2_HFM | SMO_HFM |
| AKT2_HFM | MYCN_HFM | ERBB2_HFM | CEBPA_LFM | AR_LFM | BCL6_LFM |
| SOX9_LFM | ARFRP1_HFM | DDR2_HFM | TNFRSF14_LFM | ATM_HFM | CARD11_LFM |
| NBN_LFM | TSC1_LFM | HRAS_HFM | IGF1R_HFM | SMARCB1_HFM | MEF2B_HFM |
| MAP3K1_LFM | KRAS_LFM | ERRFI1_LFM | AKT3_HFM | PIK3CA_LFM | FGF19_LFM |
| CSF1R_LFM | CDK12_HFM | GNAS_LFM | GATA6_HFM | SMAD4_LFM | ERBB4_HFM |
| HSD3B1_LFM | ATM_HFM | SOX9_HFM | SF3B1_LFM | BRCA2_LFM | PIK3C2G_HFM |
| SMARCB1_HF M | WT1_HFM | NBN_LFM | EZH2_LFM | | MEF2B_LFM |
| IRF4_LFM | MCL1_HFM | RICTOR_HFM | CASP8_LFM | | GID4_LFM |
| FGFR2_LFM | XRCC2_LFM | SPOP_HFM | FLT1_LFM | | CEBPA_HFM |
| CCND2_LFM | | TNFRSF14_LFM | GNA13_LFM | | FGF12_HFM |
| FH_HFM | | MEF2B_HFM | | | CYP17A1_LFM |
| IDH1_LFM | | TEK_LFM | | | SMARCB1_HF M |
| MYCN_HFM | | FANCC_HFM | | | IDH1_LFM |
| GATA6_HFM | | ASXL1_HFM | | | CD79A_LFM |
| JAK1_HFM | | AKT2_HFM | | | TERT_HFM |
| ERRFI1_LFM | | | | | ASXL1_HFM |
| EZH2_LFM | | | | | CSF1R_LFM |
| MEF2B_HFM | | | | | FGF14_HFM |

**Table 3A:** Cluster information for hotspot mutations

| Hotspot | | | | | |
|---|---|---|---|---|---|
| **cluster1** | **cluster2** | **cluster3** | **cluster4** | **cluster5** | **cluster6** |
| NF1_LOF | NF1_LOF | NF1_LOF | NF1_LOF | NF1_LOF | NF1_LOF |
| STK11_LOF | TSC2_LOF | KMT2A_LOF | ASXL1_LOF | STK11_LOF | ASXL1_LOF |
| ASXL1_LOF | KMT2A_LOF | BRAF_GOF _600 | TSC2_LOF | BRAF_GOF _600 | ATRX_LOF |
| KMT2A_LOF | ASXL1_LOF | STK11_LOF | BRAF_GOF _600 | ASXL1_LOF | BRAF_GOF _600 |
| PDGFRA_L OF | BRAF_GOF _600 | ATR_LOF | STK11_LOF | TSC2_LOF | STK11_LOF |
| ATR_LOF | STK11_LOF | TSC2_LOF | KMT2A_LOF | ATR_LOF | BRCA2_LOF |

35

(continued)

| Hotspot | | | | | |
|---|---|---|---|---|---|
| **cluster1** | **cluster2** | **cluster3** | **cluster4** | **cluster5** | **cluster6** |
| NFKBIA_GO F | ATR_LOF | DNMT3A_G OF_882 | ATRX_LOF | KMT2A_LOF | TSC2_LOF |
| BRAF_GOF _600 | PDGFRA_L OF | FGF19_LOF | BRCA2_LOF | BRCA2_LOF | FGF19_LOF |
| TSC2_LOF | BRCA2_LOF | ASXL1_LOF | PAX5_GOF | PDGFRA_L OF | PDK1_LOF |
| FGF19_LOF | STAG2_LOF | BRCA2_LOF | STAG2_LOF | NFKBIA_GO F | NFE2L2_GO F_24 |
| MYCN_LOF | NKX2-1_LOF | PDGFRA_L OF | FGF19_LOF | ATRX_LOF | ATR_LOF |
| NKX2-1_LOF | FGF19_LOF | CDKN2A_G OF _151 | QKI_LOF | STAG2_LOF | NFKBIA_GO F |
| BRCA2_LOF | NFKBIA_GO F | WT1_LOF | CDKN2A_G OF _151 | NFE2L2_GO F_24 | CDKN2A_G OF _151 |
| CDKN2A_G OF _151 | CDKN2A_G OF _151 | NFKBIA_GO F | U2AF1_GOF _34 | NKX2-1_LOF | NKX2-1_LOF |
| PDK1_LOF | NFE2L2_GO F_24 | NKX2-1_LOF | PDK1_LOF | PDK1_LOF | DNMT3A_G OF_882 |
| TP53_GOF_ 331 | TP53_GOF_ 282 | MYCN_LOF | NFE2L2_GO F_24 | FGF19_LOF | STAG2_LOF |
| TP53_GOF_ 282 | PDK1_LOF | PDK1_LOF | DNMT3A_G OF_882 | U2AF1_GOF _34 | MYCN_LOF |
| WT1_LOF | GATA3_GO F | TP53_GOF_ 331 | EGFR_GOF _719 | FANCG_GO F | KMT2A_LOF |
| QKI_LOF | PAX5_GOF | CDC73_GO F | CDH1_LOF | CDKN2A_G OF _151 | PPP2R1A_G OF |
| NFE2L2_GO F_24 | DNMT3A_G OF_882 | PPP2R1A_G OF | TP53_GOF_ 331 | NRAS_LOF | U2AF1_GOF _34 |
| VEGFA_LO F | U2AF1_GOF _34 | STAG2_LOF | MYD88_GO F_265 | TP53_GOF_ 282 | PAX5_GOF |
| U2AF1_GOF _34 | TP53_GOF_ 331 | U2AF1_GOF _34 | NRAS_LOF | CEBPA_LO F | JAK1_GOF |
| EGFR_GOF _719 | VEGFA_LO F | VEGFA_LO F | NKX2-1_LOF | FGF14_GOF | NFE2L2_GO F_77 |
| PPP2R1A_G OF | TP53_GOF_ 272 | TP53_GOF_ 282 | GATA3_GO F | EGFR_GOF _719 | GATA3_GO F |
| DNMT3A_G OF_882 | CEBPA_LO F | NFE2L2_GO F_77 | PPP2R1A_G OF | GATA3_GO F | EGFR_GOF _719 |
| STAG2_LOF | PTCH1_GO F_48 | NFE2L2_GO F_24 | PTEN_GOF | MDM2_LOF | TP53_GOF_ 282 |
| GATA3_GO F | STK11_GOF _291 | CCNE1_GO F | NFKBIA_GO F | EGFR_GOF _746 | TP53_GOF_ 244 |
| HGF_LOF | DIS3_GOF_ 458 | FANCG_GO F | PIK3C2G_G OF _1088 | ACVR1B_L OF | TP53_GOF_ 331 |
| PIK3CA_GO F_1043 | TP53_GOF_ 285 | TP53_GOF_ 560 | TP53_GOF_ 195 | EGFR_GOF _747 | FGF14_GOF |

(continued)

| Hotspot | | | | | |
|---|---|---|---|---|---|
| cluster1 | cluster2 | cluster3 | cluster4 | cluster5 | cluster6 |
| PIK3C2G_G OF _1088 | STK11_GOF _37 | EGFR_GOF _719 | CDKN2A_G OF_80 | TNFAIP3_G OF | BRAF_GOF _466 |
| TP53_GOF_ 159 | AKT3_GOF | STK11_GOF _291 | CD79A_LOF | CDK4_GOF | PBRM1_LO F |
| TP53_GOF_ 272 | TP53_GOF_ 244 | EGFR_GOF _858 | NBN_LOF | ZNF703_GO F | JUN_LOF |
| MTOR_GOF _1834 | PIK3C2G_G OF _1088 | BRAF_GOF _466 | TP53_GOF_ 282 | CHEK1_LOF | MUTYH_GO F_165 |
| MYCN_GOF | TP53_GOF_ 920 | HGF_LOF | EGFR_GOF _747 | ERCC4_LO F | MAP3K1_G OF |
| NRAS_LOF | PARP1_GO F | EGFR_GOF _746 | BRAF_GOF _469 | TP53_GOF_ 285 | AKT3_GOF |
| STK11_GOF _291 | KEAP1_GO F_116 | TP53_GOF_ 159 | IDH1_GOF | MTOR_GOF _1834 | TP53_GOF_ 376 |
| JUN_LOF | KDM5A_GO F | KEAP1_GO F_260 | NTRK2_LOF | PPP2R1A_G OF | PTEN_GOF |
| AR_GOF_49 3 | MAP3K1_G OF | PIK3C2G_G OF _1088 | TP53_GOF_ 244 | BRAF_GOF _469 | STK11_GOF _291 |
| CEBPA_LO F | JUN_LOF | JUN_LOF | CD274_GOF | NFE2L2_GO F_77 | RAD51B_LO F |
| ERBB2_GO F_755 | KEAP1_GO F_272 | ZNF703_GO F | FGFR4_GO F | KDM5C_GO F_1546 | EGFR_GOF _746 |
| RB1_GOF | GNAQ_GOF | AR_GOF_69 | KEAP1_GO F_260 | MYD88_GO F_265 | TP53_GOF_ 215 |
| BRAF_GOF _466 | POLD1_GO F | GATA3_GO F | STK11_GOF _181 | MAP3K13_G OF | KDM5C_GO F_1330 |
| STK11_GOF _221 | IDH1_GOF | KDM5C_GO F_1546 | STK11_GOF _291 | TP53_GOF_ 278 | AR_GOF_45 7 |
| PTEN_GOF _59 | CD274_GOF | KEAP1_GO F_234 | CDK4_GOF | SMARCA4_ GOF_1157 | EGFR_GOF _747 |
| AKT3_GOF | FH_GOF | BRCA2_GO F | RB1_GOF | CCNE1_GO F | PPARG_LO F |
| NFE2L2_GO F_77 | STK11_GOF _84 | PIK3CA_GO F_1043 | TNFAIP3_G OF | RAD52_GO F | PARP1_GO F |
| STK11_GOF _251 | MYCN_LOF | TP53_GOF_ 195 | ZNF703_GO F | IDH1_GOF | IDH1_GOF |
| SF3B1_GOF _666 | CDK8_GOF | KEAP1_GO F_332 | FGF14_GOF | NOTCH1_G OF | CDKN1A_G OF |
| ARID1A_GO F_21 | PARP3_GO F | STK11_GOF _84 | SOX2_LOF | AR_GOF_49 3 | PIK3CA_GO F_1043 |
| KEAP1_GO F | CDKN2A_G OF_80 | QKI_LOF | TP53_GOF_ 238 | TP53_GOF_ 192 | GATA6_GO F |
| CUL4A_LOF | TNFAIP3_G OF | SMARCB1_ GOF | BRD4_GOF | FLCN_GOF _306 | BRAF_GOF _469 |
| KEAP1_GO F_364 | MYD88_GO F_265 | KEAP1_GO F_364 | JAK1_GOF | BRCA2_GO F | EPHA3_GO F |

(continued)

| Hotspot | | | | | |
|---|---|---|---|---|---|
| cluster1 | cluster2 | cluster3 | cluster4 | cluster5 | cluster6 |
| MAP2K1_G OF _102 | KEAP1_GO F_483 | KLHL6_GOF | BRCA2_GO F | MYCN_LOF | MTOR_GOF _1834 |
| ALOX12B_G OF | TSC1_GOF | CEBPA_LO F | BRAF_GOF _466 | RNF43_GO F | TP53_GOF_ 275 |
| MYD88_GO F_265 | FGF14_GOF | STK11_GOF _163 | SF3B1_GOF _666 | KEAP1_GO F_470 | STK11_GOF _37 |
| ACVR1B_L OF | BRCA2_GO F | FBXW7_GO F_505 | PRKAR1A_ GOF | KEAP1_GO F_544 | TP53_GOF_ 195 |
| SMARCA4_ GOF_1160 | SF3B1_GOF _666 | AKT1_LOF | PTEN_GOF _59 | STK11_GOF _242 | ERCC4_LO F |
| TNFRSF14_ LOF | CCNE1_GO F | MET_GOF_ 3028 | AMER1_GO F_625 | SF3B1_GOF _666 | TP53_GOF_ 236 |
| TP53_GOF_ 278 | PPP2R1A_G OF | MYCN_GOF | PARP1_GO F | CD79A_LOF | ACVR1B_L OF |
| RAD51B_LO F | STK11_GOF _308 | SMARCA4_ GOF_1162 | NFE2L2_GO F_29 | HRAS_GOF _61 | DIS3_GOF_ 458 |
| FLT1_GOF | EGFR_GOF _790 | PIK3CA_GO F_345 | SDHA_GOF _531 | NTRK3_GO F | SMAD4_GO F |
| KEAP1_GO F_493 | CDKN1A_G OF | CD79A_LOF | STK11_GOF _199 | PIK3CA_GO F_1043 | MAP3K1_G OF_949 |
| MDM2_LOF | TP53_GOF_ 298 | PPARG_GO F | JAK3_GOF | RBM10_GO F | BCORL1_G OF_883 |
| PIK3R1_LO F | CDKN2A_G OF_61 | XRCC2_LO F | ACVR1B_L OF | NF1_GOF_1 642 | FH_GOF_47 6 |
| AR_GOF_46 8 | SOX2_LOF | AR_GOF_70 | KDM6A_GO F | CUL4A_LOF | PIK3C2G_G OF _129 |
| SMAD4_GO F_351 | EGFR_GOF _719 | MYD88_GO F_265 | KEAP1_GO F | TP53_GOF_ 159 | TP53_GOF_ 272 |
| POLE_GOF | MAP3K1_G OF_949 | IRF2_GOF | CUL4A_LOF | MLH1_LOF | CDKN2A_G OF_100 |
| CTNNB1_G OF_33 | PALB2_GOF | RAD52_GO F | KEAP1_GO F_364 | NFE2L2_GO F_31 | GABRA6_G OF |
| NBN_GOF_ 680 | IRF4_GOF | PIK3CB_GO F | SMARCA4_ GOF_1243 | CTNNA1_L OF | MAP2K1_G OF_102 |
| CDK4_GOF | STK11_GOF _57 | PIK3CA_GO F_1047 | KEAP1_GO F_186 | DNMT3A_G OF _749 | CDH1_LOF |
| KEAP1_GO F_417 | KEAP1_GO F_470 | AR_GOF_46 5 | EGFR_GOF _771 | STK11_GOF _734 | NRAS_LOF |
| KEAP1_GO F_116 | CHEK2_GO F_392 | TP53_GOF_ 278 | NFE2L2_GO F_77 | ARID1A_GO F_21 | FLT3_GOF |
| ATM_GOF_ 337 | MAP3K1_G OF_5 | AMER1_GO F_385 | RBM10_GO F_503 | STK11_GOF _168 | GNAQ_GOF |
| SDHA_GOF _457 | KEAP1_GO F_153 | ERBB2_GO F_755 | EGFR_GOF _861 | STK11_GOF _256 | KEAP1_GO F_135 |

(continued)

| Hotspot | | | | | |
|---------|---------|---------|---------|---------|---------|
| cluster1 | cluster2 | cluster3 | cluster4 | cluster5 | cluster6 |
| GATA4_GO F | ERBB2_GO F_755 | MSH6_GOF _1088 | TP53_GOF_ 560 | FH_GOF | TP53_GOF_ 285 |
| PDGFRB_G OF | CBFB_LOF | HSD3B1_G OF_75 | MED12_GO F | IRF4_GOF | ATM_GOF |
| FBXW7_GO F_479 | KEAP1_GO F_260 | CHEK1_GO F | RAD51D_G OF | ARID1A_GO F_343 | ZNF703_GO F |
| CTNNB1_G OF_41 | FGFR4_GO F | KEAP1_GO F_244 | PIK3CA_GO F_726 | STK11_GOF _291 | STK11_GOF _163 |
| CDKN1A_G OF | GNAS_GOF _407 | CDKN1A_G OF | KMT2D_GO F_755 | MYCN_GOF _44 | SMARCA4_ GOF_1157 |
| ZNF217_GO F_410 | SDHB_LOF | CDKN2A_G OF_69 | CTCF_LOF | RARA_GOF | RPTOR_GO F |
| BRAF_GOF _464 | PIK3CB_LO F | TP53_GOF_ 272 | ATM_GOF | PTCH1_GO F_48 | KEAP1_GO F_470 |
| TP53_GOF_ 234 | STK11_GOF _221 | BTG1_GOF | NFE2L2_GO F_30 | DNMT3A_G OF_882 | KEAP1_GO F_274 |
| NKX2-1_GOF_234 | BCOR_GOF _1526 | AR_GOF_45 7 | BCOR_GOF _679 | RAD54L_LO F | NOTCH1_G OF |
| FH_GOF_47 6 | STK11_GOF _216 | MTOR_GOF _1834 | STK11_GOF _37 | KEAP1_GO F_523 | KEAP1_GO F_509 |
| KEAP1_GO F_274 | | DDR2_GOF | HRAS_LOF | MITF_GOF | |
| NBN_GOF_ 219 | | KEAP1_GO F_362 | RAD51B_LO F | TYRO3_GO F | |
| PAX5_GOF | | SF3B1_GOF _666 | IRF4_GOF | ARID1A_GO F_515 | |
| TP53_GOF_ 195 | | KEAP1_GO F_470 | EGFR_GOF _746 | GABRA6_G OF | |
| FANCG_GO F | | NOTCH1_G OF | MUTYH_GO F_165 | RB1_GOF_5 76 | |
| NFE2L2_GO F_30 | | STK11_GOF _181 | KDM5C_GO F | | |
| | | KDM5C_GO F_1330 | EGFR_GOF _763 | | |
| | | SMAD4_GO F | BCL6_GOF | | |
| | | RAD51B_LO F | FH_GOF_47 6 | | |
| | | FGF6_GOF | | | |
| | | IDH1_GOF | | | |
| | | MLH1_GOF | | | |
| | | CEBPA_GO F_197 | | | |
| | | CHEK2_GO F_367 | | | |
| | | MAP3K1_G OF | | | |

(continued)

| cluster7 | cluster8 | cluster9 | cluster10 | cluster11 | cluster12 |
|---|---|---|---|---|---|
| NF1_LOF | NF1_LOF | NF1_LOF | STK11_LOF | NF1_LOF | NF1_LOF |
| ATR_LOF | KMT2A_LOF | KMT2A_LOF | NF1_LOF | STK11_LOF | ATR_LOF |
| STK11_LOF | STK11_LOF | BRAF_GOF _600 | BRAF_GOF _600 | BRAF_GOF _600 | STK11_LOF |
| ASXL1_LOF | BRAF_GOF _600 | STK11_LOF | ATR_LOF | ASXL1_LOF | BRAF_GOF _600 |
| BRAF_GOF _600 | ASXL1_LOF | ASXL1_LOF | BRCA2_LOF | STAG2_LOF | ASXL1_LOF |
| ATRX_LOF | PDGFRA_L OF | TSC2_LOF | TSC2_LOF | TSC2_LOF | ATRX_LOF |
| FGF19_LOF | ATR_LOF | FGF19_LOF | ASXL1_LOF | FGF19_LOF | NFKBIA_GO F |
| BRCA2_LOF | FGF19_LOF | PDGFRA_L OF | CDKN2A_G OF _151 | NFE2L2_GO F_24 | FGF19_LOF |
| TSC2_LOF | BRCA2_LOF | MYCN_LOF | STAG2_LOF | CDKN2A_G OF _151 | BRCA2_LOF |
| NKX2-1_LOF | TSC2_LOF | NFKBIA_GO F | FGF19_LOF | QKI_LOF | NKX2-1_LOF |
| NFKBIA_GO F | NFKBIA_GO F | PDK1_LOF | NFE2L2_GO F_24 | TP53_GOF_ 195 | KMT2A_LOF |
| CDKN2A_G OF _151 | PDK1_LOF | CDKN2A_G OF _151 | PDK1_LOF | NKX2-1_LOF | TSC2_LOF |
| KMT2A_LOF | NKX2-1_LOF | WT1_LOF | NKX2-1_LOF | U2AF1_GOF _34 | BRAF_GOF _466 |
| PDK1_LOF | NFE2L2_GO F_24 | TP53_GOF_ 282 | DNMT3A_G OF_882 | NRAS_LOF | STAG2_LOF |
| JAK1_GOF | MYCN_LOF | VEGFA_LO F | TP53_GOF_ 282 | BRCA2_LOF | DNMT3A_G OF_882 |
| NFE2L2_GO F_77 | TP53_GOF_ 331 | NKX2-1_LOF | NFE2L2_GO F_77 | PDK1_LOF | MUTYH_GO F_165 |
| STAG2_LOF | WT1_LOF | ATR_LOF | QKI_LOF | ATRX_LOF | CDKN2A_G OF _151 |
| BRAF_GOF _466 | STAG2_LOF | DNMT3A_G OF_882 | U2AF1_GOF _34 | NFKBIA_GO F | NFE2L2_GO F_24 |
| MUTYH_GO F_165 | TP53_GOF_ 282 | PPP2R1A_G OF | VEGFA_LO F | STK11_GOF _291 | PDK1_LOF |
| TP53_GOF_ 282 | CDKN2A_G OF _151 | STAG2_LOF | MAP3K13_G OF | FANCG_GO F | MYCN_LOF |
| NFE2L2_GO F_24 | QKI_LOF | EGFR_GOF _719 | PIK3C2G_G OF _1088 | DNMT3A_G OF_882 | TP53_GOF_ 376 |
| PTEN_GOF | PPP2R1A_G OF | NFE2L2_GO F_24 | JUN_LOF | NBN_LOF | JAK1_GOF |
| MAP3K1_G OF | EGFR_GOF _719 | U2AF1_GOF _34 | PPP2R1A_G OF | PDGFRA_L OF | NFE2L2_GO F_77 |
| DNMT3A_G OF_882 | U2AF1_GOF _34 | TP53_GOF_ 331 | KEAP1_GO F_272 | PIK3CA_GO F | PAX5_GOF |
| PAX5_GOF | DNMT3A_G OF_882 | NFE2L2_GO F_77 | RAC1_LOF | GATA3_GO F | TP53_GOF_ 282 |

(continued)

| cluster7 | cluster8 | cluster9 | cluster10 | cluster11 | cluster12 |
|----------|----------|----------|-----------|-----------|-----------|
| U2AF1_GOF _34 | GATA3_GO F | CDC73_GO F | TP53_GOF_ 195 | EGFR_GOF _719 | FGF14_GOF |
| TP53_GOF_ 376 | HGF_LOF | BRCA2_LOF | PBRM1_LO F | PPP2R1A_G OF | U2AF1_GOF _34 |
| FGF14_GOF | NRAS_LOF | KEAP1_GO F_260 | TP53_GOF_ 331 | MYCN_GOF | MAP3K1_G OF |
| TP53_GOF_ 244 | PIK3C2G_G OF _1088 | CCNE1_GO F | AKT2_GOF | RB1_GOF | IDH1_GOF |
| MYCN_LOF | FANCG_GO F | TP53_GOF_ 560 | NKX2-1_GOF_234 | JUN_LOF | PIK3CA_GO F_1043 |
| TP53_GOF_ 215 | STK11_GOF _291 | BRAF_GOF _466 | FGF14_GOF | TP53_GOF_ 282 | GATA3_GO F |
| TP53_GOF_ 331 | TP53_GOF_ 159 | TP53_GOF_ 159 | EGFR_GOF _858 | TP53_GOF_ 331 | PTEN_GOF |
| JUN_LOF | MTOR_GOF _1834 | FANCG_GO F | KEAP1_GO F_364 | PIK3C2G_G OF _1088 | TP53_GOF_ 331 |
| GATA3_GO F | VEGFA_LO F | EGFR_GOF _746 | STK11_GOF _37 | TP53_GOF_ 159 | JUN_LOF |
| STK11_GOF _291 | TP53_GOF_ 272 | MTOR_GOF _1834 | JAK1_GOF | KMT2A_LOF | AKT3_GOF |
| EGFR_GOF _747 | JUN_LOF | JUN_LOF | TP53_GOF_ 285 | TNFAIP3_G OF | TP53_GOF_ 215 |
| BRAF_GOF _469 | MYCN_GOF | HGF_LOF | EGFR_GOF _747 | KEAP1_GO F_417 | EGFR_GOF _719 |
| EGFR_GOF _719 | PIK3CA_GO F_1043 | STK11_GOF _291 | MAP3K1_G OF | NFE2L2_GO F_77 | KDM5C_GO F_1330 |
| PPP2R1A_G OF | AR_GOF_49 3 | PIK3C2G_G OF _1088 | EGFR_GOF _719 | STK11_GOF _84 | PPARG_LO F |
| IDH1_GOF | ERBB2_GO F_755 | KDM5C_GO F_1546 | MTOR_GOF _1834 | PDCD1LG2_ GOF | STK11_GOF _291 |
| AKT3_GOF | CEBPA_LO F | AR_GOF_69 | KMT2A_LOF | KLHL6_GOF | PBRM1_LO F |
| CDKN1A_G OF | BRAF_GOF _466 | EGFR_GOF _858 | BRAF_GOF _466 | PBRM1_LO F | BRAF_GOF _469 |
| PPARG_LO F | SF3B1_GOF _666 | ZNF703_GO F | CDKN1A_G OF | NTRK2_LOF | EGFR_GOF _746 |
| PBRM1_LO F | PTEN_GOF _59 | MYCN_GOF | CTNNA1_L OF | TP53_GOF_ 298 | EGFR_GOF _747 |
| KDM5C_GO F_1330 | KEAP1_GO F_364 | BRCA2_GO F | NTRK2_LOF | POLD1_GO F | RAD51B_LO F |
| AR_GOF_45 7 | STK11_GOF _221 | GATA3_GO F | ATRX_LOF | EGFR_GOF _746 | AR_GOF_45 7 |
| PIK3CA_GO F_1043 | ARID1A_GO F_21 | FGF6_GOF | MUTYH_GO F_165 | VEGFA_LO F | PPP2R1A_G OF |
| GATA6_GO F | TNFRSF14_ LOF | IRF4_GOF | SF3B1_GOF _666 | CDK4_GOF | ACVR1B_L OF |

41

(continued)

| cluster7 | cluster8 | cluster9 | cluster10 | cluster11 | cluster12 |
|---|---|---|---|---|---|
| EGFR_GOF _746 | MAP2K1_G OF_102 | STK11_GOF _84 | FGFR4_GO F | CD79B_GO F | GATA6_GO F |
| ACVR1B_L OF | STK11_GOF _251 | TP53_GOF_ 376 | ERCC4_LO F | MYD88_GO F_265 | PARP1_GO F |
| ERCC4_LO F | TP53_GOF_ 278 | KEAP1_GO F_272 | TP53_GOF_ 159 | STK11_GOF _37 | SMAD4_GO F |
| PIK3C2G_G OF _129 | ERBB2_GO F_776 | MAP3K1_G OF | MET_GOF_ 2888 | ERBB3_LOF | NRAS_LOF |
| PARP1_GO F | NFE2L2_GO F_77 | KEAP1_GO F_332 | GNAQ_GOF | KEAP1_GO F_272 | PIK3C2G_G OF _129 |
| RAD51B_LO F | KEAP1_GO F | TNFAIP3_G OF | FH_GOF | BRD4_GOF | ERCC4_LO F |
| TP53_GOF_ 236 | RB1_GOF | PIK3CA_GO F_1043 | ERBB2_GO F_776 | MAP3K1_G OF | MTOR_GOF _1834 |
| MTOR_GOF _1834 | AKT3_GOF | KEAP1_GO F_234 | PIK3CA_GO F_1043 | TP53_GOF_ 272 | TP53_GOF_ 244 |
| TP53_GOF_ 195 | MYD88_GO F_265 | KEAP1_GO F_364 | APC_GOF | GATA6_GO F | CDKN1A_G OF |
| MAP3K1_G OF_949 | KEAP1_GO F_509 | SMARCB1_ GOF | AR_GOF_46 8 | JAK1_GOF | MAP2K1_G OF_102 |
| SMAD4_GO F | ATM_GOF_ 337 | CEBPA_LO F | CDC73_GO F | FGF14_GOF | TP53_GOF_ 285 |
| MAP2K1_G OF _102 | SMARCA4_ GOF_1160 | KLHL6_GOF | NFKBIA_GO F | AKT3_GOF | GNAQ_GOF |
| CDH1_LOF | CUL4A_LOF | QKI_LOF | XRCC2_LO F | AR_GOF_69 | MAP3K1_G OF_949 |
| NRAS_LOF | FLT1_GOF | AKT1_LOF | STK11_GOF _220 | ACVR1B_L OF | EPHA3_GO F |
| RPTOR_GO F | EGFR_GOF _746 | PPARG_GO F | IDH1_GOF | AR_GOF_46 9 | KEAP1_GO F_274 |
| CDKN2A_G OF_100 | PIK3R1_LO F | ATRX_GOF | KEAP1_GO F_332 | CDKN1A_G OF | ATM_GOF |
| KEAP1_GO F_409 | KEAP1_GO F_430 | FBXW7_GO F_505 | NFE2L2_GO F_29 | BRAF_GOF _469 | RPTOR_GO F |
| TP53_GOF_ 285 | ALOX12B_G OF | TP53_GOF_ 195 | FANCL_LOF | PTCH1_GO F | KEAP1_GO F_409 |
| GNAQ_GOF | KEAP1_GO F_234 | MET_GOF_ 3028 | FBXW7_GO F_505 | KEAP1_GO F_332 | PTEN_GOF _165 |
| TP53_GOF_ 275 | APC_GOF | STK11_GOF _163 | KEAP1_GO F_234 | NFE2L2_GO F_27 | TP53_GOF_ 275 |
| DIS3_GOF_ 458 | RAF1_GOF | IRF2_GOF | CHEK2_GO F | RET_GOF_5 11 | SMAD2_LO F |
| GABRA6_G OF | PARP1_GO F | AR_GOF_45 7 | MAP2K1_G OF _121 | DIS3_GOF_ 458 | CDH1_LOF |
| ATM_GOF | TP53_GOF_ 195 | SMARCA4_ GOF_1162 | FAS_GOF | AR_GOF_59 8 | FANCL_LOF |
| STK11_GOF _37 | NFE2L2_GO F_30 | ERBB2_GO F_755 | RPTOR_GO F | MYD88_GO F | FGF6_GOF |

(continued)

| cluster7 | cluster8 | cluster9 | cluster10 | cluster11 | cluster12 |
|---|---|---|---|---|---|
| BCORL1_G OF_883 | KEAP1_GO F_272 | CREBBP_G OF_1472 | AKT3_GOF | RAD51C_G OF_21 | FLT3_GOF |
| ZNF703_GO F | CD79A_LOF | | SMAD4_GO F_351 | BRAF_GOF _466 | TP53_GOF_ 236 |
| EGFR_GOF _858 | MDM2_LOF | | KEAP1_GO F_155 | STK11_GOF _242 | TP53_GOF_ 195 |
| EPHA3_GO F | KEAP1_GO F_493 | | IRS2_GOF | ERBB2_GO F_776 | CDKN2A_G OF_100 |
| PTEN_GOF _165 | CTNNB1_G OF_33 | | KEAP1_GO F_274 | EGFR_GOF _858 | MEN1_GOF |
| GNAS_GOF _415 | STK11_GOF _464 | | PDGFRA_L OF | AKT1_GOF_ 17 | CEBPA_LO F |
| KEAP1_GO F_470 | | | SMAD2_GO F_464 | AKT2_GOF | POLD1_GO F |
| KEAP1_GO F_274 | | | TNFAIP3_G OF | TP53_GOF_ 673 | FH_GOF_47 6 |
| KEAP1_GO F_509 | | | CD79A_LOF | CBL_GOF_1 096 | DIS3_GOF_ 458 |
| NTRK2_LOF | | | MITF_GOF | BCOR_GOF _679 | KMT2A_GO F_53 |
| FH_GOF_47 6 | | | TP53_GOF_ 342 | PALB2_GOF | CDK8_GOF |
| CDKN2A_G OF_61 | | | HRAS_GOF _13 | CDKN2A_G OF_69 | PIK3CA_GO F_545 |
| MYCN_GOF | | | KEAP1_GO F_362 | KEAP1_GO F_450 | STK11_GOF _168 |
| POLD1_GO F | | | BCL2_LOF | CDKN2A_G OF_61 | KEAP1_GO F |
| FAS_GOF | | | NTRK3_GO F_610 | BTG1_GOF | STK11_GOF _176 |
| SMAD2_LO F | | | SMARCB1_ GOF | VHL_GOF | KEAP1_GO F_450 |
| | | | FH_GOF_47 6 | KEAP1_GO F_116 | GNAS_GOF _415 |
| | | | RB1_GOF_5 76 | | CDKN2A_G OF_83 |
| | | | CEBPA_LO F | | MDM2_LOF |
| | | | KLHL6_GOF | | KEAP1_GO F_236 |
| | | | AR_GOF_46 7 | | STK11_GOF _163 |
| | | | BCORL1_G OF_94 | | IDH2_GOF_ 140 |

**Table** 3B: Cluster information for hotspot mutations - updated following additional analysis

| Hotspot | | | | | |
| --- | --- | --- | --- | --- | --- |
| cluster1 | cluster2 | cluster3 | cluster4 | clusters | cluster6 |
| CDKN2B_LFM | CDKN2B_LFM | FGF10_HFM | CDKN2B_LFM | BRIP1_LFM | CDKN2B_LFM |
| FGF10_HFM | FGF10_HFM | BRIP1_LFM | FLT1_LFM | CDKN2B_LFM | BRCA2_LFM |
| BRCA2_LFM | BRIP1_LFM | BRCA2_LFM | BRIP1_LFM | PIK3CA_HFM | BRIP1_LFM |
| PTEN_LFM | DNMT3A_HFM_ 771 | CDKN2B_LFM | FGF10_HFM | BRCA2_LFM | MAP3K13_LFM |
| FLT1_LFM | TP53_HFM 331 | FLT1_LFM | KEAP1_LFM | FLT1_LFM | FLT1_LFM |
| MYC_LFM | ZNF703_HFM | KEAP1_LFM | ZNF703_HFM | FGF10_HFM | BRAF_HFM 469 |
| IKBKE_LFM | MAP3K13_LFM | TP53_HFM_331 | STK11_LFM | TSC1_LFM | TERT_HFM_ |
| BRIP1_LFM | NOTCH3_LFM | BRAF_HFM_469 | BRAF_HFM_469 | FAS_LFM | BRAF_HFM_600 |
| U2AF1_HFM_34 | MLH1_LFM | RARA_HFM | FAS_LFM | NBN_LFM | TP53_HFM_331 |
| RARA_HFM | RARA_HFM | BARD1_LFM | BRCA2_LFM | U2AF1_HFM_34 | BCL6_LFM |
| ASXL1_LFM | TSC1_LFM | BRAF_HFM 600 | PIK3CA_LFM | DNMT3A_HFM_ 771 | JAK1_HFM |
| DNMT3A_HFM_ 771 | PTEN_LFM | PIK3CA_LFM | MLH1_LFM | BRAF_HFM 600 | ALOX12B_LFM |
| ZNF703_HFM | IKBKE_LFM | PIK3C2G_HFM | TSC1_LFM | TP53_HFM_331 | EGFR_HFM_746 |
| TERT_HFM_ | BRCA2_LFM | ARFRP1_HFM | NBN_LFM | BTG1_LFM | MLH1_LFM |
| MAP3K13_LFM | EGFR_HFM_746 | AR_LFM | TP53_HFM_331 | NFE2L2_HFM_2 4 | FGFR2_LFM |
| BRAF_HFM_600 | MYC_LFM | MYC_LFM | RARA_HFM | BRAF_HFM_469 | U2AF1_HFM 34 |
| MAP3K1_LFM | BCL6_LFM | TERT_HFM | PIK3CA_HFM | ALOX12B_LFM | CTNNA1_LFM |
| ALOX12B_LFM | FAS_LFM | IKBKE_LFM | BARD1_LFM | NF2_HFM | DNMT3A_HFM_ 771 |
| BRAF_HFM 469 | PDGFRA_LFM | PTEN_LFM | PDGFRA_LFM | SOX9_LFM | PIK3C2GHFM |
| EZH2_LFM | PDGFRB_LFM | DNMT3A_HFM_ 771 | U2AF1_HFM_34 | ZNF703_HFM | FGF10_HFM |
| JAK2_LFM | ALOX12B_LFM | DDR2_HFM | PIK3C2G HFM | BARD1_LFM | XRCC2_LFM |
| BCL6_LFM | U2AF1_HFM_34 | TSC1_LFM | ALOX12B_LFM | PIK3C2G_HFM | NF1_HFM |
| XRCC2_LFM | TERT_HFM_ | XRCC2_LFM | BCL6_LFM | TP53_HFM_560 | PIK3CA_HFM |
| EGFR_HFM_746 | MAP3K1_LFM | ZNF703_HFM | XRCC2_LFM | RARA_HFM | DDR2_HFM |
| JAK1_HFM | NF2_HFM | MLH1_LFM | EGFR_HFM 746 | MAP3K13_LFM | BARD1_LFM |

44

(continued)

| Hotspot | | | | | |
|---|---|---|---|---|---|
| cluster1 | cluster2 | cluster3 | cluster4 | clusters | cluster6 |
| BARD1_LFM | XRCC2_LFM | ALOX12B_LFM | NFE2L2_HFM_2 4 | EGFR_HFM 746 | STK11_HFM |
| TSC1_LFM | DDR2_HFM | JAK2_LFM | MAP3K13_LFM | MLH1_LFM | NBN_LFM |
| PIK3C2G_HFM | EZH2_LFM | FGFR2_HFM | BRAF_HFM_600 | IKBKE_LFM | RARA_HFM |
| FGF19_LFM | FANCC_HFM | PIK3CA_HFM | FGFR2_LFM | MYC_LFM | FAS_LFM |
| NOTCH3_LFM | PIK3CA_HFM | AKT2_HFM | TP53_HFM_560 | TP53_HFM_173 | NF2_HFM |
| STK11_LFM | FGF19_LFM | JAK1_HFM | ARFRP1_HFM | ASXL1_HFM | TSC1_LFM |
| PARP2_LFM | PIK3CA_LFM | ASXL1_LFM | KLHL6_HFM | FOXL2_LFM | NFE2L2_HFM_2 4 |
| PIK3CA_HFM_5 42 | BRAF_HFM_600 | NBN_LFM | DDR2_HFM | CTNNA1_LFM | TNFRSF14_LFM |
| SMAD4_LFM | STK11_LFM | MAP3K13_LFM | DNMT3A_HFM_771 | FUBP1_LFM | TP53_HFM_173 |
| TP53_HFM_331 | TP53_HFM_560 | NFE2L2_HFM_2 4 | GNAS_LFM | SMO_LFM | ASXL1_LFM |
| PIK3CA_HFM | FLT1_LFM | CTNNA1_LFM | TSC2_LFM | FANCC_HFM | PAX5_HFM |
| MLH1_LFM | HSD3B1_LFM | PIK3CA_HFM_5 42 | BAP1_LFM | GNAS_LFM | MAP3K1_LFM |
| TP53_HFM_282 | NFE2L2_HFM_2 4 | KLHL6_HFM | CSF1R_LFM | BCL6_LFM | IKBKE_LFM |
| CDC73_HFM | PARP2_LFM | TP53_HFM_560 | MAP3K1_LFM | FGFR2_LFM | CHEK2_HFM |
| KEAP1_LFM | TSC2_LFM | STK11_LFM | TERT_HFM | ALK_HFM | GNA11_LFM |
| PAX5_HFM | TNFRSF14_LFM | TP53_HFM_280 | BTG1_LFM | TNFRSF14_LFM | MITF_LFM |
| NF1_HFM | NBN_LFM | TSC2_LFM | NF2_LFM | JAK1_HFM | PDCD1LG2_HF M |
| FGFR2_HFM | AKT2_HFM | FOXL2_LFM | FGF19_HFM | TP53_HFM 920 | FANCC_HFM |
| ARFRP1_HFM | JAK1_HFM | GABRA6_HFM | NF2_HFM | XRCC2_LFM | AXIN1_LFM |
| FOXL2_LFM | GATA6_HFM | BCL2L2_HFM | JAK1_HFM | NF2_LFM | ERBB4_HFM |
| TP53_HFM_173 | CDC73_HFM | NF1_HFM | VHL_LFM | ERBB4_HFM | HSD3B1_LFM |
| TP53_HFM_159 | GNAS_LFM | ALK_HFM | NF1_HFM | DDR2_HFM | ERBB2_LFM |
| FAS_LFM | BRAF_HFM_469 | NFE2L2_HFM_7 9 | FANCC_HFM | FGF19_LFM | ASXL1_HFM |
| NF2_HFM | CCNE1_LFM | FANCC_HFM | AKT2_HFM | TP53_HFM_294 | TP53_HFM_560 |
| NFKBIA_HFM | NF1_HFM | CDK6_LFM | STK11_HFM_24 2 | VHL LFM | CCNE1_HFM |

(continued)

| Hotspot | | | | | |
|---|---|---|---|---|---|
| **cluster1** | **cluster2** | **cluster3** | **cluster4** | **clusters** | **cluster6** |
| CTNNA1_LFM | ARFRP1_HFM | BAP1_LFM | ARAF_HFM_181 | PRKAR1A HFM | FGF19_LFM |
| FGFR2_LFM | GNA13_LFM | MAP3K1_LFM | HSD3B1_LFM | GABRA6_LFM | TERC_HFM |
| TERC_HFM | IGF1IR_HFM | GID4 LFM | CUL4A_HFM | EPHA3_HFM | PARP2_LFM |
| ERRFI1_LFM | GID4_LFM | TP53_HFM_154 | TP53_HFM_154 | GNA11_HFM | CDC73_HFM |
| ALK_HFM | AXIN1_LFM | MERTK_LFM | FGFR2_HFM | BAP1_LFM | STK11_HFM_19 4 |
| TP53_HFM_154 | SMAD4_LFM | HSD3B1_LFM | CTNNA1_LFM | CDC73_HFM | NFE2L2_HFM_2 9 |
| ERBB4_HFM | PRDM1_LFM | AKT3_HFM | AKT3_HFM | FH_HFM | GNAS_LFM |
| CSF1R_LFM | JAK3_HFM | CD79A_LFM | ERBB2_HFM_77 5 | TP53 HFM 783 | PIK3CA_HFM_1 047 |
| PDCD1LG2_HF M | GNA11_HFM | FAS_LFM | PAX5_HFM | ARAF_HFM_181 | AKT2_HFM |
| SDHC HFM | NFE2L2_HFM_7 9 | BCL6_LFM | IKBKE_LFM | H3F3A_HFM | TP53_HFM_159 |
| STK11_HFM_24 2 | TP53_HFM_173 | EGFR_HFM_746 | ASXL1_HFM | PARP2_LFM | ALK_HFM |
| JUN_LFM | FGFR2_LFM | ROS1_HFM | PRKAR1A_HFM | BCL2L2_LFM | FOXL2_LFM |
| | PIK3C2G HFM | PDGFRA_LFM | AR_LFM | LYN_HFM | GATA3_HFM |
| | TP53_HFM_280 | TP53_HFM_173 | NKX2-1_LFM | CDK12_HFM | TP53_HFM_920 |
| | CHEK2_HFM | BCL6_HFM | TP53_HFM_280 | GSK3B HFM | GNA13_LFM |
| | AKT3_HFM | ERBB4_HFM | MYC_LFM | ERRFI1_LFM | FGFR2_HFM |
| | | BTK LFM | TP53_HFM_152 | CUL4A LFM | |
| | | SDHA LFM | TP53_HFM_249 | TP53_HFM_135 | |
| | | PDCD1LG2_ HF M | GABRA6_HFM | SRC_LFM | |
| | | PRKAR1A_HFM | CHEK2_HFM | PIK3CA LFM | |
| | | ATR_LFM | PARP2_LFM | TP53_HFM 215 | |
| | | TP53_HFM_920 | KEAP1_HFM_32 0 | FGF14_HFM | |
| | | BTG1 LFM | CHEK2_HFM_15 7 | | |
| | | TP53_HFM_249 | TP53_HFM_173 | | |
| | | ERBB2_LFM | TP53_HFM_294 | | |

| Hotspot | | | | | |
|---|---|---|---|---|---|
| **cluster1** | **cluster2** | **cluster3** | **cluster4** | **clusters** | **cluster6** |
| | | CDKN2A_LFM | | | |
| | | MAP3K13_HFM | | | |
| | | FGF19_LFM | | | |
| | | GATA6 LFM | | | |
| **cluster7** | **cluster8** | **cluster9** | **cluster10** | **cluster11** | **cluster12** |
| CDKN2B_LFM | FGF10_HFM | BRCA2_LFM | CDKN2B_LFM | CDKN2B_LFM | CDKN2B_LFM |
| BRCA2_LFM | CDKN2B_LFM | CDKN2B_LFM | FGF10_HFM | BRCA2_LFM | FGF10_HFM |
| BRIP1_LFM | FLT1_LFM | FLT1_LFM | BRCA2_LFM | BRIP1_LFM | DNMT3A_HFM_ 771 |
| TERT_HFM_ | BRIP1_LFM | FGF10_HFM | FLT1_LFM | TERT_HFM_ | BRIP1_LFM |
| FLT1_LFM | BRCA2_LFM | BRIP1_LFM | BRIP1_LFM | FGF10_HFM | NOTCH3_LFM |
| PIK3C2G_HFM | MYC_LFM | ASXL1_LFM | IKBKE_LFM | FLT1_LFM | RARA_HFM |
| BARD1_LFM | BRAF_HFM_46 9 | PIK3C2G_HFM | MYC_LFM | TP53_HFM_331 | FLT1_LFM |
| FGF10_HFM | MLH1_LFM | BARD1_LFM | PTEN_LFM | MLH1_LFM | TP53_HFM_331 |
| DDR2_HFM | ASXL1_LFM | MLH1_LFM | ALOX12B_LFM | MAP3K13_LFM | ZNF703_HFM |
| ALOX12B_LFM | BRAF_HFM_60 0 | DNMT3A_HFM_ 771 | MAP3K13_LFM | DNMT3A_HFM_ 771 | MAP3K13_LFM |
| BCL6_LFM | MAP3K13_LFM | FOXL2_LFM | DNMT3A_HFM_ 771 | FAS_LFM | U2AF1_HFM_34 |
| FAS_LFM | PTEN_LFM | BRAF_HFM_46 9 | BCL6_LFM | ZNF703_HFM | TERT_HFM_ |
| TP53_HFM_331 | TSC1_LFM | TERT_HFM_ | ZNF703_HFM | BRAF_HFM_46 9 | BCL6_LFM |
| ASXL1_LFM | BARD1_LFM | BCL6_LFM | U2AF1_HFM _3 4 | ALOX12B_LFM | PDGFRB_LFM |
| TSC1_LFM | TERT_HFM_ | ALOX12B_LFM | ASXL1_LFM | XRCC2_LFM | IKBKE_LFM |
| BRAF_HFM_46 9 | AR_LFM | PIK3CA_HFM | JAK2_LFM | EGFR_HFM_74 6 | ALOX12B_LFM |
| XRCC2_LFM | DDR2_HFM | BRAF_HFM_60 0 | BRAF_HFM_60 0 | NBN_LFM | TSC1_LFM |
| PIK3CA_HFM | DNMT3A_HFM_ 771 | MAP3K13_LFM | TERT_HFM_ | TSC1_LFM | MLH1_LFM |
| BRAF_HFM_60 0 | TP53_HFM_331 | TP53_HFM_331 | RARA_HFM | U2AF1_HFM_3 4 | PTEN_LFM |
| HSD3B1_LFM | FOXL2_LFM | XRCC2_LFM | BARD1_LFM | BARD1_LFM | DDR2_HFM |

EP 4 515 000 B1

47

(continued)

| cluster7 | cluster8 | cluster9 | cluster10 | cluster11 | cluster12 |
|---|---|---|---|---|---|
| NF2_HFM | PIK3CA_HFM | RARA_HFM | TP53_HFM_331 | FANCC_HFM | XRCC2_LFM |
| ARFRP1_HFM | ALOX12B_LFM | NF2_HFM | TSC1_LFM | BRAF_HFM_60 0 | PIK3CA_HFM |
| DNMT3A_HFM_771 | RARA_HFM | NFE2L2_HFM_ 24 | KEAP1_LFM | PARP2_LFM | EGFR_HFM_746 |
| SOX2_HFM | IKBKE_LFM | MYC_LFM | MAP3K1_LFM | TP53_HFM_173 | FAS_LFM |
| TP53_HFM_173 | NBN_LFM | FGF19_LFM | PIK3C2G_HFM | FGFR2_LFM | BRCA2_LFM |
| ZNF703_HFM | U2AF1_HFM_3 4 | JAK1_HFM | STK11_LFM | PIK3CA_LFM | BRAF_HFM_600 |
| U2AF1_HFM_3 4 | PIK3CA_LFM | DDR2_HFM | EZH2_LFM | AXIN1_LFM | MAP3K1_LFM |
| RARA_HFM | PIK3C2G_HFM | AR_LFM | JAK1_HFM | HSD3B1_LFM | PIK3CA_LFM |
| NFE2L2_HFM_ 24 | CTNNA1_LFM | TP53_HFM_173 | BRAF_HFM_46 9 | EZH2_LFM | FANCC_HFM |
| NOTCH3_LFM | EGFR_HFM_74 6 | TSC1_LFM | FGF19_LFM | BCL6_LFM | STK11_LFM |
| FGF19_LFM | XRCC2_LFM | U2AF1_HFM_3 4 | EGFR_HFM_74 6 | NF1_HFM | NF2_HFM |
| EGFR_HFM_74 6 | NF2_HFM | MAP3K1_LFM | XRCC2_LFM | TNFRSF14_LF M | FGF19_LFM |
| TP53_HFM_560 | ZNF703_HFM | SOX2_HFM | MLH1_LFM | PDGFRB_LFM | PARP2_LFM |
| MLH1_LFM | SMAD4_LFM | NF2_LFM | PIK3CA_HFM | ASXL1_LFM | BRAF_HFM_469 |
| AKT3_HFM | CCNE1_HFM | SMAD4_LFM | PAX5_HFM | CHEK2_HFM | MYC_LFM |
| FANCC_HFM | MAP3K1_LFM | FGFR2_LFM | PARP2_LFM | NF2_LFM | PDGFRA_LFM |
| SOX9_LFM | FAS_LFM | PDCD1LG2_HF M | FGFR2_HFM | NFE2L2_HFM_ 24 | NFE2L2_HFM_2 4 |
| EZH2_LFM | STK11_HFM_53 | EGFR_HFM_74 6 | NF1_HFM | CDC73_HFM | NBN_LFM |
| NF2_LFM | PAX5_HFM | TP53_HFM_152 | SMAD4_LFM | ERBB3_HFM | TP53_HFM_560 |
| MAP3K13_LFM | TNFRSF14_LF M | ALK_HFM | NOTCH3_LFM | NOTCH3_LFM | TSC2_LFM |
| AR_LFM | BCL6_LFM | ERBB4_HFM | TP53_HFM_282 | RARA_HFM | GATA6_HFM |
| CEBPA_LFM | HSD3B1_LFM | EZH2_LFM | PIK3CA_HFM_5 42 | CTNNA1_LFM | EZH2_LFM |
| PIK3CA_HFM_5 42 | STK11_LFM | TP53_HFM_560 | TP53_HFM_159 | FH_HFM | AKT2_HFM |
| GID4_LFM | TP53_HFM_173 | CCNE1_HFM | FOXL2_LFM | KLHL6_HFM | HSD3B1_LFM |
| TSC2_LFM | JAK1_HFM | WT1_HFM | FAS_LFM | PIK3C2G_HFM | JAK1_HFM |
| TP53_HFM_294 | EZH2_LFM | FAS_LFM | TERC_HFM | MAP3K1_LFM | TNFRSF14_LFM |

(continued)

| cluster7 | cluster8 | cluster9 | cluster10 | cluster11 | cluster12 |
|---|---|---|---|---|---|
| CDK12_HFM | ERBB4_HFM | ZNF703_HFM | ARFRP1_HFM | ARFRP1_HFM | GNAS_LFM |
| FUBP1_LFM | IGF1R_HFM | CTNNA1_LFM | NFKBIA_HFM | JAK1_HFM | CCNE1_LFM |
| PARP2_LFM | WT1_HFM | AKT3_HFM | CDC73_HFM | CSF1R_LFM | CDC73_HFM |
| JAK2_LFM | MITF_LFM | MEF2B_LFM | CSF1R_LFM | CEBPA_LFM | GNA11_HFM |
| TNFRSF14_LF M | RAD51C_LFM | EP300_HFM | TP53_HFM_173 | TP53_HFM_560 | BARD1_LFM |
| NFE2L2_HFM_79 | NFE2L2_HFM_24 | TP53_HFM_920 | CTNNA1_LFM | FOXL2_LFM | AXIN1_LFM |
| CTNNA1_LFM | PDCD1LG2_HF M | CD79A_LFM | ERRFI1_LFM | TEK_LFM | PAX5_HFM |
| CD79A_LFM | SOX9_HFM | IKBKE_LFM | ERBB4_HFM | NF2_HFM | PIK3C2G_HFM |
| KMT2A_LFM | TP53_HFM_294 | IGF1R_LFM | NF2_HFM | STK11_HFM | JAK3_HFM |
| PRKAR1A_HFM | KEAP1_LFM | IDH1_LFM | TP53_HFM_154 | STK11_HFM_53 | TP53_HFM_294 |
| ALK_HFM | GATA3_HFM | NBN_LFM | STK11_HFM_24 2 | PDGFRA_LFM | CSF1R_LFM |
| MAP3K1_LFM | FGFR2_HFM | TNFRSF14_LF M | ERBB2_HFM_7 75 | FGFR2_HFM | GID4_LFM |
| NBN_LFM | ERBB3_HFM | PTEN_LFM | PRKAR1A_HFM | NFE2L2_HFM_28 | AKT3_HFM |
| NFE2L2_HFM_29 | PBRM1_LFM | | NFE2L2_HFM_79 | PIK3CA_HFM | TP53_HFM_272 |
| TP53_HFM_154 | CUL3_LFM | | HSD3B1_LFM | GATA6_HFM | TP53_HFM_280 |
| IKBKE_LFM | ROS1_HFM | | JUN_LFM | TP53_HFM_280 | TP53_HFM_135 |
| CCNE1_HFM | | | ALK_HFM | CUL3_LFM | IGF1R_HFM |
| CCND2_LFM | | | TP53_HFM_920 | MITF_LFM | DIS3_HFM |
| SMAD4_LFM | | | RAD51C_LFM | DDR2_HFM | CHEK2_HFM |
| TP53_HFM_159 | | | | | NF1_HFM |
| CDK6_LFM | | | | | ARFRP1_HFM |
| CSF1R_LFM | | | | | GNA13_LFM |
| MYC_LFM | | | | | |
| IGF1R_HFM | | | | | |
| CHEK2_HFM_1 57 | | | | | |
| PIK3CA_HFM_1 047 | | | | | |

| cluster7 | cluster8 | cluster9 | cluster10 | cluster11 | cluster12 |
|---|---|---|---|---|---|
| KEAP1_HFM_3 20 | | | | | |
| GABRA6_LFM | | | | | |
| BCL2L2_HFM | | | | | |
| TP53_HFM_135 | | | | | |
| FGFR2_HFM | | | | | |

**Table 4A:** Overlap between binary, GOF/LOF, hotspot and overall mutations

| Feature popularity within 12 models | | | |
|---|---|---|---|
| **Binary Overlap** | **GOF/LOF Overlap** | **Hotspot Overlap** | **Overlap Between 36 models** |
| NF1 | NF1_LOF | NF1_LOF | NF1 |
| STK11 | STK11_LOF | STK11_LOF | STK11 |
| TSC2 | ASXL1_LOF | ASXL1_LOF | TSC2 |
| BRCA2 | FGF19_LOF | KMT2A_LOF | BRCA2 |
| BRAF | TSC2_LOF | NFKBIA_GOF | BRAF |
| STAG2 | BRAF_GOF | BRAF_GOF_600 | STAG2 |
| U2AF1 | IDH1_GOF | TSC2_LOF | U2AF1 |
| PDK1 | STAG2_LOF | FGF19_LOF | PDK1 |
| ATR | BRCA2_LOF | NKX2-1_LOF | |
| | PDK1_LOF | BRCA2_LOF | |
| | U2AF1_GOF | CDKN2A_GOF_151 | |
| | NKX2-1_LOF | PDK1_LOF | |
| | PPP2R1A_GOF | TP53_GOF_282 | |
| | | NFE2L2_GOF_24 | |
| | | U2AF1_GOF_34 | |
| | | EGFR_GOF_719 | |
| | | PPP2R1A_GOF | |
| | | DNMT3A_GOF_882 | |
| | | STAG2_LOF | |

**Table 4B:** Overlap between binary, GOF/LOF, hotspot and overall mutations - updated following additional analysis

| Feature popularity within 12 models | | | |
|---|---|---|---|
| **Binary Overlap** | **GOF/LOF Overlap** | **Hotspot Overlap** | **Overlap Between 36 models** |
| STK11 | BRAF_HFM | CDKN2B_LFM | BRIP1 |
| BRIP1 | BRIP1_LFM | FGF10_HFM | FLT1 |
| CDKN2B | FGF10_HFM | BRCA2_LFM | FGF10 |
| FLT1 | NF1_HFM | FLT1_LFM | BRAF |
| FGF10 | STK11_HFM | BRIP1_LFM | |
| BRAF | MAP3K13_LFM | RARA_HFM | |
| ASXL1 | NOTCH3_LFM | DNMT3A_HFM_771 | |
| HRAS | ALOX12B_LFM | MAP3K13_LFM | |
| IDH1 | U2AF1_HFM | BRAF_HFM_600 | |
| BARD1 | RARA_HFM | ALOX12B_LFM | |
| BRCA2 | MLH1_LFM | BRAF_HFM_469 | |
| U2AF1 | FLT1_LFM | BCL6_LFM | |
| CTNNA1 | MAP3K1_LFM | XRCC2_LFM | |
| | MYC_LFM | EGFR_HFM_746 | |
| | CTNNA1_LFM | TSC1_LFM | |

(continued)

| Feature popularity within 12 models | | | |
|---|---|---|---|
| **Binary Overlap** | **GOF/LOF Overlap** | **Hotspot Overlap** | **Overlap Between 36 models** |
|  | NBN_LFM | PIK3C2G_HFM |  |
|  | ASXL1_LFM | TP53_HFM_331 |  |
|  |  | PIK3CA_HFM |  |
|  |  | MLH1_LFM |  |
|  |  | FAS_LFM |  |

**Table 5A:** Overlap between all inputs

| **Overlap Between all Inputs** |
|---|
| NF1 |
| STK11 |
| TSC2 |
| BRCA2 |
| BRAF |
| ATRX |
| STAG2 |
| U2AF1 |
| PDK1 |
| ATR |
| ASXL1 |
| ERCC4 |
| PAX5 |
| CTNNA1 |
| CD79A |
| TSC1 |
| NRAS |
| RARA |
| PDCD1LG2 |
| NBN |
| PDGFRB |
| PDGFRA |
| CCNE1 |
| JUN |
| IDH1 |
| CDK4 |
| NKX2-1 |
| PPP2R1A |
| FH |
| MDM2 |

(continued)

| Overlap Between all Inputs |
|---|
| AKT1 |
| NTRK2 |
| FANCG |
| QKI |
| BRD4 |
| CDKN1A |
| CEBPA |
| FANCL |
| SMARCA4 |

**Table 5B:** Overlap between all inputs - updated following additional analysis

| Overlap Between all Inputs |
|---|
| STK11 |
| BRIP1 |
| CDKN2B |
| FLT1 |
| FGF10 |
| BRAF |
| ASXL1 |
| TSC2 |
| IKBKE |
| PBRM1 |
| IDH1 |
| BARD1 |
| BRCA2 |
| MLH1 |
| U2AF1 |
| CEBPA |
| TSC1 |
| CSF1R |
| GABRA6 |
| RARA |
| CDK12 |
| NKX2-1 |
| CD79A |
| SOX9 |
| NOTCH3 |
| SOX2 |
| CTNNA1 |

(continued)

| Overlap Between all Inputs |
|---|
| EZH2 |
| FAS |
| ALOX12B |
| TERT |
| FOXL2 |
| GNA13 |
| CUL3 |
| CCNE1 |
| HSD3B1 |
| MYC |
| JAK1 |
| PIK3CA |
| NFE2L2 |
| PDGFRB |
| FANCC |
| XRCC2 |
| PDGFRA |
| GID4 |
| DDR2 |
| MAP3K1 |
| ZNF703 |
| AKT2 |
| ALK |
| BAP1 |
| ERRFI1 |
| NBN |
| PAX5 |
| TERC |
| CDC73 |
| PDCD1LG2 |
| ARFRP1 |

**Table 6A:** Coefficient information for the durable response group

| Durable Response | | | | |
|---|---|---|---|---|
| Gene_Input | GeneName | Contribution To Response | Input | Percent In population (n = 8768) |
| TSC2 | TSC2 | 1.559808 | binary | 5.132299 |
| U2AF1 | U2AF1 | 1.348334 | binary | 2.463504 |
| FGF23 | FGF23 | 1.266593 | binary | 3.61542 |
| IDH1 | IDH1 | 1.266593 | binary | 1.687956 |

(continued)

| | | Durable Response | | |
|---|---|---|---|---|
| Gene_Input | GeneName | Contribution To Response | Input | Percent In population (n = 8768) |
| PDCD1LG2 | PDCD1LG2 | 1.23884 | binary | 1.687956 |
| MEF2B | MEF2B | 1.215131 | binary | 1.448449 |
| PDK1 | PDK1 | 1.16345 | binary | 1.676551 |
| BRIP1 | BRIP1 | 1.152226 | binary | 4.17427 |
| QKI | QKI | 1.064153 | binary | 1.231752 |
| CTNNA1 | CTNNA1 | 1.064153 | binary | 1.859033 |
| FUBP1 | FUBP1 | 0.978551 | binary | 2.018704 |
| STAG2 | STAG2 | 0.972096 | binary | 4.493613 |
| FANCL | FANCL | 0.933488 | binary | 1.995894 |
| PAX5 | PAX5 | 0.925331 | binary | 3.193431 |
| MLH1 | MLH1 | 0.856218 | binary | 1.687956 |
| FANCG | FANCG | 0.849644 | binary | 2.212591 |
| AKT1 | AKT1 | 0.842021 | binary | 1.893248 |
| MPL | MPL | 0.842021 | binary | 2.703011 |
| BRCA2 | BRCA2 | 0.830051 | binary | 7.071168 |
| ATR | ATR | 0.803612 | binary | 8.81615 |
| POLE | POLE | 0.77999 | binary | 7.538777 |
| TSC1 | TSC1 | 0.778369 | binary | 3.033759 |
| FOXL2 | FOXL2 | 0.758752 | binary | 2.292427 |
| BRAF | BRAF | 0.717131 | binary | 7.607208 |
| ASXL1 | ASXL1 | 0.702532 | binary | 6.375456 |
| NF1 | NF1 | 0.700892 | binary | 12.44297 |
| ATRX | ATRX | 0.668535 | binary | 9.899635 |
| NRAS | NRAS | 0.659455 | binary | 1.254562 |
| PDGFRA | PDGFRA | 0.657149 | binary | 8.37135 |
| SMAD4 | SMAD4 | 0.63135 | binary | 4.64188 |
| NBN | NBN | 0.609419 | binary | 6.238595 |
| PDGFRB | PDGFRB | 0.558338 | binary | 4.539234 |
| BRCA1 | BRCA1 | 0.539297 | binary | 4.881387 |
| TP53 | TP53 | 0.520266 | binary | 66.40055 |
| SMARCA4 | SMARCA4 | 0.059468 | binary | 12.60265 |
| FGF19_LOF | FGF19 | 2.172156 | goflof | 6.021898 |
| U2AF1_GOF | U2AF1 | 1.634661 | goflof | 2.463504 |
| NBN_LOF | NBN | 1.621183 | goflof | 6.238595 |
| NRAS_LOF | NRAS | 1.456333 | goflof | 1.254562 |
| RAC1_LOF | RAC1 | 1.456333 | goflof | 3.421533 |
| RB1_GOF | RB1 | 1.456333 | goflof | 9.124088 |
| IDH1_GOF | IDH1 | 1.348334 | goflof | 1.687956 |

(continued)

| Durable Response | | | | |
|---|---|---|---|---|
| Gene_Input | GeneName | Contribution To Response | Input | Percent In population (n = 8768) |
| XRCC2_LOF | XRCC2 | 1.254342 | goflof | 1.58531 |
| PAX5_GOF | PAX5 | 1.254342 | goflof | 3.193431 |
| PDCD1LG2_GOF | PDCD1LG2 | 1.23884 | goflof | 1.687956 |
| CD274_GOF | CD274 | 1.23884 | goflof | 1.459854 |
| RAD52_GOF | RAD52 | 1.23884 | goflof | 2.771442 |
| VEGFA_LOF | VEGFA | 1.215131 | goflof | 2.577555 |
| CBFB_LOF | CBFB | 1.215131 | goflof | 1.07208 |
| AKT1_GOF | AKT1 | 1.215131 | goflof | 1.893248 |
| FGF23_LOF | FGF23 | 1.16345 | goflof | 3.61542 |
| PDK1_LOF | PDK1 | 1.16345 | goflof | 1.676551 |
| MYD88_GOF | MYD88 | 1.158903 | goflof | 1.04927 |
| CD79B_GOF | CD79B | 1.158903 | goflof | 1.60812 |
| PRKAR1A_GOF | PRKAR1A | 1.158903 | goflof | 1.380018 |
| BRCA2_GOF | BRCA2 | 1.158903 | goflof | 7.071168 |
| SDHC_LOF | SDHC | 1.158903 | goflof | 2.58896 |
| GNA13_LOF | GNA13 | 1.116719 | goflof | 1.357208 |
| FANCG_GOF | FANCG | 1.064153 | goflof | 2.212591 |
| FANCL_LOF | FANCL | 1.064153 | goflof | 1.995894 |
| SOX2_LOF | SOX2 | 1.032211 | goflof | 9.181113 |
| EZH2_LOF | EZH2 | 1.011338 | goflof | 2.531934 |
| STAG2_LOF | STAG2 | 0.972096 | goflof | 4.493613 |
| RAD51C_LOF | RAD51C | 0.954659 | goflof | 1.437044 |
| QKI_LOF | QKI | 0.954659 | goflof | 1.231752 |
| FGF6_GOF | FGF6 | 0.954659 | goflof | 3.832117 |
| FGF23_GOF | FGF23 | 0.954659 | goflof | 3.61542 |
| MUTYH_GOF | MUTYH | 0.954659 | goflof | 3.159215 |
| SYK_LOF | SYK | 0.941318 | goflof | 1.630931 |
| CTNNA1_LOF | CTNNA1 | 0.941318 | goflof | 1.859033 |
| CDH1_LOF | CDH1 | 0.928907 | goflof | 2.12135 |
| PBRM1_LOF | PBRM1 | 0.877942 | goflof | 4.812956 |
| PIK3C2G_GOF | PIK3C2G | 0.867665 | goflof | 9.42062 |
| ASXL1_LOF | ASXL1 | 0.852463 | goflof | 6.375456 |
| BRAF_GOF | BRAF | 0.844869 | goflof | 7.607208 |
| NF1_LOF | NF1 | 0.839896 | goflof | 12.44297 |
| CHEK1_LOF | CHEK1 | 0.832538 | goflof | 1.528285 |
| FUBP1_LOF | FUBP1 | 0.832538 | goflof | 2.018704 |
| ERRFI1_LOF | ERRFI1 | 0.832538 | goflof | 1.58531 |
| BRCA2_LOF | BRCA2 | 0.822594 | goflof | 7.071168 |

(continued)

| Durable Response | | | | |
|---|---|---|---|---|
| Gene_Input | GeneName | Contribution To Response | Input | Percent In population (n = 8768) |
| HSD3B1_GOF | HSD3B1 | 0.81931 | goflof | 3.113595 |
| DNMT3A_GOF | DNMT3A | 0.81931 | goflof | 8.827555 |
| WT1_LOF | WT1 | 0.800475 | goflof | 4.698905 |
| PDGFRA_LOF | PDGFRA | 0.793705 | goflof | 8.37135 |
| ATRX_LOF | ATRX | 0.743512 | goflof | 9.899635 |
| ATR_LOF | ATR | 0.739747 | goflof | 8.81615 |
| RBM10_GOF | RBM10 | 0.737032 | goflof | 10.54973 |
| MAP2K2_GOF | MAP2K2 | 0.737032 | goflof | 1.106296 |
| BRIP1_GOF | BRIP1 | 0.737032 | goflof | 4.17427 |
| PDCD1LG2_LOF | PDCD1LG2 | 0.737032 | goflof | 1.687956 |
| MEF2B_GOF | MEF2B | 0.737032 | goflof | 1.448449 |
| CREBBP_GOF | CREBBP | 0.737032 | goflof | 7.652829 |
| IRF2_GOF | IRF2 | 0.737032 | goflof | 1.448449 |
| BARD1_GOF | BARD1 | 0.737032 | goflof | 3.033759 |
| CTNNA1_GOF | CTNNA1 | 0.737032 | goflof | 1.859033 |
| MPL_GOF | MPL | 0.737032 | goflof | 2.703011 |
| ACVR1B_LOF | ACVR1B | 0.732527 | goflof | 2.018704 |
| PPARG_LOF | PPARG | 0.732527 | goflof | 1.676551 |
| TSC2_LOF | TSC2 | 0.729362 | goflof | 5.132299 |
| KMT2A_LOF | KMT2A | 0.69818 | goflof | 6.683394 |
| PTPN11_LOF | PTPN11 | 0.69437 | goflof | 1.813412 |
| PDGFRB_LOF | PDGFRB | 0.669592 | goflof | 4.539234 |
| AKT1_LOF | AKT1 | 0.610406 | goflof | 1.893248 |
| CHEK2_LOF | CHEK2 | 0.603633 | goflof | 4.12865 |
| NOTCH2_GOF | NOTCH2 | 0.559568 | goflof | 7.470347 |
| MUTYH_LOF | MUTYH | 0.559568 | goflof | 3.159215 |
| TSC2_GOF | TSC2 | 0.499286 | goflof | 5.132299 |
| MAP3K1_LOF | MAP3K1 | 0.448653 | goflof | 4.356752 |
| ARID1A_GOF | ARID1A | 0.42784 | goflof | 11.88412 |
| MED12_GOF | MED12 | 0.077416 | goflof | 8.131843 |
| CBFB_GOF | CBFB | 0.077416 | goflof | 1.07208 |
| PIK3R1_GOF | PIK3R1 | 0.077416 | goflof | 2.452099 |
| STAG2_GOF | STAG2 | 1.33E-14 | goflof | 4.493613 |
| MAP2K4_GOF | MAP2K4 | 1.33E-14 | goflof | 1.847628 |
| FLCN_GOF | FLCN | 1.33E-14 | goflof | 2.12135 |
| TSC1_GOF | TSC1 | 1.33E-14 | goflof | 3.033759 |
| SETD2_GOF | SETD2 | 1.33E-14 | goflof | 7.185219 |
| FGF19_LOF | FGF19 | 2.172156 | hotspot | 6.021898 |

(continued)

| Durable Response | | | | |
|---|---|---|---|---|
| Gene_Input | GeneName | Contribution To Response | Input | Percent In population (n = 8768) |
| TP53_GOF_331 | TP53 | 1.874747 | hotspot | 66.40055 |
| NFE2L2_GOF_24 | NFE2L2 | 1.686601 | hotspot | 6.455292 |
| PIK3C2G_GOF_108 8 | PIK3C2G | 1.686601 | hotspot | 9.42062 |
| U2AF1_GOF_34 | U2AF1 | 1.634661 | hotspot | 2.463504 |
| NBN_LOF | NBN | 1.621183 | hotspot | 6.238595 |
| NFE2L2_GOF_77 | NFE2L2 | 1.456333 | hotspot | 6.455292 |
| TP53_GOF_376 | TP53 | 1.456333 | hotspot | 66.40055 |
| TP53_GOF_244 | TP53 | 1.456333 | hotspot | 66.40055 |
| AKT1_GOF_17 | AKT1 | 1.456333 | hotspot | 1.893248 |
| RAC1_LOF | RAC1 | 1.456333 | hotspot | 3.421533 |
| MYCN_GOF | MYCN | 1.456333 | hotspot | 2.999544 |
| CDKN2A_GOF_151 | CDKN2A | 1.456333 | hotspot | 30.31478 |
| DNMT3A_GOF_882 | DNMT3A | 1.456333 | hotspot | 8.827555 |
| NRAS_LOF | NRAS | 1.456333 | hotspot | 1.254562 |
| XRCC2_LOF | XRCC2 | 1.254342 | hotspot | 1.58531 |
| PAX5_GOF | PAX5 | 1.254342 | hotspot | 3.193431 |
| BRAF_GOF_600 | BRAF | 1.254342 | hotspot | 7.607208 |
| RAD52_GOF | RAD52 | 1.23884 | hotspot | 2.771442 |
| PDCD1LG2_GOF | PDCD1LG2 | 1.23884 | hotspot | 1.687956 |
| CD274_GOF | CD274 | 1.23884 | hotspot | 1.459854 |
| TP53_GOF_159 | TP53 | 1.215131 | hotspot | 66.40055 |
| VEGFA_LOF | VEGFA | 1.215131 | hotspot | 2.577555 |
| CBFB_LOF | CBFB | 1.215131 | hotspot | 1.07208 |
| PDK1_LOF | PDK1 | 1.16345 | hotspot | 1.676551 |
| KDM5C_GOF_1330 | KDM5C | 1.158903 | hotspot | 5.782391 |
| KEAP1_GOF_332 | KEAP1 | 1.158903 | hotspot | 18.56752 |
| STK11_GOF_37 | STK11 | 1.158903 | hotspot | 17.49544 |
| BRCA2_GOF | BRCA2 | 1.158903 | hotspot | 7.071168 |
| KEAP1_GOF_116 | KEAP1 | 1.158903 | hotspot | 18.56752 |
| MYD88_GOF_265 | MYD88 | 1.158903 | hotspot | 1.04927 |
| IDH1_GOF | IDH1 | 1.158903 | hotspot | 1.687956 |
| AR_GOF_457 | AR | 1.158903 | hotspot | 8.177464 |
| RB1_GOF | RB1 | 1.158903 | hotspot | 9.124088 |
| MUTYH_GOF_165 | MUTYH | 1.158903 | hotspot | 3.159215 |
| BRAF_GOF_466 | BRAF | 1.158903 | hotspot | 7.607208 |
| POLE_GOF | POLE | 1.158903 | hotspot | 7.538777 |
| TP53_GOF_673 | TP53 | 1.158903 | hotspot | 66.40055 |
| CD79B_GOF | CD79B | 1.158903 | hotspot | 1.60812 |

(continued)

| Durable Response | | | | |
|---|---|---|---|---|
| Gene_Input | GeneName | Contribution To Response | Input | Percent In population (n = 8768) |
| ARID1A_GOF_21 | ARID1A | 1.158903 | hotspot | 11.88412 |
| PRKAR1A_GOF | PRKAR1A | 1.158903 | hotspot | 1.380018 |
| DIS3_GOF_458 | DIS3 | 1.158903 | hotspot | 3.501369 |
| CDKN2A_GOF_69 | CDKN2A | 1.158903 | hotspot | 30.31478 |
| FANCL_LOF | FANCL | 1.064153 | hotspot | 1.995894 |
| FANCG_GOF | FANCG | 1.064153 | hotspot | 2.212591 |
| SOX2_LOF | SOX2 | 1.032211 | hotspot | 9.181113 |
| SMAD4_GOF | SMAD4 | 1.026984 | hotspot | 4.64188 |
| BRAF_GOF_469 | BRAF | 1.026984 | hotspot | 7.607208 |
| TP53_GOF_275 | TP53 | 1.026984 | hotspot | 66.40055 |
| TP53_GOF_192 | TP53 | 1.026984 | hotspot | 66.40055 |
| STAG2_LOF | STAG2 | 0.972096 | hotspot | 4.493613 |
| QKI_LOF | QKI | 0.954659 | hotspot | 1.231752 |
| FGF6_GOF | FGF6 | 0.954659 | hotspot | 3.832117 |
| CTNNA1_LOF | CTNNA1 | 0.941318 | hotspot | 1.859033 |
| MLH1_LOF | MLH1 | 0.928907 | hotspot | 1.687956 |
| CDH1_LOF | CDH1 | 0.928907 | hotspot | 2.12135 |
| ERBB3_LOF | ERBB3 | 0.926409 | hotspot | 3.706661 |
| PBRM1_LOF | PBRM1 | 0.877942 | hotspot | 4.812956 |
| ASXL1_LOF | ASXL1 | 0.852463 | hotspot | 6.375456 |
| KDM5A_GOF | KDM5A | 0.842021 | hotspot | 5.885037 |
| NF1_LOF | NF1 | 0.839896 | hotspot | 12.44297 |
| CHEK1_LOF | CHEK1 | 0.832538 | hotspot | 1.528285 |
| BRCA2_LOF | BRCA2 | 0.822594 | hotspot | 7.071168 |
| TP53_GOF_560 | TP53 | 0.81931 | hotspot | 66.40055 |
| WT1_LOF | WT1 | 0.800475 | hotspot | 4.698905 |
| SDHB_LOF | SDHB | 0.796717 | hotspot | 0.638686 |
| EGFR_GOF_858 | EGFR | 0.796717 | hotspot | 19.34307 |
| TP53_GOF_215 | TP53 | 0.796717 | hotspot | 66.40055 |
| PDGFRA_LOF | PDGFRA | 0.793705 | hotspot | 8.37135 |
| ATRX_LOF | ATRX | 0.743512 | hotspot | 9.899635 |
| ATR_LOF | ATR | 0.739747 | hotspot | 8.81615 |
| IRF2_GOF | IRF2 | 0.737032 | hotspot | 1.448449 |
| EGFR_GOF_771 | EGFR | 0.737032 | hotspot | 19.34307 |
| EGFR_GOF_861 | EGFR | 0.737032 | hotspot | 19.34307 |
| MLH1_GOF | MLH1 | 0.737032 | hotspot | 1.687956 |
| AR_GOF_465 | AR | 0.737032 | hotspot | 8.177464 |
| STK11_GOF_221 | STK11 | 0.737032 | hotspot | 17.49544 |

(continued)

| Durable Response | | | | |
|---|---|---|---|---|
| Gene_Input | GeneName | Contribution To Response | Input | Percent In population (n = 8768) |
| FAS_GOF | FAS | 0.737032 | hotspot | 1.00365 |
| AMER1_GOF_385 | AMER1 | 0.737032 | hotspot | 7.960766 |
| RBM10_GOF_503 | RBM10 | 0.737032 | hotspot | 10.54973 |
| KEAP1_GOF_153 | KEAP1 | 0.737032 | hotspot | 18.56752 |
| STK11_GOF_199 | STK11 | 0.737032 | hotspot | 17.49544 |
| PTCH1_GOF | PTCH1 | 0.737032 | hotspot | 4.550639 |
| STK11_GOF_163 | STK11 | 0.737032 | hotspot | 17.49544 |
| ATRX_GOF | ATRX | 0.737032 | hotspot | 9.899635 |
| KEAP1_GOF_509 | KEAP1 | 0.737032 | hotspot | 18.56752 |
| TP53_GOF_236 | TP53 | 0.737032 | hotspot | 66.40055 |
| KEAP1_GOF_362 | KEAP1 | 0.737032 | hotspot | 18.56752 |
| HSD3B1_GOF_75 | HSD3B1 | 0.737032 | hotspot | 3.113595 |
| RB1_GOF_576 | RB1 | 0.737032 | hotspot | 9.124088 |
| STK11_GOF_216 | STK11 | 0.737032 | hotspot | 17.49544 |
| FBXW7_GOF_479 | FBXW7 | 0.737032 | hotspot | 3.934763 |
| KEAP1_GOF_523 | KEAP1 | 0.737032 | hotspot | 18.56752 |
| JAK3_GOF | JAK3 | 0.737032 | hotspot | 4.276916 |
| PPARG_LOF | PPARG | 0.732527 | hotspot | 1.676551 |
| PIK3CB_GOF | PIK3CB | 0.732527 | hotspot | 3.980383 |
| ACVR1B_LOF | ACVR1B | 0.732527 | hotspot | 2.018704 |
| TSC2_LOF | TSC2 | 0.729362 | hotspot | 5.132299 |
| KMT2A_LOF | KMT2A | 0.69818 | hotspot | 6.683394 |
| TP53_GOF_238 | TP53 | 0.631163 | hotspot | 66.40055 |
| AKT1_LOF | AKT1 | 0.610406 | hotspot | 1.893248 |
| PIK3R1_LOF | PIK3R1 | 0.567122 | hotspot | 2.452099 |
| IRS2_GOF | IRS2 | 0.499286 | hotspot | 8.200274 |
| HRAS_GOF_61 | HRAS | 0.499286 | hotspot | 0.969434 |
| PIK3CA_GOF_726 | PIK3CA | 0.499286 | hotspot | 14.18796 |
| AR_GOF_468 | AR | 0.499286 | hotspot | 8.177464 |
| TNFRSF14_LOF | TNFRSF14 | 0.400896 | hotspot | 1.094891 |
| NFE2L2_GOF_29 | NFE2L2 | 0.103465 | hotspot | 6.455292 |
| MEN1_GOF | MEN1 | 0.103465 | hotspot | 2.007299 |
| BCL2_LOF | BCL2 | 0.103465 | hotspot | 0.809763 |
| AR_GOF_467 | AR | 0.077416 | hotspot | 8.177464 |
| ATM_GOF_337 | ATM | 0.077416 | hotspot | 11.91834 |
| KEAP1_GOF_544 | KEAP1 | 0.077416 | hotspot | 18.56752 |
| NTRK3_GOF | NTRK3 | 0.077416 | hotspot | 9.876825 |
| NTRK3_GOF_610 | NTRK3 | 0.077416 | hotspot | 9.876825 |

(continued)

| | | Durable Response | | |
|---|---|---|---|---|
| Gene_Input | GeneName | Contribution To Response | Input | Percent In population (n = 8768) |
| HRAS_LOF | HRAS | 0.077416 | hotspot | 0.969434 |
| CTNNB1_GOF_41 | CTNNB1 | 1.33E-14 | hotspot | 4.15146 |
| SMARCA4_GOF_11 60 | SMARCA4 | 1.33E-14 | hotspot | 12.60265 |
| KEAP1_GOF_409 | KEAP1 | 1.33E-14 | hotspot | 18.56752 |
| SMAD2_GOF_464 | SMAD2 | 1.33E-14 | hotspot | 1.5625 |
| PTEN_GOF_59 | PTEN | 1.33E-14 | hotspot | 6.683394 |
| CTNNB1_GOF_33 | CTNNB1 | 1.33E-14 | hotspot | 4.15146 |
| CREBBP_GOF_147 2 | CREBBP | 1.33E-14 | hotspot | 7.652829 |
| APC_GOF | APC | 1.33E-14 | hotspot | 7.937956 |
| PTEN_GOF_165 | PTEN | 1.33E-14 | hotspot | 6.683394 |
| TSC1_GOF | TSC1 | 1.33E-14 | hotspot | 3.033759 |
| KEAP1_GOF_417 | KEAP1 | 1.33E-14 | hotspot | 18.56752 |
| RBM10_GOF | RBM10 | 1.33E-14 | hotspot | 10.54973 |
| NBN_GOF_219 | NBN | 1.33E-14 | hotspot | 6.238595 |
| PIK3CA_GOF_345 | PIK3CA | 1.33E-14 | hotspot | 14.18796 |
| AR_GOF_493 | AR | 1.33E-14 | hotspot | 8.177464 |
| ZNF217_GOF_410 | ZNF217 | 1.33E-14 | hotspot | 4.345347 |
| AR_GOF_598 | AR | 1.33E-14 | hotspot | 8.177464 |
| STK11_GOF_256 | STK11 | 1.33E-14 | hotspot | 17.49544 |
| ARID1A_GOF_343 | ARID1A | 1.33E-14 | hotspot | 11.88412 |
| RET_GOF_511 | RET | 1.33E-14 | hotspot | 6.592153 |
| KEAP1_GOF_155 | KEAP1 | 1.33E-14 | hotspot | 18.56752 |
| MITF_GOF | MITF | 1.33E-14 | hotspot | 1.836223 |
| CDK8_GOF | CDK8 | 1.33E-14 | hotspot | 1.186131 |
| DNMT3A_GOF_749 | DNMT3A | 1.33E-14 | hotspot | 8.827555 |
| MYD88_GOF | MYD88 | 1.33E-14 | hotspot | 1.04927 |
| KEAP1_GOF_430 | KEAP1 | 1.33E-14 | hotspot | 18.56752 |
| GNAS_GOF_415 | GNAS | 1.33E-14 | hotspot | 10.28741 |
| KDM5C_GOF | KDM5C | 1.33E-14 | hotspot | 5.782391 |
| FLT1_GOF | FLT1 | 1.33E-14 | hotspot | 7.036953 |
| NF1_GOF_1642 | NF1 | 1.33E-14 | hotspot | 12.44297 |
| KMT2A_GOF_53 | KMT2A | 1.33E-14 | hotspot | 6.683394 |
| SDHA_GOF_531 | SDHA | 1.33E-14 | hotspot | 12.10082 |
| CDKN2A_GOF_61 | CDKN2A | 1.33E-14 | hotspot | 30.31478 |
| CEBPA_GOF_197 | CEBPA | 1.33E-14 | hotspot | 3.854927 |
| STK11_GOF_220 | STK11 | 1.33E-14 | hotspot | 17.49544 |
| NBN_GOF_680 | NBN | 1.33E-14 | hotspot | 6.238595 |
| KEAP1_GOF_450 | KEAP1 | 1.33E-14 | hotspot | 18.56752 |

(continued)

| Durable Response | | | | |
|---|---|---|---|---|
| Gene_Input | GeneName | Contribution To Response | Input | Percent In population (n = 8768) |
| CHEK2_GOF_392 | CHEK2 | 1.33E-14 | hotspot | 4.12865 |
| NKX2-1_GOF_234 | NKX2-1 | 1.33E-14 | hotspot | 10.20757 |
| SMARCA4_GOF_11 57 | SMARCA4 | 1.33E-14 | hotspot | 12.60265 |
| BCORL1_GOF_94 | BCORL1 | 1.33E-14 | hotspot | 8.32573 |
| KEAP1_GOF_493 | KEAP1 | 1.33E-14 | hotspot | 18.56752 |
| FLCN_GOF_306 | FLCN | 1.33E-14 | hotspot | 2.12135 |
| STK11_GOF_168 | STK11 | 1.33E-14 | hotspot | 17.49544 |
| MSH6_GOF_1088 | MSH6 | 1.33E-14 | hotspot | 3.410128 |

**Table 6B:** Coefficient information for the durable response group - updated following additional analysis

| Durable Response | | |
|---|---|---|
| Binary gene input | | |
| Gene Feature | Contribution to response | Popularity* |
| CTNNA1 | 1.896 | 1 |
| ALOX12B | 1.52 | 0.75 |
| HRAS | 1.4 | 1 |
| FAS | 1.346 | 0.833 |
| U2AF1 | 1.286 | 1 |
| TSC1 | 1.251 | 0.75 |
| MLH1 | 1.251 | 0.75 |
| BRIP1 | 1.177 | 1 |
| GNA13 | 1.117 | 0.667 |
| ERRFI1 | 1.117 | 0.167 |
| ACVR1B | 1.004 | 0.417 |
| IDH1 | 0.937 | 1 |
| XRCC2 | 0.892 | 0.167 |
| BRAF | 0.882 | 1 |
| SOX9 | 0.874 | 0.417 |
| HNF1A | 0.824 | 0.083 |
| PDCD1LG2 | 0.803 | 0.083 |
| ESR1 | 0.77 | 0.083 |
| BRCA2 | 0.769 | 1 |
| ASXL1 | 0.767 | 1 |
| KEL | 0.741 | 0.417 |
| TERT | 0.727 | 0.583 |
| TSC2 | 0.723 | 0.833 |
| BARD1 | 0.721 | 1 |
| BCL2 | 0.703 | 0.083 |

(continued)

| Durable Response | | |
| --- | --- | --- |
| **Binary gene input** | | |
| **Gene Feature** | **Contribution to response** | **Popularity*** |
| QKI | 0.701 | 0.083 |
| PDGFRB | 0.691 | 0.5 |
| BTK | 0.663 | 0.667 |
| FOXL2 | 0.645 | 0.5 |
| CARD11 | 0.634 | 0.667 |
| PBRM1 | 0.625 | 0.583 |
| EZH2 | 0.607 | 0.25 |
| RAD51C | 0.598 | 0.667 |
| ABL1 | 0.594 | 0.333 |
| ALK | 0.593 | 0.167 |
| HSD3B1 | 0.574 | 0.333 |
| POLE | 0.543 | 0.417 |
| FLT1 | 0.52 | 1 |
| NOTCH3 | 0.511 | 0.583 |
| PAX5 | 0.491 | 0.083 |
| MAP3K1 | 0.471 | 0.167 |
| STAT3 | 0.416 | 0.167 |
| GABRA6 | 0.409 | 0.5 |
| TP53 | 0.396 | 0.25 |
| CUL3 | 0.362 | 0.25 |
| NBN | 0.349 | 0.083 |
| PDGFRA | 0.34 | 0.083 |
| SDHD | 0.291 | 0.083 |
| RBM10 | 0.276 | 0.333 |
| KRAS | 0.081 | 0.25 |
| **HFM/LFM input** | | |
| **Gene Feature** | **Contribution to response** | **Popularity** |
| ALOX12B_LFM | 2.215 | 1 |
| CTNNA1_LFM | 1.725 | 1 |
| HRAS_HFM | 1.618 | 0.75 |
| MEF2B_LFM | 1.52 | 0.167 |
| TNFRSF14_LFM | 1.4 | 0.833 |
| XRCC2_LFM | 1.4 | 0.333 |
| BRIP1_LFM | 1.354 | 1 |
| U2AF1_HFM | 1.31 | 1 |
| NF1_HFM | 1.31 | 1 |
| FAS_LFM | 1.275 | 0.5 |

(continued)

| HFM/LFM input | | |
|---|---|---|
| Gene Feature | Contribution to response | Popularity |
| TSC1_LFM | 1.251 | 0.917 |
| ERRFI1_LFM | 1.22 | 0.333 |
| EZH2_LFM | 1.199 | 0.583 |
| NBN_LFM | 1.179 | 1 |
| MLH1_LFM | 1.121 | 1 |
| GNA13_LFM | 1.113 | 0.333 |
| FGF19_LFM | 1.113 | 0.667 |
| ALK_HFM | 1.108 | 0.167 |
| AMER1_HFM | 1.108 | 0.167 |
| BRAF_HFM | 1.052 | 1 |
| IDH1_HFM | 1.028 | 0.25 |
| DNMT3A_HFM | 0.943 | 0.083 |
| CDK6_LFM | 0.924 | 0.333 |
| BCL6_LFM | 0.866 | 0.75 |
| WT1_HFM | 0.857 | 0.333 |
| SOX9_LFM | 0.847 | 0.083 |
| SMAD4_LFM | 0.847 | 0.583 |
| HNF1A_LFM | 0.824 | 0.083 |
| JAK2_LFM | 0.816 | 0.5 |
| PAX5_HFM | 0.803 | 0.417 |
| HSD3B1_LFM | 0.803 | 0.333 |
| PARP2_LFM | 0.803 | 0.333 |
| ASXL1_HFM | 0.781 | 0.917 |
| ESR1_LFM | 0.77 | 0.417 |
| PIK3C2G_HFM | 0.77 | 0.667 |
| BRCA2_LFM | 0.768 | 0.75 |
| ASXL1_LFM | 0.762 | 1 |
| TERT_HFM | 0.727 | 0.917 |
| WT1_LFM | 0.723 | 0.333 |
| BARD1_LFM | 0.721 | 0.917 |
| MAP3K1_LFM | 0.718 | 1 |
| FGFR2_LFM | 0.714 | 0.583 |
| PPARG_LFM | 0.71 | 0.083 |
| AXIN1_LFM | 0.707 | 0.083 |
| PDCD1LG2_HFM | 0.705 | 0.167 |
| SOX9_HFM | 0.701 | 0.083 |
| KRAS_LFM | 0.701 | 0.083 |
| PRKAR1A_HFM | 0.699 | 0.167 |

(continued)

| HFM/LFM input | | |
|---|---|---|
| **Gene Feature** | **Contribution to response** | **Popularity** |
| PDGFRB_LFM | 0.691 | 0.917 |
| TSC2_LFM | 0.681 | 0.667 |
| NF2_LFM | 0.663 | 0.333 |
| FGF6_LFM | 0.655 | 0.083 |
| PBRM1_LFM | 0.654 | 0.25 |
| KEL_LFM | 0.648 | 0.167 |
| FOXL2_LFM | 0.645 | 0.833 |
| CARD11 LFM | 0.633 | 0.167 |
| MAP3K13_LFM | 0.619 | 1 |
| FGF6_HFM | 0.618 | 0.083 |
| NOTCH3_LFM | 0.593 | 1 |
| SF3B1_LFM | 0.592 | 0.333 |
| CUL3_LFM | 0.562 | 0.167 |
| FLT1_LFM | 0.537 | 1 |
| CD274_HFM | 0.521 | 0.167 |
| IDH1_LFM | 0.516 | 0.25 |
| SPOP_LFM | 0.516 | 0.083 |
| MYCN_HFM | 0.516 | 0.167 |
| SRC_LFM | 0.516 | 0.083 |
| AR_LFM | 0.512 | 0.25 |
| ERBB2_LFM | 0.509 | 0.083 |
| CASP8_LFM | 0.485 | 0.083 |
| PDGFRA_LFM | 0.464 | 0.667 |
| IRF4_LFM | 0.454 | 0.083 |
| PTEN_LFM | 0.447 | 0.917 |
| GNAS_LFM | 0.443 | 0.417 |
| CCNE1_LFM | 0.413 | 0.083 |
| NOTCH2_LFM | 0.365 | 0.083 |
| ATR_LFM | 0.363 | 0.083 |
| RBM10_LFM | 0.336 | 0.083 |
| FANCG_HFM | 0.229 | 0.167 |
| SMO_HFM | 0.227 | 0.167 |
| EGFR_LFM | 0.002 | 0.083 |
| SPOP_HFM | 0.002 | 0.167 |
| Hotspot granular input | | |
| **Gene Feature** | **Contribution to response** | **Popularity** |
| ALOX12B_LFM | 2.215 | 1 |
| DNMT3A_HFM_771 | 1.803 | 1 |

(continued)

| Hotspot granular input | | |
|---|---|---|
| Gene Feature | Contribution to response | Popularity |
| CTNNA1_LFM | 1.725 | 0.833 |
| NFE2L2_HFM_24 | 1.618 | 0.833 |
| MEF2B_LFM | 1.52 | 0.083 |
| TP53_HFM_331 | 1.52 | 1 |
| BRAF_HFM_469 | 1.52 | 1 |
| XRCC2_LFM | 1.4 | 1 |
| TNFRSF14_LFM | 1.4 | 0.667 |
| BRIP1_LFM | 1.354 | 1 |
| FUBP1_LFM | 1.317 | 0.167 |
| U2AF1_HFM_34 | 1.31 | 0.917 |
| FAS_LFM | 1.275 | 1 |
| TP53_HFM 173 | 1.264 | 0.917 |
| TSC1_LFM | 1.251 | 1 |
| ERRFI1_LFM | 1.22 | 0.25 |
| EZH2_LFM | 1.199 | 0.667 |
| NBN_LFM | 1.179 | 0.833 |
| MLH1_LFM | 1.121 | 1 |
| BRAF_HFM_600 | 1.117 | 1 |
| FGF19_LFM | 1.113 | 0.75 |
| GNA13_LFM | 1.113 | 0.25 |
| ALK_HFM | 1.108 | 0.583 |
| TP53_HFM _15 | 1.108 | 0.083 |
| TP53 HFM_783 | 1.103 | 0.083 |
| TP53_HFM_560 | 0.993 | 0.75 |
| CDK6_LFM | 0.924 | 0.167 |
| BCL6_LFM | 0.866 | 1 |
| TP53_HFM_280 | 0.857 | 0.417 |
| BTG1_LFM | 0.857 | 0.25 |
| WT1_HFM_ | 0.857 | 0.167 |
| SOX9_LFM | 0.847 | 0.167 |
| SMAD4_LFM | 0.847 | 0.5 |
| NF1_HFM | 0.823 | 0.667 |
| JAK2_LFM | 0.816 | 0.333 |
| HSD3B1_LFM | 0.803 | 0.75 |
| PARP2_LFM | 0.803 | 0.75 |
| PIK3C2G HFM | 0.77 | 1 |
| BRCA2_LFM | 0.768 | 1 |
| ASXL1_LFM | 0.762 | 0.667 |

(continued)

| Hotspot granular input | | |
|---|---|---|
| Gene Feature | Contribution to response | Popularity |
| TERT_HFM | 0.727 | 0.917 |
| BARD1_LFM | 0.721 | 0.917 |
| MAP3K1_LFM | 0.718 | 0.917 |
| FGFR2_LFM | 0.714 | 0.583 |
| SDHA_LFM | 0.712 | 0.083 |
| IGF1R_LFM | 0.71 | 0.083 |
| AXIN1_LFM | 0.707 | 0.333 |
| PDCD1LG2_HFM | 0.705 | 0.417 |
| PAX5_HFM | 0.705 | 0.5 |
| TP53_HFM 294 | 0.703 | 0.417 |
| RAD51C_LFM | 0.703 | 0.167 |
| JUN_LFM | 0.701 | 0.167 |
| PRKAR1A_HFM | 0.699 | 0.417 |
| PDGFRB_LFM | 0.691 | 0.25 |
| TSC2_LFM | 0.681 | 0.417 |
| NF2_LFM | 0.663 | 0.417 |
| BTK_LFM | 0.663 | 0.083 |
| PBRM1_LFM | 0.654 | 0.083 |
| FOXL2_LFM | 0.645 | 0.667 |
| MAP3K13_LFM | 0.619 | 1 |
| NOTCH3_LFM | 0.593 | 0.5 |
| GABRA6_LFM | 0.587 | 0.167 |
| PIK3CA HFM 542 | 0.567 | 0.333 |
| TP53_HFM_159 | 0.567 | 0.333 |
| CUL3_LFM | 0.562 | 0.167 |
| ASXL1_HFM | 0.551 | 0.25 |
| FLT1_LFM | 0.537 | 1 |
| MITF_LFM | 0.521 | 0.25 |
| TP53_HFM_249 | 0.521 | 0.167 |
| IDH1_LFM | 0.516 | 0.083 |
| SRC_LFM | 0.516 | 0.083 |
| AR_LFM | 0.512 | 0.417 |
| ERBB2_LFM | 0.509 | 0.167 |
| SMO_LFM | 0.491 | 0.083 |
| KEAP1_LFM | 0.467 | 0.417 |
| PDGFRA_LFM | 0.464 | 0.417 |
| GATA6 LFM | 0.448 | 0.083 |
| PTEN_LFM | 0.447 | 0.583 |

(continued)

| Hotspot granular input | | |
|---|---|---|
| **Gene Feature** | **Contribution to response** | **Popularity** |
| GNAS_LFM | 0.443 | 0.417 |
| CCNE1_LFM | 0.413 | 0.167 |
| ATR_LFM | 0.363 | 0.083 |
| MERTK_LFM | 0.32 | 0.083 |
| KMT2A_LFM | 0.32 | 0.083 |
| SOX9_HFM | 0.291 | 0.083 |
| GSK3B_HFM | 0.227 | 0.083 |
| CUL4A_LFM | 0.002 | 0.083 |
| BCL2L2_LFM | 0.002 | 0.083 |
| * Popularity is the occurrence frequency of the gene feature in the 12 genetic signature outputs for each input type. | | |

**Table 7A:** Coefficient information for the innate CPI resistance group

| Innate Resistance | | | | |
|---|---|---|---|---|
| **Gene_input** | **Gene Name** | **Contribution To Response** | **Input** | **Percent In population (n = 8768)** |
| GID4 | GID4 | -1.56175 | binary | 1.060675 |
| JUN | JUN | -0.82472 | binary | 1.345803 |
| RAD51 | RAD51 | -0.82472 | binary | 0.752737 |
| CD79A | CD79A | -0.69758 | binary | 1.414234 |
| STK11 | STK11 | -0.66955 | binary | 17.49544 |
| TET2 | TET2 | -0.58201 | binary | 6.626369 |
| MAP2K1 | MAP2K1 | -0.57806 | binary | 1.893248 |
| CCNE1 | CCNE1 | -0.574 | binary | 4.208485 |
| CDK4 | CDK4 | -0.57279 | binary | 3.820712 |
| ERCC4 | ERCC4 | -0.57279 | binary | 2.988139 |
| CEBPA | CEBPA | -0.52738 | binary | 3.854927 |
| RARA | RARA | -0.50609 | binary | 2.383668 |
| CDKN1A | CDKN1A | -0.4431 | binary | 1.02646 |
| RAD51B | RAD51B | -0.4431 | binary | 1.334398 |
| PPP2R1A | PPP2R1A | -0.38647 | binary | 2.349453 |
| CSF1R | CSF1R | -0.38397 | binary | 3.832117 |
| FH | FH | -0.36521 | binary | 3.63823 |
| NKX2-1 | NKX2-1 | -0.358 | binary | 10.20757 |
| NTRK2 | NTRK2 | -0.34154 | binary | 3.558394 |
| FGFR1 | FGFR1 | -0.23631 | binary | 6.25 |
| CDK6 | CDK6 | -0.15361 | binary | 3.467153 |
| MDM2 | MDM2 | -0.15062 | binary | 5.964872 |
| BRD4 | BRD4 | -0.06751 | binary | 3.980383 |
| MAP3K1_GOF | MAP3K1 | -1.74432 | goflof | 4.356752 |

(continued)

| | | Innate Resistance | | |
|---|---|---|---|---|
| **Gene_input** | **Gene Name** | **Contribution To Response** | **Input** | **Percent In population (n = 8768)** |
| FGF14_GOF | FGF14 | -1.56175 | goflof | 2.976734 |
| PPP2R1A_GOF | PPP2R1A | -1.56175 | goflof | 2.349453 |
| CDKN1A_GOF | CDKN1A | -1.33962 | goflof | 1.02646 |
| JAK1_GOF | JAK1 | -1.33962 | goflof | 2.714416 |
| IRF4_GOF | IRF4 | -1.33962 | goflof | 2.782847 |
| JUN_LOF | JUN | -1.1624 | goflof | 1.345803 |
| AKT3_GOF | AKT3 | -1.1624 | goflof | 3.489964 |
| NKX2-1_LOF | NKX2-1 | -1.15045 | goflof | 10.20757 |
| TGFBR2_GOF | TGFBR2 | -1.05544 | goflof | 2.953923 |
| GABRA6_GOF | GABRA6 | -1.05544 | goflof | 4.881387 |
| BCORL1_GOF | BCORL1 | -1.05544 | goflof | 8.32573 |
| TNFAIP3_GOF | TNFAIP3 | -1.05544 | goflof | 2.395073 |
| POLD1_GOF | POLD1 | -1.05544 | goflof | 4.71031 |
| GNAQ_GOF | GNAQ | -1.05544 | goflof | 0.775547 |
| RAD51C_GOF | RAD51C | -1.05544 | goflof | 1.437044 |
| BRD4_GOF | BRD4 | -1.05544 | goflof | 3.980383 |
| RNF43_GOF | RNF43 | -1.05544 | goflof | 2.657391 |
| FGFR4_GOF | FGFR4 | -1.05544 | goflof | 3.387318 |
| GATA6_GOF | GATA6 | -1.00728 | goflof | 3.832117 |
| GATA3_GOF | GATA3 | -1.00728 | goflof | 4.037409 |
| MDM2_LOF | MDM2 | -1.00728 | goflof | 5.964872 |
| MYC_LOF | MYC | -0.99501 | goflof | 8.565237 |
| RPTOR_GOF | RPTOR | -0.82472 | goflof | 4.19708 |
| CD79A_LOF | CD79A | -0.82472 | goflof | 1.414234 |
| CCNE1_GOF | CCNE1 | -0.73203 | goflof | 4.208485 |
| STK11_LOF | STK11 | -0.72345 | goflof | 17.49544 |
| MYCN_LOF | MYCN | -0.71132 | goflof | 2.999544 |
| CEBPA_LOF | CEBPA | -0.69758 | goflof | 3.854927 |
| PARP1_GOF | PARP1 | -0.69758 | goflof | 3.330292 |
| NOTCH3_GOF | NOTCH3 | -0.65962 | goflof | 8.30292 |
| SDHD_GOF | SDHD | -0.65962 | goflof | 0.55885 |
| LTK_GOF | LTK | -0.65962 | goflof | 2.63458 |
| DAXX_GOF | DAXX | -0.65962 | goflof | 2.61177 |
| ABL1_GOF | ABL1 | -0.65962 | goflof | 3.284672 |
| PDGFRB_GOF | PDGFRB | -0.65962 | goflof | 4.539234 |
| BTG1_GOF | BTG1 | -0.65962 | goflof | 0.775547 |
| CHEK1_GOF | CHEK1 | -0.65962 | goflof | 1.528285 |
| GATA4_GOF | GATA4 | -0.65962 | goflof | 2.862683 |

(continued)

| | Innate Resistance | | | |
|---|---|---|---|---|
| Gene_input | Gene Name | Contribution To Response | Input | Percent In population (n = 8768) |
| JUN_GOF | JUN | -0.65962 | goflof | 1.345803 |
| FLT3_GOF | FLT3 | -0.65962 | goflof | 4.562044 |
| CUL3_GOF | CUL3 | -0.65962 | goflof | 3.067975 |
| CDK4_GOF | CDK4 | -0.65552 | goflof | 3.820712 |
| AKT2_GOF | AKT2 | -0.63635 | goflof | 3.546989 |
| KDM6A_GOF | KDM6A | -0.60259 | goflof | 4.812956 |
| MTOR_GOF | MTOR | -0.60259 | goflof | 6.090329 |
| BCL2L1_LOF | BCL2L1 | -0.60259 | goflof | 2.09854 |
| CD274_LOF | CD274 | -0.60259 | goflof | 1.459854 |
| NF2_GOF | NF2 | -0.58541 | goflof | 2.908303 |
| SMARCA4_GOF | SMARCA 4 | -0.57806 | goflof | 12.60265 |
| NFKBIA_GOF | NFKBIA | -0.57663 | goflof | 6.979927 |
| ERCC4_LOF | ERCC4 | -0.57314 | goflof | 2.988139 |
| ZNF703_GOF | ZNF703 | -0.52122 | goflof | 5.554288 |
| STK11_GOF | STK11 | -0.51839 | goflof | 17.49544 |
| KEAP1_GOF | KEAP1 | -0.50849 | goflof | 18.56752 |
| ERBB2_GOF | ERBB2 | -0.49443 | goflof | 6.421077 |
| FH_GOF | FH | -0.4839 | goflof | 3.63823 |
| REL_LOF | REL | -0.4431 | goflof | 1.859033 |
| RARA_GOF | RARA | -0.4431 | goflof | 2.383668 |
| RAD51_LOF | RAD51 | -0.40285 | goflof | 0.752737 |
| PTEN_GOF | PTEN | -0.40285 | goflof | 6.683394 |
| NTRK2_LOF | NTRK2 | -0.4019 | goflof | 3.558394 |
| CDKN2B_LOF | CDKN2B | -0.37499 | goflof | 17.4042 |
| BAP1_LOF | BAP1 | -0.36521 | goflof | 2.372263 |
| RARA_LOF | RARA | -0.35811 | goflof | 2.383668 |
| AXL_GOF | AXL | -0.34779 | goflof | 3.341697 |
| CCND2_LOF | CCND2 | -0.34779 | goflof | 2.828467 |
| CUL4A_LOF | CUL4A | -0.34779 | goflof | 2.543339 |
| H3F3A_GOF | H3F3A | -0.34779 | goflof | 1.197537 |
| GRM3_GOF | GRM3 | -0.32465 | goflof | 8.759124 |
| CDK6_GOF | CDK6 | -0.32465 | goflof | 3.467153 |
| ARFRP1_LOF | ARFRP1 | -0.31841 | goflof | 2.497719 |
| SUFU_LOF | SUFU | -0.28798 | goflof | 1.391423 |
| RAD54L_LOF | RAD54L | -0.27916 | goflof | 1.881843 |
| PIK3CB_LOF | PIK3CB | -0.27448 | goflof | 3.980383 |
| KLHL6_GOF | KLHL6 | -0.2703 | goflof | 9.021442 |
| MAP3K13_GOF | MAP3K13 | -0.2318 | goflof | 7.527372 |

(continued)

| Gene_input | Gene Name | Contribution To Response | Input | Percent In population (n = 8768) |
|---|---|---|---|---|
| | | **Innate Resistance** | | |
| PIK3CA_GOF | PIK3CA | -0.22932 | goflof | 14.18796 |
| CDKN2A_LOF | CDKN2A | -0.22309 | goflof | 30.31478 |
| EZH2_GOF | EZH2 | -0.21284 | goflof | 2.531934 |
| FANCA_LOF | FANCA | -0.20211 | goflof | 4.482208 |
| HGF_LOF | HGF | -0.1972 | goflof | 9.990876 |
| FBXW7_GOF | FBXW7 | -0.18072 | goflof | 3.934763 |
| FGFR2_GOF | FGFR2 | -0.18072 | goflof | 2.782847 |
| AURKA_LOF | AURKA | -0.18072 | goflof | 1.744982 |
| HSD3B1_LOF | HSD3B1 | -0.15965 | goflof | 3.113595 |
| SDHC_GOF | SDHC | -0.10371 | goflof | 2.58896 |
| EGFR_GOF_746 | EGFR | -1.89944 | hotspot | 19.34307 |
| TP53_GOF_282 | TP53 | -1.74432 | hotspot | 66.40055 |
| EGFR_GOF_747 | EGFR | -1.56175 | hotspot | 19.34307 |
| EGFR_GOF_719 | EGFR | -1.56175 | hotspot | 19.34307 |
| TP53_GOF_195 | TP53 | -1.56175 | hotspot | 66.40055 |
| FGF14_GOF | FGF14 | -1.56175 | hotspot | 2.976734 |
| PPP2R1A_GOF | PPP2R1A | -1.56175 | hotspot | 2.349453 |
| IRF4_GOF | IRF4 | -1.33962 | hotspot | 2.782847 |
| MAP3K1_GOF | MAP3K1 | -1.33962 | hotspot | 4.356752 |
| STK11_GOF_291 | STK11 | -1.33962 | hotspot | 17.49544 |
| ERBB2_GOF_776 | ERBB2 | -1.33962 | hotspot | 6.421077 |
| JAK1_GOF | JAK1 | -1.33962 | hotspot | 2.714416 |
| CDKN1A_GOF | CDKN1A | -1.33962 | hotspot | 1.02646 |
| KEAP1_GOF_272 | KEAP1 | -1.33962 | hotspot | 18.56752 |
| JUN_LOF | JUN | -1.1624 | hotspot | 1.345803 |
| AKT3_GOF | AKT3 | -1.1624 | hotspot | 3.489964 |
| NKX2-1_LOF | NKX2-1 | -1.15045 | hotspot | 10.20757 |
| ATM_GOF | ATM | -1.05544 | hotspot | 11.91834 |
| CDKN2A_GOF_100 | CDKN2A | -1.05544 | hotspot | 30.31478 |
| STK11_GOF_84 | STK11 | -1.05544 | hotspot | 17.49544 |
| FGFR4_GOF | FGFR4 | -1.05544 | hotspot | 3.387318 |
| KEAP1_GOF_483 | KEAP1 | -1.05544 | hotspot | 18.56752 |
| KEAP1_GOF_234 | KEAP1 | -1.05544 | hotspot | 18.56752 |
| GABRA6_GOF | GABRA6 | -1.05544 | hotspot | 4.881387 |
| CDKN2A_GOF_80 | CDKN2A | -1.05544 | hotspot | 30.31478 |
| MYCN_GOF_44 | MYCN | -1.05544 | hotspot | 2.999544 |
| KEAP1_GOF_260 | KEAP1 | -1.05544 | hotspot | 18.56752 |
| MAP2K1_GOF_102 | MAP2K1 | -1.05544 | hotspot | 1.893248 |

(continued)

| Gene_input | Gene Name | Contribution To Response | Input | Percent In population (n = 8768) |
|---|---|---|---|---|
| | | **Innate Resistance** | | |
| PTEN_GOF | PTEN | -1.05544 | hotspot | 6.683394 |
| PTCH1_GOF_48 | PTCH1 | -1.05544 | hotspot | 4.550639 |
| GNAQ_GOF | GNAQ | -1.05544 | hotspot | 0.775547 |
| KEAP1_GOF_364 | KEAP1 | -1.05544 | hotspot | 18.56752 |
| POLD1_GOF | POLD1 | -1.05544 | hotspot | 4.71031 |
| PIK3CA_GOF_1043 | PIK3CA | -1.05544 | hotspot | 14.18796 |
| BCOR_GOF_679 | BCOR | -1.05544 | hotspot | 8.462591 |
| FH_GOF_476 | FH | -1.05544 | hotspot | 3.63823 |
| STK11_GOF_181 | STK11 | -1.05544 | hotspot | 17.49544 |
| TP53_GOF_285 | TP53 | -1.05544 | hotspot | 66.40055 |
| TNFAIP3_GOF | TNFAIP3 | -1.05544 | hotspot | 2.395073 |
| BRD4_GOF | BRD4 | -1.05544 | hotspot | 3.980383 |
| KDM5C_GOF_1546 | KDM5C | -1.05544 | hotspot | 5.782391 |
| KEAP1_GOF_274 | KEAP1 | -1.05544 | hotspot | 18.56752 |
| SMAD4_GOF_351 | SMAD4 | -1.05544 | hotspot | 4.64188 |
| RNF43_GOF | RNF43 | -1.05544 | hotspot | 2.657391 |
| SF3B1_GOF_666 | SF3B1 | -1.05544 | hotspot | 4.607664 |
| MTOR_GOF_1834 | MTOR | -1.05544 | hotspot | 6.090329 |
| NOTCH1_GOF | NOTCH1 | -1.05544 | hotspot | 9.272354 |
| TP53_GOF_272 | TP53 | -1.00728 | hotspot | 66.40055 |
| GATA6_GOF | GATA6 | -1.00728 | hotspot | 3.832117 |
| MDM2_LOF | MDM2 | -1.00728 | hotspot | 5.964872 |
| GATA3_GOF | GATA3 | -1.00728 | hotspot | 4.037409 |
| PIK3CA_GOF_1047 | PIK3CA | -0.82472 | hotspot | 14.18796 |
| TP53_GOF_278 | TP53 | -0.82472 | hotspot | 66.40055 |
| CD79A_LOF | CD79A | -0.82472 | hotspot | 1.414234 |
| RPTOR_GOF | RPTOR | -0.82472 | hotspot | 4.19708 |
| ZNF703_GOF | ZNF703 | -0.8035 | hotspot | 5.554288 |
| CCNE1_GOF | CCNE1 | -0.73203 | hotspot | 4.208485 |
| STK11_LOF | STK11 | -0.72345 | hotspot | 17.49544 |
| MYCN_LOF | MYCN | -0.71132 | hotspot | 2.999544 |
| PARP1_GOF | PARP1 | -0.69758 | hotspot | 3.330292 |
| CEBPA_LOF | CEBPA | -0.69758 | hotspot | 3.854927 |
| MAP2K1_GOF_121 | MAP2K1 | -0.65962 | hotspot | 1.893248 |
| SMARCA4_GOF_1243 | SMARCA 4 | -0.65962 | hotspot | 12.60265 |
| MED12_GOF | MED12 | -0.65962 | hotspot | 8.131843 |
| KEAP1_GOF_135 | KEAP1 | -0.65962 | hotspot | 18.56752 |
| AR_GOF_69 | AR | -0.65962 | hotspot | 8.177464 |

| Innate Resistance | | | | |
|---|---|---|---|---|
| Gene_input | Gene Name | Contribution To Response | Input | Percent In population (n = 8768) |
| BTG1_GOF | BTG1 | -0.65962 | hotspot | 0.775547 |
| MAP3K1_GOF_5 | MAP3K1 | -0.65962 | hotspot | 4.356752 |
| TYRO3_GOF | TYRO3 | -0.65962 | hotspot | 3.740876 |
| ERBB2_GOF_755 | ERBB2 | -0.65962 | hotspot | 6.421077 |
| CBL_GOF_1096 | CBL | -0.65962 | hotspot | 3.649635 |
| STK11_GOF_176 | STK11 | -0.65962 | hotspot | 17.49544 |
| EGFR_GOF_790 | EGFR | -0.65962 | hotspot | 19.34307 |
| RAF1_GOF | RAF1 | -0.65962 | hotspot | 1.494069 |
| KEAP1_GOF_244 | KEAP1 | -0.65962 | hotspot | 18.56752 |
| STK11_GOF_464 | STK11 | -0.65962 | hotspot | 17.49544 |
| STK11_GOF_308 | STK11 | -0.65962 | hotspot | 17.49544 |
| KDM6A_GOF | KDM6A | -0.65962 | hotspot | 4.812956 |
| PDGFRB_GOF | PDGFRB | -0.65962 | hotspot | 4.539234 |
| BRAF_GOF_464 | BRAF | -0.65962 | hotspot | 7.607208 |
| PIK3C2G_GOF_129 | PIK3C2G | -0.65962 | hotspot | 9.42062 |
| FBXW7_GOF_505 | FBXW7 | -0.65962 | hotspot | 3.934763 |
| KEAP1_GOF_470 | KEAP1 | -0.65962 | hotspot | 18.56752 |
| ALOX12B_GOF | ALOX12B | -0.65962 | hotspot | 1.505475 |
| FLT3_GOF | FLT3 | -0.65962 | hotspot | 4.562044 |
| AMER1_GOF_625 | AMER1 | -0.65962 | hotspot | 7.960766 |
| IDH2_GOF_140 | IDH2 | -0.65962 | hotspot | 1.311588 |
| GNAS_GOF_407 | GNAS | -0.65962 | hotspot | 10.28741 |
| KEAP1_GOF_186 | KEAP1 | -0.65962 | hotspot | 18.56752 |
| BCOR_GOF_1526 | BCOR | -0.65962 | hotspot | 8.462591 |
| NFE2L2_GOF_27 | NFE2L2 | -0.65962 | hotspot | 6.455292 |
| STK11_GOF_251 | STK11 | -0.65962 | hotspot | 17.49544 |
| CHEK1_GOF | CHEK1 | -0.65962 | hotspot | 1.528285 |
| HRAS_GOF_13 | HRAS | -0.65962 | hotspot | 0.969434 |
| KEAP1_GOF_236 | KEAP1 | -0.65962 | hotspot | 18.56752 |
| GATA4_GOF | GATA4 | -0.65962 | hotspot | 2.862683 |
| MET_GOF_2888 | MET | -0.65962 | hotspot | 8.257299 |
| KMT2D_GOF_755 | KMT2D | -0.65962 | hotspot | 17.66651 |
| RAD51C_GOF_21 | RAD51C | -0.65962 | hotspot | 1.437044 |
| SMARCA4_GOF_1162 | SMARCA 4 | -0.65962 | hotspot | 12.60265 |
| STK11_GOF_734 | STK11 | -0.65962 | hotspot | 17.49544 |
| MAP3K1_GOF_949 | MAP3K1 | -0.65962 | hotspot | 4.356752 |
| PALB2_GOF | PALB2 | -0.65962 | hotspot | 3.216241 |
| BCORL1_GOF_883 | BCORL1 | -0.65962 | hotspot | 8.32573 |

(continued)

| Innate Resistance | | | | |
|---|---|---|---|---|
| Gene_input | Gene Name | Contribution To Response | Input | Percent In population (n = 8768) |
| KEAP1_GOF | KEAP1 | -0.65962 | hotspot | 18.56752 |
| ARID1A_GOF_515 | ARID1A | -0.65962 | hotspot | 11.88412 |
| AR_GOF_469 | AR | -0.65962 | hotspot | 8.177464 |
| EGFR_GOF_763 | EGFR | -0.65962 | hotspot | 19.34307 |
| AR_GOF_70 | AR | -0.65962 | hotspot | 8.177464 |
| PPARG_GOF | PPARG | -0.65962 | hotspot | 1.676551 |
| VHL_GOF | VHL | -0.65962 | hotspot | 1.00365 |
| PARP3_GOF | PARP3 | -0.65962 | hotspot | 1.106296 |
| CDK4_GOF | CDK4 | -0.65552 | hotspot | 3.820712 |
| AKT2_GOF | AKT2 | -0.63635 | hotspot | 3.546989 |
| TP53_GOF_920 | TP53 | -0.60259 | hotspot | 66.40055 |
| NFE2L2_GOF_30 | NFE2L2 | -0.60259 | hotspot | 6.455292 |
| CHEK2_GOF | CHEK2 | -0.60259 | hotspot | 4.12865 |
| SMAD2_LOF | SMAD2 | -0.58541 | hotspot | 1.5625 |
| SMARCB1_GOF | SMARCB 1 | -0.58541 | hotspot | 1.630931 |
| TP53_GOF_298 | TP53 | -0.58541 | hotspot | 66.40055 |
| SDHA_GOF_457 | SDHA | -0.58541 | hotspot | 12.10082 |
| FH_GOF | FH | -0.57806 | hotspot | 3.63823 |
| CDC73_GOF | CDC73 | -0.57806 | hotspot | 3.136405 |
| NFKBIA_GOF | NFKBIA | -0.57663 | hotspot | 6.979927 |
| ERCC4_LOF | ERCC4 | -0.57314 | hotspot | 2.988139 |
| RARA_GOF | RARA | -0.4431 | hotspot | 2.383668 |
| RAD51B_LOF | RAD51B | -0.4431 | hotspot | 1.334398 |
| NTRK2_LOF | NTRK2 | -0.4019 | hotspot | 3.558394 |
| CUL4A_LOF | CUL4A | -0.34779 | hotspot | 2.543339 |
| STK11_GOF_57 | STK11 | -0.31841 | hotspot | 17.49544 |
| TP53_GOF_234 | TP53 | -0.31841 | hotspot | 66.40055 |
| STK11_GOF_242 | STK11 | -0.31841 | hotspot | 17.49544 |
| CHEK2_GOF_367 | CHEK2 | -0.31841 | hotspot | 4.12865 |
| RAD51D_GOF | RAD51D | -0.31841 | hotspot | 1.471259 |
| CTCF_LOF | CTCF | -0.28798 | hotspot | 2.144161 |
| EPHA3_GOF | EPHA3 | -0.28798 | hotspot | 11.04015 |
| RAD54L_LOF | RAD54L | -0.27916 | hotspot | 1.881843 |
| PIK3CB_LOF | PIK3CB | -0.27448 | hotspot | 3.980383 |
| KLHL6_GOF | KLHL6 | -0.2703 | hotspot | 9.021442 |
| PIK3CA_GOF | PIK3CA | -0.26748 | hotspot | 14.18796 |
| DDR2_GOF | DDR2 | -0.23599 | hotspot | 6.443887 |
| MAP3K13_GOF | MAP3K13 | -0.2318 | hotspot | 7.527372 |

(continued)

| Innate Resistance | | | | |
|---|---|---|---|---|
| Gene_input | Gene Name | Contribution To Response | Input | Percent In population (n = 8768) |
| PIK3CA_GOF_545 | PIK3CA | -0.20301 | hotspot | 14.18796 |
| HGF_LOF | HGF | -0.1972 | hotspot | 9.990876 |
| NFE2L2_GOF_31 | NFE2L2 | -0.18072 | hotspot | 6.455292 |
| MET_GOF_3028 | MET | -0.18072 | hotspot | 8.257299 |
| BCL6_GOF | BCL6 | -0.16433 | hotspot | 7.162409 |
| TP53_GOF_342 | TP53 | -0.10542 | hotspot | 66.40055 |
| CDKN2A_GOF_83 | CDKN2A | -0.05772 | hotspot | 30.31478 |

**Table 7B:** Coefficient information for the innate CPI resistance group - updated following additional analysis

| Innate Resistance | | |
|---|---|---|
| Binary gene input | | |
| Gene Feature | Contribution to response | Popularity* |
| RARA | -0.812 | 0.417 |
| VHL | -0.7 | 0.667 |
| GID4 | -0.698 | 0.167 |
| IKBKE | -0.666 | 0.917 |
| CEBPA | -0.657 | 0.667 |
| CD79A | -0.63 | 0.833 |
| CDK12 | -0.62 | 0.333 |
| STK11 | -0.551 | 1 |
| BAP1 | -0.543 | 0.25 |
| CDKN2B | -0.527 | 1 |
| FGF10 | -0.514 | 1 |
| ARFRP1 | -0.481 | 0.083 |
| CSF1R | -0.481 | 0.25 |
| FANCC | -0.45 | 0.083 |
| CDC73 | -0.39 | 0.083 |
| ZNF703 | -0.381 | 0.25 |
| PIK3CA | -0.379 | 0.083 |
| NKX2-1 | -0.374 | 0.75 |
| TNFAIP3 | -0.374 | 0.167 |
| CCNE1 | -0.358 | 0.333 |
| SOX2 | -0.342 | 0.167 |
| SDHC | -0.334 | 0.5 |
| MYC | -0.316 | 0.167 |
| TERC | -0.296 | 0.083 |
| PARP3 | -0.254 | 0.333 |
| JAK1 | -0.227 | 0.083 |

(continued)

| Innate Resistance | | |
| --- | --- | --- |
| Binary gene input | | |
| Gene Feature | Contribution to response | Popularity* |
| DDR2 | -0.219 | 0.083 |
| PARP1 | -0.212 | 0.083 |
| AKT2 | -0.144 | 0.167 |
| PDK1 | -0.134 | 0.25 |
| NFE2L2 | -0.127 | 0.333 |
| MDM4 | -0.074 | 0.083 |
| HFM/LFM input | | |
| Gene Feature | Contribution to response | Popularity |
| MYC_LFM | -1.957 | 1 |
| FANCC_HFM | -1.617 | 0.583 |
| JAK1_HFM | -1.617 | 0.75 |
| FGFR2_HFM | -1.397 | 0.917 |
| GABRA6_HFM | -1.392 | 0.167 |
| RARA_HFM | -1.314 | 1 |
| ERBB4_HFM | -1.262 | 0.417 |
| CD79A_LFM | -1.262 | 0.583 |
| GID4_LFM | -1.105 | 0.333 |
| IGF1R_HFM | -1.105 | 0.25 |
| GNA11_HFM | -1.105 | 0.167 |
| GATA6_HFM | -0.99 | 0.833 |
| CDC73_HFM | -0.927 | 0.083 |
| CHEK2_HFM | -0.888 | 0.917 |
| ARFRP1_HFM | -0.862 | 0.917 |
| SMARCB1 HFM | -0.854 | 0.417 |
| FGF10_HFM | -0.791 | 1 |
| CDK12_HFM | -0.786 | 0.083 |
| IKBKE_LFM | -0.777 | 0.917 |
| DDR2_HFM | -0.712 | 0.833 |
| NF2_HFM | -0.702 | 0.083 |
| AKT3_HFM | -0.702 | 0.083 |
| CEBPA_LFM | -0.7 | 0.5 |
| FGF14_HFM | -0.696 | 0.083 |
| EP300_HFM | -0.696 | 0.083 |
| CCNE1_HFM | -0.659 | 0.25 |
| AKT2_HFM | -0.659 | 0.5 |
| BAP1_LFM | -0.625 | 0.333 |
| FH_HFM | -0.615 | 0.417 |

(continued)

| HFM/LFM input | | |
|---|---|---|
| Gene Feature | Contribution to response | Popularity |
| CEBPA_HFM | -0.59 | 0.083 |
| ERBB2_HFM | -0.571 | 0.667 |
| CCND2_LFM | -0.564 | 0.167 |
| ATM_HFM | -0.564 | 0.25 |
| PIK3CA_LFM | -0.558 | 0.833 |
| STK11_HFM_ | -0.542 | 1 |
| CDKN2B_LFM | -0.526 | 0.917 |
| PRDM1_LFM | -0.517 | 0.083 |
| NOTCH3_HFM | -0.513 | 0.083 |
| CDK12_LFM | -0.505 | 0.083 |
| ZNF703_HFM | -0.487 | 0.917 |
| CSF1R_LFM | -0.481 | 0.333 |
| KLHL6_HFM | -0.464 | 0.5 |
| BCL2L2_HFM | -0.458 | 0.083 |
| LYN_HFM | -0.458 | 0.083 |
| RICTOR_HFM | -0.449 | 0.417 |
| SOX2_HFM | -0.433 | 0.083 |
| STK11_LFM | -0.426 | 0.083 |
| KEAP1_HFM | -0.422 | 0.5 |
| CYP17A1_LFM | -0.41 | 0.083 |
| CUL4A_HFM | -0.408 | 0.083 |
| PRKCI_HFM | -0.343 | 0.167 |
| PIK3CA HFM | -0.323 | 0.083 |
| TERC_HFM | -0.296 | 0.167 |
| IKZF1_HFM | -0.294 | 0.167 |
| MEF2B_HFM | -0.288 | 0.417 |
| NFE2L2_HFM | -0.273 | 0.083 |
| CDKN2A_LFM | -0.262 | 0.167 |
| NKX2-1_HFM | -0.259 | 0.167 |
| TEK_LFM | -0.254 | 0.083 |
| MYCL_LFM | -0.224 | 0.083 |
| ROS1_HFM | -0.224 | 0.083 |
| MCL1_HFM | -0.218 | 0.083 |
| FGF12_HFM | -0.199 | 0.083 |
| EZH2_HFM | -0.154 | 0.083 |
| MDM4_HFM | -0.134 | 0.083 |

(continued)

| Hotspot granular input | | |
| --- | --- | --- |
| **Gene Feature** | **Contribution to response** | **Popularity** |
| EGFR_HFM_746 | -2.213 | 1 |
| MYC_LFM | -1.957 | 0.833 |
| FANCC_HFM | -1.617 | 0.667 |
| JAK1_HFM | -1.617 | 0.917 |
| NFE2L2_HFM_79 | -1.617 | 0.333 |
| GABRA6_HFM_ | -1.392 | 0.167 |
| RARA_HFM_ | -1.314 | 1 |
| ERBB4_HFM | -1.262 | 0.583 |
| CD79A_LFM | -1.262 | 0.25 |
| NF2_HFM | -1.217 | 0.917 |
| GNA11_HFM | -1.105 | 0.333 |
| CHEK2_HFM | -1.105 | 0.417 |
| GID4 LFM | -1.105 | 0.333 |
| NFE2L2_HFM 29 | -1.105 | 0.167 |
| IGF1R_HFM | -1.105 | 0.333 |
| GATA6_HFM | -0.99 | 0.25 |
| CDC73_HFM | -0.927 | 0.583 |
| FGFR2_HFM | -0.921 | 0.667 |
| CHEK2_HFM_157 | -0.921 | 0.167 |
| STK11_HFM_242 | -0.921 | 0.25 |
| TP53_HFM_152 | -0.921 | 0.167 |
| STK11_HFM_53 | -0.921 | 0.167 |
| ARFRP1_HFM_ | -0.862 | 0.667 |
| TP53_HFM 282 | -0.854 | 0.167 |
| FGF10_HFM | -0.791 | 1 |
| CDK12_HFM | -0.786 | 0.167 |
| IKBKE_LFM | -0.777 | 0.917 |
| DDR2_HFM | -0.712 | 0.833 |
| NKX2-1 LFM | -0.704 | 0.083 |
| AKT3_HFM | -0.702 | 0.5 |
| ERBB2_HFM_775 | -0.7 | 0.167 |
| TP53_HFM_920 | -0.7 | 0.417 |
| CEBPA_LFM | -0.7 | 0.167 |
| DIS3_HFM | -0.7 | 0.083 |
| GATA3_HFM | -0.698 | 0.167 |
| EP300 HFM | -0.696 | 0.083 |
| PIK3CA_HFM_1047 | -0.696 | 0.167 |
| FGF14_HFM | -0.696 | 0.083 |

(continued)

| Hotspot granular input | | |
|---|---|---|
| Gene Feature | Contribution to response | Popularity |
| ARAF_HFM_181 | -0.696 | 0.167 |
| AKT2_HFM | -0.659 | 0.417 |
| CCNE1_HFM | -0.659 | 0.333 |
| BAP1_LFM | -0.625 | 0.25 |
| FH_HFM | -0.615 | 0.167 |
| TP53_HFM 154 | -0.615 | 0.417 |
| PIK3CA HFM | -0.57 | 1 |
| VHL LFM | -0.564 | 0.167 |
| ERBB3_HFM | -0.564 | 0.167 |
| H3F3A HFM | -0.564 | 0.083 |
| CCND2_LFM | -0.564 | 0.083 |
| PIK3CA_LFM | -0.558 | 0.583 |
| CDKN2B_LFM | -0.526 | 1 |
| PRDM1_LFM | -0.517 | 0.083 |
| STK11_HFM_194 | -0.513 | 0.083 |
| TP53_HFM_135 | -0.513 | 0.25 |
| KEAP1_HFM 320 | -0.513 | 0.167 |
| ZNF703_HFM | -0.487 | 0.917 |
| CSF1R_LFM | -0.481 | 0.5 |
| KLHL6_HFM | -0.464 | 0.25 |
| BCL2L2_HFM | -0.458 | 0.167 |
| LYN_HFM | -0.458 | 0.083 |
| SOX2_HFM | -0.433 | 0.167 |
| STK11_LFM | -0.426 | 0.583 |
| SDHCHFM | -0.416 | 0.083 |
| EPHA3 HFM | -0.41 | 0.083 |
| CUL4A_HFM | -0.408 | 0.083 |
| STK11 HFM | -0.408 | 0.167 |
| MAP3K13 HFM | -0.344 | 0.083 |
| BCL6 HFM | -0.335 | 0.083 |
| TERC_HFM | -0.296 | 0.25 |
| NFE2L2_HFM_28 | -0.288 | 0.083 |
| JAK3_HFM | -0.288 | 0.167 |
| TP53_HFM_272 | -0.288 | 0.083 |
| CDKN2A_LFM | -0.262 | 0.083 |
| TEK_LFM | -0.254 | 0.083 |
| ROS1_HFM | -0.224 | 0.167 |
| NFKBIA HFM | -0.198 | 0.167 |

(continued)

| Hotspot granular input | | |
|---|---|---|
| **Gene Feature** | **Contribution to response** | **Popularity** |
| FGF19 HFM | -0.071 | 0.083 |
| * Popularity is the occurrence frequency of the gene feature in the 12 genetic signature outputs for each input type. | | |

**Table 8A**: feature set scores for binary gene input

| Binary Gene Inputs Feature Set 1 | | | |
|---|---|---|---|
| **Train Validation Score** | **Train Validation Score** | **Test Score** | **Chemo Test Score** |
| Accuracy | 0.695 +- 0.008 | 0.575 +- 0.002 | 0.471 +- 0.002 |
| F1 | 0.704 +- 0.011 | 0.599 +- 0.003 | 0.536 +- 0.002 |
| Precision | 0.677 +- 0.025 | 0.566 +- 0.002 | 0.475 +- 0.001 |
| Recall | 0.742 +- 0.012 | 0.650 +- 0.005 | 0.613 +- 0.003 |
| Matthews CorrCoef | 0.394 +- 0.028 | 0.152 +- 0.005 | -0.068 +- 0.004 |
| Area under ROC Curve | 0.704 +- 0.011 | 0.596 +- 0.002 | 0.457 +- 0.002 |
| Binary Gene Inputs Feature Set 2 | | | |
|  | **Train Validation Score** | **Test Score** | **Chemo Test Score** |
| Accuracy | 0.697 +- 0.014 | 0.573 +- 0.002 | 0.461 +- 0.001 |
| F1 | 0.709 +- 0.014 | 0.612 +- 0.002 | 0.543 +- 0.002 |
| Precision | 0.686 +- 0.016 | 0.553 +- 0.002 | 0.469 +- 0.001 |

| | | | |
|---|---|---|---|
| Recall | 0.742 +- 0.024 | 0.686 +- 0.005 | 0.641 +- 0.004 |
| Matthews CorrCoef | 0.418 +- 0.058 | 0.137 +- 0.005 | -0.084 +- 0.004 |
| Area under ROC Curve | 0.720 +- 0.006 | 0.588 +- 0.002 | 0.451 +- 0.002 |

| Binary Gene Inputs Feature Set 3 | | | |
|---|---|---|---|
| | Train Validation Score | Test Score | Chemo Test Score |
| Accuracy | 0.703 +- 0.014 | 0.604 +- 0.002 | 0.498 +- 0.002 |
| F1 | 0.719 +- 0.017 | 0.636 +- 0.002 | 0.570 +- 0.001 |
| Precision | 0.661 +- 0.005 | 0.595 +- 0.002 | 0.502 +- 0.001 |
| Recall | 0.770 +- 0.027 | 0.677 +- 0.005 | 0.665 +- 0.003 |
| Matthews CorrCoef | 0.410 +- 0.020 | 0.216 +- 0.004 | -0.005 +- 0.003 |
| Area under ROC Curve | 0.720 +- 0.015 | 0.629 +- 0.002 | 0.487 +- 0.002 |

| Binary Gene Inputs Feature Set 4 | | | |
|---|---|---|---|
| | Train Validation Score | Test Score | Chemo Test Score |
| Accuracy | 0.688 +- 0.025 | 0.595 +- 0.002 | 0.484 +- 0.002 |
| F1 | 0.716 +- 0.016 | 0.624 +- 0.002 | 0.567 +- 0.002 |
| Precision | 0.685 +- 0.022 | 0.577 +- 0.002 | 0.490 +- 0.001 |
| Recall | 0.753 +- 0.020 | 0.673 +- 0.004 | 0.670 +- 0.004 |
| Matthews CorrCoef | 0.405 +- 0.030 | 0.184 +- 0.005 | -0.031 +- 0.004 |
| Area under ROC Curve | 0.718 +- 0.017 | 0.601 +- 0.002 | 0.471 +- 0.002 |

| Binary Gene Inputs Feature Set 5 | | | |
|---|---|---|---|
| | Train Validation Score | Test Score | Chemo Test Score |
| Accuracy | 0.701 +- 0.008 | 0.592 +- 0.002 | 0.507 +- 0.001 |
| F1 | 0.710 +- 0.009 | 0.626 +- 0.002 | 0.583 +- 0.002 |
| Precision | 0.682 +- 0.006 | 0.577 +- 0.002 | 0.503 +- 0.001 |
| Recall | 0.756 +- 0.027 | 0.677 +- 0.004 | 0.687 +- 0.004 |
| Matthews CorrCoef | 0.369 +- 0.033 | 0.188 +- 0.005 | 0.008 +- 0.003 |
| Area under ROC Curve | 0.709 +- 0.009 | 0.617 +- 0.002 | 0.505 +- 0.001 |

| Binary Gene Inputs Feature Set 6 | | | |
|---|---|---|---|

|  | Train Validation Score | Test Score | Chemo Test Score |
|---|---|---|---|
| Accuracy | 0.700 +- 0.013 | 0.615 +- 0.002 | 0.491 +- 0.002 |
| F1 | 0.705 +- 0.019 | 0.647 +- 0.002 | 0.567 +- 0.002 |
| Precision | 0.668 +- 0.019 | 0.605 +- 0.002 | 0.497 +- 0.001 |
| Recall | 0.759 +- 0.022 | 0.694 +- 0.004 | 0.662 +- 0.004 |
| Matthews CorrCoef | 0.397 +- 0.039 | 0.240 +- 0.004 | -0.013 +- 0.003 |
| Area under ROC Curve | 0.721 +- 0.019 | 0.644 +- 0.002 | 0.492 +- 0.002 |

| Binary Gene Inputs Feature Set 7 | | | |
|---|---|---|---|
|  | Train Validation Score | Test Score | Chemo Test Score |
| Accuracy | 0.700 +- 0.014 | 0.599 +- 0.002 | 0.493 +- 0.002 |
| F1 | 0.707 +- 0.013 | 0.627 +- 0.002 | 0.567 +- 0.002 |
| Precision | 0.678 +- 0.013 | 0.593 +- 0.002 | 0.495 +- 0.002 |
| Recall | 0.751 +- 0.037 | 0.677 +- 0.004 | 0.666 +- 0.004 |
| Matthews CorrCoef | 0.367 +- 0.018 | 0.202 +- 0.004 | -0.020 +- 0.004 |
| Area under ROC Curve | 0.717 +- 0.006 | 0.609 +- 0.002 | 0.469 +- 0.002 |

| Binary Gene Inputs Feature Set 8 | | | |
|---|---|---|---|
|  | Train Validation Score | Test Score | Chemo Test Score |
| Accuracy | 0.692 +- 0.006 | 0.594 +- 0.002 | 0.465 +- 0.001 |
| F1 | 0.707 +- 0.022 | 0.621 +- 0.002 | 0.540 +- 0.002 |
| Precision | 0.680 +- 0.022 | 0.584 +- 0.002 | 0.475 +- 0.001 |
| Recall | 0.737 +- 0.016 | 0.667 +- 0.004 | 0.632 +- 0.004 |
| Matthews CorrCoef | 0.402 +- 0.033 | 0.193 +- 0.005 | -0.076 +- 0.003 |
| Area under ROC Curve | 0.713 +- 0.019 | 0.623 +- 0.002 | 0.464 +- 0.002 |

| Binary Gene Inputs Feature Set 9 | | | |
|---|---|---|---|
|  | Train Validation Score | Test Score | Chemo Test Score |
| Accuracy | 0.690 +- 0.015 | 0.596 +- 0.002 | 0.461 +- 0.001 |
| F1 | 0.720 +- 0.010 | 0.635 +- 0.002 | 0.542 +- 0.002 |
| Precision | 0.671 +- 0.009 | 0.577 +- 0.002 | 0.472 +- 0.001 |
| Recall | 0.748 +- 0.027 | 0.709 +- 0.004 | 0.636 +- 0.004 |

| Matthews CorrCoef | 0.372 +- 0.019 | 0.206 +- 0.004 | -0.089 +- 0.003 |
|---|---|---|---|
| Area under ROC Curve | 0.722 +- 0.008 | 0.621 +- 0.002 | 0.449 +- 0.001 |

**Binary Gene Inputs Feature Set 10**

|  | Train Validation Score | Test Score | Chemo Test Score |
|---|---|---|---|
| Accuracy | 0.681 +- 0.015 | 0.564 +- 0.002 | 0.477 +- 0.002 |
| F1 | 0.713 +- 0.023 | 0.613 +- 0.003 | 0.553 +- 0.002 |
| Precision | 0.656 +- 0.013 | 0.552 +- 0.002 | 0.480 +- 0.001 |
| Recall | 0.759 +- 0.029 | 0.690 +- 0.005 | 0.645 +- 0.004 |
| Matthews CorrCoef | 0.409 +- 0.032 | 0.139 +- 0.005 | -0.059 +- 0.003 |
| Area under ROC Curve | 0.699 +- 0.012 | 0.577 +- 0.002 | 0.479 +- 0.002 |

**Binary Gene Inputs Feature Set 11**

|  | Train Validation Score | Test Score | Chemo Test Score |
|---|---|---|---|
| Accuracy | 0.707 +- 0.009 | 0.584 +- 0.003 | 0.472 +- 0.002 |
| F1 | 0.721 +- 0.014 | 0.635 +- 0.002 | 0.554 +- 0.002 |
| Precision | 0.700 +- 0.015 | 0.570 +- 0.002 | 0.478 +- 0.001 |
| Recall | 0.740 +- 0.026 | 0.724 +- 0.004 | 0.660 +- 0.003 |
| Matthews CorrCoef | 0.408 +- 0.042 | 0.183 +- 0.005 | -0.064 +- 0.003 |
| Area under ROC Curve | 0.724 +- 0.010 | 0.610 +- 0.002 | 0.476 +- 0.002 |

**Binary Gene Inputs Feature Set 12**

|  | Train Validation Score | Test Score | Chemo Test Score |
|---|---|---|---|
| Accuracy | 0.686 +- 0.019 | 0.579 +- 0.002 | 0.467 +- 0.002 |
| F1 | 0.707 +- 0.020 | 0.617 +- 0.002 | 0.554 +- 0.002 |
| Precision | 0.665 +- 0.016 | 0.567 +- 0.002 | 0.478 +- 0.001 |
| Recall | 0.773 +- 0.020 | 0.683 +- 0.005 | 0.667 +- 0.004 |
| Matthews CorrCoef | 0.392 +- 0.032 | 0.162 +- 0.004 | -0.074 +- 0.004 |
| Area under ROC Curve | 0.717 +- 0.020 | 0.595 +- 0.002 | 0.468 +- 0.002 |

**Table 8B:** feature set scores for binary gene input - updated following additional analysis

| Binary gene genetic signatures | | | |
|---|---|---|---|
| Genetic signature | Train Validation ROC AUC | CPI test ROC AUC | Chemo test ROC AUC |
| 1 | 0.695 ± 0.024 | 0.601 | 0.489 |
| 2 | 0.685 ± 0.014 | 0.589 | 0.489 |
| 3 | 0.662 ± 0.03 | 0.601 | 0.496 |
| 4 | 0.651 ± 0.011 | 0.641 | 0.477 |
| 5 | 0.675 ± 0.031 | 0.618 | 0.488 |
| 6 | 0.675 ± 0.03 | 0.63 | 0.462 |
| 7 | 0.694 ± 0.027 | 0.6 | 0.493 |
| 8 | 0.679 ± 0.046 | 0.635 | 0.517 |
| 9 | 0.677 ± 0.018 | 0.63 | 0.472 |
| 10 | 0.68 ± 0.012 | 0.67 | 0.472 |
| 11 | 0.669 ± 0.017 | 0.606 | 0.468 |
| 12 | 0.666 ± 0.041 | 0.624 | 0.501 |
| average | 0.676 ± 0.013 | 0.62 | 0.485 |

**Table 9A**: feature set scores for GOF/LOF gene input

| Gain or Loss of Function Feature Set 1 | | | |
|---|---|---|---|
| | Train Validation Score | Test Score | Chemo Test Score |
| Accuracy | 0.722 +- 0.009 | 0.583 +- 0.002 | 0.498 +- 0.002 |
| F1 | 0.746 +- 0.009 | 0.598 +- 0.002 | 0.553 +- 0.002 |
| Precision | 0.714 +- 0.015 | 0.582 +- 0.002 | 0.498 +- 0.001 |
| Recall | 0.797 +- 0.010 | 0.609 +- 0.004 | 0.626 +- 0.003 |
| Matthews CorrCoef | 0.485 +- 0.031 | 0.171 +- 0.004 | -0.005 +- 0.003 |
| Area under ROC Curve | 0.744 +- 0.021 | 0.612 +- 0.002 | 0.501 +- 0.002 |
| Gain or Loss of Function Feature Set 2 | | | |
| | Train Validation Score | Test Score | Chemo Test Score |
| Accuracy | 0.736 +- 0.020 | 0.571 +- 0.003 | 0.505 +- 0.002 |
| F1 | 0.738 +- 0.013 | 0.599 +- 0.002 | 0.565 +- 0.002 |

| | Train Validation Score | Test Score | Chemo Test Score |
|---|---|---|---|
| Precision | 0.719 +- 0.007 | 0.562 +- 0.002 | 0.504 +- 0.001 |
| Recall | 0.778 +- 0.020 | 0.630 +- 0.004 | 0.651 +- 0.003 |
| Matthews CorrCoef | 0.474 +- 0.018 | 0.142 +- 0.004 | 0.009 +- 0.004 |
| Area under ROC Curve | 0.752 +- 0.016 | 0.580 +- 0.002 | 0.514 +- 0.002 |
| Gain or Loss of Function Feature Set 3 | | | |
| | Train Validation Score | Test Score | Chemo Test Score |
| Accuracy | 0.732 +- 0.014 | 0.565 +- 0.002 | 0.507 +- 0.002 |
| F1 | 0.737 +- 0.011 | 0.588 +- 0.002 | 0.571 +- 0.002 |
| Precision | 0.697 +- 0.008 | 0.556 +- 0.002 | 0.508 +- 0.001 |
| Recall | 0.786 +- 0.016 | 0.642 +- 0.005 | 0.649 +- 0.003 |
| Matthews CorrCoef | 0.443 +- 0.037 | 0.134 +- 0.004 | 0.022 +- 0.004 |
| Area under ROC Curve | 0.754 +- 0.015 | 0.603 +- 0.002 | 0.491 +- 0.002 |
| Gain or Loss of Function Feature Set 4 | | | |
| | Train Validation Score | Test Score | Chemo Test Score |
| Accuracy | 0.730 +- 0.013 | 0.557 +- 0.002 | 0.501 +- 0.002 |
| F1 | 0.732 +- 0.014 | 0.582 +- 0.003 | 0.560 +- 0.002 |
| Precision | 0.698 +- 0.011 | 0.549 +- 0.002 | 0.499 +- 0.001 |
| Recall | 0.797 +- 0.015 | 0.617 +- 0.004 | 0.635 +- 0.003 |
| Matthews CorrCoef | 0.444 +- 0.025 | 0.113 +- 0.004 | -0.004 +- 0.004 |
| Area under ROC Curve | 0.749 +- 0.013 | 0.606 +- 0.002 | 0.508 +- 0.002 |
| Gain or Loss of Function Feature Set 5 | | | |

|  | Train Validation Score | Test Score | Chemo Test Score |
|---|---|---|---|
| Accuracy | 0.724 +- 0.023 | 0.560 +- 0.002 | 0.508 +- 0.002 |
| F1 | 0.735 +- 0.011 | 0.572 +- 0.002 | 0.570 +- 0.002 |
| Precision | 0.716 +- 0.015 | 0.552 +- 0.002 | 0.505 +- 0.001 |
| Recall | 0.773 +- 0.013 | 0.596 +- 0.005 | 0.645 +- 0.004 |
| Matthews CorrCoef | 0.450 +- 0.041 | 0.115 +- 0.004 | 0.019 +- 0.003 |
| Area under ROC Curve | 0.740 +- 0.013 | 0.613 +- 0.002 | 0.498 +- 0.002 |

Gain or Loss of Function Feature Set 6

|  | Train Validation Score | Test Score | Chemo Test Score |
|---|---|---|---|
| Accuracy | 0.721 +- 0.017 | 0.583 +- 0.002 | 0.509 +- 0.002 |
| F1 | 0.740 +- 0.009 | 0.606 +- 0.002 | 0.583 +- 0.002 |
| Precision | 0.691 +- 0.014 | 0.578 +- 0.002 | 0.506 +- 0.001 |
| Recall | 0.770 +- 0.016 | 0.640 +- 0.004 | 0.682 +- 0.003 |
| Matthews CorrCoef | 0.482 +- 0.035 | 0.170 +- 0.003 | 0.020 +- 0.004 |
| Area under ROC Curve | 0.752 +- 0.030 | 0.602 +- 0.002 | 0.503 +- 0.002 |

Gain or Loss of Function Feature Set 7

|  | Train Validation Score | Test Score | Chemo Test Score |
|---|---|---|---|
| Accuracy | 0.728 +- 0.012 | 0.558 +- 0.002 | 0.528 +- 0.002 |
| F1 | 0.738 +- 0.015 | 0.588 +- 0.002 | 0.578 +- 0.002 |
| Precision | 0.704 +- 0.021 | 0.547 +- 0.002 | 0.523 +- 0.001 |
| Recall | 0.778 +- 0.033 | 0.621 +- 0.005 | 0.639 +- 0.004 |

| | Train Validation Score | Test Score | Chemo Test Score |
|---|---|---|---|
| Matthews CorrCoef | 0.471 +- 0.041 | 0.117 +- 0.004 | 0.060 +- 0.003 |
| Area under ROC Curve | 0.733 +- 0.017 | 0.587 +- 0.002 | 0.515 +- 0.002 |
| Gain or Loss of Function Feature Set 8 | | | |
| | Train Validation Score | Test Score | Chemo Test Score |
| Accuracy | 0.733 +- 0.015 | 0.598 +- 0.002 | 0.498 +- 0.001 |
| F1 | 0.737 +- 0.011 | 0.610 +- 0.003 | 0.558 +- 0.002 |
| Precision | 0.711 +- 0.012 | 0.589 +- 0.002 | 0.498 +- 0.001 |
| Recall | 0.762 +- 0.011 | 0.639 +- 0.004 | 0.635 +- 0.004 |
| Matthews CorrCoef | 0.446 +- 0.031 | 0.200 +- 0.005 | 0.003 +- 0.003 |
| Area under ROC Curve | 0.760 +- 0.015 | 0.622 +- 0.002 | 0.483 +- 0.002 |
| Gain or Loss of Function Feature Set 9 | | | |
| | Train Validation Score | Test Score | Chemo Test Score |
| Accuracy | 0.723 +- 0.006 | 0.560 +- 0.002 | 0.514 +- 0.002 |
| F1 | 0.752 +- 0.020 | 0.585 +- 0.002 | 0.572 +- 0.002 |
| Precision | 0.727 +- 0.017 | 0.551 +- 0.002 | 0.512 +- 0.001 |
| Recall | 0.748 +- 0.033 | 0.617 +- 0.004 | 0.651 +- 0.004 |
| Matthews CorrCoef | 0.463 +- 0.019 | 0.117 +- 0.005 | 0.030 +- 0.004 |
| Area under ROC Curve | 0.739 +- 0.031 | 0.572 +- 0.003 | 0.522 +- 0.002 |
| Gain or Loss of Function Feature Set 10 | | | |
| | Train Validation Score | Test Score | Chemo Test Score |
| Accuracy | 0.722 +- 0.008 | 0.550 +- 0.002 | 0.477 +- 0.002 |

| | 0.736 +- | | |
|---|---|---|---|
| F1 | 0.736 +- 0.011 | 0.568 +- 0.002 | 0.548 +- 0.002 |
| Precision | 0.718 +- 0.020 | 0.544 +- 0.002 | 0.483 +- 0.001 |
| Recall | 0.778 +- 0.020 | 0.593 +- 0.003 | 0.637 +- 0.003 |
| Matthews CorrCoef | 0.432 +- 0.045 | 0.102 +- 0.004 | -0.043 +- 0.003 |
| Area under ROC Curve | 0.751 +- 0.009 | 0.554 +- 0.002 | 0.468 +- 0.002 |

Gain or Loss of Function Feature Set 11

| | Train Validation Score | Test Score | Chemo Test Score |
|---|---|---|---|
| Accuracy | 0.697 +- 0.016 | 0.582 +- 0.002 | 0.501 +- 0.001 |
| F1 | 0.737 +- 0.006 | 0.598 +- 0.002 | 0.553 +- 0.002 |
| Precision | 0.694 +- 0.019 | 0.578 +- 0.002 | 0.501 +- 0.001 |
| Recall | 0.792 +- 0.016 | 0.615 +- 0.004 | 0.619 +- 0.003 |
| Matthews CorrCoef | 0.462 +- 0.021 | 0.169 +- 0.004 | 0.005 +- 0.003 |
| Area under ROC Curve | 0.748 +- 0.007 | 0.607 +- 0.002 | 0.488 +- 0.001 |

Gain or Loss of Function Feature Set 12

| | Train Validation Score | Test Score | Chemo Test Score |
|---|---|---|---|
| Accuracy | 0.726 +- 0.010 | 0.576 +- 0.002 | 0.490 +- 0.001 |
| F1 | 0.746 +- 0.010 | 0.584 +- 0.003 | 0.549 +- 0.001 |
| Precision | 0.707 +- 0.028 | 0.567 +- 0.002 | 0.493 +- 0.001 |
| Recall | 0.792 +- 0.006 | 0.615 +- 0.005 | 0.617 +- 0.003 |
| Matthews CorrCoef | 0.453 +- 0.023 | 0.160 +- 0.004 | -0.012 +- 0.003 |
| Area under ROC Curve | 0.750 +- 0.017 | 0.588 +- 0.002 | 0.481 +- 0.001 |

**Table 9B:** feature set scores for GOF/LOF gene input - updated following additional analysis

| HFM/LFM genetic signatures | | | |
|---|---|---|---|
| Genetic signature | Train Validation ROC AUC | CPI test ROC AUC | Chemo test ROC AUC |
| 1 | $0.718 \pm 0.015$ | 0.601 | 0.515 |
| 2 | $0.703 \pm 0.029$ | 0.636 | 0.523 |
| 3 | $0.709 \pm 0.016$ | 0.59 | 0.512 |
| 4 | $0.693 \pm 0.016$ | 0.607 | 0.505 |
| 5 | $0.724 \pm 0.017$ | 0.578 | 0.529 |
| 6 | $0.71 \pm 0.023$ | 0.595 | 0.501 |
| 7 | $0.734 \pm 0.017$ | 0.601 | 0.502 |
| 8 | $0.724 \pm 0.037$ | 0.607 | 0.507 |
| 9 | $0.743 \pm 0.018$ | 0.584 | 0.509 |
| 10 | $0.688 \pm 0.011$ | 0.618 | 0.52 |
| 11 | $0.744 \pm 0.014$ | 0.642 | 0.522 |
| 12 | $0.716 \pm 0.025$ | 0.584 | 0.505 |
| average | $0.717 \pm 0.018$ | 0.6 | 0.51 |

**Table 10A**: feature set scores for Hotspot gene input

| Hotspot Granular Feature Set 1 | | | |
|---|---|---|---|
| | Train Validation Score | Test Score | Chemo Test Score |
| Accuracy | 0.775 +- 0.022 | 0.555 +- 0.002 | 0.490 +- 0.002 |
| F1 | 0.758 +- 0.008 | 0.579 +- 0.002 | 0.570 +- 0.002 |
| Precision | 0.749 +- 0.013 | 0.553 +- 0.002 | 0.494 +- 0.001 |
| Recall | 0.786 +- 0.027 | 0.609 +- 0.004 | 0.679 +- 0.004 |
| Matthews CorrCoef | 0.508 +- 0.039 | 0.107 +- 0.004 | -0.021 +- 0.003 |
| Area under ROC Curve | 0.774 +- 0.013 | 0.561 +- 0.002 | 0.491 +- 0.002 |
| Hotspot Granular Feature Set 2 | | | |
| | Train Validation Score | Test Score | Chemo Test Score |
| Accuracy | 0.773 +- 0.022 | 0.579 +- 0.002 | 0.490 +- 0.001 |
| F1 | 0.766 +- 0.011 | 0.589 +- 0.004 | 0.562 +- 0.001 |

| | | Test Score | Chemo Test Score |
|---|---|---|---|
| Precision | 0.746 +- 0.013 | 0.574 +- 0.002 | 0.492 +- 0.001 |
| Recall | 0.797 +- 0.023 | 0.610 +- 0.003 | 0.661 +- 0.003 |
| Matthews CorrCoef | 0.503 +- 0.033 | 0.158 +- 0.004 | -0.022 +- 0.003 |
| Area under ROC Curve | 0.775 +- 0.014 | 0.562 +- 0.003 | 0.478 +- 0.002 |

**Hotspot Granular Feature Set 3**

| | Train Validation Score | Test Score | Chemo Test Score |
|---|---|---|---|
| Accuracy | 0.764 +- 0.015 | 0.585 +- 0.003 | 0.480 +- 0.001 |
| F1 | 0.788 +- 0.003 | 0.601 +- 0.003 | 0.554 +- 0.003 |
| Precision | 0.731 +- 0.022 | 0.577 +- 0.003 | 0.485 +- 0.001 |
| Recall | 0.808 +- 0.030 | 0.631 +- 0.004 | 0.645 +- 0.004 |
| Matthews CorrCoef | 0.541 +- 0.018 | 0.168 +- 0.005 | -0.036 +- 0.003 |
| Area under ROC Curve | 0.781 +- 0.014 | 0.611 +- 0.002 | 0.478 +- 0.002 |

**Hotspot Granular Feature Set 4**

| | Train Validation Score | Test Score | Chemo Test Score |
|---|---|---|---|
| Accuracy | 0.765 +- 0.021 | 0.554 +- 0.002 | 0.500 +- 0.002 |
| F1 | 0.770 +- 0.004 | 0.576 +- 0.003 | 0.573 +- 0.002 |
| Precision | 0.754 +- 0.027 | 0.548 +- 0.002 | 0.503 +- 0.001 |
| Recall | 0.805 +- 0.033 | 0.612 +- 0.005 | 0.668 +- 0.003 |
| Matthews CorrCoef | 0.535 +- 0.043 | 0.111 +- 0.004 | 0.006 +- 0.003 |
| Area under ROC Curve | 0.786 +- 0.008 | 0.566 +- 0.002 | 0.505 +- 0.002 |

**Hotspot Granular Feature Set 5**

|  | Train Validation Score | Test Score | Chemo Test Score |
|---|---|---|---|
| Accuracy | 0.760 +- 0.012 | 0.598 +- 0.002 | 0.499 +- 0.002 |
| F1 | 0.759 +- 0.014 | 0.606 +- 0.002 | 0.573 +- 0.002 |
| Precision | 0.747 +- 0.015 | 0.599 +- 0.002 | 0.500 +- 0.001 |
| Recall | 0.795 +- 0.022 | 0.615 +- 0.004 | 0.684 +- 0.003 |
| Matthews CorrCoef | 0.528 +- 0.040 | 0.201 +- 0.004 | -0.003 +- 0.004 |
| Area under ROC Curve | 0.777 +- 0.017 | 0.614 +- 0.002 | 0.521 +- 0.002 |
| Hotspot Granular Feature Set 6 |  |  |  |
|  | Train Validation Score | Test Score | Chemo Test Score |
| Accuracy | 0.754 +- 0.023 | 0.580 +- 0.002 | 0.472 +- 0.001 |
| F1 | 0.770 +- 0.017 | 0.587 +- 0.002 | 0.541 +- 0.002 |
| Precision | 0.756 +- 0.021 | 0.580 +- 0.002 | 0.478 +- 0.001 |
| Recall | 0.792 +- 0.015 | 0.595 +- 0.004 | 0.628 +- 0.003 |
| Matthews CorrCoef | 0.542 +- 0.010 | 0.154 +- 0.004 | -0.063 +- 0.002 |
| Area under ROC Curve | 0.786 +- 0.009 | 0.590 +- 0.002 | 0.476 +- 0.001 |
| Hotspot Granular Feature Set 7 |  |  |  |
|  | Train Validation Score | Test Score | Chemo Test Score |
| Accuracy | 0.764 +- 0.015 | 0.568 +- 0.002 | 0.489 +- 0.001 |
| F1 | 0.779 +- 0.009 | 0.579 +- 0.003 | 0.548 +- 0.002 |
| Precision | 0.754 +- 0.015 | 0.567 +- 0.002 | 0.493 +- 0.001 |
| Recall | 0.805 +- 0.010 | 0.593 +- 0.005 | 0.617 +- 0.003 |

| | Train Validation Score | Test Score | Chemo Test Score |
|---|---|---|---|
| Matthews CorrCoef | 0.524 +- 0.034 | 0.149 +- 0.004 | -0.021 +- 0.003 |
| Area under ROC Curve | 0.778 +- 0.010 | 0.585 +- 0.002 | 0.487 +- 0.002 |

**Hotspot Granular Feature Set 8**

| | Train Validation Score | Test Score | Chemo Test Score |
|---|---|---|---|
| Accuracy | 0.761 +- 0.010 | 0.559 +- 0.002 | 0.486 +- 0.001 |
| F1 | 0.773 +- 0.013 | 0.579 +- 0.002 | 0.564 +- 0.003 |
| Precision | 0.756 +- 0.017 | 0.558 +- 0.002 | 0.489 +- 0.001 |
| Recall | 0.797 +- 0.019 | 0.600 +- 0.004 | 0.670 +- 0.003 |
| Matthews CorrCoef | 0.540 +- 0.012 | 0.123 +- 0.004 | -0.030 +- 0.003 |
| Area under ROC Curve | 0.786 +- 0.026 | 0.565 +- 0.002 | 0.477 +- 0.002 |

**Hotspot Granular Feature Set 9**

| | Train Validation Score | Test Score | Chemo Test Score |
|---|---|---|---|
| Accuracy | 0.763 +- 0.020 | 0.587 +- 0.002 | 0.489 +- 0.001 |
| F1 | 0.775 +- 0.017 | 0.602 +- 0.002 | 0.555 +- 0.002 |
| Precision | 0.753 +- 0.024 | 0.583 +- 0.002 | 0.493 +- 0.001 |
| Recall | 0.797 +- 0.024 | 0.623 +- 0.004 | 0.636 +- 0.004 |
| Matthews CorrCoef | 0.533 +- 0.029 | 0.180 +- 0.004 | -0.023 +- 0.003 |
| Area under ROC Curve | 0.784 +- 0.016 | 0.607 +- 0.002 | 0.489 +- 0.002 |

**Hotspot Granular Feature Set 10**

| | Train Validation Score | Test Score | Chemo Test Score |
|---|---|---|---|
| Accuracy | 0.763 +- 0.014 | 0.578 +- 0.002 | 0.496 +- 0.002 |

| | | | |
|---|---|---|---|
| F1 | 0.765 +- 0.027 | 0.599 +- 0.003 | 0.540 +- 0.002 |
| Precision | 0.737 +- 0.025 | 0.571 +- 0.002 | 0.495 +- 0.001 |
| Recall | 0.789 +- 0.033 | 0.619 +- 0.004 | 0.598 +- 0.004 |
| Matthews CorrCoef | 0.487 +- 0.023 | 0.162 +- 0.004 | -0.007 +- 0.003 |
| Area under ROC Curve | 0.770 +- 0.019 | 0.591 +- 0.002 | 0.482 +- 0.002 |

Hotspot Granular Feature Set 11

| | Train Validation Score | Test Score | Chemo Test Score |
|---|---|---|---|
| Accuracy | 0.761 +- 0.015 | 0.590 +- 0.002 | 0.496 +- 0.001 |
| F1 | 0.796 +- 0.009 | 0.606 +- 0.003 | 0.543 +- 0.002 |
| Precision | 0.751 +- 0.020 | 0.587 +- 0.002 | 0.498 +- 0.001 |
| Recall | 0.805 +- 0.019 | 0.627 +- 0.004 | 0.600 +- 0.003 |
| Matthews CorrCoef | 0.542 +- 0.030 | 0.185 +- 0.004 | -0.006 +- 0.003 |
| Area under ROC Curve | 0.796 +- 0.013 | 0.608 +- 0.002 | 0.482 +- 0.002 |

Hotspot Granular Feature Set 12

| | Train Validation Score | Test Score | Chemo Test Score |
|---|---|---|---|
| Accuracy | 0.771 +- 0.009 | 0.564 +- 0.002 | 0.465 +- 0.001 |
| F1 | 0.763 +- 0.005 | 0.567 +- 0.002 | 0.537 +- 0.002 |
| Precision | 0.751 +- 0.010 | 0.565 +- 0.002 | 0.473 +- 0.001 |
| Recall | 0.770 +- 0.036 | 0.584 +- 0.004 | 0.624 +- 0.004 |
| Matthews CorrCoef | 0.532 +- 0.019 | 0.132 +- 0.004 | -0.073 +- 0.003 |
| Area under ROC Curve | | 0.559 +- 0.002 | 0.467 +- 0.002 |

**Table 10B:** feature set scores for Hotspot gene input - updated following additional analysis

| Hotspot granular genetic signatures | | | |
|---|---|---|---|
| Genetic signature | Train Validation ROC AUC | CPI test ROC AUC | Chemo test ROC AUC |
| 1 | 0.742 ± 0.018 | 0.59 | 0.514 |
| 2 | 0.746 ± 0.021 | 0.624 | 0.541 |
| 3 | 0.747 ± 0.025 | 0.561 | 0.529 |
| 4 | 0.733 ± 0.02 | 0.578 | 0.547 |
| 5 | 0.726 ± 0.024 | 0.595 | 0.514 |
| 6 | 0.692 ± 0.029 | 0.63 | 0.5 |
| 7 | 0.737 ± 0.007 | 0.595 | 0.517 |
| 8 | 0.727 ± 0.041 | 0.584 | 0.529 |
| 9 | 0.739 ± 0.033 | 0.561 | 0.537 |
| 10 | 0.741 ± 0.048 | 0.572 | 0.52 |
| 11 | 0.709 ± 0.043 | 0.63 | 0.511 |
| 12 | 0.747 ± 0.024 | 0.607 | 0.515 |
| average | 0.732 ± 0.017 | 0.59 | 0.52 |

**Table 11: Gene ID information**

| Gene_name | Transcript ID |
|---|---|
| ABL1 | NM_005157 |
| ABL1 | NM_007313 |
| ACVR1B | NM_020328 |
| AKT1 | NM_001014431 |
| AKT2 | NM_001626 |
| AKT3 | NM_005465 |
| AKT3 | NM_181690 |
| ALK | NM_004304 |
| ALOX12B | NM_001139 |
| APC | NM_000038 |
| AR | NM_000044 |
| AR | NM_001011645 |
| ARAF | NM_001654 |
| ARFRP1 | NM_003224 |
| ARFRP1 | NM_001134758 |
| ARID1A | NM_006015 |
| ARID1A | NM_139135 |
| ASXL1 | NM_015338 |
| ASXL1 | NM_001164603 |
| ATM | NM_000051 |
| ATR | NM_001184 |
| ATRX | NM_000489 |

(continued)

| Gene_name | Transcript ID |
|-----------|---------------|
| ATRX | NM_138270 |
| AURKA | NM_003600 |
| AURKB | NM_004217 |
| AXIN1 | NM_003502 |
| AXL | NM_001699 |
| AXL | NM_021913 |
| BAP1 | NM_004656 |
| BARD1 | NM_000465 |
| BCL2 | NM_000633 |
| BCL2 | NM_000657 |
| BCL2L1 | NM_138578 |
| BCL2L2 | NM_004050 |
| BCL6 | NM_001706 |
| BCOR | NM_017745 |
| BCOR | NM_001123385 |
| BCORL1 | NM_021946 |
| BRAF | NM_004333 |
| BRCA1 | NM_007294 |
| BRCA1 | NM_007300 |
| BRCA1 | NM_007299 |
| BRCA2 | NM_000059 |
| BRD4 | NM_014299 |
| BRD4 | NM_058243 |
| BRIP1 | NM_032043 |
| BTG1 | NM_001731 |
| BTK | NM_000061 |
| CARD11 | NM_032415 |
| CASP8 | NM_001228 |
| CASP8 | NM_033358 |
| CASP8 | NM_001080125 |
| CBFB | NM_022845 |
| CBFB | NM_001755 |
| CBL | NM_005188 |
| CCND1 | NM_053056 |
| CCND2 | NM_001759 |
| CCND3 | NM_001760 |
| CCND3 | NM_001136017 |
| CCNE1 | NM_001238 |
| CD274 | NM_014143 |

(continued)

| Gene_name | Transcript ID |
|---|---|
| CD79A | NM_001783 |
| CD79B | NM_000626 |
| CDC73 | NM_024529 |
| CDH1 | NM_004360 |
| CDK12 | NM_016507 |
| CDK4 | NM_000075 |
| CDK6 | NM_001259 |
| CDK8 | NM_001260 |
| CDKN1A | NM_000389 |
| CDKN1B | NM_004064 |
| CDKN2A | NM_000077 |
| CDKN2A | NM_058195 |
| CDKN2A | NM_058197 |
| CDKN2B | NM_078487 |
| CDKN2B | NM_004936 |
| CDKN2C | NM_001262 |
| CEBPA | NM_004364 |
| CHEK1 | NM_001274 |
| CHEK2 | NM_007194 |
| CHEK2 | NM_001005735 |
| CIC | NM_015125 |
| CIC | NM_001304815 |
| CREBBP | NM_004380 |
| CRKL | NM_005207 |
| CSF1R | NM_005211 |
| CTCF | NM_006565 |
| CTCF | NM_001191022 |
| CTNNA1 | NM_001903 |
| CTNNB1 | NM_001904 |
| CUL3 | NM_003590 |
| CUL4A | NM_003589 |
| CUL4A | NM_001008895 |
| CYP17A1 | NM_000102 |
| DAXX | NM_001350 |
| DAXX | NM_001141970 |
| DDR1 | NM_001954 |
| DDR1 | NM_001202523 |
| DDR1 | NM_013994 |
| DDR1 | NM_001202522 |

(continued)

| Gene_name | Transcript ID |
|-----------|---------------|
| DDR2_ | NM_006182 |
| DIS3 | NM_001128226 |
| DIS3 | NM_014953 |
| DNMT3A | NM_022552 |
| DNMT3A | NM_175630 |
| DOT1L | NM_032482 |
| EGFR | NM_005228 |
| EGFR | NM_201284 |
| EGFR | NM_201283 |
| EMSY | NM_020193 |
| EP300 | NM_001429 |
| EPHA3 | NM_005233 |
| EPHA3 | NM_182644 |
| EPHB1 | NM_004441 |
| EPHB4 | NM_004444 |
| ERBB2 | NM_004448 |
| ERBB3 | NM_001982 |
| ERBB3 | NM_001005915 |
| ERBB4 | NM_005235 |
| ERCC4 | NM_005236 |
| ERG | NM_182918 |
| ERG | NM_001136154 |
| ERRFI1 | NM_018948 |
| ESR1 | NM_000125 |
| EZH2 | NM_004456 |
| EZH2 | NM_001203249 |
| FANCA | NM_000135 |
| FANCC | NM_000136 |
| FANCG | NM_004629 |
| FANCL | NM_018062 |
| FANCL | NM_001114636 |
| FAS | NM_000043 |
| FAS | NM_152872 |
| FBXW7 | NM_033632 |
| FBXW7 | NM_018315 |
| FBXW7 | NM_001013415 |
| FGF10 | NM_004465 |
| FGF12 | NM_021032 |
| FGF12 | NM_004113 |

(continued)

| Gene_name | Transcript ID |
|-----------|---------------|
| FGF14 | NM_004115 |
| FGF14 | NM_175929 |
| FGF19 | NM_005117 |
| FGF23 | NM_020638 |
| FGF3 | NM_005247 |
| FGF4 | NM_002007 |
| FGF6 | NM_020996 |
| FGFR1 | NM_023110 |
| FGFR1 | NM_001174067 |
| FGFR1 | NM_001174065 |
| FGFR2 | NM_022970 |
| FGFR2 | NM_000141 |
| FGFR2 | NM_001144919 |
| FGFR3 | NM_000142 |
| FGFR4 | NM_022963 |
| FGFR4 | NM_002011 |
| FGFR4 | NM_213647 |
| FH | NM_000143 |
| FLCN | NM_144997 |
| FLCN | NM_144606 |
| FLT1 | NM_002019 |
| FLT1 | NM_001159920 |
| FLT1 | NM_001160030 |
| FLT3 | NM_004119 |
| FOXL2 | NM_023067 |
| FUBP1 | NM_003902 |
| GABRA6 | NM_000811 |
| GATA3 | NM_001002295 |
| GATA3 | NM_002051 |
| GATA4 | NM_002052 |
| GATA6 | NM_005257 |
| GNA11 | NM_002067 |
| GNA13 | NM_006572 |
| GNAQ | NM_002072 |
| GNAS | NM_000516 |
| GNAS | NM_080425 |
| GNAS | NM_016592 |
| GNAS | NM_001077490 |
| GRM3 | NM_000840 |

(continued)

| Gene_name | Transcript ID |
|---|---|
| GSK3B | NM_002093 |
| H3F3A | NM_002107 |
| HGF | NM_000601 |
| HGF | NM_001010931 |
| HNF1A | NM_000545 |
| HRAS | NM_176795 |
| HRAS | NM_005343 |
| HSD3B1 | NM_000862 |
| IDH1 | NM_005896 |
| IDH2 | NM_002168 |
| IGF1R | NM_000875 |
| IKBKE | NM_014002 |
| IKZF1 | NM_006060 |
| INPP4B | NM_003866 |
| IRF2 | NM_002199 |
| IRF4 | NM_002460 |
| IRS2 | NM_003749 |
| JAK1 | NM_002227 |
| JAK2 | NM_004972 |
| JAK3 | NM_000215 |
| JUN | NM_002228 |
| KDM5A | NM_001042603 |
| KDM5C | NM_004187 |
| KDM5C | NM_001146702 |
| KDM6A | NM_021140 |
| KDR | NM_002253 |
| KEAP1 | NM_012289 |
| KEL | NM_000420 |
| KIT | NM_000222 |
| KLH L6 | NM_130446 |
| KMT2D | NM_003482 |
| KRAS | NM_004985 |
| KRAS | NM_033360 |
| LTK | NM_002344 |
| LTK | NM_001135685 |
| LYN | NM_002350 |
| LYN | NM_001111097 |
| MAP2K1 | NM_002755 |
| MAP2K2 | NM_030662 |

(continued)

| Gene_name | Transcript ID |
|---|---|
| MAP2K4 | NM_003010 |
| MAP3K1 | NM_005921 |
| MAP3K13 | NM_004721 |
| MCL1 | NM_182763 |
| MCL1 | NM_001197320 |
| MDM2 | NM_002392 |
| MDM4 | NM_002393 |
| MED12 | NM_005120 |
| MEF2B | NM_001145785 |
| MEN1 | NM_130801 |
| MERTK | NM_006343 |
| MET | NM_000245 |
| MET | NM_001127500 |
| MITF | NM_198159 |
| MITF | NM_006722 |
| MITF | NM_000248 |
| MITF | NM_198177 |
| MKNK1 | NM_003684 |
| MKNK1 | NM_198973 |
| MLH1 | NM_000249 |
| MPL | NM_005373 |
| MSH2 | NM_000251 |
| MSH6 | NM_000179 |
| MST1R | NM_002447 |
| MTOR | NM_004958 |
| MUTYH | NM_001048171 |
| MUTYH | NM_001128425 |
| MUTYH | NM_001048172 |
| MYC | NM_002467 |
| MYCN | NM_005378 |
| MYD88 | NM_002468 |
| MYD88 | NM_001172568 |
| MYD88 | NM_001172567 |
| NBN | NM_002485 |
| NF1 | NM_001042492 |
| NF1 | NM_001128147 |
| NF2 | NM_000268 |
| NF2 | NM_181830 |
| NFE2L2 | NM_006164 |

(continued)

| Gene_name | Transcript ID |
|---|---|
| NFE2L2 | NM_001145412 |
| NFKBIA | NM_020529 |
| NKX2-1 | NM_003317 |
| NKX2-1 | NM_001079668 |
| NOTCH1 | NM_017617 |
| NOTCH2 | NM_024408 |
| NOTCH2 | NM_001200001 |
| NOTCH3 | NM_000435 |
| NPM1 | NM_002520 |
| NPM1 | NM_001037738 |
| NRAS | NM_002524 |
| NTRK1 | NM_002529 |
| NTRK1 | NM_001007792 |
| NTRK2 | NM_006180 |
| NTRK3 | NM_001007156 |
| NTRK3 | NM_001012338 |
| NTRK3 | NM_002530 |
| PALB2 | NM_024675 |
| PARP1 | NM_001618 |
| PARP2 | NM_005484 |
| PARP3 | NM_005485 |
| PARP3 | NM_001003931 |
| PAX5 | NM_016734 |
| PBRM1 | NM_018313 |
| PBRM1 | NM_181042 |
| PDCD1LG2 | NM_025239 |
| PDGFRA | NM_006206 |
| PDGFRB | NM_002609 |
| PDK1 | NM_002610 |
| PIK3C2B | NM_002646 |
| PIK3C2G | NM_004570 |
| PIK3CA | NM_006218 |
| PIK3CB | NM_006219 |
| PIK3R1 | NM_181523 |
| PIK3R1 | NM_181504 |
| PIK3R1 | NM_181524 |
| PMS2 | NM_000535 |
| POLD1 | NM_002691 |
| POLE | NM_006231 |

(continued)

| Gene_name | Transcript ID |
|-----------|---------------|
| PPARG | NM_015869 |
| PPP2R1A | NM_014225 |
| PRDM1 | NM_001198 |
| PRKAR1A | NM_212472 |
| PRKCI | NM_002740 |
| PTCH1 | NM_000264 |
| PTCH1 | NM_001083603 |
| PTEN | NM_000314 |
| PTPN 11 | NM_002834 |
| QKI | NM_206854 |
| QKI | NM_006775 |
| QKI | NM_206853 |
| QKI | NM_206855 |
| RAC1 | NM_006908 |
| RAC1 | NM_018890 |
| RAD51 | NM_133487 |
| RAD51 | NM_001164270 |
| RAD51 | NM_002875 |
| RAD51B | NM_133509 |
| RAD51C | NM_058216 |
| RAD51D | NM_002878 |
| RAD51D | NM_001142571 |
| RAD52 | NM_134424 |
| RAD54L | NM_003579 |
| RAF1 | NM_002880 |
| RARA | NM_000964 |
| RARA | NM_001024809 |
| RB1 | NM_000321 |
| RBM10 | NM_005676 |
| REL | NM_002908 |
| RET | NM_020975 |
| RET | NM_020630 |
| RICTOR | NM_152756 |
| RNF43 | NM_017763 |
| ROS1 | NM_002944 |
| RPTOR | NM_020761 |
| SDHA | NM_004168 |
| SDHB | NM_003000 |
| SDHC | NM_003001 |

## EP 4 515 000 B1

(continued)

| Gene_name | Transcript ID |
| --- | --- |
| SDHC | NM_001035511 |
| SDHD | NM_003002 |
| SETD2 | NM_014159 |
| SF3B1 | NM_012433 |
| SF3B1 | NM_001005526 |
| SMAD2 | NM_005901 |
| SMAD4 | NM_005359 |
| SMARCA4 | NM_003072 |
| MYCL | NM_001033082 |
| MYCL | NM_005376 |
| KMT2A | NM_005933 |
| GID4 | NM_024052 |
| AMER1 | NM_152424 |

Note that where a given gene name appears more than once in the above table, this indicates that the gene panel is designed to capture mutations from different isoforms of the gene in question (e.g. STAG2).

### References

A number of publications are cited above in order to more fully describe and disclose the invention and the state of the art to which the invention pertains.

1. Rotow, J. & Bivona, T. G. Understanding and targeting resistance mechanisms in NSCLC. Nature Reviews Cancer (2017) doi:10.1038/nrc.2017.84.

2. Inamura, K. Lung cancer: understanding its molecular pathology and the 2015 wHO classification. Front. Oncol. (2017) doi:10.3389/fonc.2017.00193.

3. Schadendorf, D. et al. Efficacy and safety outcomes in patients with advanced melanoma who discontinued treatment with nivolumab and ipilimumab because of adverse events: A pooled analysis of randomized phase II and III trials. J. Clin. Oncol. (2017) doi: 1 0.1200/JCO.2017. 73.2289.

4. Fehrenbacher, L. et al. Updated Efficacy Analysis Including Secondary Population Results for OAK: A Randomized Phase III Study of Atezolizumab versus Docetaxel in Patients with Previously Treated Advanced Non-Small Cell Lung Cancer. J. Thorac. Oncol. (2018) doi:10.1016/j.jtho.2018.04.039.

5. Bernard-Tessier, A. et al. Outcomes of long-term responders to anti-programmed death 1 and anti-programmed death ligand 1 when being rechallenged with the same anti-programmed death 1 and anti-programmed death ligand 1 at progression. Eur. J. Cancer (2018) doi: 1 0.1 016/j.ejca.2018.06.005.

6. Mazein, A., Watterson, S., Hsieh, W. Y., Griffiths, W. J. & Ghazal, P. A comprehensive machine-readable view of the mammalian cholesterol biosynthesis pathway. Biochem. Pharmacol. (2013) doi:10.1016/j.bcp.2013.03.021.

7. Antonia, S. J. et al. Four-year survival with nivolumab in patients with previously treated advanced non-small-cell lung cancer: a pooled analysis. Lancet Oncol. (2019) doi:10.1016/S14 70-2045(19)30407-3.

8. Schoenfeld, A. J. & Hellmann, M. D. Acquired Resistance to Immune Checkpoint Inhibitors. Cancer Cell vol. 37 443-455 (2020).

9. Sharma, P., Hu-Lieskovan, S., Wargo, J. A. & Ribas, A. Primary, Adaptive, and Acquired Resistance to Cancer Immunotherapy. Cell (2017) doi:10.1016/j.cell.2017.01.017.

10. Walsh, R. J. & Soo, R. A. Resistance to immune checkpoint inhibitors in non-small cell lung cancer: biomarkers and therapeutic strategies. Ther. Adv. Med. Oncol. 12, 1-22 (2020).

11. Lagos, G. G., Izar, B. & Rizvi, N. A. Beyond Tumor PD-L1: Emerging Genomic Biomarkers for Checkpoint Inhibitor Immunotherapy. Am. Soc. Clin. Oncol. Educ. B. (2020) doi:10.1200/edbk_289967.

12. Kalbasi, A. & Ribas, A. Tumour-intrinsic resistance to immune checkpoint blockade. Nature Reviews Immunology vol. 20 25-39 (2020).

13. Jiang, P. et al. Signatures of T cell dysfunction and exclusion predict cancer immunotherapy response. Nat. Med.

**105**

(2018) doi:10.1038/s41591-018-0136-1.

14. Hugo, W. et al. Genomic and Transcriptomic Features of Response to Anti-PD-1 Therapy in Metastatic Melanoma. Cell (2016) doi:10.1016/j.cell.2016.02.065.

15. Auslander, N. et al. Robust prediction of response to immune checkpoint blockade therapy in metastatic melanoma. Nat. Med. (2018) doi:10.1038/s41591-018-0157-9.

16. Anagnostou, V. et al. Integrative Tumor and Immune Cell Multi-omic Analyses Predict Response to Immune Checkpoint Blockade in Melanoma. Cell Reports Med. 1, (2020).

17. Singal, G. et al. Association of Patient Characteristics and Tumor Genomics With Clinical Outcomes Among Patients With Non-Small Cell Lung Cancer Using a Clinicogenomic Database. JAMA (2019) doi:10.1001/jama.2019.3241.

18. Frampton, G. M. et al. Development and validation of a clinical cancer genomic profiling test based on massively parallel DNA sequencing. Nat. Biotechnol. (2013) doi:10.1038/nbt.2696.

19. Kugel, C. H. et al. Age correlates with response to anti-PD1, reflecting age-related differences in intratumoral effector and regulatory T-cell populations. Clin. Cancer Res. (2018) doi:10.1158/1078-0432.CCR-18-1116.

20. Rizvi, N. A. et al. Mutational landscape determines sensitivity to PD-1 blockade in non-small cell lung cancer. Science (80-. ). (2015) doi:10.1126/science.aaa1348.

21. Norum, J. & Nieder, C. Tobacco smoking and cessation and PD-L1 inhibitors in non-small cell lung cancer (NSCLC): A review of the literature. ESMO Open (2018) doi: 1 0.1136/esmoopen-2018-000406.

22. Xu, J. et al. Heterogeneity of Li-Fraumeni Syndrome links to unequal gain-of-function effects of p53 mutations. Sci. Rep. (2014) doi:10.1038/srep04223.

23. Brosh, R. & Rotter, V. When mutants gain new powers: News from the mutant p53 field. Nature Reviews Cancer (2009) doi:10.1038/nrc2693.

24. Summers, M. G. et al. BRAF and NRAS locus-specific variants have different outcomes on survival to colorectal cancer. Clin. Cancer Res. (2017) doi:10.1158/1078-0432.CCR-16-1541.

25. De Roock, W. et al. Association of KRAS p.G13D mutation with outcome in patients with chemotherapy-refractory metastatic colorectal cancer treated with cetuximab. JAMA - J. Am. Med. Assoc. (2010) doi:10.1001/jama.2010.1535.

26. Kadosh, E. et al. The gut microbiome switches mutant p53 from tumour-suppressive to oncogenic. Nature (2020) doi:10.1038/s41586-020-2541-0.

27. Vogelstein, B. et al. Cancer genome landscapes. Science (2013) doi: 1 0.1126/science.1235122.

28. Skoulidis, F. et al. STK11/LKB1 mutations and PD-1 inhibitor resistance in KRAS-mutant lung adenocarcinoma. Cancer Discov. (2018) doi:10.1158/2159-8290.CD-18-0099.

29. Skoulidis, F. et al. Co-occurring genomic alterations define major subsets of KRAS-mutant lung adenocarcinoma with distinct biology, immune profiles, and therapeutic vulnerabilities. Cancer Discov. (2015) doi:10.1158/2159-8290.CD-14-1236.

30. Blumenthal, G. M. et al. Overall response rate, progression-free survival, and overall survival with targeted and standard therapies in advanced non-small-cell lung cancer: US Food and Drug Administration trial-level and patient-level analyses. J. Clin. Oncol. (2015) doi:10.1200/JCO.2014.59.0489.

31. Solomon, B. J. et al. Correlation between overall response rate and progression-free survival/overall survival in comparative trials involving targeted therapies in molecularly enriched populations. J. Clin. Oncol. (2020) doi:10.1200/jco.2020.38.15_suppl.3588.

32. Papillon-Cavanagh, S., Doshi, P., Dobrin, R., Szustakowski, J. & Walsh, A. M. STK11 and KEAP1 mutations as prognostic biomarkers in an observational real-world lung adenocarcinoma cohort. ESMO Open 5, e000706 (2020).

33. Rish, I. An empirical study of the naive Bayes classifier. IJCAI 2001 Work. Empir. methods Artif. Intell. (2001) doi:10.1039/b104835).

34. Van Allen, E. M. et al. Genomic correlates of response to CTLA-4 blockade in metastatic melanoma. Science (80-. ). (2015) doi:10.1126/science.aad0095.

35. Miao, D. et al. Genomic correlates of response to immune checkpoint blockade in microsatellite-stable solid tumors. Nat. Genet. (2018) doi:10.1038/s41588-018-0200-2.

36. Ng, A. Y. & Jordan, M. I. On discriminative vs. Generative classifiers: A comparison of logistic regression and naive bayes. in Advances in Neural Information Processing Systems (2002).

37. Hill, A. et al. Benchmarking network algorithms for contextualizing genes of interest. PLoS Comput. Biol. (2019) doi:10.1371/journal.pcbi.1007403.

38. O'Donnell, J. S., Long, G. V., Scolyer, R. A., Teng, M. W. L. & Smyth, M. J. Resistance to PD1/PDL1 checkpoint inhibition. Cancer Treatment Reviews (2017) doi:10.1016/j.ctrv.2016.11.007.

39. Jackson, C. M., Choi, J. & Lim, M. Mechanisms of immunotherapy resistance: lessons from glioblastoma. Nature Immunology (2019) doi:10.1038/s41590-019-0433-y.

40. Baugh, E. H., Ke, H., Levine, A. J., Bonneau, R. A. & Chan, C. S. Why are there hotspot mutations in the TP53 gene in human cancers? Cell Death and Differentiation (2018) doi:10.1038/cdd.2017.180.

41. Levine, A. J. p53: 800 million years of evolution and 40 years of discovery. Nature Reviews Cancer www.nature.com/nrc doi:10.1038/s41568-020-0262-1.

42. Bargonetti, J. & Prives, C. Gain-of-function mutant p53: History and speculation. Journal of Molecular Cell Biology (2019) doi:10.1093/jmcb/mjz067.

43. Cormedi, M. C. V., Van Allen, E. M. & Colli, L. M. Predicting immunotherapy response through genomics. Current Opinion in Genetics and Development vol. 66 1-9 (2021).

44. Liu, H. J. et al. TSC2-deficient tumors have evidence of T cell exhaustion and respond to anti-PD-1/anti-CTLA-4 immunotherapy. JCI insight (2018) doi:10.1172/jci.insight.98674.

45. Torrejon, D. Y. et al. Overcoming Genetically Based Resistance Mechanisms to PD-1 Blockade. Cancer Discov. (2020) doi:10.1158/2159-8290.CD-19-1409.

46. Liang, Y. et al. Targeting IFN$\alpha$ to tumor by anti-PD-L1 creates feedforward antitumor responses to overcome checkpoint blockade resistance. Nat. Commun. (2018) doi:10.1038/s41467-018-06890-y.

47. Reisländer, T., Groelly, F. J. & Tarsounas, M. DNA Damage and Cancer Immunotherapy: A STING in the Tale. Molecular Cell (2020) doi:10.1016/j.molcel.2020.07.026.

48. Gao, S. P. et al. Mutations in the EGFR kinase domain mediate STAT3 activation via IL-6 production in human lung adenocarcinomas. J. Clin. Invest. 117, 3846-3856 (2007).

49. Jiang, L. et al. Continuous targeted kinase inhibitors treatment induces upregulation of PD-L1 in resistant NSCLC. Sci. Rep. (2019) doi:10.1038/s41598-018-38068-3.

50. Liu, H., Shen, J. & Lu, K. IL-6 and PD-L1 blockade combination inhibits hepatocellular carcinoma cancer development in mouse model. Biochem. Biophys. Res. Commun. 486, 239-244 (2017).

51. Bialkowski, L. et al. Immune checkpoint blockade combined with IL-6 and TGF-$\beta$ inhibition improves the therapeutic outcome of mRNA-based immunotherapy. Int. J. Cancer 143, 686-698 (2018).

52. Keegan, A. et al. Plasma IL-6 changes correlate to PD-1 inhibitor responses in NSCLC. J. Immunother. Cancer (2020) doi:10.1136/jitc-2020-000678.

53. Garbers, C., Heink, S., Korn, T. & Rose-John, S. Interleukin-6: Designing specific therapeutics for a complex cytokine. Nature Reviews Drug Discovery (2018) doi:10.1038/nrd.2018.45.

54. Takeuchi, T. et al. Considering new lessons about the use of IL-6 inhibitors in arthritis. Considerations Med. (2018) doi:10.1136/conmed-2018-000002.

55. Yuen, K. C. et al. Abstract 2676: Associations of peripheral biomarkers to outcomes to anti-PD-L1 immune checkpoint blockade in metastatic urothelial cancer. in (2019). doi:10.1158/1538-7445.am2019-2676.

56. Çelik, A. et al. Angiogenic and Immune-Related Biomarkers and Outcomes Following Axitinib/Pembrolizumab Treatment in Patients with Advanced Renal Cell Carcinoma. J. Mater. Process. Technol. 1, 1-8 (2018).

57. Zhao, J. et al. Immune and genomic correlates of response to anti-PD-1 immunotherapy in glioblastoma. Nat. Med. (2019) doi:10.1038/s41591-019-0349-y.

58. Luke, J. J., Bao, R., Sweis, R. F., Spranger, S. & Gajewski, T. F. WNT/b-catenin pathway activation correlates with immune exclusion across human cancers. Clin. Cancer Res. (2019) doi:10.1158/1078-0432.CCR-18-1942.

59. Ohlund, D. et al. Distinct populations of inflammatory fibroblasts and myofibroblasts in pancreatic cancer. J. Exp. Med. (2017) doi:10.1084/jem.20162024.

60. Evans, E. K. et al. C A N C E R A precision therapy against cancers driven by KIT/PDGFRA mutations. www.cellsignal.com (2017).

61. Kim, G. & Ko, Y. T. Small molecule tyrosine kinase inhibitors in glioblastoma. Archives of Pharmacal Research vol. 43 385-394 (2020).

62. Tassell, V. et al. Preliminary biomarker analysis of sitravatinib in combination with nivolumab in NSCLC patients progressing on prior checkpoint inhibitor. J. Immunother. Cancer (2018).

63. Brose, M. S. et al. A phase Ib/II trial of lenvatinib plus pembrolizumab in non-small cell lung cancer. J. Clin. Oncol. (2019) doi:10.1200/jco.2019.37.8_suppl.16.

64. Du, W., Huang, H., Sorrelle, N. & Brekken, R. A. Sitravatinib potentiates immune checkpoint blockade in refractory cancer models. JCI insight (2018) doi: 1 0.1172/jci. insight.124184.

For standard molecular biology techniques, see Sambrook, J., Russel, D.W. Molecular Cloning, A Laboratory Manual. 3 ed. 2001, Cold Spring Harbor, New York: Cold Spring Harbor Laboratory Press

**Claims**

1. A computer-implemented method for predicting the treatment response of a subject having a lung cancer to an immune checkpoint inhibitor (CPI) therapy, the method comprising:

providing a mutation profile of the subject, said profile comprising the presence or absence of mutations at one or more locations in at least five, six, or at least seven genes selected from the group consisting of: NF1, STK11, TSC2, BRCA2, BRAF, STAG2, U2AF1, BRIP1, PDGFRA, CTNNA1 PDK1, FGF10 and FLT1;

analysing the mutation profile to classify the profile as matching the mutation profile of a response signature or a resistance signature,

wherein the subject is predicted to be likely to respond to the CPI therapy if the mutation profile for the subject is classified as matching the mutation profile of the response signature and is predicted to be likely not to respond to the CPI therapy if the mutation profile for the subject is classified as matching the mutation profile of the resistance signature.

2. The method of claim 1, wherein said mutations are cancer-specific mutations.

3. The method of claim 1 or claim 2, wherein said at least five genes comprise all of the genes set forth in one of the following gene sets:

(i) NF1, STK11, TSC2, STAG2, U2AF1, BRCA2, PDK1;
(ii) STK11, BRAF, BRIP1, U2AF1 and NF1;
(iii) STK11, PDGFRA, BRAF, BRIP1 and CTNNA1;
(iv) BRAF, BRIP1, CTNNA1, FGF10, FLT1, PDGFRA and STK11;
(v) NF1, STK11, TSC2, BRCA2, BRAF, STAG2, U2AF1, and PDK1;
(vi) BRAF, BRIP1, STK11, CDK12, CTNNA1, FAS, NRAS, NOTCH3, PIK3CA, and RAD51C;
(vii) STK11, BRIP1, CDKN2B, FLT1, FGF10, BRAF, ASXL1, HRAS, IDH1, BARD1, BRCA2, U2AF1 and CTNNA1;
(viii) NF1, STK11, TSC2, BRCA2, BRAF, STAG2, U2AF1, PDK1, and ATR;
(ix) NF1, STK11, ASXL1, FGF19, TSC2, BRAF, IDH1, STAG2, BRCA2, PDK1, U2AF1, NKX2-1, and PPP2R1A, and wherein the mutations are gain of function (GOF) and/or loss of function (LOF) mutations selected from: NF1_LOF, STK11_LOF, ASXL1_LOF, FGF19_LOF, TSC2_LOF, BRAF_GOF, IDH1_GOF, STAG2_LOF, BRCA2_LOF, PDK1_LOF, U2AF1_GOF, NKX2-1_LOF, and PPP2R1A_GOF;
(x) BRAF, BRIP1, FGF10, NF1, STK11, MAP3K13, NOTCH3, ALOX12B, U2AF1, RARA, MLH1, FLT1, MAP3K1, MYC, CTNNA1, NBN and ASXL1, and wherein the mutations are high frequency mutations ("HFM") and/or low frequency mutations ("LFM") selected from: BRAF_HFM, BRIP1_LFM, FGF10_HFM, NF1_HFM, STK11_HFM, MAP3K13_LFM, NOTCH3_LFM, ALOX12B_LFM, U2AF1_HFM, RARA_HFM, MLH1_LFM, FLT1_LFM, MAP3K1_LFM, MYC_LFM, CTNNA1_LFM, NBN_LFM, and ASXL1_LFM;
(xi) PBRM1, BRIP1, PTEN, CDKN2A, STK11, CDKN2B, U2AF1, CTNNA1, FGF10, FGF19, AKT2, NBN, ALOX12B, BRAF, and NF1, and wherein the mutations are gain of function (GOF) and/or loss of function (LOF) mutations selected from: PBRM1_LOF, BRIP1_LOF, PTEN_LOF, CDKN2A_LOF, STK11_GOF, CDKN2B_LOF, U2AF1_GOF, CTNNA1_LOF, FGF10_GOF, FGF19_LOF, AKT2_GOF, NBN_LOF, ALOX12-B_LOF, BRAF_GOF, and NF1_GOF;
(xii) NF1, STK11, ASXL1, KMT2A, NFKBIA, BRAF, TSC2, FGF19, NKX2-1, BRCA2, CDKN2A, PDK1, TP53, NFE2L2, U2AF1, EGFR, PPP2R1A, DNMT3A, and STAG2, and wherein the cancer-specific mutations are GOF and/or LOF mutations selected from: NF1_LOF, STK11_LOF, ASXL1_LOF, KMT2A_LOF, NFKBIA_GOF, BRAF_GOF_600, TSC2_LOF, FGF19_LOF, NKX2-1_LOF, BRCA2_LOF, CDKN2A_GOF_151, PDK1_LOF, TP53_GOF_282, NFE2L2_GOF_24, U2AF1_GOF_34, EGFR_GOF_719, PPP2R1A_GOF, DNMT3A_GOF_882, and STAG2_LOF, wherein the number following GOF indicates the position of the mutation in the amino acid sequence of the protein encoded by the gene;
(xiii) CDKN2B, FGF10, BRCA2, FLT1, BRIP1, RARA, DNMT3A_771, MAP3K13, BRAF_600, ALOX12B, BRAF_469, BCL6, XRCC2, EGFR_746, TSC1, PIK3C2G, TP53_331, PIK3CA, MLH1 and FAS, and wherein the mutations are HFM and/or LFM mutations selected from: CDKN2B_LFM, FGF10_HFM, BRCA2_LFM, FLT1_LFM, BRIP1_LFM, RARA_HFM, DNMT3A_HFM_771, MAP3K13_LFM, BRAF_HFM_600, ALOX12B_LFM, BRAF_HFM_469, BCL6_LFM, XRCC2_LFM, EGFR_HFM_746, TSC1_LFM, PIK3C2G_HFM, TP53_HFM_331, PIK3CA_HFM, MLH1_LFM and FAS_LFM;
(xiv) BRIP1, CDKN2B, U2AF1, CTNNA1, ALOX12B, EGFR, FAS, and KMT2A, and wherein the cancer-specific mutations are GOF and/or LOF mutations selected from: BRIP1_LOF, CDKN2B_LOF, U2AF1_GOF_34, CTNNA1_LOF, ALOX12B_LOF, EGFR_GOF_746, FAS_LOF, and KMT2A_LOF;
(xv) NF1, STK11, TSC2, BRCA2, BRAF, ATRX, STAG2, U2AF1, PDK1, ATR, ASXL1, ERCC4, PAX5, CTNNA1, CD79A, TSC1, NRAS, RARA, PDCD1LG2, NBN, PDGFRB, PDGFRA, CCNE1, JUN, IDH1, CDK4, NKX2-1, PPP2R1A, FH, MDM2, AKT1, NTRK2, FANCG, QKI, BRD4, CDKN1A, CEBPA, FANCL, and SMARCA4; and/or

(xvi) the set of genes set forth in Table 1A or Table 1B:
binary cluster 1; binary cluster 2; binary cluster 3; binary cluster 4; binary cluster 5; binary cluster 6; binary cluster 7; binary cluster 8; binary cluster 9; binary cluster 10; binary cluster 11; and/or binary cluster 12.

4. The method of any one of the preceding claims, wherein said mutations are cancer-specific mutations that are GOF and/or LOF mutations selected from

(i) the set of mutations set forth in Table 2A or Table 2B:
GOF/LOF cluster 1; GOF/LOF cluster 2; GOF/LOF cluster 3; GOF/LOF cluster 4; GOF/LOF cluster 5; GOF/LOF cluster 6; GOF/LOF cluster 7; GOF/LOF cluster 8; GOF/LOF cluster 9; GOF/LOF cluster 10; GOF/LOF cluster 11; and/or GOF/LOF cluster 12; and/or
(ii) the set of mutations set forth in Table 3A or Table 3B:
Hotspot cluster 1; Hotspot cluster 2; Hotspot cluster 3; Hotspot cluster 4; Hotspot cluster 5; Hotspot cluster 6; Hotspot cluster 7; Hotspot cluster 8; Hotspot cluster 9; Hotspot cluster 10; Hotspot cluster 11; and/or Hotspot cluster 12.

5. The method of any one of the preceding claims, wherein said analysing the mutation profile to classify the profile comprises:

inputting data representing the mutation profile of the subject into a machine learning classifier, wherein said machine learning classifier has been trained on a training data set comprising the mutation profiles of at least the same genes as those forming the mutation profile of the subject, wherein the mutation profiles of the training set are from a plurality of samples derived from lung cancer (e.g. NSCLC) patients known to have responded to CPI therapy and from a plurality of samples derived from lung cancer (e.g. NSCLC) patients known to have been resistant to CPI therapy; and
causing the machine learning classifier to classify the mutation profile of the subject as belonging to the response group or the resistance group.

6. The method of claim 5, wherein the training data set comprises mutation profiles of at least 50, at least 100 or at least 200 samples derived from lung cancer patients known to have responded to CPI therapy and mutation profiles of at least 50, at least 100 or at least 200 samples derived from lung cancer patients known to have been resistant to CPI therapy and/or

wherein the training data set comprises the Flatiron Health de-identified Clinico-Genomic Database (CGDB) as available on January 1, 2020 and/or
wherein the machine learning classifier is selected from the group consisting of: a Naïve Bayes model; a logistic regression model; an artificial neural network, a support vector machine (SVM); a random forest; and a perceptron.

7. The method of any one of the preceding claims,

(i) wherein the presence of a mutation in one or more of the following genes contributes to classifying the profile as matching the mutation profile of a response signature:
TSC2, U2AF1, FGF23, IDH1, PDCD1LG2, MEF2B, PDK1, BRIP1, QKI, CTNNA1, FUBP1, STAG2, FANCL, PAX5, MLH1, FANCG, AKT1, MPL, BRCA2, ATR, POLE, TSC1, FOXL2, BRAF, ASXL1, NF1, ATRX, NRAS, PDGFRA, SMAD4, NBN, PDGFRB, BRCA1, TP53, and SMARCA4;
(ii) wherein the presence of a mutation in one or more of the following genes contributes to classifying the profile as matching the mutation profile of a response signature:
CTNNA1, ALOX12B, HRAS, FAS, U2AF1, TSC1, MLH1, BRIP1, GNA13, ERRFI1, ACVR1B, IDH1, XRCC2, BRAF, SOX9, HNF1A, PDCD1LG2, ESR1, BRCA2, ASXL1, KEL, TERT, TSC2, BARD1, BCL2, QKI, PDGFRB, BTK, FOXL2, CARD11, PBRM1, EZH2, RAD51C, ABL1, ALK, HSD3B1, POLE, FLT1, NOTCH3, PAX5, MAP3K1, STAT3, GABRA6, TP53, CUL3, NBN, PDGFRA, SDHD, RBM10 and KRAS;
(iii) wherein the presence of one or more of the following GOF and/or LOF mutations contributes to classifying the profile as matching the mutation profile of a response signature:
FGF19_LOF, U2AF1_GOF, NBN_LOF, NRAS_LOF, RAC1_LOF, RB1_GOF, IDH1_GOF, XRCC2_LOF, PAX5_GOF, PDCD1LG2_GOF, CD274_GOF, RAD52_GOF, VEGFA_LOF, CBFB_LOF, AKT1_GOF, FGF23_LOF, PDK1_LOF, MYD88_GOF, CD79B_GOF, PRKAR1A_GOF, BRCA2_GOF, SDHC_LOF, GNA13_LOF, FANCG_GOF, FANCL_LOF, SOX2_LOF, EZH2_LOF, STAG2_LOF, RAD51C_LOF, QKI_LOF, FGF6_GOF, FGF23_GOF, MUTYH_GOF, SYK_LOF, CTNNA1_LOF, CDH1_LOF, PBRM1_LOF, PIK3C2G_GOF, ASXL1_LOF, BRAF_GOF, NF1_LOF, CHEK1_LOF, FUBP1_LOF, ERRFI1_LOF, BRCA2_LOF, HSD3B1_GOF, DNMT3A_GOF, WT1_LOF, PDGFRA_LOF, ATRX_LOF, ATR_LOF, RBM10_GOF, MAP2K2_GOF, BRIP1_GOF, PDCD1LG2_LOF, MEF2B_GOF, CREBBP_GOF, IRF2_GOF, BARD1_GOF, CTNNA1_GOF, MPL_GOF, ACVR1B_LOF, PPARG_LOF, TSC2_LOF, KMT2A_LOF, PTPN11_LOF, PDGFRB_LOF, AKT1_LOF, CHEK2_LOF, NOTCH2_GOF, MUTYH_LOF, TSC2_GOF, MAP3K1_LOF, ARID1A_GOF, MED12_GOF, CBFB_GOF, PIK3R1_GOF, STAG2_GOF, MAP2K4_GOF, FLCN_GOF, TSC1_GOF, and SETD2_GOF;

(iv) wherein the presence of one or more of the following GOF and/or LOF mutations contributes to classifying the profile as matching the mutation profile of a response signature:

ALOX12B_LFM, CTNNA1_LFM, HRAS_HFM, MEF2B_LFM, TNFRSF14_LFM, XRCC2_LFM, BRIP1_LFM, U2AF1_HFM, NF1_HFM, FAS_LFM, TSC1_LFM, ERRFI1_LFM, EZH2_LFM, NBN_LFM, MLH1_LFM, GNA13_LFM, FGF19_LFM, ALK_HFM, AMER1_HFM, BRAF_HFM, IDH1_HFM, DNMT3A_HFM, CDK6_LFM, BCL6_LFM, WT1_HFM, SOX9_LFM, SMAD4_LFM, HNF1A_LFM, JAK2_LFM, PAX5_HFM, HSD3B1_LFM, PARP2_LFM, ASXL1_HFM, ESR1_LFM, PIK3C2G_HFM, BRCA2_LFM, ASXL1_LFM, TERT_HFM, WT1_LFM, BARD1_LFM, MAP3K1_LFM, FGFR2_LFM, PPARG_LFM, AXIN1_LFM, PDCD1LG2_HFM, SOX9_HFM, KRAS_LFM, PRKAR1A_HFM, PDGFRB_LFM, TSC2_LFM, NF2_LFM, FGF6_LFM, PBRM1_LFM, KEL_LFM, FOXL2_LFM, CARD11_LFM, MAP3K13_LFM, FGF6_HFM, NOTCH3_LFM, SF3B1_LFM, CUL3_LFM, FLT1_LFM, CD274_HFM, IDH1_LFM, SPOP_LFM, MYCN_HFM, SRC_LFM, AR_LFM, ERBB2_LFM, CASP8_LFM, PDGFRA_LFM, IRF4_LFM, PTEN_LFM, GNAS_LFM, CCNE1_LFM, NOTCH2_LFM, ATR_LFM, RBM10_LFM, FANCG_HFM, SMO_HFM, EGFR_LFM and SPOP_HFM;

(v) wherein the presence of one or more of the following GOF and/or LOF mutations contributes to classifying the profile as matching the mutation profile of a response signature:

FGF19_LOF, TP53_GOF_331, NFE2L2_GOF_24, PIK3C2G_GOF_1088, U2AF1_GOF_34, NBN_LOF, NFE2L2_GOF_77, TP53_GOF_376, TP53_GOF_244, AKT1_GOF_17, RAC1_LOF, MYCN_GOF, CDKN2A_GOF_151, DNMT3A_GOF_882, NRAS_LOF, XRCC2_LOF, PAX5_GOF, BRAF_GOF_600, RAD52_GOF, PDCD1LG2_GOF, CD274_GOF, TP53_GOF_159, VEGFA_LOF, CBFB_LOF, PDK1_LOF, KDM5C_GOF_1330, KEAP1_GOF_332, STK11_GOF_37, BRCA2_GOF, KEAP1_GOF_116, MYD88_GOF_265, IDH1_GOF, AR_GOF_457, RB1_GOF, MUTYH_GOF_165, BRAF_GOF_466, POLE_GOF, TP53_GOF_673, CD79B_GOF, ARID1A_GOF_21, PRKAR1A_GOF, DIS3_GOF_458, CDKN2A_GOF_69, FANCL_LOF, FANCG_GOF, SOX2_LOF, SMAD4_GOF, BRAF_GOF_469, TP53_GOF_275, TP53_GOF_192, STAG2_LOF, QKI_LOF, FGF6_GOF, CTNNA1_LOF, MLH1_LOF, CDH1_LOF, ERBB3_LOF, PBRM1_LOF, ASXL1_LOF, KDM5A_GOF, NF1_LOF, CHEK1_LOF, BRCA2_LOF, TP53_GOF_560, WT1_LOF, SDHB_LOF, EGFR_GOF_858, TP53_GOF_215, PDGFRA_LOF, ATRX_LOF, ATR_LOF, IRF2_GOF, EGFR_GOF_771, EGFR_GOF_861, MLH1_GOF, AR_GOF_465, STK11_GOF_221, FAS_GOF, AMER1_GOF_385, RBM10_GOF_503, KEAP1_GOF_153, STK11_GOF_199, PTCH1_GOF, STK11_GOF_163, ATRX_GOF, KEAP1_GOF_509, TP53_GOF_236, KEAP1_GOF_362, HSD3B1_GOF_75, RB1_GOF_576, STK11_GOF_216, FBXW7_GOF_479, KEAP1_GOF_523, JAK3_GOF, PPARG_LOF, PIK3CB_GOF, ACVR1B_LOF, TSC2_LOF, KMT2A_LOF, TP53_GOF_238, AKT1_LOF, PIK3R1_LOF, IRS2_GOF, HRAS_GOF_61, PIK3CA_GOF_726, AR_GOF_468, TNFRSF14_LOF, NFE2L2_GOF_29, MEN1_GOF, BCL2_LOF, AR_GOF_467, ATM_GOF_337, KEAP1_GOF_544, NTRK3_GOF, NTRK3_GOF_610, HRAS_LOF, CTNNB1_GOF_41, SMARCA4_GOF_1160, KEAP1_GOF_409, SMAD2_GOF_464, PTEN_GOF_59, CTNNB1_GOF_33, CREBBP_GOF_1472, APC_GOF, PTEN_GOF_165, TSC1_GOF, KEAP1_GOF_417, RBM10_GOF, NBN_GOF_219, PIK3CA_GOF_345, AR_GOF_493, ZNF217_GOF_410, AR_GOF_598, STK11_GOF_256, ARID1A_GOF_343, RET_GOF_511, KEAP1_GOF_155, MITF_GOF, CDK8_GOF, DNMT3A_GOF_749, MYD88_GOF, KEAP1_GOF_430, GNAS_GOF_415, KDM5C_GOF, FLT1_GOF, NF1_GOF_1642, KMT2A_GOF_53, SDHA_GOF_531, CDKN2A_GOF_61, CEBPA_GOF_197, STK11_GOF_220, NBN_GOF_680, KEAP1_GOF_450, CHEK2_GOF_392, NKX2-1_GOF_234, SMARCA4_GOF_1157, BCORL1_GOF_94, KEAP1_GOF_493, FLCN_GOF_306, STK11_GOF_168, and MSH6_GOF_1088;

(vi) wherein the presence of one or more of the following GOF and/or LOF mutations contributes to classifying the profile as matching the mutation profile of a response signature:

ALOX12B_LFM, DNMT3A_HFM_771, CTNNA1_LFM, NFE2L2_HFM_24, MEF2B_LFM, TP53_HFM_331, BRAF_HFM_469, XRCC2_LFM, TNFRSF14_LFM, BRIP1_LFM, FUBP1_LFM, U2AF1_HFM_34, FAS_LFM, TP53_HFM_173, TSC1_LFM, ERRFI1_LFM, EZH2_LFM, NBN_LFM, MLH1_LFM, BRAF_HFM_600, FGF19_LFM, GNA13_LFM, ALK_HFM, TP53_HFM_215, TP53_HFM_783, TP53_HFM_560, CDK6_LFM, BCL6_LFM, TP53_HFM_280, BTG1_LFM, WT1_HFM, SOX9_LFM, SMAD4_LFM, NF1_HFM, JAK2_LFM, HSD3B1_LFM, PARP2_LFM, PIK3C2G_HFM, BRCA2_LFM, ASXL1_LFM, TERT_HFM_, BARD1_LFM, MAP3K1_LFM, FGFR2_LFM, SDHA_LFM, IGF1R_LFM, AXIN1_LFM, PDCD1LG2_HFM, PAX5_HFM, TP53_HFM_294, RAD51C_LFM, JUN_LFM, PRKAR1A_HFM, PDGFRB_LFM, TSC2_LFM, NF2_LFM, BTK_LFM, PBRM1_LFM, FOXL2_LFM, MAP3K13_LFM, NOTCH3_LFM, GABRA6_LFM, PIK3CA_HFM_542, TP53_HFM_159, CUL3_LFM, ASXL1_HFM, FLT1_LFM, MITF_LFM, TP53_HFM_249, IDH1_LFM, SRC_LFM, AR_LFM, ERBB2_LFM, SMO_LFM, KEAP1_LFM, PDGFRA_LFM, GATA6_LFM, PTEN_LFM, GNAS_LFM, CCNE1_LFM, ATR_LFM, MERTK_LFM, KMT2A_LFM, SOX9_HFM, GSK3B_HFM, CUL4A_LFM and BCL2L2_LFM;

(vii) wherein the presence of a mutation in one or more of the following genes contributes to classifying the profile as matching the mutation profile of a resistance signature:

GID4, JUN, RAD51, CD79A, STK11, TET2, MAP2K1, CCNE1, CDK4, ERCC4, CEBPA, RARA, CDKN1A, RAD51B, PPP2R1A, CSF1R, FH, NKX2-1, NTRK2, FGFR1, CDK6, MDM2, and BRD4;

(viii) wherein the presence of a mutation in one or more of the following genes contributes to classifying the profile as matching the mutation profile of a resistance signature:

RARA, VHL, GID4, IKBKE, CEBPA, CD79A, CDK12, STK11, BAP1, CDKN2B, FGF10, ARFRP1, CSF1R, FANCC, CDC73, ZNF703, PIK3CA, NKX2-1, TNFAIP3, CCNE1, SOX2, SDHC, MYC, TERC, PARP3, JAK1, DDR2, PARP1, AKT2, PDK1, NFE2L2 and MDM4;

(ix) wherein the presence of one or more of the following GOF and/or LOF mutations contributes to classifying the profile as matching the mutation profile of a resistance signature:

MAP3K1_GOF, FGF14_GOF, PPP2R1A_GOF, CDKN1A_GOF, JAK1_GOF, IRF4_GOF, JUN_LOF, AKT3_GOF, NKX2-1_LOF, TGFBR2_GOF, GABRA6_GOF, BCORL1_GOF, TNFAIP3_GOF, POLD1_GOF, GNAQ_GOF, RAD51C_GOF, BRD4_GOF, RNF43_GOF, FGFR4_GOF, GATA6_GOF, GATA3_GOF, MDM2_LOF, MYC_LOF, RPTOR_GOF, CD79A_LOF, CCNE1_GOF, STK11_LOF, MYCN_LOF, CEBPA_LOF, PARP1_GOF, NOTCH3_GOF, SDHD_GOF, LTK_GOF, DAXX_GOF, ABL1_GOF, PDGFRB_GOF, BTG1_GOF, CHEK1_GOF, GATA4_GOF, JUN_GOF, FLT3_GOF, CUL3_GOF, CDK4_GOF, AKT2_GOF, KDM6A_GOF, MTOR_GOF, BCL2L1_LOF, CD274_LOF, NF2_GOF, SMARCA4_GOF, NFKBIA_GOF, ERCC4_LOF, ZNF703_GOF, STK11_GOF, KEAP1_GOF, ERBB2_GOF, FH_GOF, REL_LOF, RARA_GOF, RAD51_LOF, PTEN_GOF, NTRK2_LOF, CDKN2B_LOF, BAP1_LOF, RARA_LOF, AXL_GOF, CCND2_LOF, CUL4A_LOF, H3F3A_GOF, GRM3_GOF, CDK6_GOF, ARFRP1_LOF, SUFU_LOF, RAD54L_LOF, PIK3CB_LOF, KLHL6_GOF, MAP3K13_GOF, PIK3CA_GOF, CDKN2A_LOF, EZH2_GOF, FANCA_LOF, HGF_LOF, FBXW7_GOF, FGFR2_GOF, AURKA_LOF, HSD3B1_LOF, and SDHC_GOF;

(x) wherein the presence of one or more of the following GOF and/or LOF mutations contributes to classifying the profile as matching the mutation profile of a resistance signature:

MYC_LFM, FANCC_HFM, JAK1_HFM, FGFR2_HFM, GABRA6_HFM, RARA_HFM, ERBB4_HFM, CD79A_LFM, GID4_LFM, IGF1R_HFM, GNA11_HFM, GATA6_HFM, CDC73_HFM, CHEK2_HFM, ARFRP1_HFM, SMARCB1_HFM, FGF10_HFM, CDK12_HFM, IKBKE_LFM, DDR2_HFM, NF2_HFM, AKT3_HFM, CEBPA_LFM, FGF14_HFM, EP300_HFM, CCNE1_HFM, AKT2_HFM, BAP1_LFM, FH_HFM, CEBPA_HFM, ERBB2_HFM, CCND2_LFM, ATM_HFM, PIK3CA_LFM, STK11_HFM, CDKN2B_LFM, PRDM1_LFM, NOTCH3_HFM, CDK12_LFM, ZNF703_HFM, CSF1R_LFM, KLHL6_HFM, BCL2L2_HFM, LYN_HFM, RICTOR_HFM, SOX2_HFM, STK11_LFM, KEAP1_HFM, CYP17A1_LFM, CUL4A_HFM, PRKCI_HFM, PIK3CA_HFM, TERC_HFM, IKZF1_HFM, MEF2B_HFM, NFE2L2_HFM, CDKN2A_LFM, NKX2-1_HFM, TEK_LFM, MYCL_LFM, ROS1_HFM, MCL1_HFM, FGF12_HFM, EZH2_HFM and MDM4_HFM;

(xi) wherein the presence of one or more of the following GOF and/or LOF mutations contributes to classifying the profile as matching the mutation profile of a resistance signature:

EGFR_GOF_746, TP53_GOF_282, EGFR_GOF_747, EGFR_GOF_719, TP53_GOF_195, FGF14_GOF, PPP2R1A_GOF, IRF4_GOF, MAP3K1_GOF, STK11_GOF_291, ERBB2_GOF_776, JAK1_GOF, CDKN1A_GOF, KEAP1_GOF_272, JUN_LOF, AKT3_GOF, NKX2-1_LOF, ATM_GOF, CDKN2A_GOF_100, STK11_GOF_84, FGFR4_GOF, KEAP1_GOF_483, KEAP1_GOF_234, GABRA6_GOF, CDKN2A_GOF_80, MYCN_GOF_44, KEAP1_GOF_260, MAP2K1_GOF_102, PTEN_GOF, PTCH1_GOF_48, GNAQ_GOF, KEAP1_GOF_364, POLD1_GOF, PIK3CA_GOF_1043, BCOR_GOF_679, FH_GOF_476, STK11_GOF_181, TP53_GOF_285, TNFAIP3_GOF, BRD4_GOF, KDM5C_GOF_1546, KEAP1_GOF_274, SMAD4_GOF_351, RNF43_GOF, SF3B1_GOF_666, MTOR_GOF_1834, NOTCH1_GOF, TP53_GOF_272, GATA6_GOF, MDM2_LOF, GATA3_GOF, PIK3CA_GOF_1047, TP53_GOF_278, CD79A_LOF, RPTOR_GOF, ZNF703_GOF, CCNE1_GOF, STK11_LOF, MYCN_LOF, PARP1_GOF, CEBPA_LOF, MAP2K1_GOF_121, SMARCA4_GOF_1243, MED12_GOF, KEAP1_GOF_135, AR_GOF_69, BTG1_GOF, MAP3K1_GOF_5, TYRO3_GOF, ERBB2_GOF_755, CBL_GOF_1096, STK11_GOF_176, EGFR_GOF_790, RAF1_GOF, KEAP1_GOF_244, STK11_GOF_464, STK11_GOF_308, KDM6A_GOF, PDGFRB_GOF, BRAF_GOF_464, PIK3C2G_GOF_129, FBXW7_GOF_505, KEAP1_GOF_470, ALOX12B_GOF, FLT3_GOF, AMER1_GOF_625, IDH2_GOF_140, GNAS_GOF_407, KEAP1_GOF_186, BCOR_GOF_1526, NFE2L2_GOF_27, STK11_GOF_251, CHEK1_GOF, HRAS_GOF_13, KEAP1_GOF_236, GATA4_GOF, MET_GOF_2888, KMT2D_GOF_755, RAD51C_GOF_21, SMARCA4_GOF_1162, STK11_GOF_734, MAP3K1_GOF_949, PALB2_GOF, BCORL1_GOF_883, KEAP1_GOF, ARID1A_GOF_515, AR_GOF_469, EGFR_GOF_763, AR_GOF_70, PPARG_GOF, VHL_GOF, PARP3_GOF, CDK4_GOF, AKT2_GOF, TP53_GOF_920, NFE2L2_GOF_30, CHEK2_GOF, SMAD2_LOF, SMARCB1_GOF, TP53_GOF_298, SDHA_GOF_457, FH_GOF, CDC73_GOF, NFKBIA_GOF, ERCC4_LOF, RARA_GOF, RAD51B_LOF, NTRK2_LOF, CUL4A_LOF, STK11_GOF_57, TP53_GOF_234, STK11_GOF_242, CHEK2_GOF_367, RAD51D_GOF, CTCF_LOF, EPHA3_GOF, RAD54L_LOF, PIK3CB_LOF, KLHL6_GOF, PIK3CA_GOF, DDR2_GOF, MAP3K13_GOF, PIK3CA_GOF_545, HGF_LOF, NFE2L2_GOF_31, MET_GOF_3028, BCL6_GOF, TP53_GOF_342, and CDKN2A_GOF_83; and/or

(xii) wherein the presence of one or more of the following GOF and/or LOF mutations contributes to classifying the profile as matching the mutation profile of a resistance signature:

EGFR_HFM_746, MYC_LFM, FANCC_HFM, JAK1_HFM, NFE2L2_HFM_79, GABRA6_HFM, RARA_HFM, ERBB4_HFM, CD79A_LFM, NF2_HFM, GNA11_HFM, CHEK2_HFM, GID4_LFM, NFE2L2_HFM_29, IGF1R_HFM, GATA6_HFM, CDC73_HFM, FGFR2_HFM, CHEK2_HFM_157, STK11_HFM_242, TP53_HFM_152, STK11_HFM_53, ARFRP1_HFM, TP53_HFM_282, FGF10_HFM, CDK12_HFM, IKBKE_LFM, DDR2_HFM, NKX2-1_LFM, AKT3_HFM, ERBB2_HFM_775, TP53_HFM_920, CEBPA_LFM, DIS3_HFM, GATA3_HFM, EP300_HFM, PIK3CA_HFM_1047, FGF14_HFM, ARAF_HFM_181, AKT2_HFM, CCNE1_HFM, BAP1_LFM, FH_HFM, TP53_HFM_154, PIK3CA_HFM, VHL_LFM, ERBB3_HFM, H3F3A_HFM, CCND2_LFM, PIK3CA_LFM, CDKN2B_LFM, PRDM1_LFM, STK11_HFM_194, TP53_HFM_135, KEAP1_HFM_320, ZNF703_HFM, CSF1R_LFM, KLHL6_HFM, BCL2L2_HFM, LYN_HFM, SOX2_HFM, STK11_LFM, SDHC_HFM, EPHA3_HFM, CUL4A_HFM, STK11_HFM, MAP3K13_HFM, BCL6_HFM, TERC_HFM, NFE2L2_HFM_28, JAK3_HFM, TP53_HFM_272, CDKN2A_LFM, TEK_LFM, ROS1_HFM, NFKBIA_HFM and FGF19_HFM.

8.  The method of any one of the preceding claims, (i) wherein said response is a durable response, optionally wherein said durable response is a lack of disease progression for at least 270 days following CPI therapy; and/or

    (ii) wherein said resistance is an innate resistance, optionally wherein said innate resistance is a lack of response to CPI therapy and/or disease progression with 270 days of CPI therapy; and/or
    (iii) wherein the total number of genes making up the mutation profile does not exceed 100 genes.

9.  A method for classifying a sample obtained from a subject having a lung cancer, said sample comprising nucleic acid, optionally wherein said sample is derived from one or more cancer cells of the subject, the method comprising:

    analysing the sample to obtain a mutation profile for the subject, said mutation profile comprising the presence or absence of mutations at one or more locations in at least five, six, or at least seven genes selected from the group consisting of: NF1, STK11, TSC2, BRCA2, BRAF, STAG2, U2AF1, BRIP1, PDGFRA, CTNNA1, PDK1, FGF10, and FLT1; and
    analysing the mutation profile to classify the profile as matching the mutation profile of a checkpoint inhibitor (CPI) response signature or a CPI resistance signature,
    wherein the sample is classified as being derived from a CPI therapy responsive subject if the mutation profile is classified as matching the mutation profile of the CPI response signature and is characterised as being derived from a CPI therapy resistant subject if the mutation profile is classified as matching the mutation profile of the CPI resistance signature.

10. The method of claim 9, wherein said mutations at one or more locations are cancer-specific mutations and/or

    wherein analysing the sample to obtain the mutation profile for the subject comprises nucleic acid sequencing, optionally wherein the sample is sequenced to provide at least exonic coverage of the at least five, six, seven or all of the genes as defined in any of the preceding claims and/or
    wherein the sample comprises a tumour tissue sample, a circulating tumour cell or a cell-free sample comprising circulating tumour DNA (ctDNA) and/or circulating tumour RNA (ctRNA) and/or
    wherein analysing the mutation profile to classify the profile as matching the mutation profile of a response signature or a resistance signature comprises carrying out the method of any one of claims 1 to 8.

11. The method of any one of the preceding claims, wherein (i) said lung cancer is Non-Small Cell Lung Cancer or Small-Cell Lung Cancer optionally wherein said lung cancer is Non-Small Cell Lung Cancer; and/or

(ii) said CPI therapy comprises an inhibitor of PD-L1, PD-1, and/or CTLA-4 optionally wherein said CPI therapy comprises an agent selected from the group consisting of: nivolumab, pembrolizumab, atezolizumab, durvalumab, avelumab, ipilimumab, and cemiplimab; and/or

(iii) the method further comprises analysing one or more additional markers of CPI response derived from the subject to supplement and/or corroborate the prediction of CPI response or resistance optionally wherein the one or more additional markers of CPI response are selected from the group consisting of: age, disease stage, histology, smoking history, race, gender, tumour mutational burden (TMB), microsatellite instability (MSI), PD-L1 expression, JAK1/JAK2, IFNg, PTEN loss, PBRM1, STK11/KEAP1 mutations, antigen processing/presentation loss, and WNT/b-catenin signalling.

12. A system for predicting the treatment response of a subject having a lung cancer to an immune checkpoint inhibitor (CPI) therapy, the system comprising:

at least one processor; and
at least one non-transitory computer readable medium containing instructions that, when executed by the at least one processor, cause the at least one processor to perform operations comprising:

obtaining a mutation profile for the subject, said profile comprising the presence or absence of one or more mutations at one or more locations in at least five, six or seven genes selected from the group consisting of: NF1, STK11, TSC2, BRCA2, BRAF, STAG2, U2AF1, BRIP1, PDGFRA, CTNNA1, PDK1, FGF10, and FLT1; analysing the mutation profile to classify the profile as matching the mutation profile of a CPI response signature or a CPI resistance signature,

wherein the subject is predicted to be likely to respond to the CPI therapy if the mutation profile for the subject is classified as matching the mutation profile of the CPI response signature and is predicted to be likely not to respond to the CPI therapy if the mutation profile for the subject is classified as matching the mutation profile of the CPI resistance signature optionally wherein said instructions, when executed by the at least one processor, cause the at least one processor to perform the method of any one of claims 1 to 8.

13. One or more computer readable media comprising instructions that, when executed by one or more processors, cause the one or more processors to perform the steps of the method of any of claims 1 to 8.

14. A pharmaceutical composition comprising an immune checkpoint inhibitor (CPI) for use in a method of treatment of a subject having a lung cancer, wherein the subject has been predicted to respond to said CPI therapy by a method of any one of claims 1 to 8.

15. The composition for use of claim 14, wherein the method of treatment comprises the step of predicting whether the subject will respond to the CPI therapy by a method of any one of claims 1 to 8 and/or wherein the immune checkpoint inhibitor comprises an inhibitor of PD-L1, PD-1, and/or CTLA-4, optionally wherein the inhibitor comprises an antibody, further optionally wherein said immune checkpoint inhibitor is selected from the group consisting of: nivolumab, pembrolizumab, atezolizumab, durvalumab, avelumab, ipilimumab, and cemiplimab.

**Patentansprüche**

1. Computerimplementiertes Verfahren zu Vorhersage des Behandlungsansprechens eines Individuums mit Lungenkrebs auf eine Immun-Checkpoint-Inhibitor-(CPI)-Therapie, wobei das Verfahren Folgendes umfasst:

Bereitstellen eines Mutationsprofils des Individuums, wobei das Profil das Vorhandensein oder Fehlen von Mutationen an einer oder mehreren Stellen in mindestens fünf, sechs oder mindestens sieben Genen umfasst, ausgewählt der Gruppe, bestehend aus: NF1, STK11, TSC2, BRCA2, BRAF, STAG2, U2AF1, BRIP1, PDGFRA, CTNNA1, PDK1, FGF10 und FLT1;
Analysieren des Mutationsprofils, um das Profil als mit dem Mutationsprofil einer Ansprechsignatur oder einer Resistenzsignatur übereinstimmend zu klassifizieren,

wobei vorhergesagt wird, dass das Individuum wahrscheinlich auf die CPI-Therapie anspricht, wenn das Mutations-

**EP 4 515 000 B1**

profil für das Individuum als mit dem Mutationsprofil der Ansprechsignatur übereinstimmend klassifiziert wird, und vorhergesagt wird, dass es wahrscheinlich nicht auf die CPI-Therapie anspricht, wenn das Mutationsprofil für das Individuum als mit dem Mutationsprofil der Resistenzsignatur übereinstimmend klassifiziert wird.

**2.** Verfahren nach Anspruch 1, wobei die Mutationen krebsspezifische Mutationen sind.

**3.** Verfahren nach Anspruch 1 oder Anspruch 2, wobei die mindestens fünf Gene alle Gene umfassen, die in einem der folgenden Gensätze aufgeführt sind:

(i) NF1, STK11, TSC2, STAG2, U2AF1, BRCA2, PDK1;

(ii) STK11, BRAF, BRIP1, U2AF1 und NF1;

(iii) STK11, PDGFRA, BRAF, BRIP1 und CTNNA1;

(iv) BRAF, BRIP1, CTNNA1, FGF10, FLT1, PDGFRA und STK11;

(v) NF1, STK11, TSC2, BRCA2, BRAF, STAG2, U2AF1 und PDK1;

(vi) BRAF, BRIP1, STK11, CDK12, CTNNA1, FAS, NRAS, NOTCH3, PIK3CA und RAD51C;

(vii) STK11, BRIP1, CDKN2B, FLT1, FGF10, BRAF, ASXL1, HRAS, IDH1, BARD1, BRCA2, U2AF1 und CTNNA1;

(viii) NF1, STK11, TSC2, BRCA2, BRAF, STAG2, U2AF1, PDK1 und ATR;

(ix) NF1, STK11, ASXL1, FGF19, TSC2, BRAF, IDH1, STAG2, BRCA2, PDK1, U2AF1, NKX2-1 und PPP2R1A, und wobei die Mutationen Funktionsgewinn(GOF)- und/oder Funktionsverlust(LOF)-Mutationen sind, ausgewählt aus: NF1_LOF, STK11_LOF, ASXL1_LOF, FGF19_LOF, TSC2_LOF, BRAF_GOF, IDH1_GOF, STAG2_LOF, BRCA2_LOF, PDK1_LOF, U2AF1_GOF, NKX2-1_LOF und PPP2R1A_GOF;

(x) BRAF, BRIP1, FGF10, NF1, STK11, MAP3K13, NOTCH3, ALOX12B, U2AF1, RARA, MLH1, FLT1, MAP3K1, MYC, CTNNA1, NBN und ASXL1, und wobei die Mutationen Hochfrequenzmutationen ("HFM") und/oder Niederfrequenzmutationen ("LFM") sind, ausgewählt aus: BRAF_HFM, BRIP1_LFM, FGF10_HFM, NF1_HFM, STK11_HFM, MAP3K13_LFM, NOTCH3_LFM, ALOX12B_LFM, U2AF1_HFM, RARA_HFM, MLH1_LFM, FLT1_LFM, MAP3K1_LFM, MYC_LFM, CTNNA1_LFM, NBN_LFM und ASXL1_LFM;

(xi) PBRM1, BRIP1, PTEN, CDKN2A, STK11, CDKN2B, U2AF1, CTNNA1, FGF10, FGF19, AKT2, NBN, ALOX12B, BRAF und NF1, und wobei die Mutationen Funktionsgewinn(GOF)- und/oder Funktionsverlust(LOF)-Mutationen sind, ausgewählt aus: PBRM1_LOF, BRIP1_LOF, PTEN_LOF, CDKN2A_LOF, STK11_GOF, CDKN2B_LOF, U2AF1_GOF, CTNNA1_LOF, FGF10_GOF, FGF19_LOF, AKT2_GOF, NBN_LOF, ALOX12B_LOF, BRAF_GOF und NF1_GOF;

(xii) NF1, STK11, ASXL1, KMT2A, NFKBIA, BRAF, TSC2, FGF19, NKX2-1, BRCA2, CDKN2A, PDK1, TP53, NFE2L2, U2AF1, EGFR, PPP2R1A, DNMT3A und STAG2, und wobei die krebsspezifischen Mutationen GOF- und/oder LOF-Mutationen sind, ausgewählt aus: NF1_LOF, STK11_LOF, ASXL1_LOF, KMT2A_LOF, NFKBIA_GOF, BRAF_GOF_600, TSC2_LOF, FGF19_LOF, NKX2-1_LOF, BRCA2_LOF, CDKN2A_GOF_151, PDK1_LOF, TP53_GOF_282, NFE2L2_GOF_24, U2AF1_GOF_34, EGFR_GOF_719, PPP2R1A_GOF, DNMT3A_GOF_882 und STAG2_LOF, wobei die Zahl nach GOF die Position der Mutation in der Aminosäuresequenz des durch das Gen codierten Proteins anzeigt;

(xiii) CDKN2B, FGF10, BRCA2, FLT1, BRIP1, RARA, DNMT3A_771, MAP3K13, BRAF_600, ALOX12B, BRAF_469, BCL6, XRCC2, EGFR_746, TSC1, PIK3C2G, TP53_331, PIK3CA, MLH1 und FAS, und wobei die Mutationen HFM- und/oder LFM-Mutationen sind, ausgewählt aus: CDKN2B_LFM, FGF10_HFM, BRCA2_LFM, FLT1_LFM, BRIP1_LFM, RARA_HFM, DNMT3A_HFM_771, MAP3K13_LFM, BRAF_HFM_600, ALOX12B_LFM, BRAF_HFM_469, BCL6_LFM, XRCC2_LFM, EGFR_HFM_746, TSC1_LFM, PIK3C2G_HFM, TP53_HFM_331, PIK3CA_HFM, MLH1_LFM und FAS_LFM;

(xiv) BRIP1, CDKN2B, U2AF1, CTNNA1, ALOX12B, EGFR, FAS und KMT2A, und wobei die krebsspezifischen Mutationen GOF- und/oder LOF-Mutationen sind, ausgewählt aus: BRIP1_LOF, CDKN2B_LOF, U2AF1_GOF_34, CTNNA1_LOF, ALOX12B_LOF, EGFR_GOF_746, FAS_LOF und KMT2A_LOF;

(xv) NF1, STK11, TSC2, BRCA2, BRAF, ATRX, STAG2, U2AF1, PDK1, ATR, ASXL1, ERCC4, PAX5, CTNNA1, CD79A, TSC1, NRAS, RARA, PDCD1LG2, NBN, PDGFRB, PDGFRA, CCNE1, JUN, IDH1, CDK4, NKX2-1, PPP2R1A, FH, MDM2, AKT1, NTRK2, FANCG, QKI, BRD4, CDKN1A, CEBPA, FANCL und SMARCA4; und/oder

(xvi) dem in Tabelle 1A oder Tabelle 1B aufgeführten Gensatz:

binärer Cluster 1; binärer Cluster 2; binärer Cluster 3; binärer Cluster 4; binärer Cluster 5; binärer Cluster 6; binärer Cluster 7; binärer Cluster 8; binärer Cluster 9; binärer Cluster 10; binärer Cluster 11; und/oder binärer Cluster 12.

**4.** Verfahren nach einem der vorhergehenden Ansprüche, wobei die Mutationen krebsspezifische Mutationen sind, die GOF- und/oder LOF-Mutationen sind, ausgewählt aus

(i) dem in Tabelle 2A oder Tabelle 2B aufgeführten Gensatz:
GOF/LOF-Cluster 1; GOF/LOF-Cluster 2; GOF/LOF-Cluster 3; GOF/LOF-Cluster 4; GOF/LOF-Cluster 5; GOF/LOF-Cluster 6; GOF/LOF-Cluster 7; GOF/LOF-Cluster 8; GOF/LOF-Cluster 9; GOF/LOF-Cluster 10; GOF/LOF-Cluster 11; und/oder GOF/LOF-Cluster 12; und/oder
(ii) dem in Tabelle 3A oder Tabelle 3B aufgeführten Gensatz:
Hotspot-Cluster 1; Hotspot-Cluster 2; Hotspot-Cluster 3; Hotspot-Cluster 4; Hotspot-Cluster 5; Hotspot-Cluster 6; Hotspot-Cluster 7; Hotspot-Cluster 8; Hotspot-Cluster 9; Hotspot-Cluster 10; Hotspot-Cluster 11; und/oder Hotspot-Cluster 12.

5. Verfahren nach einem der vorhergehenden Ansprüche, wobei das Analysieren des Mutationsprofils, um das Profil zu klassifizieren, Folgendes umfasst:

Eingeben von Daten, die das Mutationsprofil des Individuums darstellen, in einen Klassifikator für maschinelles Lernen, wobei der Klassifikator für maschinelles Lernen an einem Trainingsdatensatz trainiert wurde, der die Mutationsprofile von mindestens denselben Genen wie jenen umfasst, die das Mutationsprofil des Individuums bilden, wobei die Mutationsprofile des Trainingssatzes aus einer Vielzahl von Proben stammen, die von Lungenkrebs- (z. B. NSCLC-) Patienten stammen, von denen bekannt ist, dass sie auf die CPI-Therapie angesprochen haben, und aus einer Vielzahl von Proben stammen, die von Lungenkrebs- (z. B. NSCLC-) Patienten stammen, von denen bekannt ist, dass sie gegen die CPI-Therapie resistent waren; und
Bewirken, dass der Klassifikator für maschinelles Lernen das Mutationsprofil des Individuums als zu der Ansprechgruppe oder der Resistenzgruppe gehörend klassifiziert.

6. Verfahren nach Anspruch 5, wobei der Trainingsdatensatz Mutationsprofile von mindestens 50, mindestens 100 oder mindestens 200 Proben umfasst, die von Lungenkrebspatienten stammen, von denen bekannt ist, dass sie auf die CPI-Therapie angesprochen haben, und Mutationsprofile von mindestens 50, mindestens 100 oder mindestens 200 Proben umfasst, die von Lungenkrebspatienten stammen, von denen bekannt ist, dass sie gegen die CPI-Therapie resistent waren, und/oder

wobei der Trainingsdatensatz die Flatiron Health de-identified Clinico-Genomic Database (CGDB) umfasst, wie am 1. Januar 2020 verfügbar, und/oder
wobei der Klassifikator für maschinelles Lernen ausgewählt ist aus der Gruppe, bestehend aus: einem Naive Bayes-Modell; einem logistischen Regressionsmodell; einem künstlichen neuronalen Netzwerk, einer Support Vector Machine (SVM); einem Zufallswald; und einem Perzeptron.

7. Verfahren nach einem der vorhergehenden Ansprüche,

(i) wobei das Vorhandensein einer Mutation in einem oder mehreren der folgenden Gene dazu beiträgt, das Profil als mit dem Mutationsprofil einer Ansprechsignatur übereinstimmend zu klassifizieren:
TSC2, U2AF1, FGF23, IDH1, PDCD1LG2, MEF2B, PDK1, BRIP1, QKI, CTNNA1, FUBP1, STAG2, FANCL, PAX5, MLH1, FANCG, AKT1, MPL, BRCA2, ATR, POLE, TSC1, FOXL2, BRAF, ASXL1, NF1, ATRX, NRAS, PDGFRA, SMAD4, NBN, PDGFRB, BRCA1, TP53 und SMARCA4;
(ii) wobei das Vorhandensein einer Mutation in einem oder mehreren der folgenden Gene dazu beiträgt, das Profil als mit dem Mutationsprofil einer Ansprechsignatur übereinstimmend zu klassifizieren:
CTNNA1, ALOX12B, HRAS, FAS, U2AF1, TSC1, MLH1, BRIP1, GNA13, ERRFI1, ACVR1B, IDH1, XRCC2, BRAF, SOX9, HNF1A, PDCD1LG2, ESR1, BRCA2, ASXL1, KEL, TERT, TSC2, BARD1, BCL2, QKI, PDGFRB, BTK, FOXL2, CARD11, PBRM1, EZH2, RAD51C, ABL1, ALK, HSD3B1, POLE, FLT1, NOTCH3, PAX5, MAP3K1, STAT3, GABRA6, TP53, CUL3, NBN, PDGFRA, SDHD, RBM10 und KRAS;
(iii) wobei das Vorhandensein einer oder mehrerer der folgenden GOF- und/oder LOF-Mutationen dazu beiträgt, das Profil als mit dem Mutationsprofil einer Ansprechsignatur übereinstimmend zu klassifizieren:
FGF19_LOF, U2AF1_GOF, NBN_LOF, NRAS_LOF, RAC1_LOF, RB1_GOF, IDH1_GOF, XRCC2_LOF, PAX5_GOF, PDCD1LG2_GOF, CD274_GOF, RAD52_GOF, VEGFA_LOF, CBFB_LOF, AKT1_GOF, FGF23_LOF, PDK1_LOF, MYD88_GOF, CD79B_GOF, PRKAR1A_GOF, BRCA2_GOF, SDHC_LOF, GNA13_LOF, FANCG_GOF, FANCL_LOF, SOX2_LOF, EZH2_LOF, STAG2_LOF, RAD51C_LOF, QKI_LOF, FGF6_GOF, FGF23_GOF, MUTYH_GOF, SYK_LOF, CTNNA1_LOF, CDH1_LOF, PBRM1_LOF, PIK3C2G_GOF, ASXL1_LOF, BRAF_GOF, NF1_LOF, CHEK1_LOF, FUBP1_LOF, ERRFI1_LOF, BRCA2_LOF, HSD3B1_GOF, DNMT3A_GOF, WT1_LOF, PDGFRA_LOF, ATRX_LOF, ATR_LOF, RBM10_GOF, MAP2K2_GOF, BRIP1_GOF, PDCD1LG2_LOF, MEF2B_GOF, CREBBP_GOF, IRF2_GOF, BARD1_GOF, CTNNA1_GOF, MPL_GOF, ACVR1B_LOF, PPARG_LOF, TSC2_LOF, KMT2A_LOF, PTPN11_LOF, PDGFRB_LOF, AKT1_LOF, CHEK2_LOF, NOTCH2_GOF, MUTYH_LOF, TSC2_GOF, MAP3K1_LOF, ARID1A_GOF, MED12_GOF, CBFB_GOF, PIK3R1_GOF, STAG2_GOF, MAP2K4_GOF, FLCN_GOF, TSC1_GOF und SETD2_GOF;

(iv) wobei das Vorhandensein einer oder mehrerer der folgenden GOF- und/oder LOF-Mutationen dazu beiträgt, das Profil als mit dem Mutationsprofil einer Ansprechsignatur übereinstimmend zu klassifizieren:
ALOX12B_LFM, CTNNA1_LFM, HRAS_HFM, MEF2B_LFM, TNFRSF14_LFM, XRCC2_LFM, BRIP1_LFM, U2AF1_HFM, NF1_HFM, FAS_LFM, TSC1_LFM, ERRFI1_LFM, EZH2_LFM, NBN_LFM, MLH1_LFM, GNA13_LFM, FGF19_LFM, ALK_HFM, AMER1_HFM, BRAF_HFM, IDH1_HFM, DNMT3A_HFM, CDK6_LFM, BCL6_LFM, WT1_HFM, SOX9_LFM, SMAD4_LFM, HNF1A_LFM, JAK2_LFM, PAX5_HFM, HSD3B1_LFM, PARP2_LFM, ASXL1_HFM, ESR1_LFM, PIK3C2G_HFM, BRCA2_LFM, ASXL1_LFM, TERT_HFM, WT1_LFM, BARD1_LFM, MAP3K1_LFM, FGFR2_LFM, PPARG_LFM, AX1N1_LFM, PDCD1LG2_HFM, SOX9_HFM, KRAS_LFM, PRKAR1A_HFM, PDGFRB_LFM, TSC2_LFM, NF2_LFM, FGF6_LFM, PBRM1_LFM, KEL_LFM, FOXL2_LFM, CARD11_LFM, MAP3K13_LFM, FGF6_HFM, NOTCH3_LFM, SF3B1_LFM, CUL3_LFM, FLT1_LFM, CD274_HFM, IDH1_LFM, SPOP_LFM, MYCN_HFM, SRC_LFM, AR_LFM, ERBB2_LFM, CASP8_LFM, PDGFRA_LFM, IRF4_LFM, PTEN_LFM, GNAS_LFM, CCNE1_LFM, NOTCH2_LFM, ATR_LFM, RBM10_LFM, FANCG_HFM, SMO_HFM, EGFR_LFM und SPOP_HFM;

(v) wobei das Vorhandensein einer oder mehrerer der folgenden GOF- und/oder LOF-Mutationen dazu beiträgt, das Profil als mit dem Mutationsprofil einer Ansprechsignatur übereinstimmend zu klassifizieren:
FGF19_LOF, TP53_GOF_331, NFE2L2_GOF_24, PIK3C2G_GOF_1088, U2AF1_GOF_34, NBN_LOF, NFE2L2_GOF_77, TP53_GOF_376, TP53_GOF_244, AKT1_GOF_17, RAC1_LOF, MYCN_GOF, CDKN2A_GOF_151, DNMT3A_GOF_882, NRAS_LOF, XRCC2_LOF, PAX5_GOF, BRAF_GOF_600, RAD52_GOF, PDCD1LG2_GOF, CD274_GOF, TP53_GOF_159, VEGFA_LOF, CBFB_LOF, PDK1_LOF, KDM5C_GOF_1330, KEAP1_GOF_332, STK11_GOF_37, BRCA2_GOF, KEAP1_GOF_116, MYD88_GOF_265, IDH1_GOF, AR_GOF_457, RB1_GOF, MUTYH_GOF_165, BRAF_GOF_466, POLE_GOF, TP53_GOF_673, CD79B_GOF, ARID1A_GOF_21, PRKAR1A_GOF, DIS3_GOF_458, CDKN2A_GOF_69, FANCL_LOF, FANCG_GOF, SOX2_LOF, SMAD4_GOF, BRAF_GOF_469, TP53_GOF_275, TP53_GOF_192, STAG2_LOF, QKI_LOF, FGF6_GOF, CTNNA1_LOF, MLH1_LOF, CDH1_LOF, ERBB3_LOF, PBRM1_LOF, ASXL1_LOF, KDM5A_GOF, NF1_LOF, CHEK1_LOF, BRCA2_LOF, TP53_GOF_560, WT1_LOF, SDHB_LOF, EGFR_GOF_858, TP53_GOF_215, PDGFRA_LOF, ATRX_LOF, ATR_LOF, IRF2_GOF, EGFR_GOF_771, EGFR_GOF_861, MLH1_GOF, AR_GOF_465, STK11_GOF_221, FAS_GOF, AMER1_GOF_385, RBM10_GOF_503, KEAP1_GOF_153, STK11_GOF_199, PTCH1_GOF, STK11_GOF_163, ATRX_GOF, KEAP1_GOF_509, TP53_GOF_236, KEAP1_GOF_362, HSD3B1_GOF_75, RB1_GOF_576, STK11_GOF_216, FBXW7_GOF_479, KEAP1_GOF_523, JAK3_GOF, PPARG_LOF, PIK3CB_GOF, ACVR1B_LOF, TSC2_LOF, KMT2A_LOF, TP53_GOF_238, AKT1_LOF, PIK3R1_LOF, IRS2_GOF, HRAS_GOF_61, PIK3CA_GOF_726, AR_GOF_468, TNFRSF14_LOF, NFE2L2_GOF_29, MEN1_GOF, BCL2_LOF, AR_GOF_467, ATM_GOF_337, KEAP1_GOF_544, NTRK3_GOF, NTRK3_GOF_610, HRAS_LOF, CTNNB1_GOF_41, SMARCA4_GOF_1160, KEAP1_GOF_409, SMAD2_GOF_464, PTEN_GOF_59, CTNNB1_GOF_33, CREBBP_GOF_1472, APC_GOF, PTEN_GOF_165, TSC1_GOF, KEAP1_GOF_417, RBM10_GOF, NBN_GOF_219, PIK3CA_GOF_345, AR_GOF_493, ZNF217_GOF_410, AR_GOF_598, STK11_GOF_256, ARID1A_GOF_343, RET_GOF_511, KEAP1_GOF_155, MITF_GOF, CDK8_GOF, DNMT3A_GOF_749, MYD88_GOF, KEAP1_GOF_430, GNAS_GOF_415, KDM5C_GOF, FLT1_GOF, NF1_GOF_1642, KMT2A_GOF_53, SDHA_GOF_531, CDKN2A_GOF_61, CEBPA_GOF_197, STK11_GOF_220, NBN_GOF_680, KEAP1_GOF_450, CHEK2_GOF_392, NKX2-1_GOF_234, SMARCA4_GOF_1157, BCORL1_GOF_94, KEAP1_GOF_493, FLCN_GOF_306, STK11_GOF_168 und MSH6_GOF_1088;

(vi) wobei das Vorhandensein einer oder mehrerer der folgenden GOF- und/oder LOF-Mutationen dazu beiträgt, das Profil als mit dem Mutationsprofil einer Ansprechsignatur übereinstimmend zu klassifizieren:
ALOX12B_LFM, DNMT3A_HFM_771, CTNNA1_LFM, NFE2L2_HFM_24, MEF2B_LFM, TP53_HFM_331, BRAF_HFM_469, XRCC2_LFM, TNFRSF14_LFM, BRIP1_LFM, FUBP1_LFM, U2AF1_HFM_34, FAS_LFM, TP53_HFM_173, TSC1_LFM, ERRFI1_LFM, EZH2_LFM, NBN_LFM, MLH1_LFM, BRAF_HFM_600, FGF19_LFM, GNA13_LFM, ALK_HFM, TP53_HFM_215, TP53_HFM_783, TP53_HFM_560, CDK6_LFM, BCL6_LFM, TP53_HFM_280, BTG1_LFM, WT1_HFM, SOX9_LFM, SMAD4_LFM, NF1_HFM, JAK2_LFM, HSD3B1_LFM, PARP2_LFM, PIK3C2G_HFM, BRCA2_LFM, ASXL1_LFM, TERT_HFM, BARD1_LFM, MAP3K1_LFM, FGFR2_LFM, SDHA_LFM, IGF1R_LFM, AXIN1_LFM, PDCD1LG2_HFM, PAX5_HFM, TP53_HFM_294, RAD51C_LFM, JUN_LFM, PRKAR1A_HFM, PDGFRB_LFM, TSC2_LFM, NF2_LFM, BTK_LFM, PBRM1_LFM, FOXL2_LFM, MAP3K13_LFM, NOTCH3_LFM, GABRA6_LFM, PIK3CA_HFM_542, TP53_HFM_159, CUL3_LFM, ASXL1_HFM, FLT1_LFM, MITF_LFM, TP53_HFM_249, IDH1_LFM, SRC_LFM, AR_LFM, ERBB2_LFM, SMO_LFM, KEAP1_LFM, PDGFRA_LFM, GATA6_LFM, PTEN_LFM, GNAS_LFM, CCNE1_LFM, ATR_LFM, MERTK_LFM, KMT2A_LFM, SOX9_HFM, GSK3B_HFM, CUL4A_LFM und BCL2L2_LFM;

(vii) wobei das Vorhandensein einer Mutation in einem oder mehreren der folgenden Gene dazu beiträgt, das Profil als mit dem Mutationsprofil einer Resistenzsignatur übereinstimmend zu klassifizieren:
GID4, JUN, RAD51, CD79A, STK11, TET2, MAP2K1, CCNE1, CDK4, ERCC4, CEBPA, RARA, CDKN1A, RAD51B, PPP2R1A, CSF1R, FH, NKX2-1, NTRK2, FGFR1, CDK6, MDM2 und BRD4;

(viii) wobei das Vorhandensein einer Mutation in einem oder mehreren der folgenden Gene dazu beiträgt, das Profil als mit dem Mutationsprofil einer Resistenzsignatur übereinstimmend zu klassifizieren:
RARA, VHL, GID4, IKBKE, CEBPA, CD79A, CDK12, STK11, BAP1, CDKN2B, FGF10, ARFRP1, CSF1R, FANCC, CDC73, ZNF703, PIK3CA, NKX2-1, TNFAIP3, CCNE1, SOX2, SDHC, MYC, TERC, PARP3, JAK1, DDR2, PARP1, AKT2, PDK1, NFE2L2 und MDM4;

(ix) wobei das Vorhandensein einer oder mehrerer der folgenden GOF- und/oder LOF-Mutationen dazu beiträgt, das Profil als mit dem Mutationsprofil einer Resistenzsignatur übereinstimmend zu klassifizieren:
MAP3K1_GOF, FGF14_GOF, PPP2R1A_GOF, CDKN1A_GOF, JAK1_GOF, IRF4_GOF, JUN_LOF, AKT3_GOF, NKX2-1_LOF, TGFBR2_GOF, GABRA6_GOF, BCORL1_GOF, TNFAIP3_GOF, POLD1_GOF, GNAQ_GOF, RAD51C_GOF, BRD4_GOF, RNF43_GOF, FGFR4_GOF, GATA6_GOF, GATA3_GOF, MDM2_LOF, MYC_LOF, RPTOR_GOF, CD79A_LOF, CCNE1_GOF, STK11_LOF, MYCN_LOF, CEBPA_LOF, PARP1_GOF, NOTCH3_GOF, SDHD_GOF, LTK_GOF, DAXX_GOF, ABL1_GOF, PDGFRB_GOF, BTG1_GOF, CHEK1_GOF, GATA4_GOF, JUN_GOF, FLT3_GOF, CUL3_GOF, CDK4_GOF, AKT2_GOF, KDM6A_GOF, MTOR_GOF, BCL2L1_LOF, CD274_LOF, NF2_GOF, SMARCA4_GOF, NFKBIA_GOF, ERCC4_LOF, ZNF703_GOF, STK11_GOF, KEAP1_GOF, ERBB2_GOF, FH_GOF, REL_LOF, RARA_GOF, RAD51_LOF, PTEN_GOF, NTRK2_LOF, CDKN2B_LOF, BAP1_LOF, RARA_LOF, AXL_GOF, CCND2_LOF, CUL4A_LOF, H3F3A_GOF, GRM3_GOF, CDK6_GOF, ARFRP1_LOF, SUFU_LOF, RAD54L_LOF, PIK3CB_LOF, KLHL6_GOF, MAP3K13_GOF, PIK3CA_GOF, CDKN2A_LOF, EZH2_GOF, FANCA_LOF, HGF_LOF, FBXW7_GOF, FGFR2_GOF, AURKA_LOF, HSD3B1_LOF und SDHC_GOF;

(x)
MYC_LFM, FANCC_HFM, JAK1_HFM, FGFR2_HFM, GABRA6_HFM, RARA_HFM, ERBB4_HFM, CD79A_LFM, GID4_LFM, IGF1R_HFM, GNA11_HFM, GATA6_HFM, CDC73_HFM, CHEK2_HFM, ARFRP1_HFM, SMARCB1_HFM, FGF10_HFM, CDK12_HFM, IKBKE_LFM, DDR2_HFM, NF2_HFM, AKT3_HFM, CEBPA_LFM, FGF14_HFM, EP300_HFM, CCNE1_HFM, AKT2_HFM, BAP1_LFM, FH_HFM, CEBPA_HFM, ERBB2_HFM, CCND2_LFM, ATM_HFM, PIK3CA_LFM, STK11_HFM, CDKN2B_LFM, PRDM1_LFM, NOTCH3_HFM, CDK12_LFM, ZNF703_HFM, CSF1R_LFM, KLHL6_HFM, BCL2L2_HFM, LYN_HFM, RICTOR_HFM, SOX2_HFM, STK11_LFM, KEAP1_HFM, CYP17A1_LFM, CUL4A_HFM, PRKCI_HFM, PIK3CA_HFM, TERC_HFM, IKZF1_HFM, MEF2B_HFM, NFE2L2_HFM, CDKN2A_LFM, NKX2-1_HFM, TEK_LFM, MYCL_LFM, ROS1_HFM, MCL1_HFM, FGF12_HFM, EZH2_HFM und MDM4_HFM;

(xi) wobei das Vorhandensein einer oder mehrerer der folgenden GOF- und/oder LOF-Mutationen dazu beiträgt, das Profil als mit dem Mutationsprofil einer Resistenzsignatur übereinstimmend zu klassifizieren:
EGFR_GOF_746, TP53_GOF_282, EGFR_GOF_747, EGFR_GOF_719, TP53_GOF_195, FGF14_GOF, PPP2R1A_GOF, IRF4_GOF, MAP3K1_GOF, STK11_GOF_291, ERBB2_GOF_776, JAK1_GOF, CDKN1A_GOF, KEAP1_GOF_272, JUN_LOF, AKT3_GOF, NKX2-1_LOF, ATM_GOF, CDKN2A_GOF_100, STK11_GOF_84, FGFR4_GOF, KEAP1_GOF_483, KEAP1_GOF_234, GABRA6_GOF, CDKN2A_GOF_80, MYCN_GOF_44, KEAP1_GOF_260, MAP2K1_GOF_102, PTEN_GOF, PTCH1_GOF_48, GNAQ_GOF, KEAP1_GOF_364, POLD1_GOF, PIK3CA_GOF_1043, BCOR_GOF_679, FH_GOF_476, STK11_GOF_181, TP53_GOF_285, TNFAIP3_GOF, BRD4_GOF, KDM5C_GOF_1546, KEAP1_GOF_274, SMAD4_GOF_351, RNF43_GOF, SF3B1_GOF_666, MTOR_GOF_1834, NOTCH1_GOF, TP53_GOF_272, GATA6_GOF, MDM2_LOF, GATA3_GOF, PIK3CA_GOF_1047, TP53_GOF_278, CD79A_LOF, RPTOR_GOF, ZNF703_GOF, CCNE1_GOF, STK11_LOF, MYCN_LOF, PARP1_GOF, CEBPA_LOF, MAP2K1_GOF_121, SMARCA4_GOF_1243, MED12_GOF, KEAP1_GOF_135, AR_GOF_69, BTG1_GOF, MAP3K1_GOF_5, TYRO3_GOF, ERBB2_GOF_755, CBL_GOF_1096, STK11_GOF_176, EGFR_GOF_790, RAF1_GOF, KEAP1_GOF_244, STK11_GOF_464, STK11_GOF_308, KDM6A_GOF, PDGFRB_GOF, BRAF_GOF_464, PIK3C2G_GOF_129, FBXW7_GOF_505, KEAP1_GOF_470, ALOX12B_GOF, FLT3_GOF, AMER1_GOF_625, IDH2_GOF_140, GNAS_GOF_407, KEAP1_GOF_186, BCOR_GOF_1526, NFE2L2_GOF_27, STK11_GOF_251, CHEK1_GOF, HRAS_GOF_13, KEAP1_GOF_236, GATA4_GOF, MET_GOF_2888, KMT2D_GOF_755, RAD51C_GOF_21, SMARCA4_GOF_1162, STK11_GOF_734, MAP3K1_GOF_949, PALB2_GOF, BCORL1_GOF_883, KEAP1_GOF, ARID1A_GOF_515, AR_GOF_469, EGFR_GOF_763, AR_GOF_70, PPARG_GOF, VHL_GOF, PARP3_GOF, CDK4_GOF, AKT2_GOF, TP53_GOF_920, NFE2L2_GOF_30, CHEK2_GOF, SMAD2_LOF, SMARCB1_GOF, TP53_GOF_298, SDHA_GOF_457, FH_GOF, CDC73_GOF, NFKBIA_GOF, ERCC4_LOF, RARA_GOF, RAD51B_LOF, NTRK2_LOF, CUL4A_LOF, STK11_GOF_57, TP53_GOF_234, STK11_GOF_242, CHEK2_GOF_367, RAD51D_GOF, CTCF_LOF, EPHA3_GOF, RAD54L_LOF, PIK3CB_LOF, KLHL6_GOF, PIK3CA_GOF, DDR2_GOF, MAP3K13_GOF, PIK3CA_GOF_545, HGF_LOF, NFE2L2_GOF_31, MET_GOF_3028, BCL6_GOF, TP53_GOF_342 und CDKN2A_GOF_83; und/oder

(xii) wobei das Vorhandensein einer oder mehrerer der folgenden GOF- und/oder LOF-Mutationen dazu beiträgt, das Profil als mit dem Mutationsprofil einer Resistenzsignatur übereinstimmend zu klassifizieren:

EGFR_HFM_746, MYC_LFM, FANCC_HFM, JAK1_HFM, NFE2L2_HFM_79, GABRA6_HFM, RARA_HFM, ERBB4_HFM, CD79A_LFM, NF2_HFM, GNA11_HFM, CHEK2_HFM, GID4_LFM, NFE2L2_HFM_29, IGF1R_HFM, GATA6_HFM, CDC73_HFM, FGFR2_HFM, CHEK2_HFM_157, STK11_HFM_242, TP53_HFM_152, STK11_HFM_53, ARFRP1_HFM, TP53_HFM_282, FGF10_HFM, CDK12_HFM, IKBKE_LFM, DDR2_HFM, NKX2-1_LFM, AKT3_HFM, ERBB2_HFM_775, TP53_HFM_920, CEBPA_LFM, DIS3_HFM, GATA3_HFM, EP300_HFM, PIK3CA_HFM_1047, FGF14_HFM, ARAF_HFM_181, AKT2_HFM, CCNE1_HFM, BAP1_LFM, FH_HFM, TP53_HFM_154, PIK3CA_HFM, VHL_LFM, ERBB3_HFM, H3F3A_HFM, CCND2_LFM, PIK3CA_LFM, CDKN2B_LFM, PRDM1_LFM, STK11_HFM_194, TP53_HFM_135, KEAP1_HFM_320, ZNF703_HFM, CSF1R_LFM, KLHL6_HFM, BCL2L2_HFM, LYN_HFM, SOX2_HFM, STK11_LFM, SDHC_HFM, EPHA3_HFM, CUL4A_HFM, STK11_HFM, MAP3K13_HFM, BCL6_HFM, TERC_HFM, NFE2L2_HFM_28, JAK3_HFM, TP53_HFM_272, CDKN2A_LFM, TEK_LFM, ROS1_HFM, NFKBIA_HFM und FGF19_HFM.

8. Verfahren nach einem der vorhergehenden Ansprüche, (i) wobei das Ansprechen ein dauerhaftes Ansprechen ist, wobei das dauerhafte Ansprechen gegebenenfalls ein Mangel an Krankheitsprogression für mindestens 270 Tage nach einer CPI-Therapie ist; und/oder

(ii) wobei die Resistenz eine angeborene Resistenz ist, wobei die angeborene Resistenz gegebenenfalls ein Mangel an Ansprechen auf eine CPI-Therapie und/oder Krankheitsprogression mit 270 Tagen einer CPI-Therapie ist; und/oder

(iii) wobei die Gesamtzahl der Gene, aus denen das Mutationsprofil besteht, 100 Gene nicht überschreitet.

9. Verfahren zum Klassifizieren einer Probe, die von einem Individuum mit Lungenkrebs erhalten wurde, wobei die Probe Nukleinsäure umfasst, wobei die Probe gegebenenfalls von einer oder mehreren Krebszellen des Individuums stammt, wobei das Verfahren Folgendes umfasst:

Analysieren der Probe, um ein Mutationsprofil für das Individuum zu erhalten, wobei das Mutationsprofil das Vorhandensein oder Fehlen von Mutationen an einer oder mehreren Stellen in mindestens fünf, sechs oder mindestens sieben Genen umfasst, ausgewählt der Gruppe, bestehend aus: NF1, STK11, TSC2, BRCA2, BRAF, STAG2, U2AF1, BRIP1, PDGFRA, CTNNA1, PDK1, FGF10 und FLT1; und

Analysieren des Mutationsprofils, um das Profil als mit dem Mutationsprofil einer Checkpoint-Inhibitor-(CPI)-Ansprechsignatur oder einer CPI-Resistenzsignatur übereinstimmend zu klassifizieren, wobei die Probe als von einem auf eine CPI-Therapie ansprechenden Individuum stammend klassifiziert wird, wenn das Mutationsprofil als mit dem Mutationsprofil der CPI-Ansprechsignatur übereinstimmend klassifiziert wird, und **dadurch gekennzeichnet ist, dass** sie von einem gegen eine CPI-Therapie resistenten Individuum stammt, wenn das Mutationsprofil als mit dem Mutationsprofil der CPI-Resistenzsignatur übereinstimmend klassifiziert wird.

10. Verfahren nach Anspruch 9, wobei die Mutationen an einer oder mehreren Stellen krebsspezifische Mutationen sind und/oder

wobei das Analysieren der Probe, um das Mutationsprofil für das Individuum zu erhalten, Nukleinsäuresequenzierung umfasst, wobei die Probe gegebenenfalls sequenziert wird, um mindestens eine exonische Abdeckung der mindestens fünf, sechs, sieben oder aller Gene bereitzustellen, wie in einem der vorhergehenden Ansprüche definiert, und/oder

wobei die Probe eine Tumorgewebeprobe, eine zirkulierende Tumorzelle oder eine zellfreie Probe umfasst, die zirkulierende Tumor-DNA (ctDNA) und/oder zirkulierende Tumor-RNA (ctRNA) umfasst, und/oder

wobei das Analysieren des Mutationsprofils, um das Profil als mit dem Mutationsprofil einer Ansprechsignatur oder einer Resistenzsignatur übereinstimmend zu klassifizieren, das Durchführen des Verfahrens nach einem der Ansprüche 1 bis 8 umfasst.

11. Verfahren nach einem der vorhergehenden Ansprüche, wobei (i) der Lungenkrebs nichtkleinzelliger Lungenkrebs oder kleinzelliger Lungenkrebs ist, wobei der Lungenkrebs gegebenenfalls nichtkleinzelliger Lungenkrebs ist; und/oder

(ii) die CPI-Therapie einen Inhibitor von PD-L1, PD-1 und/oder CTLA-4 umfasst, wobei die CPI-Therapie gegebenenfalls ein Mittel umfasst, ausgewählt aus der Gruppe, bestehend aus: Nivolumab, Pembrolizumab, Atezolizumab, Durvalumab, Avelumab, Ipilimumab und Cemiplimab; und/oder

(iii) das Verfahren ferner das Analysieren eines oder mehrerer zusätzlicher Marker von CPI-Ansprechen umfasst, die von dem Individuum stammen, um die Vorhersage von CPI-Ansprechen oder -Resistenz zu ergänzen und/oder zu bestätigen, wobei der eine oder die mehreren zusätzlichen Marker von CPI-Ansprechen gegebenenfalls ausgewählt sind aus der Gruppe, bestehend aus: Alter, Krankheitsstadium, Histologie, Rauchervorgeschichte, Rasse, Geschlecht, Tumormutationsbelastung (TMB), Mikrosatelliteninstabilität (MSI), PD-L1-Expression, JAK1/JAK2, IFNg, PTEN-Verlust, PBRM1, STK11/KEAP1-Mutationen, Antigenverarbeitungs-/Präsentationsverlust und WNT/b-Catenin-Signalisierung.

12. System zum Vorhersagen des Behandlungsansprechens eines Individuums mit Lungenkrebs auf eine Immun-Checkpoint-Inhibitor-(CPI)-Therapie, wobei das System Folgendes umfasst:

mindestens einen Prozessor; und
mindestens ein nichtflüchtiges computerlesbares Medium, das Anweisungen enthält, die, wenn sie von dem mindestens einen Prozessor ausgeführt werden, bewirken, dass der mindestens eine Prozessor Operationen durchführt, die Folgendes umfassen:

Erhalten eines Mutationsprofils für das Individuum, wobei das Profil das Vorhandensein oder Fehlen von einer oder mehreren Mutationen an einer oder mehreren Stellen in mindestens fünf, sechs oder sieben Genen umfasst, ausgewählt der Gruppe, bestehend aus: NF1, STK11, TSC2, BRCA2, BRAF, STAG2, U2AF1, BRIP1, PDGFRA, CTNNA1, PDK1, FGF10 und FLT1;
Analysieren des Mutationsprofils, um das Profil als mit dem Mutationsprofil einer CPI-Ansprechsignatur oder einer CPI-Resistenzsignatur übereinstimmend zu klassifizieren,

wobei vorhergesagt wird, dass das Individuum wahrscheinlich auf die CPI-Therapie anspricht, wenn das Mutationsprofil für das Individuum als mit dem Mutationsprofil der CPI-Ansprechsignatur übereinstimmend klassifiziert wird, und vorhergesagt wird, dass es wahrscheinlich nicht auf die CPI-Therapie anspricht, wenn das Mutationsprofil für das Individuum als mit dem Mutationsprofil der CPI-Resistenzsignatur übereinstimmend klassifiziert wird, optional wobei die Anweisungen, wenn sie von dem mindestens einen Prozessor ausgeführt werden, bewirken, dass der mindestens eine Prozessor das Verfahren nach einem der Ansprüche 1 bis 8 durchführt.

13. Ein oder mehrere computerlesbare Medium/Medien, das/die Anweisungen umfasst/umfassen, die bewirken, dass der eine oder die mehreren Prozessoren die Schritte des Verfahrens nach einem der Ansprüche 1 bis 8 durchführen, wenn sie von einem oder mehreren Prozessoren ausgeführt werden.

14. Pharmazeutische Zusammensetzung, umfassend einen Immun-Checkpoint-Inhibitor (CPI), zur Verwendung in einem Verfahren zur Behandlung eines Individuums mit Lungenkrebs, wobei vorhergesagt wurde, dass das Individuum durch ein Verfahren nach einem der Ansprüche 1 bis 8 auf die CPI-Therapie anspricht.

15. Zusammensetzung zur Verwendung nach Anspruch 14, wobei das Behandlungsverfahren den Schritt des Vorhersagens umfasst, ob das Individuum durch ein Verfahren nach einem der Ansprüche 1 bis 8 auf die CPI-Therapie anspricht, und/oder wobei der Immun-Checkpoint-Inhibitor einen Inhibitor von PD-L1, PD-1 und/oder CTLA-4 umfasst, gegebenenfalls wobei der Inhibitor einen Antikörper umfasst, ferner optional wobei der Immun-Checkpoint-Inhibitor ausgewählt ist aus der Gruppe, bestehend aus: Nivolumab, Pembrolizumab, Atezolizumab, Durvalumab, Avelumab, Ipilimumab und Cemiplimab.

**Revendications**

1. Procédé mis en œuvre par ordinateur de prédiction de la réponse au traitement d'un sujet atteint d'un cancer du poumon à une thérapie par inhibiteur de point de contrôle immunitaire (IPCI), le procédé comprenant :

la fourniture d'un profil de mutation du sujet, ledit profil comprenant la présence ou l'absence de mutations en un ou plusieurs emplacements dans au moins cinq, six, ou au moins sept gènes choisis dans le groupe constitué par : NF1, STK11, TSC2, BRCA2, BRAF, STAG2, U2AF1, BRIP1, PDGFRA, CTNNA1, PDK1, FGF10 et FLT1 ; l'analyse du profil de mutation pour classer le profil comme correspondant au profil de mutation d'une signature de réponse ou d'une signature de résistance,

dans lequel il est prédit que le sujet est susceptible de répondre à la thérapie par IPCI si le profil de mutation pour le sujet est classé comme correspondant au profil de mutation de la signature de réponse et il est prédit qu'il n'est pas susceptible de répondre à la thérapie par IPCI si le profil de mutation pour le sujet est classé comme correspondant au profil de mutation de la signature de résistance.

2. Procédé selon la revendication 1, dans lequel lesdites mutations sont des mutations spécifiques du cancer.

3. Procédé selon la revendication 1 ou la revendication 2, dans lequel lesdits au moins cinq gènes comprennent la totalité des gènes énoncés dans l'un des ensembles de gènes suivants :

(i) NF1, STK11, TSC2, STAG2, U2AF1, BRCA2, PDK1 ;
(ii) STK11, BRAF, BRIP1, U2AF1 et NF1 ;
(iii) STK11, PDGFRA, BRAF, BRIP1 et CTNNA1 ;
(iv) BRAF, BRIP1, CTNNA1, FGF10, FLT1, PDGFRA et STK11 ;
(v) NF1, STK11, TSC2, BRCA2, BRAF, STAG2, U2AF1 et PDK1 ;
(vi) BRAF, BRIP1, STK11, CDK12, CTNNA1, FAS, NRAS, NOTCH3, PIK3CA et RAD51C ;
(vii) STK11, BRIP1, CDKN2B, FLT1, FGF10, BRAF, ASXL1, HRAS, IDH1, BARD1, BRCA2, U2AF1 et CTNNA1 ;
(viii) NF1, STK11, TSC2, BRCA2, BRAF, STAG2, U2AF1, PDK1 et ATR ;
(ix) NF1, STK11, ASXL1, FGF19, TSC2, BRAF, IDH1, STAG2, BRCA2, PDK1, U2AF1, NKX2-1 et PPP2R1A, et dans lequel les mutations sont des mutations de gain de fonction (GOF) et/ou de perte de fonction (LOF) choisies parmi : NF1_LOF, STK11_LOF, ASXL1_LOF, FGF19_LOF, TSC2_LOF, BRAF_GOF, IDH1_GOF, STAG2_LOF, BRCA2_LOF, PDK1_LOF, U2AF1_GOF, NKX2-1_LOF et PPP2R1A_GOF ;
(x) BRAF, BRIP1, FGF10, NF1, STK11, MAP3K13, NOTCH3, ALOX12B, U2AF1, RARA, MLH1, FLT1, MAP3K1, MYC, CTNNA1, NBN et ASXL1, et dans lequel les mutations sont des mutations haute fréquence (« HFM ») et/ou des mutations basse fréquence (« LFM ») choisies parmi : BRAF_HFM, BRIP1_LFM, FGF10_HFM, NF1_HFM, STK11_HFM, MAP3K13_LFM, NOTCH3_LFM, ALOX12B_LFM, U2AF1_HFM, RARA_HFM, MLH1_LFM, FLT1_LFM, MAP3K1_LFM, MYC_LFM, CTNNA1_LFM, NBN_LFM et ASXL1_LFM ;
(xi) PBRM1, BRIP1, PTEN, CDKN2A, STK11, CDKN2B, U2AF1, CTNNA1, FGF10, FGF19, AKT2, NBN, ALOX12B, BRAF et NF1, et dans lequel les mutations sont des mutations de gain de fonction (GOF) et/ou des mutations de perte de fonction (LOF) choisies parmi : PBRM1_LOF, BRIP1_LOF, PTEN_LOF, CDKN2A_LOF, STK11_GOF, CDKN2B_LOF, U2AF1_GOF, CTNNA1_LOF, FGF10_GOF, FGF19_LOF, AKT2_GOF, NBN_LOF, ALOX12B_LOF, BRAF_GOF et NF1_GOF ;
(xii) NF1, STK11, ASXL1, KMT2A, NFKBIA, BRAF, TSC2, FGF19, NKX2-1, BRCA2, CDKN2A, PDK1, TP53, NFE2L2, U2AF1, EGFR, PPP2R1A, DNMT3A et STAG2, et dans lequel les mutations spécifiques du cancer sont des mutations GOF et/ou LOF choisies parmi : NF1_LOF, STK11_LOF, ASXL1_LOF, KMT2A_LOF, NFKBIA_GOF, BRAF_GOF_600, TSC2_LOF, FGF19_LOF, NKX2-1_LOF, BRCA2_LOF, CDKN2A_GOF_151, PDK1_LOF, TP53_GOF_282, NFE2L2_GOF_24, U2AF1_GOF_34, EGFR_GOF_719, PPP2R1A_GOF, DNMT3A_GOF_882 et STAG2_LOF, dans lequel le nombre après GOF indique la position de la mutation dans la séquence d'acides aminés de la protéine codée par le gène ;
(xiii) CDKN2B, FGF10, BRCA2, FLT1, BRIP1, RARA, DNMT3A_771, MAP3K13, BRAF_600, ALOX12B, BRAF_469, BCL6, XRCC2, EGFR_746, TSC1, PIK3C2G, TP53_331, PIK3CA, MLH1 et FAS, et dans lequel les mutations sont des mutations HFM et/ou LFM choisies parmi : CDKN2B_LFM, FGF10_HFM, BRCA2_LFM, FLT1_LFM, BRIP1_LFM, RARA_HFM, DNMT3A_HFM_771, MAP3K13_LFM, BRAF_HFM_600, ALOX12B_LFM, BRAF_HFM_469, BCL6_LFM, XRCC2_LFM, EGFR_HFM_746, TSC1_LFM, PIK3C2G_HFM, TP53_HFM_331, PIK3CA_HFM, MLH1_LFM et FAS_LFM ;
(xiv) BRIP1, CDKN2B, U2AF1, CTNNA1, ALOX12B, EGFR, FAS et KMT2A, et dans lequel les mutations spécifiques au cancer sont des mutations GOF et/ou LOF choisies parmi : BRIP1_LOF, CDKN2B_LOF, U2AF1_GOF_34, CTNNA1_LOF, ALOX12B_LOF, EGFR_GOF_746, FAS_LOF et KMT2A_LOF ;
(xv) NF1, STK11, TSC2, BRCA2, BRAF, ATRX, STAG2, U2AF1, PDK1, ATR, ASXL1, ERCC4, PAX5, CTNNA1, CD79A, TSC1, NRAS, RARA, PDCD1LG2, NBN, PDGFRB, PDGFRA, CCNE1, JUN, IDH1, CDK4, NKX2-1,

PPP2R1A, FH, MDM2, AKT1, NTRK2, FANCG, QKI, BRD4, CDKN1A, CEBPA, FANCL et SMARCA4 ; et/ou
(xvi) l'ensemble de gènes énoncés dans le tableau 1A ou le tableau 1B :
cluster binaire 1 ; cluster binaire 2 ; cluster binaire 3 ; cluster binaire 4 ; cluster binaire 5 ; cluster binaire 6 ; cluster binaire 7 ; cluster binaire 8 ; cluster binaire 9 ; cluster binaire 10 ; cluster binaire 11 ; et/ou cluster binaire 12.

**4.** Procédé selon l'une quelconque des revendications précédentes, dans lequel lesdites mutations sont des mutations spécifiques au cancer qui sont des mutations GOF et/ou LOF choisies parmi

(i) l'ensemble de mutations énoncées dans le tableau 2A ou le tableau 2B :
cluster GOF/LOF 1 ; cluster GOF/LOF 2 ; cluster GOF/LOF 3 ; cluster GOF/LOF 4 ; cluster GOF/LOF 5 ; cluster GOF/LOF 6 ; cluster GOF/LOF 7 ; cluster GOF/LOF 8 ; cluster GOF/LOF 9 ; cluster GOF/LOF 10 ; cluster GOF/LOF 11 ; et/ou cluster GOF/LOF 12 ; et/ou
(ii) l'ensemble de mutations énoncées dans le tableau 3A ou le tableau 3B :
cluster de points chauds 1 ; cluster de points chauds 2 ; cluster de points chauds 3 ; cluster de points chauds 4 ; cluster de points chauds 5 ; cluster de points chauds 6 ; cluster de points chauds 7 ; cluster de points chauds 8 ; cluster de points chauds 9 ; cluster de points chauds 10 ; cluster de points chauds 11 ; et/ou cluster de points chauds 12.

**5.** Procédé selon l'une quelconque des revendications précédentes, dans lequel ladite analyse du profil de mutation pour classer le profil comprend :

la saisie de données représentant le profil de mutation du sujet dans un classificateur d'apprentissage automatique, ledit classificateur d'apprentissage automatique ayant été entraîné sur un ensemble de données d'entraînement comprenant les profils de mutation d'au moins les mêmes gènes que ceux formant le profil de mutation du sujet, les profils de mutation de l'ensemble d'entraînement provenant d'une pluralité d'échantillons issus de patients atteints de cancer du poumon (p. ex. CPNPC) connus pour avoir répondu à la thérapie par IPCI et d'une pluralité d'échantillons issus de patients atteints de cancer du poumon (p. ex. CPNPC) connus pour être résistants à la thérapie par IPCI ; et
le fait d'amener le classificateur d'apprentissage automatique à classer le profil de mutation du sujet comme appartenant au groupe de réponse ou au groupe de résistance.

**6.** Procédé selon la revendication 5, dans lequel l'ensemble de données d'entraînement comprend des profils de mutation d'au moins 50, d'au moins 100 ou d'au moins 200 échantillons issus de patients atteints de cancer du poumon connus pour avoir répondu à la thérapie par IPCI et des profils de mutation d'au moins 50, d'au moins 100 ou d'au moins 200 échantillons issus de patients atteints de cancer du poumon connus pour être résistants à la thérapie par IPCI et/ou

dans lequel l'ensemble de données d'entraînement comprend la base de données clinico-génomiques (CGDB) dé-identifiée de Flatiron Health telle que disponible au 1er janvier 2020 et/ou
dans lequel le classificateur d'apprentissage automatique est choisi dans le groupe constitué par : un modèle de Bayes naïf ; un modèle de régression logistique ; un réseau neuronal artificiel, une machine à vecteur de support (SVM) ; une forêt aléatoire ; et un perceptron.

**7.** Procédé selon l'une quelconque des revendications précédentes,

(i) dans lequel la présence d'une mutation dans un ou plusieurs des gènes suivants contribue au classement du profil comme correspondant au profil de mutation d'une signature de réponse :
TSC2, U2AF1, FGF23, IDH1, PDCD1LG2, MEF2B, PDK1, BRIP1, QKI, CTNNA1, FUBP1, STAG2, FANCL, PAX5, MLH1, FANCG, AKT1, MPL, BRCA2, ATR, POLE, TSC1, FOXL2, BRAF, ASXL1, NF1, ATRX, NRAS, PDGFRA, SMAD4, NBN, PDGFRB, BRCA1, TP53 et SMARCA4 ;
(ii) dans lequel la présence d'une mutation dans un ou plusieurs des gènes suivants contribue au classement du profil comme correspondant au profil de mutation d'une signature de réponse :
CTNNA1, ALOX12B, HRAS, FAS, U2AF1, TSC1, MLH1, BRIP1, GNA13, ERRFI1, ACVR1B, IDH1, XRCC2, BRAF, SOX9, HNF1A, PDCD1LG2, ESR1, BRCA2, ASXL1, KEL, TERT, TSC2, BARD1, BCL2, QKI, PDGFRB, BTK, FOXL2, CARD11, PBRM1, EZH2, RAD51C, ABL1, ALK, HSD3B1, POLE, FLT1, NOTCH3, PAX5, MAP3K1, STAT3, GABRA6, TP53, CUL3, NBN, PDGFRA, SDHD, RBM10 et KRAS ;
(iii) dans lequel la présence d'une ou de plusieurs des mutations GOF et/ou LOF suivantes contribue au classement du profil comme correspondant au profil de mutation d'une signature de réponse :

FGF19_LOF, U2AF1_GOF, NBN_LOF, NRAS_LOF, RAC1_LOF, RB1_GOF, IDH1_GOF, XRCC2_LOF, PAX5_GOF, PDCD1LG2_GOF, CD274_GOF, RAD52_GOF, VEGFA_LOF, CBFB_LOF, AKT1_GOF, FGF23_LOF, PDK1_LOF, MYD88_GOF, CD79B_GOF, PRKAR1A_GOF, BRCA2_GOF, SDHC_LOF, GNA13_LOF, FANCG_GOF, FANCL_LOF, SOX2_LOF, EZH2_LOF, STAG2_LOF, RAD51C_LOF, QKI_LOF, FGF6_GOF, FGF23_GOF, MUTYH_GOF, SYK_LOF, CTNNA1_LOF, CDH1_LOF, PBRM1_LOF, PIK3C2G_GOF, ASXL1_LOF, BRAF_GOF, NF1_LOF, CHEK1_LOF, FUBP1_LOF, ERRFI1_LOF, BRCA2_LOF, HSD3B1_GOF, DNMT3A_GOF, WT1_LOF, PDGFRA_LOF, ATRX_LOF, ATR_LOF, RBM10_GOF, MAP2K2_GOF, BRIP1_GOF, PDCD1LG2_LOF, MEF2B_GOF, CREBBP_GOF, IRF2_GOF, BARD1_GOF, CTNNA1_GOF, MPL_GOF, ACVR1B_LOF, PPARG_LOF, TSC2_LOF, KMT2A_LOF, PTPN11_LOF, PDGFRB_LOF, AKT1_LOF, CHEK2_LOF, NOTCH2_GOF, MUTYH_LOF, TSC2_GOF, MAP3K1_LOF, ARID1A_GOF, MED12_GOF, CBFB_GOF, PIK3R1_GOF, STAG2_GOF, MAP2K4_GOF, FLCN_GOF, TSC1_GOF et SETD2_GOF ;

(iv) dans lequel la présence d'une ou de plusieurs des mutations GOF et/ou LOF suivantes contribue au classement du profil comme correspondant au profil de mutation d'une signature de réponse :

ALOX12B_LFM, CTNNA1_LFM, HRAS_HFM, MEF2B_LFM, TNFRSF14_LFM, XRCC2_LFM, BRIP1_LFM, U2AF1_HFM, NF1_HFM, FAS_LFM, TSC1_LFM, ERRFI1_LFM, EZH2_LFM, NBN_LFM, MLH1_LFM, GNA13_LFM, FGF19_LFM, ALK_HFM, AMER1_HFM, BRAF_HFM, IDH1_HFM, DNMT3A_HFM, CDK6_LFM, BCL6_LFM, WT1_HFM, SOX9_LFM, SMAD4_LFM, HNF1A_LFM, JAK2_LFM, PAX5_HFM, HSD3B1_LFM, PARP2_LFM, ASXL1_HFM, ESR1_LFM, PIK3C2G_HFM, BRCA2_LFM, ASXL1_LFM, TERT_HFM, WT1_LFM, BARD1_LFM, MAP3K1_LFM, FGFR2_LFM, PPARG_LFM, AXIN1_LFM, PDCD1LG2_HFM, SOX9_HFM, KRAS_LFM, PRKAR1A_HFM, PDGFRB_LFM, TSC2_LFM, NF2_LFM, FGF6_LFM, PBRM1_LFM, KEL_LFM, FOXL2_LFM, CARD11_LFM, MAP3K13_LFM, FGF6_HFM, NOTCH3_LFM, SF3B1_LFM, CUL3_LFM, FLT1_LFM, CD274_HFM, IDH1_LFM, SPOP_LFM, MYCN_HFM, SRC_LFM, AR_LFM, ERBB2_LFM, CASP8_LFM, PDGFRA_LFM, IRF4_LFM, PTEN_LFM, GNAS_LFM, CCNE1_LFM, NOTCH2_LFM, ATR_LFM, RBM10_LFM, FANCG_HFM, SMO_HFM, EGFR_LFM et SPOP_HFM ;

(v) dans lequel la présence d'une ou de plusieurs des mutations GOF et/ou LOF suivantes contribue au classement du profil comme correspondant au profil de mutation d'une signature de réponse :

FGF19_LOF, TP53_GOF_331, NFE2L2_GOF_24, PIK3C2G_GOF_1088, U2AF1_GOF_34, NBN_LOF, NFE2L2_GOF_77, TP53_GOF_376, TP53_GOF_244, AKT1_GOF_17, RAC1_LOF, MYCN_GOF, CDKN2A_GOF_151, DNMT3A_GOF_882, NRAS_LOF, XRCC2_LOF, PAX5_GOF, BRAF_GOF_600, RAD52_GOF, PDCD1LG2_GOF, CD274_GOF, TP53_GOF_159, VEGFA_LOF, CBFB_LOF, PDK1_LOF, KDM5C_GOF_1330, KEAP1_GOF_332, STK11_GOF_37, BRCA2_GOF, KEAP1_GOF_116, MYD88_GOF_265, IDH1_GOF, AR_GOF_457, RB1_GOF, MUTYH_GOF_165, BRAF_GOF_466, POLE_GOF, TP53_GOF_673, CD79B_GOF, ARID1A_GOF_21, PRKAR1A_GOF, DIS3_GOF_458, CDKN2A_GOF_69, FANCL_LOF, FANCG_GOF, SOX2_LOF, SMAD4_GOF, BRAF_GOF_469, TP53_GOF_275, TP53_GOF_192, STAG2_LOF, QKI_LOF, FGF6_GOF, CTNNA1_LOF, MLH1_LOF, CDH1_LOF, ERBB3_LOF, PBRM1_LOF, ASXL1_LOF, KDM5A_GOF, NF1_LOF, CHEK1_LOF, BRCA2_LOF, TP53_GOF_560, WT1_LOF, SDHB_LOF, EGFR_GOF_858, TP53_GOF_215, PDGFRA_LOF, ATRX_LOF, ATR_LOF, IRF2_GOF, EGFR_GOF_771, EGFR_GOF_861, MLH1_GOF, AR_GOF_465, STK11_GOF_221, FAS_GOF, AMER1_GOF_385, RBM10_GOF_503, KEAP1_GOF_153, STK11_GOF_199, PTCH1_GOF, STK11_GOF_163, ATRX_GOF, KEAP1_GOF_509, TP53_GOF_236, KEAP1_GOF_362, HSD3B1_GOF_75, RB1_GOF_576, STK11_GOF_216, FBXW7_GOF_479, KEAP1_GOF_523, JAK3_GOF, PPARG_LOF, PIK3CB_GOF, ACVR1B_LOF, TSC2_LOF, KMT2A_LOF, TP53_GOF_238, AKT1_LOF, PIK3R1_LOF, IRS2_GOF, HRAS_GOF_61, PIK3CA_GOF_726, AR_GOF_468, TNFRSF14_LOF, NFE2L2_GOF_29, MEN1_GOF, BCL2_LOF, AR_GOF_467, ATM_GOF_337, KEAP1_GOF_544, NTRK3_GOF, NTRK3_GOF_610, HRAS_LOF, CTNNB1_GOF_41, SMARCA4_GOF_1160, KEAP1_GOF_409, SMAD2_GOF_464, PTEN_GOF_59, CTNNB1_GOF_33, CREBBP_GOF_1472, APC_GOF, PTEN_GOF_165, TSC1_GOF, KEAP1_GOF_417, RBM10_GOF, NBN_GOF_219, PIK3CA_GOF_345, AR_GOF_493, ZNF217_GOF_410, AR_GOF_598, STK11_GOF_256, ARID1A_GOF_343, RET_GOF_511, KEAP1_GOF_155, MITF_GOF, CDK8_GOF, DNMT3A_GOF_749, MYD88_GOF, KEAP1_GOF_430, GNAS_GOF_415, KDM5C_GOF, FLT1_GOF, NF1_GOF_1642, KMT2A_GOF_53, SDHA_GOF_531, CDKN2A_GOF_61, CEBPA_GOF_197, STK11_GOF_220, NBN_GOF_680, KEAPI_GOF_450, CHEK2_GOF_392, NKX2-1_GOF_234, SMARCA4_GOF_1157, BCORL1_GOF_94, KEAP1_GOF_493, FLCN_GOF_306, STK11_GOF_168 et MSH6_GOF_1088 ;

(vi) dans lequel la présence d'une ou de plusieurs des mutations GOF et/ou LOF suivantes contribue au classement du profil comme correspondant au profil de mutation d'une signature de réponse :

ALOX12B_LFM, DNMT3A_HFM_771, CTNNA1_LFM, NFE2L2_HFM_24, MEF2B_LFM, TP53_HFM_331, BRAF_HFM_469, XRCC2_LFM, TNFRSF 14_LFM, BRIP1_LFM, FUBP1_LFM, U2AF1_HFM_34, FAS_LFM, TP53_HFM_173, TSC1_LFM, ERRFI1_LFM, EZH2_LFM, NBN_LFM, MLH1_LFM, BRAF_HFM_600, FGF19_LFM, GNA13_LFM, ALK_HFM, TP53_HFM_215, TP53_HFM_783, TP53_HFM_560, CDK6_LFM, BCL6_LFM, TP53_HFM_280, BTG1_LFM, WT1_HFM, SOX9_LFM, SMAD4_LFM, NF1_HFM, JAK2_LFM, HSD3B1_LFM, PARP2_LFM, PIK3C2G_HFM, BRCA2_LFM, ASXL1_LFM, TERT_HFM_, BARD1_LFM, MAP3K1_LFM, FGFR2_LFM, SDHA_LFM, IGF1R_LFM, AXIN1_LFM, PDCD1LG2_HFM, PAX5_HFM, TP53_HFM_294, RAD51C_LFM, JUN_LFM, PRKAR1A _HFM, PDGFRB_LFM, TSC2_LFM, NF2_LFM, BTK_LFM, PBRM1_LFM, FOXL2_LFM, MAP3K13_LFM, NOTCH3_LFM, GABRA6_LFM, PIK3CA_HFM_542, TP53_HFM_159, CUL3_LFM, ASXL1_HFM, FLT1_LFM, MITF_LFM, TP53_HFM_249, IDH1_LFM, SRC_LFM, AR_LFM, ERBB2_LFM, SMO_LFM, KEAP1_LFM, PDGFRA_LFM, GATA6_LFM, PTEN_LFM, GNAS_LFM, CCNE1_LFM, ATR_LFM, MERTK_LFM, KMT2A_LFM, SOX9_HFM, GSK3B_HFM, CUL4A _LFM et BCL2L2_LFM ;

(vii) dans lequel la présence d'une mutation dans un ou plusieurs des gènes suivants contribue au classement du profil comme correspondant au profil de mutation d'une signature de résistance :
GID4, JUN, RAD51, CD79A, STK11, TET2, MAP2K1, CCNE1, CDK4, ERCC4, CEBPA, RARA, CDKN1A, RAD51B, PPP2R1A, CSF1R, FH, NKX2-1, NTRK2, FGFR1, CDK6, MDM2 et BRD4 ;

(viii) dans lequel la présence d'une mutation dans un ou plusieurs des gènes suivants contribue au classement du profil comme correspondant au profil de mutation d'une signature de résistance :
RARA, VHL, GID4, IKBKE, CEBPA, CD79A, CDK12, STK11, BAP1, CDKN2B, FGF10, ARFRP1, CSF1R, FANCC, CDC73, ZNF703, PIK3CA, NKX2-1, TNFAIP3, CCNE1, SOX2, SDHC, MYC, TERC, PARP3, JAK1, DDR2, PARP1, AKT2, PDK1, NFE2L2 et MDM4 ;

(ix) dans lequel la présence d'une ou de plusieurs des mutations GOF et/ou LOF suivantes contribue au classement du profil comme correspondant au profil de mutation d'une signature de résistance :
MAP3K1_GOF, FGF14_GOF, PPP2R1A_GOF, CDKN1A_GOF, JAK1_GOF, IRF4_GOF, JUN_LOF, AKT3_GOF, NKX2-1_LOF, TGFBR2_GOF, GABRA6_GOF, BCORL1_GOF, TNFAIP3_GOF, POLD1_GOF, GNAQ_GOF, RAD51C_GOF, BRD4_GOF, RNF43_GOF, FGFR4_GOF, GATA6_GOF, GATA3_GOF, MDM2_LOF, MYC_LOF, RPTOR_GOF, CD79A_LOF, CCNE1_GOF, STK11_LOF, MYCN_LOF, CEBPA_LOF, PARP1_GOF, NOTCH3_GOF, SDHD_GOF, LTK_GOF, DAXX_GOF, ABL1_GOF, PDGFRB_GOF, BTG1_GOF, CHEK1_GOF, GATA4_GOF, JUN_GOF, FLT3_GOF, CUL3_GOF, CDK4_GOF, AKT2_GOF, KDM6A_GOF, MTOR_GOF, BCL2L1_LOF, CD274_LOF, NF2_GOF, SMARCA4_GOF, NFKBIA_GOF, ERCC4_LOF, ZNF703_GOF, STK11_GOF, KEAP1_GOF, ERBB2_GOF, FH_GOF, REL_LOF, RARA_GOF, RAD51_LOF, PTEN_GOF, NTRK2_LOF, CDKN2B_LOF, BAP1_LOF, RARA_LOF, AXL_GOF, CCND2_LOF, CUL4A_LOF, H3F3A_GOF, GRM3_GOF, CDK6_GOF, ARFRP1_LOF, SUFU_LOF, RAD54L_LOF, PIK3CB_LOF, KLHL6_GOF, MAP3K13_GOF, PIK3CA_GOF, CDKN2A_LOF, EZH2_GOF, FANCA_LOF, HGF_LOF, FBXW7_GOF, FGFR2_GOF, AURKA_LOF, HSD3B1_LOF et SDHC_GOF;

(x) dans lequel la présence d'une ou de plusieurs des mutations GOF et/ou LOF suivantes contribue au classement du profil comme correspondant au profil de mutation d'une signature de résistance :
MYC_LFM, FANCC_HFM, JAK1_HFM, FGFR2_HFM, GABRA6_HFM, RARA_HFM, ERBB4_HFM, CD79A_LFM, GID4_LFM, IGF1R_HFM, GNA11_HFM, GATA6_HFM, CDC73_HFM, CHEK2_HFM, ARFRP1_HFM, SMARCB1_HFM, FGF10_HFM, CDK12_HFM, IKBKE_LFM, DDR2_HFM, NF2_HFM, AKT3_HFM, CEBPA_LFM, FGF14_HFM, EP300_HFM, CCNE1_HFM, AKT2_HFM, BAP1_LFM, FH_HFM, CEBPA_HFM, ERBB2_HFM, CCND2_LFM, ATM_HFM, PIK3CA_LFM, STK11_HFM, CDKN2B_LFM, PRDM1_LFM, NOTCH3_HFM, CDK12_LFM, ZNF703_HFM, CSF1R_LFM, KLHL6_HFM, BCL2L2_HFM, LYN_HFM, RICTOR_HFM, SOX2_HFM, STK11_LFM, KEAP1_HFM, CYP17A1_LFM, CUL4A_HFM, PRKCI_HFM, PIK3CA_HFM, TERC_HFM, IKZF1_HFM, MEF2B_HFM, NFE2L2_HFM, CDKN2A_ LFM, NKX2-1_HFM, TEK_LFM, MYCL_LFM, ROS1_HFM, MCL1_HFM, FGF12_HFM, EZH2_HFM et MDM4_HFM ;

(xi) dans lequel la présence d'une ou de plusieurs des mutations GOF et/ou LOF suivantes contribue au classement du profil comme correspondant au profil de mutation d'une signature de résistance :
EGFR_GOF_746, TP53_GOF_282, EGFR_GOF_747, EGFR_GOF_719, TP53_GOF_195, FGF14_GOF, PPP2R1A_GOF, IRF4_GOF, MAP3K1_GOF, STK11_GOF_291, ERBB2_GOF_776, JAK1_GOF, CDKN1A_GOF, KEAP1_GOF_272, JUN_LOF, AKT3_GOF, NKX2-1_LOF, ATM_GOF, CDKN2A_GOF_100, STK11_GOF_84, FGFR4_GOF, KEAP1_GOF_483, KEAP1_GOF_234, GABRA6_GOF, CDKN2A_GOF_80, MYCN_GOF_44, KEAP1_GOF_260, MAP2K1_GOF_102, PTEN_GOF, PTCH1_GOF_48, GNAQ_GOF, KEAP1_GOF_364, POLD1_GOF, PIK3CA_GOF_1043, BCOR_GOF_679, FH_GOF_476, STK11_GOF_181, TP53_GOF_285, TNFAIP3_GOF, BRD4_GOF, KDM5C_GOF_1546, KEAP1_GOF_274, SMAD4_GOF_351, RNF43_GOF, SF3B1_GOF_666, MTOR_GOF_1834, NOTCH1_GOF, TP53_GOF_272, GATA6_GOF, MDM2_LOF, GATA3_GOF, PIK3CA_GOF_1047, TP53_GOF_278, CD79A_LOF, RPTOR_GOF, ZNF703_GOF, CCNE1_GOF, STK11_LOF, MYCN_LOF, PARP1_GOF, CEBPA_LOF, MAP2K1_GOF_121, SMARCA4_GOF_1243, MED12_GOF, KEAP1_GOF_135, AR_GOF_69, BTG1_GOF, MAP3K1_GOF_5, TYRO3_GOF, ERBB2_GOF_755, CBL_GOF_1096, STK11_GOF_176, EGFR_GOF_790, RAF1_GOF, KEAP1_GOF_244, STK11_GOF_464, STK11_GOF_308, KDM6A_GOF, PDGFRB_GOF, BRAF_GOF_464, PIK3C2G_GOF_129, FBXW7_GOF_505, KEAP1_GOF_470, ALOX12B_GOF, FLT3_GOF, AMER1_GOF_625, IDH2_GOF_140, GNAS_GOF_407, KEAP1_GOF_186, BCOR_GOF_1526, NFE2L2_GOF_27, STK11_GOF_251, CHEK1_GOF, HRAS_GOF_13, KEAP1_GOF_236, GATA4_GOF, MET_GOF_2888, KMT2D_GOF_755, RAD51C_GOF_21, SMARCA4_GOF_1162, STK11_GOF_734, MAP3K1_GOF_949, PALB2_GOF, BCORL1_GOF_883, KEAP1_GOF, ARID1A_GOF_515, AR_GOF_469, EGFR_GOF_763, AR_GOF_70, PPARG_GOF, VHL_GOF, PARP3_GOF, CDK4_GOF, AKT2_GOF, TP53_GOF_920, NFE2L2_GOF_30, CHEK2_GOF, SMAD2_LOF, SMARCB1_GOF, TP53_GOF_298, SDHA_GOF_457, FH_GOF, CDC73_GOF, NFKBIA_GOF, ERCC4_LOF, RARA_GOF, RAD51B_LOF, NTRK2_LOF, CUL4A_LOF, STK11_GOF_57, TP53_GOF_234, STK11_GOF_242, CHEK2_GOF_367, RAD51D_GOF, CTCF_LOF, EPHA3_GOF, RAD54L_LOF, PIK3CB_LOF, KLHL6_GOF, PIK3CA_GOF, DDR2_GOF, MAP3K13_GOF, PIK3CA_GOF_545, HGF_LOF, NFE2L2_GOF_31, MET_GOF_3028, BCL6_GOF, TP53_GOF_342 et CDKN2A _GOF_83 ; et/ou

(xii) dans lequel la présence d'une ou de plusieurs des mutations GOF et/ou LOF suivantes contribue au classement du profil comme correspondant au profil de mutation d'une signature de résistance :
EGFR_HFM_746, MYC_LFM, FANCC_HFM, JAK1_HFM, NFE2L2_HFM_79, GABRA6_HFM, RARA_HFM, ERBB4_HFM, CD79A_LFM, NF2_HFM, GNA11_HFM, CHEK2_HFM, GID4_LFM, NFE2L2_HFM_29, IGF1R_HFM, GATA6_HFM, CDC73_HFM, FGFR2_HFM, CHEK2_HFM_157, STK11_HFM_242, TP53_HFM_152, STK11_HFM_53, ARFRP1_HFM, TP53_HFM_282, FGF10_HFM, CDK12_HFM, IKBKE_LFM, DDR2_HFM, NKX2-1_LFM, AKT3_HFM, ERBB2_HFM_775, TP53_HFM_920, CEBPA_LFM, DIS3_HFM, GATA3_HFM, EP300_HFM, PIK3CA_HFM_1047, FGF14_HFM, ARAF_HFM_181, AKT2_HFM, CCNE1_HFM, BAP1_LFM, FH_HFM, TP53_HFM_154, PIK3CA_HFM, VHL_LFM, ERBB3_HFM, H3F3A_HFM, CCND2_LFM, PIK3CA _LFM, CDKN2B_LFM, PRDM1_LFM, STK11_HFM_194, TP53_HFM_135, KEAP1_HFM_320, ZNF703_HFM, CSF1R_LFM, KLHL6_HFM, BCL2L2_HFM, LYN_HFM, SOX2_HFM, STK11 _LFM, SDHC_HFM, EPHA3_HFM, CUL4A_HFM, STK11_HFM, MAP3K13_HFM, BCL6_HFM, TERC_HFM, NFE2L2_HFM_28, JAK3_HFM, TP53_HFM_272, CDKN2A_LFM, TEK_LFM, ROS1_HFM, NFKBIA_HFM et FGF19_HFM.

8. Procédé selon l'une quelconque des revendications précédentes, (i) dans lequel ladite réponse est une réponse durable, éventuellement dans lequel ladite réponse durable est une absence de progression de la maladie pendant au moins 270 jours après une thérapie par IPCI ; et/ou

(ii) dans lequel ladite résistance est une résistance innée, éventuellement dans lequel ladite résistance innée est une absence de réponse à une thérapie par IPCI et/ou de progression de la maladie avec 270 jours de thérapie par IPCI ; et/ou
(iii) dans lequel le nombre total de gènes constituant le profil de mutation ne dépasse pas 100 gènes.

9. Procédé de classification d'un échantillon obtenu auprès d'un sujet atteint d'un cancer du poumon, ledit échantillon comprenant un acide nucléique, éventuellement dans lequel ledit échantillon est issu d'une ou de plusieurs cellules cancéreuses du sujet, le procédé comprenant :

l'analyse de l'échantillon pour obtenir un profil de mutation pour le sujet, ledit profil de mutation comprenant la présence ou l'absence de mutations en un ou plusieurs emplacements dans au moins cinq, six, ou au moins sept gènes choisis dans le groupe constitué par : NF1, STK11, TSC2, BRCA2, BRAF, STAG2, U2AF1, BRIP1, PDGFRA, CTNNA1, PDK1, FGF10 et FLT1 ; et
l'analyse du profil de mutation pour classer le profil comme correspondant au profil de mutation d'une signature de réponse à l'inhibiteur de point de contrôle immunitaire (IPCI) ou d'une signature de résistance à l'IPCI, dans lequel l'échantillon est classé comme étant issu d'un sujet sensible à une thérapie par IPCI si le profil de mutation est classé comme correspondant au profil de mutation de la signature de réponse à l'IPCI et est caractérisé comme étant issu d'un sujet résistant à une thérapie par IPCI si le profil de mutation est classé comme correspondant au profil de mutation de la signature de résistance à l'IPCI.

10. Procédé selon la revendication 9, dans lequel lesdites mutations en un ou de plusieurs emplacements sont des mutations spécifiques au cancer et/ou

dans lequel l'analyse de l'échantillon pour obtenir le profil de mutation pour le sujet comprend le séquençage d'acides nucléiques, éventuellement dans lequel l'échantillon est séquencé pour fournir au moins une couverture exonique des au moins cinq, six, sept gènes ou de tous les gènes comme défini dans l'une quelconque des revendications précédentes et/ou

dans lequel l'échantillon comprend un échantillon de tissu tumoral, une cellule tumorale circulante ou un échantillon acellulaire comprenant de l'ADN tumoral circulant (ADNtc) et/ou de l'ARN tumoral circulant (ARNtc) et/ou

dans lequel l'analyse du profil de mutation pour classer le profil comme correspondant au profil de mutation d'une signature de réponse ou d'une signature de résistance comprend la mise en œuvre du procédé selon l'une quelconque des revendications 1 à 8.

11. Procédé selon l'une quelconque des revendications précédentes, dans lequel (i) ledit cancer du poumon est un cancer du poumon non à petites cellules ou un cancer du poumon à petites cellules éventuellement dans lequel ledit cancer du poumon est un cancer du poumon non à petites cellules ; et/ou

(ii) ladite thérapie par IPCI comprend un inhibiteur de PD-L1, PD-1 et/ou CTLA-4 éventuellement dans lequel ladite thérapie par IPCI comprend un agent choisi dans le groupe constitué par : le nivolumab, le pembrolizumab, l'atézolizumab, le durvalumab, l'avélumab, l'ipilimumab et le cémiplimab ; et/ou

(iii) le procédé comprend en outre l'analyse d'un ou de plusieurs marqueurs supplémentaires de réponse à l'IPCI issus du sujet pour compléter et/ou confirmer la prédiction de réponse ou de résistance à l'IPCI éventuellement dans lequel le ou les marqueurs supplémentaires de réponse à l'IPCI sont choisis dans le groupe constitué par : l'âge, le stade de la maladie, l'histologie, les antécédents de tabagisme, l'ethnie, le sexe, la charge mutationnelle tumorale (TMB), l'instabilité des microsatellites (MSI), l'expression de PD-L1, JAK1/JAK2, IFNg, la perte de PTEN, PBRM1, les mutations STK11/KEAP1, la perte de traitement/présentation de l'antigène et la signalisation WNT/b-caténine.

12. Système de prédiction de la réponse au traitement d'un sujet atteint d'un cancer du poumon à une thérapie par inhibiteur de point de contrôle immunitaire (IPCI), le système comprenant :

au moins un processeur ; et

au moins un support lisible par ordinateur non transitoire contenant des instructions qui, lorsqu'elles sont exécutées par l'au moins un processeur, amènent l'au moins un processeur à réaliser des opérations comprenant :

l'obtention d'un profil de mutation pour le sujet, ledit profil comprenant la présence ou l'absence d'une ou de plusieurs mutations en un ou plusieurs emplacements dans au moins cinq, six ou sept gènes choisis dans le groupe constitué par : NF1, STK11, TSC2, BRCA2, BRAF, STAG2, U2AF1, BRIP1, PDGFRA, CTNNA1, PDK1, FGF10 et FLT1 ;

l'analyse du profil de mutation pour classer le profil comme correspondant au profil de mutation d'une signature de réponse à l'IPCI ou d'une signature de résistance à l'IPCI,

dans lequel il est prédit que le sujet est susceptible de répondre à la thérapie par IPCI si le profil de mutation pour le sujet est classé comme correspondant au profil de mutation de la signature de réponse à l'IPCI et il est prédit qu'il n'est pas susceptible de répondre à la thérapie par IPCI si le profil de mutation pour le sujet est classé comme correspondant au profil de mutation de la signature de résistance à l'IPCI, éventuellement dans lequel lesdites instructions, lorsqu'elles sont exécutées par l'au moins un processeur, amènent l'au moins un processeur à réaliser le procédé selon l'une quelconque des revendications 1 à 8.

13. Support(s) lisible(s) par ordinateur comprenant des instructions qui, lorsqu'elles sont exécutées par un ou plusieurs processeurs, amènent le ou les processeurs à réaliser les étapes du procédé selon l'une quelconque des revendications 1 à 8.

14. Composition pharmaceutique comprenant un inhibiteur de point de contrôle immunitaire (IPCI) pour une utilisation dans une méthode de traitement d'un sujet atteint d'un cancer du poumon, dans laquelle il a été prédit que le sujet réponde à ladite thérapie par IPCI par un procédé selon l'une quelconque des revendications 1 à 8.

15. Composition pour une utilisation selon la revendication 14, dans laquelle la méthode de traitement comprend l'étape de prédiction si le sujet va répondre à la thérapie par IPCI par un procédé selon l'une quelconque des revendications 1 à 8 et/ou dans laquelle l'inhibiteur de point de contrôle immunitaire comprend un inhibiteur de PD-L1, PD-1 et/ou CTLA-4, éventuellement dans laquelle l'inhibiteur comprend un anticorps, en outre éventuellement dans laquelle ledit inhibiteur de point de contrôle immunitaire est choisi dans le groupe constitué par : le nivolumab, le pembrolizumab, l'atézolizumab, le durvalumab, l'avélumab, l'ipilimumab et le cémiplimab.

A

Study Duration (270 days)

pt #1

Durable-
response

First CPI

○

pt #2

Innate-
resistance

✕

14 Days

B

Flatiron-FMI Clinico-Genomic database (CGDB)

8752

Filter → liquid samples, old baits, patients without outcome definition, combo-therapy, acq-resistance, early death

Durable-response
372

Innate-resistance
427

FIG. 1

| Drugcat | | dura-response (N=472) | inn-resistant (N=631) | Total (N=1103) | p value |
|---|---|---|---|---|---|
| cpi | **Age** | | | | **0.038** |
| | N-Miss | 0 | 2 | 2 | |
| | Mean (SD) | 68.419 (9.524) | 67.021 (9.476) | 67.674 (9.518) | |
| | Range | 38.000 - 84.000 | 35.000 - 85.000 | 35.000 - 85.000 | |
| | **Gender** | | | | **0.606** |
| | N-Miss | 0 | 2 | 2 | |
| | F | 177 (47.6%) | 210 (49.4%) | 387 (48.6%) | |
| | M | 195 (52.4%) | 215 (50.6%) | 410 (51.4%) | |
| | **Race** | | | | **0.439** |
| | N-Miss | 0 | 2 | 2 | |
| | Caucasian | 251 (67.5%) | 311 (73.2%) | 562 (70.5%) | |
| | African American | 33 (8.9%) | 33 (7.8%) | 66 (8.3%) | |
| | Asian | 6 (1.6%) | 7 (1.6%) | 13 (1.6%) | |
| | Other | 57 (15.3%) | 48 (11.3%) | 105 (13.2%) | |
| | Unknown | 25 (6.7%) | 26 (6.1%) | 51 (6.4%) | |
| | **Disease Stage** | | | | **0.687** |
| | N-Miss | 0 | 2 | 2 | |
| | Mean (SD) | 3.628 (1.010) | 3.599 (1.008) | 3.613 (1.009) | |
| | Range | 1.200 - 4.800 | 1.200 - 4.800 | 1.200 - 4.800 | |
| | **Smoking History** | | | | **0.006** |
| | N-Miss | 0 | 2 | 2 | |
| | HistoryOfSmoking | 346 (93.0%) | 366 (86.1%) | 712 (89.3%) | |
| | NoHistoryOfSmoking | 26 (7.0%) | 58 (13.6%) | 84 (10.5%) | |

FIG. 1C

| | | | | |
|---|---|---|---|---|
| | UnknownNotDocumented | 0 (0.0%) | 1 (0.2%) | 1 (0.1%) | |
| | **Histology** | | | | **0.582** |
| | N-Miss | 0 | 2 | 2 | |
| | NonSquamousCellCarcinoma | 266 (71.5%) | 292 (68.7%) | 558 (70.0%) | |
| | SquamousCellCarcinoma | 94 (25.3%) | 121 (28.5%) | 215 (27.0%) | |
| | NSCLCHistologyNOS | 12 (3.2%) | 12 (2.8%) | 24 (3.0%) | |
| | **TMB** | | | | **< 0.001** |
| | N-Miss | 0 | 2 | 2 | |
| | Mean (SD) | 15.055 (14.691) | 9.922 (10.059) | 12.317 (12.691) | |
| | Range | 0.000 - 105.269 | 0.000 - 88.764 | 0.000 - 105.269 | |
| **chemo** | **Age** | | | | **0.822** |
| | N-Miss | 35 | 94 | 129 | |
| | Mean (SD) | 64.908 (9.084) | 65.245 (9.887) | 65.120 (9.571) | |
| | Range | 33.000 - 82.000 | 43.000 - 83.000 | 33.000 - 83.000 | |
| | **Gender** | | | | **0.979** |
| | N-Miss | 35 | 94 | 129 | |
| | F | 35 (53.8%) | 59 (53.6%) | 94 (53.7%) | |
| | M | 30 (46.2%) | 51 (46.4%) | 81 (46.3%) | |
| | **Race** | | | | **0.333** |
| | N-Miss | 35 | 94 | 129 | |
| | Caucasian | 47 (72.3%) | 73 (66.4%) | 120 (68.6%) | |
| | African American | 6 (9.2%) | 4 (3.6%) | 10 (5.7%) | |
| | Asian | 3 (4.6%) | 8 (7.3%) | 11 (6.3%) | |
| | Other | 6 (9.2%) | 16 (14.5%) | 22 (12.6%) | |
| | Unknown | 3 (4.6%) | 9 (8.2%) | 12 (6.9%) | |
| | **Disease Stage** | | | | **0.839** |

FIG. 1C (Continued)

| | | | |
|---|---|---|---|
| N-Miss | 35 | 94 | 129 |
| Mean (SD) | 3.842 (0.955) | 3.872 (0.948) | 3.861 (0.948) |
| Range | 1.200 - 4.400 | 1.200 - 4.800 | 1.200 - 4.800 |
| **Smoking History** | | | **0.199** |
| N-Miss | 35 | 94 | 129 |
| HistoryOfSmoking | 51 (78.5%) | 73 (66.4%) | 124 (70.9%) |
| NoHistoryOfSmoking | 14 (21.5%) | 36 (32.7%) | 50 (28.6%) |
| UnknownNotDocumented | 0 (0.0%) | 1 (0.9%) | 1 (0.6%) |
| **Histology** | | | **0.074** |
| N-Miss | 35 | 94 | 129 |
| NonSquamousCellCarcinoma | 62 (95.4%) | 95 (86.4%) | 157 (89.7%) |
| SquamousCellCarcinoma | 1 (1.5%) | 12 (10.9%) | 13 (7.4%) |
| NSCLCHistologyNOS | 2 (3.1%) | 3 (2.7%) | 5 (2.9%) |
| **TMB** | | | **0.245** |
| N-Miss | 35 | 94 | 129 |
| Mean (SD) | 8.986 (9.140) | 7.438 (8.072) | 8.013 (8.492) |
| **Range** | **0.000 - 37.410** | **0.000 - 43.500** | **0.000 - 43.500** |

FIG. 1C (Continued)

FIG. 2A

FIG. 2B

Top Deleted Genes

FIG. 2C

Top Rearrangements

FIG. 2D

## Single Gene

### Binary

| Mutation | Pvalue | Corrected Pvalue | DR with mutation | IR with mutation | Freq % (n=8768) |
|---|---|---|---|---|---|
| TSC2 | .0001 | .029 | 29 (76.4%) | 9 (23.6%) | 5.1 |
| STK11 | .0002 | .029 | 51 (33.2%) | 103 (66.8%) | 17.5 |
| TP53 | .0007 | .069 | 277 (50.6%) | 270 (49.4%) | 66.4 |
| PDGFRA | .0011 | .081 | 50 (64.2%) | 28 (35.8%) | 8.4 |

### HFM/LFM

| Mutation | Pvalue | Corrected Pvalue | DR with mutation | IR with mutation | Freq % (n=8768) |
|---|---|---|---|---|---|
| TP53_HFM | .0002 | .046 | 206 (53.3%) | 181 (46.7%) | 66.4 |
| NF1_LFM | .0004 | .046 | 65 (63.2%) | 38 (36.8%) | 12.4 |
| NBN_LFM | .0004 | .046 | 19 (82.6%) | 4 (17.4%) | 6.2 |
| PDGFRA_LFM | .0004 | 046 | 51 (65.4%) | 27 (34.6%) | 8.4 |

### HotSpot

| Mutation | Pvalue | Corrected Pvalue | DR with mutation | IR with mutation | Freq % (n=8768) |
|---|---|---|---|---|---|
| NF1_LFM | .0004 | .064 | 65 (63.1%) | 38 (36.9%) | 12.4 |
| NBN_LFM | .0004 | .064 | 19 (82.6%) | 4 (17.4%) | 6.2 |
| PDGFRA_LFM | .0004 | .064 | 51 (65.4%) | 27 (34.6%) | 8.4 |
| FGFR4_LFM | .0057 | .200 | 28 (68.3%) | 13 (31.7%) | 6.3 |

## Pair Co-occurence

### Binary

| Mutation | Pvalue | Corrected Pvalue | DR with mutation | IR with mutation | Freq % (n=8768) |
|---|---|---|---|---|---|
| STK11 AND KRAS | 1.38E-06 | .004 | 17 (22.1%) | 60 (77.9%) | 17.5/31.2 |

## Triplet Co-occurence

### Binary

| Mutation | Pvalue | Corrected Pvalue | DR with mutation | IR with mutation | Freq % (n=8768) |
|---|---|---|---|---|---|
| KRAS AND TP53 AND NF1 | 1.80E-05 | .076 | 18 (94.7%) | 1 (5.3%) | 31.2/66.4/12.4 |

## FIG. 3

STK11

Number at risk

| | | | | | | | | | | | | | | | | |
|---|---|---|---|---|---|---|---|---|---|---|---|---|---|---|---|---|
| 34 | 29 | 15 | 8 | 7 | 7 | 5 | 5 | 4 | 3 | 2 | 1 | 1 | 0 | 0 | 0 | 0 |
| 119 | 93 | 55 | 32 | 20 | 14 | 10 | 5 | 1 | 1 | 0 | 0 | 0 | 0 | 0 | 0 | 0 |
| 236 | 191 | 122 | 83 | 58 | 41 | 28 | 22 | 20 | 13 | 9 | 4 | 2 | 1 | 1 | 1 | 1 |
| 639 | 534 | 403 | 295 | 192 | 123 | 78 | 41 | 10 | 3 | 0 | 0 | 0 | 0 | 0 | 0 | 0 |
| 0 | 6 | 12 | 18 | 24 | 30 | 36 | 42 | 48 | 54 | 60 | 66 | 72 | 78 | 84 | 90 | 96 |

Time

| Group1 | Group2 | p.value |
|---|---|---|
| Mut cpi | Mut chemo | 0.8685232 |
| WT chemo | Mut chemo | 0.8685232 |
| WT cpi | Mut chemo | 0.6340901 |
| WT chemo | Mut cpi | 0.6878333 |
| WT cpi | Mut cpi | 0.04478 |
| WT cpi | WT chemo | 0.0683325 |

FIG. 4A

PDGFRA

| Group1 | Group2 | p.value |
|--------|--------|---------|
| Mut cpi | Mut chemo | 0.024453 |
| WT chemo | Mut chemo | 0.5136703 |
| WT cpi | Mut chemo | 0.5136703 |
| WT chemo | Mut cpi | 0.0007967 |
| WT cpi | Mut cpi | 0.0051198 |
| WT cpi | WT chemo | 0.288129 |

FIG. 4A (Continued)

NBN

Number at risk

| | | | | | | | | | | | | | | | | |
|---|---|---|---|---|---|---|---|---|---|---|---|---|---|---|---|---|
| 7 | 7 | 6 | 4 | 2 | 1 | 0 | 0 | 0 | 0 | 0 | 0 | 0 | 0 | 0 | 0 | 0 |
| 22 | 21 | 19 | 17 | 13 | 6 | 4 | 2 | 2 | 0 | 0 | 0 | 0 | 0 | 0 | 0 | 0 |
| 263 | 213 | 131 | 87 | 63 | 47 | 33 | 27 | 24 | 16 | 11 | 5 | 3 | 1 | 1 | 1 | 1 |
| 736 | 606 | 439 | 310 | 199 | 131 | 84 | 44 | 9 | 4 | 0 | 0 | 0 | 0 | 0 | 0 | 0 |
| 0 | 6 | 12 | 18 | 24 | 30 | 36 | 42 | 48 | 54 | 60 | 66 | 72 | 78 | 84 | 90 | 96 |

Time

| Group1 | Group2 | p.value |
|---|---|---|
| Mut cpi | Mut chemo | 0.2904107 |
| WT chemo | Mut chemo | 0.5553062 |
| WT cpi | Mut chemo | 0.8229528 |
| WT chemo | Mut cpi | 0.040179 |
| WT cpi | Mut cpi | 0.0947282 |
| WT cpi | WT chemo | 0.1293699 |

FIG. 4A (Continued)

NF1

| | Group1 | Group2 | p.value |
|---|---|---|---|
| | Mut cpi | Mut chemo | 0.0960741 |
| | WT chemo | Mut chemo | 0.819423 |
| | WT cpi | Mut chemo | 0.9383144 |
| | WT chemo | Mut cpi | 0.0011369 |
| | WT cpi | Mut cpi | 0.0019406 |
| | WT cpi | WT chemo | 0.5158266 |

FIG. 4A (Continued)

Mut TP53 + Mut KRAS + Mut NF1 VS WT

Number at risk

| | | | | | | | | | | | | | | | | | |
|---|---|---|---|---|---|---|---|---|---|---|---|---|---|---|---|---|---|
| — | 16 | 16 | 15 | 13 | 10 | 8 | 5 | 4 | 3 | 0 | 0 | 0 | 0 | 0 | 0 | 0 | 0 |
| — | 270 | 220 | 137 | 91 | 65 | 48 | 33 | 27 | 24 | 16 | 11 | 5 | 3 | 1 | 1 | 1 | 1 |
| — | 742 | 611 | 443 | 314 | 202 | 129 | 83 | 42 | 8 | 4 | 0 | 0 | 0 | 0 | 0 | 0 | 0 |
| | 0 | 6 | 12 | 18 | 24 | 30 | 36 | 42 | 48 | 54 | 60 | 66 | 72 | 78 | 84 | 90 | 96 |

Time

| Group1 | Group2 | p.value |
|---|---|---|
| WT chemo | Mut cpi | 0.0013309 |
| WT cpi | Mut cpi | 0.0013309 |
| WT cpi | WT chemo | 0.0930456 |

FIG. 4A (Continued)

## STK11

Number at risk

| ---- | 194 | 99 | 66 | 43 | 28 | 25 | 18 | 13 | 10 | 8 | 4 | 3 | 3 | 0 | 0 | 0 | 0 | 0 | 0 | 0 | 0 | 0 |
| —— | 377 | 210 | 130 | 75 | 45 | 32 | 24 | 12 | 5 | 1 | 0 | 0 | 0 | 0 | 0 | 0 | 0 | 0 | 0 | 0 | 0 | 0 |
| —— | 1164 | 674 | 440 | 318 | 248 | 185 | 137 | 102 | 79 | 64 | 46 | 30 | 22 | 18 | 14 | 10 | 8 | 6 | 4 | 1 | 1 | 1 |
| —— | 2098 | 1325 | 902 | 588 | 377 | 234 | 137 | 67 | 22 | 6 | 0 | 0 | 0 | 0 | 0 | 0 | 0 | 0 | 0 | 0 | 0 | 0 |

0 6 12 18 24 30 36 42 48 54 60 66 72 78 84 90 96 102 108 114 120 126

Time

| Group1 | Group2 | p.value |
|---------|-----------|-----------|
| Mut cpi | Mut chemo | 0.8551525 |
| WT chemo | Mut chemo | 0.1345701 |
| WT cpi | Mut chemo | 0.0668087 |
| WT chemo | Mut cpi | 0.1345701 |
| WT cpi | Mut cpi | 0.0064787 |
| WT cpi | WT chemo | 0.1345701 |

FIG. 4B

PDGFRA

| Group1 | Group2 | p.value |
|---|---|---|
| Mut cpi | Mut chemo | 0.0890152 |
| WT chemo | Mut chemo | 0.8732425 |
| WT cpi | Mut chemo | 0.8732425 |
| WT chemo | Mut cpi | 0.0008786 |
| WT cpi | Mut cpi | 0.0010007 |
| WT cpi | WT chemo | 0.4831006 |

FIG. 4B (Continued)

## NBN

Number at risk

| | | | | | | | | | | | | | | | | | | | | | |
|---|---|---|---|---|---|---|---|---|---|---|---|---|---|---|---|---|---|---|---|---|---|
| 33 | 20 | 15 | 11 | 5 | 3 | 0 | 0 | 0 | 0 | 0 | 0 | 0 | 0 | 0 | 0 | 0 | 0 | 0 | 0 | 0 | 0 |
| 66 | 42 | 33 | 25 | 19 | 10 | 6 | 3 | 2 | 0 | 0 | 0 | 0 | 0 | 0 | 0 | 0 | 0 | 0 | 0 | 0 | 0 |
| 1325 | 753 | 491 | 350 | 271 | 207 | 155 | 115 | 89 | 72 | 50 | 33 | 25 | 18 | 14 | 10 | 8 | 6 | 4 | 1 | 1 | 1 |
| 2409 | 1493 | 999 | 638 | 403 | 256 | 155 | 76 | 25 | 7 | 0 | 0 | 0 | 0 | 0 | 0 | 0 | 0 | 0 | 0 | 0 | 0 |

Time: 0 6 12 18 24 30 36 42 48 54 60 66 72 78 84 90 96 102 108 114 120 126

| Group1 | Group2 | p.value |
|---|---|---|
| Mut cpi | Mut chemo | 0.0425708 |
| WT chemo | Mut chemo | 0.7774666 |
| WT cpi | Mut chemo | 0.5388 |
| WT chemo | Mut cpi | 0.0425708 |
| WT cpi | Mut cpi | 0.0425708 |
| WT cpi | WT chemo | 0.2577424 |

## FIG. 4B (Continued)

NF1

| Group1 | Group2 | p.value |
|---|---|---|
| Mut cpi | Mut chemo | 0.2347139 |
| WT chemo | Mut chemo | 0.5910192 |
| WT cpi | Mut chemo | 0.7174128 |
| WT chemo | Mut cpi | 0.0195562 |
| WT cpi | Mut cpi | 0.0278578 |
| WT cpi | WT chemo | 0.5604957 |

FIG. 4B (Continued)

Mut TP53 + Mut KRAS + Mut NF1 VS WT

| Group1 | Group2 | p.value |
|--------|--------|---------|
| Mut cpi | Mut chemo | 0.4198845 |
| WT chemo | Mut chemo | 0.5338764 |
| WT cpi | Mut chemo | 0.5338764 |
| WT chemo | Mut cpi | 0.0210464 |
| WT cpi | Mut cpi | 0.0210464 |
| WT cpi | WT chemo | 0.2963864 |

FIG. 4B (Continued)

Machine Learning

Genetic Algorithm

Mutation Calling

Copy Number

Somatic

Fusions/Rearrangements

Targeted Sequencing

Tumor Sample

Innate-Resistance

Durable-Response

Prediction Models

| Binary | HFM/LFM | HotSpot |
|---|---|---|
| 12 Mutation Signatures | 12 Mutation Signatures | 12 Mutation Signatures |

FIG. 5A

145

FIG. 5B

FIG. 5C

FIG. 6A

FIG. 6B

FIG. 7

FIG. 7 (Continued)

A

Base line IL6
LUNG: NON SMALL CELL

| | PR | SD | PD |
|---|---|---|---|
| Count | 11 | 9 | 15 |
| Median | 6.8 | 10 | 9.4 |
| Outliers | 0 | 1 | 2 |

B

Overall survival of NSCLC patients with high/low IL6 levels at C1D1 predose
PCD4989G (unbalanced)

— high IL6  — low IL6

Log-rank
p = 0.032

Number at risk

| | | | | | |
|---|---|---|---|---|---|
| 43 | 17 | 10 | 9 | 7 | 0 |
| 43 | 34 | 17 | 11 | 8 | 0 |
| 0 | 12 | 24 | 36 | 48 | 60 |

Month

FIG. 8

FIG. 9A

FIG. 9B

PDGFRB

| Group1 | Group2 | p.value |
|---|---|---|
| Mut cpi | Mut chemo | 0.4600658 |
| WT chemo | Mut chemo | 0.6451737 |
| WT cpi | Mut chemo | 0.6451737 |
| WT chemo | Mut cpi | 0.1197546 |
| WT cpi | Mut cpi | 0.2797656 |
| WT cpi | WT chemo | 0.27976 |

FIG. 9C

TSC2

Number at risk

| | | | | | | | | | | | | | | | | |
|---|---|---|---|---|---|---|---|---|---|---|---|---|---|---|---|---|
| 8 | 6 | 4 | 4 | 4 | 1 | 1 | 1 | 1 | 1 | 0 | 0 | 0 | 0 | 0 | 0 | 0 |
| 34 | 32 | 26 | 14 | 5 | 4 | 3 | 2 | 0 | 0 | 0 | 0 | 0 | 0 | 0 | 0 | 0 |
| 262 | 214 | 133 | 87 | 61 | 47 | 32 | 26 | 23 | 15 | 11 | 5 | 3 | 1 | 1 | 1 | 1 |
| 724 | 595 | 432 | 313 | 207 | 133 | 85 | 44 | 11 | 4 | 0 | 0 | 0 | 0 | 0 | 0 | 0 |
| 0 | 6 | 12 | 18 | 24 | 30 | 36 | 42 | 48 | 54 | 60 | 66 | 72 | 78 | 84 | 90 | 96 |

Time

| Group1 | Group2 | p.value |
|---|---|---|
| Mut cpi | Mut chemo | 0.5755961 |
| WT chemo | Mut chemo | 0.4944493 |
| WT cpi | Mut chemo | 0.5339737 |
| WT chemo | Mut cpi | 0.2654397 |
| WT cpi | Mut cpi | 0.5339737 |
| WT cpi | WT chemo | 0.2406549 |

FIG. 10A

## Mut KRAS + Mut STK11 VS WT KRAS + WT STK11

Number at risk

| | | | | | | | | | | | | | | | | |
|---|---|---|---|---|---|---|---|---|---|---|---|---|---|---|---|---|
| 22 | 19 | 9 | 4 | 3 | 3 | 2 | 2 | 1 | 1 | 0 | 0 | 0 | 0 | 0 | 0 | 0 |
| 248 | 201 | 128 | 87 | 62 | 45 | 31 | 25 | 23 | 15 | 11 | 5 | 3 | 1 | 1 | 1 | 1 |
| 63 | 46 | 28 | 15 | 7 | 6 | 5 | 2 | 0 | 0 | 0 | 0 | 0 | 0 | 0 | 0 | 0 |
| 695 | 581 | 430 | 312 | 205 | 131 | 83 | 44 | 11 | 4 | 0 | 0 | 0 | 0 | 0 | 0 | 0 |
| 0 | 6 | 12 | 18 | 24 | 30 | 36 | 42 | 48 | 54 | 60 | 66 | 72 | 78 | 84 | 90 | 96 |

Time

| Group1 | Group2 | p.value |
|---|---|---|
| WT chemo | Mut chemo | 0.137788 |
| Mut cpi | Mut chemo | 0.7529143 |
| WT cpi | Mut chemo | 0.0720382 |
| Mut cpi | WT chemo | 0.2271161 |
| WT cpi | WT chemo | 0.1120598 |
| WT cpi | Mut cpi | 0.0461295 |

## FIG. 10A (Continued)

TP53

| Group1 | Group2 | p.value |
|---|---|---|
| Mut cpi | Mut chemo | 0.0255248 |
| WT chemo | Mut chemo | 0.1511777 |
| WT cpi | Mut chemo | 0.1511777 |
| WT chemo | Mut cpi | 0.8474235 |
| WT cpi | Mut cpi | 0.7147789 |
| WT cpi | WT chemo | 0.7414105 |

FIG. 10A (Continued)

FIG. 10A (Continued)

TSC2

Number at risk

| | | | | | | | | | | | | | | | | | | | | | |
|---|---|---|---|---|---|---|---|---|---|---|---|---|---|---|---|---|---|---|---|---|---|
| 55 | 30 | 17 | 11 | 10 | 6 | 4 | 4 | 3 | 3 | 2 | 2 | 2 | 2 | 2 | 2 | 2 | 1 | 1 | 0 | 0 | 0 |
| 95 | 63 | 44 | 25 | 14 | 9 | 7 | 5 | 0 | 0 | 0 | 0 | 0 | 0 | 0 | 0 | 0 | 0 | 0 | 0 | 0 | 0 |
| 1303 | 743 | 489 | 350 | 266 | 204 | 151 | 111 | 86 | 69 | 48 | 31 | 23 | 16 | 12 | 8 | 6 | 5 | 3 | 1 | 1 | 1 |
| 2380 | 1472 | 988 | 638 | 408 | 257 | 154 | 74 | 27 | 7 | 0 | 0 | 0 | 0 | 0 | 0 | 0 | 0 | 0 | 0 | 0 | 0 |

| Group1 | Group2 | p.value |
|---|---|---|
| Mut cpi | Mut chemo | 0.4961567 |
| WT chemo | Mut chemo | 0.6396036 |
| WT cpi | Mut chemo | 0.5200897 |
| WT chemo | Mut cpi | 0.4961567 |
| WT cpi | Mut cpi | 0.6150799 |
| WT cpi | WT chemo | 0.4961567 |

FIG. 10B

160

Mut KRAS + Mut STK11 VS WT KRAS + WT STK11

Number at risk

| | | | | | | | | | | | | | | | | | | | | | |
|---|---|---|---|---|---|---|---|---|---|---|---|---|---|---|---|---|---|---|---|---|---|
| 94 | 50 | 32 | 20 | 15 | 12 | 7 | 5 | 2 | 2 | 0 | 0 | 0 | 0 | 0 | 0 | 0 | 0 | 0 | 0 | 0 | 0 |
| 1264 | 723 | 474 | 341 | 261 | 198 | 148 | 110 | 87 | 70 | 50 | 33 | 25 | 18 | 14 | 10 | 8 | 6 | 4 | 1 | 1 | 1 |
| 181 | 107 | 70 | 36 | 20 | 16 | 11 | 6 | 3 | 0 | 0 | 0 | 0 | 0 | 0 | 0 | 0 | 0 | 0 | 0 | 0 | 0 |
| 2294 | 1428 | 962 | 627 | 402 | 250 | 150 | 73 | 24 | 7 | 0 | 0 | 0 | 0 | 0 | 0 | 0 | 0 | 0 | 0 | 0 | 0 |

0  6  12  18  24  30  36  42  48  54  60  66  72  78  84  90  96  102 108 114 120 126

Time

| Group1 | Group2 | p.value |
|---|---|---|
| WT chemo | Mut chemo | 0.1177226 |
| Mut cpi | Mut chemo | 0.3272536 |
| WT cpi | Mut chemo | 0.0531283 |
| Mut cpi | WT chemo | 0.4349782 |
| WT cpi | WT chemo | 0.2342549 |
| WT cpi | Mut cpi | 0.1519346 |

FIG. 10B (Continued)

## TP53

Number at risk

| | | | | | | | | | | | | | | | | | | | | | | |
|---|---|---|---|---|---|---|---|---|---|---|---|---|---|---|---|---|---|---|---|---|---|---|
| ---- | 915 | 503 | 317 | 220 | 156 | 120 | 86 | 60 | 47 | 38 | 29 | 18 | 12 | 9 | 7 | 5 | 4 | 4 | 3 | 1 | 1 | 1 |
| — | 1693 | 1032 | 709 | 450 | 284 | 180 | 102 | 52 | 19 | 7 | 0 | 0 | 0 | 0 | 0 | 0 | 0 | 0 | 0 | 0 | 0 | 0 |
| — | 433 | 261 | 184 | 138 | 117 | 87 | 67 | 53 | 42 | 34 | 21 | 15 | 13 | 9 | 7 | 5 | 4 | 2 | 1 | 0 | 0 | 0 |
| — | 763 | 492 | 315 | 207 | 135 | 83 | 57 | 27 | 8 | 0 | 0 | 0 | 0 | 0 | 0 | 0 | 0 | 0 | 0 | 0 | 0 | 0 |

0   6   12   18   24   30   36   42   48   54   60   66   72   78   84   90   96   102  108  114  120  126

Time

| Group1 | Group2 | p.value |
|---|---|---|
| Mut cpi | Mut chemo | 0.0051268 |
| WT chemo | Mut chemo | 0.0051268 |
| WT cpi | Mut chemo | 0.0070098 |
| WT chemo | Mut cpi | 0.1025126 |
| WT cpi | Mut cpi | 0.7792904 |
| WT cpi | WT chemo | 0.2094732 |

FIG. 10B (Continued)

## TP53 LFM vs TP53 HFM

Number at risk

| | | | | | | | | | | | | | | | | | | | | | | |
|---|---|---|---|---|---|---|---|---|---|---|---|---|---|---|---|---|---|---|---|---|---|---|
| ---- | 338 | 186 | 114 | 80 | 57 | 43 | 31 | 22 | 16 | 12 | 10 | 6 | 4 | 2 | 2 | 2 | 1 | 1 | 1 | 0 | 0 | 0 |
| — | 590 | 352 | 239 | 144 | 82 | 57 | 28 | 17 | 8 | 2 | 0 | 0 | 0 | 0 | 0 | 0 | 0 | 0 | 0 | 0 | 0 | 0 |
| — | 568 | 314 | 200 | 138 | 98 | 77 | 55 | 38 | 31 | 26 | 19 | 12 | 8 | 7 | 5 | 3 | 3 | 3 | 2 | 1 | 1 | 1 |
| — | 1088 | 671 | 465 | 301 | 199 | 120 | 72 | 34 | 11 | 5 | 0 | 0 | 0 | 0 | 0 | 0 | 0 | 0 | 0 | 0 | 0 | 0 |

| Group1 | Group2 | p.value |
|---|---|---|
| LFM cpi | LFM chemo | 0.2420947 |
| HFM chemo | LFM chemo | 0.7106382 |
| HFM cpi | LFM chemo | 0.0348502 |
| HFM chemo | LFM cpi | 0.2397376 |
| HFM cpi | LFM cpi | 0.3506029 |
| HFM cpi | HFM chemo | 0.0302755 |

FIG. 10B (Continued)

## NF1 LFM vs NF1 HFM

| Group1 | Group2 | p.value |
|---|---|---|
| LFM cpi | LFM chemo | 0.7542487 |
| HFM chemo | LFM chemo | 0.991805 |
| HFM cpi | LFM chemo | 0.991805 |
| HFM chemo | LFM cpi | 0.991805 |
| HFM cpi | LFM cpi | 0.991805 |
| HFM cpi | HFM chemo | 0.991805 |

FIG. 10B (Continued)

FIG. 11

EP 4 515 000 B1

FIG. 12

166

FIG. 12 (Continued)

FIG. 12 (Continued)

FIG. 12 (Continued)

FIG. 12 (Continued)

FIG. 12 (Continued)

FIG. 12 (Continued)

HotSpot input results

Innate-Resistance

Durable-Response

Feature Name

FIG. 12 (Continued)

FIG. 12 (Continued)

Mean accuracy across five train/val splits

FIG. 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 22170144
- US 2019219586 A
- WO 2018183928 A
- US 2020109204 A
- WO 2019207030 A
- US 2019352723 A
- US 2020109455 A

**Non-patent literature cited in the description**

- **SINGAL G ; MILLER PG ; AGARWALA V et al.** Association of Patient Characteristics and Tumor Genomics With Clinical Outcomes Among Patients With Non-Small Cell Lung Cancer Using a Clinicogenomic Database (CGDB). *JAMA*, 2019, vol. 321 (14), 1391-1399
- *JAMA*, 2019, vol. 321 (14), 1391-1399
- *Journal of Thoracic Oncology*, 2018, vol. 13, 1156-1170
- *Advances in Therapy*, 2019, vol. 36 (8), 2122-2136
- *Advances in Therapy*, 2021, vol. 38, 1843-1859
- *JAMA*, 2019
- *Genome Med.*, 2017
- **ROTOW, J. ; BIVONA, T. G.** Understanding and targeting resistance mechanisms in NSCLC. *Nature Reviews Cancer*, 2017
- **INAMURA, K**. Lung cancer: understanding its molecular pathology and the 2015 wHO classification. *Front. Oncol.*, 2017
- **SCHADENDORF, D. et al.** Efficacy and safety outcomes in patients with advanced melanoma who discontinued treatment with nivolumab and ipilimumab because of adverse events: A pooled analysis of randomized phase II and III trials. *J. Clin. Oncol.*, 2017
- **FEHRENBACHER, L. et al.** Updated Efficacy Analysis Including Secondary Population Results for OAK: A Randomized Phase III Study of Atezolizumab versus Docetaxel in Patients with Previously Treated Advanced Non-Small Cell Lung Cancer. *J. Thorac. Oncol.*, 2018
- **BERNARD-TESSIER, A. et al.** Outcomes of long-term responders to anti-programmed death 1 and anti-programmed death ligand 1 when being rechallenged with the same anti-programmed death 1 and anti-programmed death ligand 1 at progression. *Eur. J. Cancer*, 2018
- **MAZEIN, A.** ; **WATTERSON, S.** ; **HSIEH, W. Y.** ; **GRIFFITHS, W. J.** ; **GHAZAL, P.** A comprehensive machine-readable view of the mammalian cholesterol biosynthesis pathway. *Biochem. Pharmacol.*, 2013
- **ANTONIA, S. J. et al.** Four-year survival with nivolumab in patients with previously treated advanced non-small-cell lung cancer: a pooled analysis. *Lancet Oncol.*, 2019
- **SCHOENFELD, A. J.** ; **HELLMANN, M. D.** Acquired Resistance to Immune Checkpoint Inhibitors. *Cancer Cell*, 2020, vol. 37, 443-455
- **SHARMA, P.** ; **HU-LIESKOVAN, S.** ; **WARGO, J. A.** ; **RIBAS, A.** Primary, Adaptive, and Acquired Resistance to Cancer Immunotherapy. *Cell*, 2017
- **WALSH, R. J.** ; **SOO, R. A.** Resistance to immune checkpoint inhibitors in non-small cell lung cancer: biomarkers and therapeutic strategies. *Ther. Adv. Med. Oncol.*, 2020, vol. 12, 1-22
- **LAGOS, G. G.** ; **IZAR, B.** ; **RIZVI, N. A.** Beyond Tumor PD-L1: Emerging Genomic Biomarkers for Checkpoint Inhibitor Immunotherapy. *Am. Soc. Clin. Oncol. Educ. B.*, 2020
- **KALBASI, A.** ; **RIBAS, A.** Tumour-intrinsic resistance to immune checkpoint blockade. *Nature Reviews Immunology*, 2020, vol. 20, 25-39
- **JIANG, P. et al.** Signatures of T cell dysfunction and exclusion predict cancer immunotherapy response. *Nat. Med.*, 2018
- **HUGO, W. et al.** Genomic and Transcriptomic Features of Response to Anti-PD-1 Therapy in Metastatic Melanoma. *Cell*, 2016
- **AUSLANDER, N. et al.** Robust prediction of response to immune checkpoint blockade therapy in metastatic melanoma. *Nat. Med.*, 2018
- **ANAGNOSTOU, V. et al.** Integrative Tumor and Immune Cell Multi-omic Analyses Predict Response to Immune Checkpoint Blockade in Melanoma. *Cell Reports Med.*, 2020, vol. 1

- **SINGAL, G. et al.** Association of Patient Characteristics and Tumor Genomics With Clinical Outcomes Among Patients With Non-Small Cell Lung Cancer Using a Clinicogenomic Database. *JAMA*, 2019
- **FRAMPTON, G. M. et al.** Development and validation of a clinical cancer genomic profiling test based on massively parallel DNA sequencing. *Nat. Biotechnol.*, 2013
- **KUGEL, C. H. et al.** Age correlates with response to anti-PD1, reflecting age-related differences in intratumoral effector and regulatory T-cell populations. *Clin. Cancer Res.*, 2018
- **RIZVI, N. A. et al.** Mutational landscape determines sensitivity to PD-1 blockade in non-small cell lung cancer. *Science*, 2015, vol. 80
- **NORUM, J.** ; **NIEDER, C.** Tobacco smoking and cessation and PD-L1 inhibitors in non-small cell lung cancer (NSCLC): A review of the literature. *ESMO Open*, 2018
- **XU, J. et al.** Heterogeneity of Li-Fraumeni Syndrome links to unequal gain-of-function effects of p53 mutations. *Sci. Rep.*, 2014
- **BROSH, R.** ; **ROTTER, V.** When mutants gain new powers: News from the mutant p53 field. *Nature Reviews Cancer*, 2009
- **SUMMERS, M. G. et al.** BRAF and NRAS locus-specific variants have different outcomes on survival to colorectal cancer. *Clin. Cancer Res.*, 2017
- **DE ROOCK, W. et al.** Association of KRAS p.G13D mutation with outcome in patients with chemotherapy-refractory metastatic colorectal cancer treated with cetuximab. *JAMA - J. Am. Med. Assoc.*, 2010
- **KADOSH, E. et al.** The gut microbiome switches mutant p53 from tumour-suppressive to oncogenic. *Nature*, 2020
- **VOGELSTEIN, B. et al.** Cancer genome landscapes. *Science*, 2013
- **SKOULIDIS, F. et al.** STK11/LKB1 mutations and PD-1 inhibitor resistance in KRAS-mutant lung adenocarcinoma. *Cancer Discov.*, 2018
- **SKOULIDIS, F. et al.** Co-occurring genomic alterations define major subsets of KRAS-mutant lung adenocarcinoma with distinct biology, immune profiles, and therapeutic vulnerabilities. *Cancer Discov.*, 2015
- **BLUMENTHAL, G. M. et al.** Overall response rate, progression-free survival, and overall survival with targeted and standard therapies in advanced non-small-cell lung cancer: US Food and Drug Administration trial-level and patient-level analyses. *J. Clin. Oncol.*, 2015
- **SOLOMON, B. J. et al.** Correlation between overall response rate and progression-free survival/overall survival in comparative trials involving targeted therapies in molecularly enriched populations. *J. Clin. Oncol.*, 2020
- **PAPILLON-CAVANAGH, S.** ; **DOSHI, P.** ; **DOBRIN, R.** ; **SZUSTAKOWSKI, J.** ; **WALSH, A. M.** STK11 and KEAP1 mutations as prognostic biomarkers in an observational real-world lung adenocarcinoma cohort. *ESMO Open*, 2020, vol. 5, e000706
- **RISH, I.** An empirical study of the naive Bayes classifier. *IJCAI 2001 Work. Empir. methods Artif. Intell.*, 2001
- **VAN ALLEN, E. M. et al.** Genomic correlates of response to CTLA-4 blockade in metastatic melanoma. *Science*, 2015, vol. 80
- **MIAO, D. et al.** Genomic correlates of response to immune checkpoint blockade in microsatellite-stable solid tumors. *Nat. Genet.*, 2018
- **NG, A. Y.** ; **JORDAN, M. I.** On discriminative vs. Generative classifiers: A comparison of logistic regression and naive bayes. *Advances in Neural Information Processing Systems*, 2002
- **HILL, A. et al.** Benchmarking network algorithms for contextualizing genes of interest. *PLoS Comput. Biol.*, 2019
- **O'DONNELL, J. S.** ; **LONG, G. V.** ; **SCOLYER, R. A.** ; **TENG, M. W. L.** ; **SMYTH, M. J.** Resistance to PD1/PDL1 checkpoint inhibition. *Cancer Treatment Reviews*, 2017
- **JACKSON, C. M.** ; **CHOI, J.** ; **LIM, M.** Mechanisms of immunotherapy resistance: lessons from glioblastoma. *Nature Immunology*, 2019
- **BAUGH, E. H.** ; **KE, H.** ; **LEVINE, A. J.** ; **BONNEAU, R. A.** ; **CHAN, C. S.** Why are there hotspot mutations in the TP53 gene in human cancers?. *Cell Death and Differentiation*, 2018
- **LEVINE, A. J.** 800 million years of evolution and 40 years of discovery. *Nature Reviews Cancer*, 53, www.nature.com/nrc doi:10.1038/s41568-020-0262-1
- **BARGONETTI, J.** ; **PRIVES, C.** Gain-of-function mutant p53: History and speculation. *Journal of Molecular Cell Biology*, 2019
- **CORMEDI, M. C. V.** ; **VAN ALLEN, E. M.** ; **COLLI, L. M.** Predicting immunotherapy response through genomics. *Current Opinion in Genetics and Development*, 2021, vol. 66, 1-9
- **LIU, H. J. et al.** TSC2-deficient tumors have evidence of T cell exhaustion and respond to anti-PD-1/anti-CTLA-4 immunotherapy. *JCI insight*, 2018
- **TORREJON, D. Y. et al.** Overcoming Genetically Based Resistance Mechanisms to PD-1 Blockade. *Cancer Discov.*, 2020
- **LIANG, Y. et al.** Targeting IFNα to tumor by anti-PD-L1 creates feedforward antitumor responses to overcome checkpoint blockade resistance. *Nat. Commun.*, 2018
- **REISLÄNDER, T.** ; **GROELLY, F. J.** ; **TARSOUNAS, M.** DNA Damage and Cancer Immunotherapy: A STING in the Tale. *Molecular Cell*, 2020

- **GAO, S. P. et al.** Mutations in the EGFR kinase domain mediate STAT3 activation via IL-6 production in human lung adenocarcinomas. *J. Clin. Invest.*, 2007, vol. 117, 3846-3856
- **JIANG, L. et al.** Continuous targeted kinase inhibitors treatment induces upregulation of PD-L1 in resistant NSCLC. *Sci. Rep.*, 2019
- **LIU, H.** ; **SHEN, J.** ; **LU, K.** IL-6 and PD-L1 blockade combination inhibits hepatocellular carcinoma cancer development in mouse model. *Biochem. Biophys. Res. Commun.*, 2017, vol. 486, 239-244
- **BIALKOWSKI, L. et al.** Immune checkpoint blockade combined with IL-6 and TGF-β inhibition improves the therapeutic outcome of mRNA-based immunotherapy. *Int. J. Cancer*, 2018, vol. 143, 686-698
- **KEEGAN, A. et al.** Plasma IL-6 changes correlate to PD-1 inhibitor responses in NSCLC. *J. Immunother. Cancer*, 2020
- **GARBERS, C.** ; **HEINK, S.** ; **KORN, T.** ; **ROSE-JOHN, S.** Interleukin-6: Designing specific therapeutics for a complex cytokine. *Nature Reviews Drug Discovery*, 2018
- **TAKEUCHI, T. et al.** Considering new lessons about the use of IL-6 inhibitors in arthritis. *Considerations Med.*, 2018
- **YUEN, K. C. et al.** *Abstract 2676: Associations of peripheral biomarkers to outcomes to anti-PD-L1 immune checkpoint blockade in metastatic urothelial cancer*, 2019
- **ÇELIK, A. et al.** Angiogenic and Immune-Related Biomarkers and Outcomes Following Axitinib/Pembrolizumab Treatment in Patients with Advanced Renal Cell Carcinoma. *J. Mater. Process. Technol.*, 2018, vol. 1, 1-8
- **ZHAO, J. et al.** Immune and genomic correlates of response to anti-PD-1 immunotherapy in glioblastoma. *Nat. Med.*, 2019
- **LUKE, J. J.** ; **BAO, R.** ; **SWEIS, R. F.** ; **SPRANGER, S.** ; **GAJEWSKI, T. F.** WNT/b-catenin pathway activation correlates with immune exclusion across human cancers. *Clin. Cancer Res.*, 2019
- **OHLUND, D. et al.** Distinct populations of inflammatory fibroblasts and myofibroblasts in pancreatic cancer. *J. Exp. Med.*, 2017
- **EVANS, E. K. et al.** *C A N C E R A precision therapy against cancers driven by KIT/PDGFRA mutations*, 2017, www.cellsignal.com
- **KIM, G.** ; **KO, Y. T.** Small molecule tyrosine kinase inhibitors in glioblastoma. *Archives of Pharmacal Research*, 2020, vol. 43, 385-394
- **TASSELL, V. et al.** Preliminary biomarker analysis of sitravatinib in combination with nivolumab in NSCLC patients progressing on prior checkpoint inhibitor. *J. Immunother. Cancer*, 2018
- **BROSE, M. S. et al.** A phase Ib/II trial of lenvatinib plus pembrolizumab in non-small cell lung cancer. *J. Clin. Oncol.*, 2019
- **DU, W.** ; **HUANG, H.** ; **SORRELLE, N.** ; **BREKKEN, R. A.** Sitravatinib potentiates immune checkpoint blockade in refractory cancer models. *JCI insight*, 2018
- **SAMBROOK, J.** ; **RUSSEL, D.W.** Molecular Cloning, A Laboratory Manual. Cold Spring Harbor Laboratory Press, 2001